# EUROPEAN PATENT APPLICATION

(11) **EP 3 828 174 A1**
(43) Date of publication of application: **02.06.2021**
(21) Application number: 19838697.1
(22) Date of filing: 18.07.2019
(51) Int. Cl.: C07D 237/14

(54) **PYRIDAZINONE DERIVATIVE**

(30) Priority: 19.07.2018 JP 2018136152
(71) Applicant: Sumitomo Dainippon Pharma Co., Ltd., Chuo-ku Osaka-shi Osaka 541-8524 (JP)
(72) Inventor: NISHIDA, Tomoaki, Osaka-shi, Osaka 554-0022 (JP); UEMACHI, Hiro, Osaka-shi, Osaka 554-0022 (JP); IWATA, Masato, Osaka-shi, Osaka 554-0022 (JP); SHIBATA, Hajime, Osaka-shi, Osaka 554-0022 (JP); NISHIMAKI, Takuya, Tokyo 104-8356 (JP); KIYOSHIGE, Saori, Osaka-shi, Osaka 554-0022 (JP)
(74) Representative: Vossius & Partner Patentanwälte Rechtsanwälte mbB
(86) International application number: PCT/JP2019/028256
(87) International publication number: WO 2020/017587

(57) **Abstract**

Provided are: a pyridazinone derivative and/or a pharmaceutically acceptable salt thereof, which is useful as a therapeutic agent and/or a prophylactic agent for diseases in which Nav1.1 is involved and various central nervous system diseases; and a medicine containing the pyridazinone derivative and/or the pharmaceutically acceptable salt thereof as an active ingredient. A compound represented by formula (1) or a pharmaceutically acceptable salt thereof. 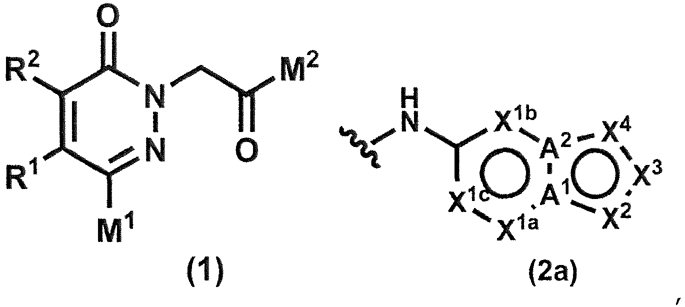 [In the formula, M¹ represents a saturated or partially unsaturated C₄₋₁₂ carbocyclic group or the like; R¹ and R² independently represent a hydrogen atom or the like; M² represents a group represented by formula (2a) or the like; X^{1a}, X^{1b} and X^{1c} independently represent N or the like; X², X³ and X⁴ independently represent CR³ or the like; A¹ and A² independently represent N or the like; and R³ represents a hydrogen atom or the like.]

## Description

### TECHNICAL FIELD

The present invention is directed to a pyridazinone derivative or a pharmaceutically acceptable salt thereof that is useful as a medicament for treating and/or preventing diseases involving sodium channel (especially Nav1.1) and various central nervous system diseases, and a medicament comprising them as an active ingredient.

### BACKGROUND ART

Nav1.1 is one of voltage-gated sodium channels (VGSC), and expressed in, for example, palvalbmin-positive GABA neurons (PV-GABA neurons). It is known that Nav1.1 is important for the function of neuronal firing in the neurons.

It has been suggested that patients suffering from a central nervous system disease such as schizophrenia, autism spectrum disorder (ASD), and attention deficit hyperactivity disorder (ADHD) have dysfunctions in GABA-ergic neurons which express Nav1.1 (Non Patent Literatures 1 and 2).

It has also been reported that heterozygous loss-of-function mutation in SCN1A gene leads to epileptic syndromes such as Dravet syndrome (severe myoclonic epilepsy of infancy) and generalized epilepsy with febrile seizure plus (GEFS+) (Non Patent Literature 1).

Dravet syndrome develops in infancy under 1 year old, and it is a serious epileptic encephalopathy in children which causes, for example, various epileptic seizures such as febrile seizures and status epilepticus. As a first-line drug, valproic acid has been used as in pharmacotherapy of Dravet syndrome, but it is less effective for epileptic seizures. As a second-line drug, clobazam and stiripentol have been used, but they are less effective for epileptic seizures. Stiripentol is only available in combination therapy with valproic acid or clobazam, which limits the number of patients who receive the drug.

Thus, a medicament that activates Nav1.1 functions is expected to ameliorate diseases such as schizophrenia, ASD, ADHD, and epilepsy, as well as their associated pathological conditions such as cognitive dysfunction and epileptic seizures, and to treat a wide variety of central nervous system diseases.

N,N'-(1,3-Phenylene)bis(2-methylbenzamide) (Non Patent Literature 3) and PF-05661014 (Non Patent Literature 4) are known as exemplary compounds that regulate Nav1.1 functions, but these compounds are different from compounds of the present invention in terms of their chemical structures.

Nav1.5, which is another subtype of voltage-dependent sodium channels, is predominantly expressed in heart, and it is known that Nav1.5 contributes to the formation of PR interval, QRS width, and QT interval in electrocardiogram, and involves the electrical conduction between atria and ventricles and the contraction and relaxation of ventricular myocardium. It is also known that antiarrhythmic agents which have inhibitory effect of Nav1.5 prolong the PR interval and QRS width in electrocardiogram. Thus, it is believed that activation of Nav1.5 may affect the PR interval, QRS width, and QT interval in electrocardiogram, the electrical conduction between atria and ventricles, and the contraction and relaxation of ventricular myocardium.

### CITATION LIST

### NON PATENT LITERATURE

[Non Patent Literature 1] Trends in Pharmacological Sciences 2014, 35, 113.
[Non Patent Literature 2] Curr. Med. Chem. 2015, 22, 1850.
[Non Patent Literature 3] ACS Chemical Neuroscience 2015, 6, 1302.
[Non Patent Literature 4] British Journal of Pharmacology 2015, 172, 4905.

### SUMMARY OF INVENTION

### PROBLEMS TO BE SOLVED BY THE INVENTION

One of the problems to be solved by the present invention is to provide a pyridazinone derivative and/or a pharmaceutically acceptable salt thereof that is useful as a medicament for treating and/or preventing diseases involving Nav1.1 and various central nervous system diseases, and a medicament comprising them as an active ingredient.

### MEANS OF SOLVING THE PROBLEMS

The present inventors have extensively studied to find that a compound of the following formula (1) or a pharmaceutically acceptable salt thereof (hereinafter, referred to as "the present compound") has a potent activation effect of Nav1.1, and that it can be a medicament that is effective for treating and/or preventing diseases involving Nav1.1 and various central nervous system diseases, and thus, they have accomplished the present invention.

Accordingly, the present invention is described as follows:
[1] A compound according to Formula (1): 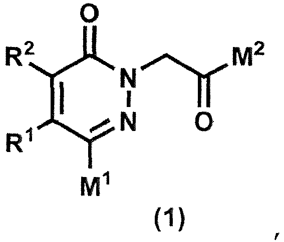 or a pharmaceutically acceptable salt thereof, wherein M¹ is
   (1-1) saturated or partially-unsaturated C₄₋₁₂ carbocyclyl, wherein the carbocyclyl may be optionally substituted with 1 to 4 the same or different substituents selected from the group consisting of:
      (a) halogen atom, and
      (b) C₁₋₆ alkyl optionally substituted with 1 to 3 the same or different substituents selected from the group consisting of halogen atom, hydroxy, and C₁₋₆ alkoxy,
   (1-2) 4- to 12-membered saturated or partially-unsaturated heterocyclyl, wherein the heterocyclyl is optionally substituted with 1 to 4 the same or different substituents selected from the group consisting of:
      (a) halogen atom,
      (b) hydroxy,
      (c) methoxy,
      (d) C₁₋₆ alkyl optionally substituted with 1 to 3 the same or different substituents selected from the group consisting of halogen atom, hydroxy, and C₁₋₆ alkoxy, and
      (e) amino-carbonyl optionally substituted with 1 to 2 the same or different C₁₋₆ alkyl, wherein the C₁₋₆ alkyl may be optionally substituted with 1 to 3 the same or different halogen atoms,
      provided that the heterocyclyl is not morpholinyl,
   (1-3) 4-methylphenyl, wherein a phenyl part of the group may be optionally substituted with 1 to 4 the same or different substituents selected from the group consisting of halogen atom, hydroxy, C₁₋₆ alkyl optionally substituted with 1 to 3 the same or different halogen atoms, and C₁₋₆ alkoxy optionally substituted with 1 to 3 the same or different halogen atoms; and wherein a methyl part of the group may be optionally substituted with 1 to 3 the same or different halogen atoms,
   (1-4) amino, wherein the amino may be optionally substituted with 1 to 2 the same or different substituents selected from the group consisting of:
      (a) C₁₋₆ alkyl optionally substituted with 1 to 3 the same or different halogen atoms,
      (b) C₃₋₁₀ cycloalkyl optionally substituted with 1 to 3 the same or different substituents selected from the group consisting of halogen atom, C₁₋₆ alkyl, and C₃₋₆ cycloalkyl, and
      (c) C₃₋₁₀ cycloalkyl-C₁₋₄ alkyl optionally substituted with 1 to 3 the same or different substituents selected from the group consisting of halogen atom, C₁₋₆ alkyl, and C₃₋₆ cycloalkyl,
   (1-5) 6-methylpyridin-3-yl or 6-trifluoromethylpyridin-3-yl,
   (1-6) 4-chlorothiophen-2-yl, 5-methylthiophen-2-yl, or 3-cyanothiophen-2-yl, provided that when M¹ is 5-methylthiophen-2-yl, M² is not a group shown in the following formula (4-2), or
   (1-7) 4-methylphenyloxy,
      R¹ and R² are each independently
   (2-1) hydrogen atom,
   (2-2) halogen atom,
   (2-3) cyano,
   (2-4) C₁₋₆ alkyl, wherein the C₁₋₆ alkyl may be optionally substituted with 1 to 3 the same or different substituents selected from the group consisting of:
      (a) halogen atom,
      (b) hydroxy,
      (c) saturated or partially-unsaturated C₃₋₇ carbocyclyl,
      (d) C₁₋₆ alkoxy, and
      (e) amino optionally substituted with 1 to 2 the same or different substituents selected from the group consisting of C₁₋₆ alkyl optionally substituted with 1 to 3 the same or different halogen atoms; saturated or partially-unsaturated C₃₋₇ carbocyclyl; and C₂₋₇ alkylcarbonyl,
   (2-5) saturated or partially-unsaturated C₃₋₇ carbocyclyl optionally substituted with 1 to 4 the same or different substituents selected from the group consisting of halogen atom, hydroxy, C₁₋₆ alkyl, and C₁₋₆ alkoxy,
   (2-6) C₂₋₆ alkenyl optionally substituted with 1 to 4 the same or different halogen atoms,
   (2-7) C₁₋₆ alkoxy optionally substituted with 1 to 3 the same or different substituents selected from the group consisting of halogen atom, hydroxy, saturated or partially-unsaturated C₃₋₇ carbocyclyl, and C₁₋₆ alkoxy, or
   (2-8) amino optionally substituted with 1 to 2 the same or different substituents selected from the group consisting of C₁₋₆ alkyl optionally substituted with 1 to 3 the same or different halogen atoms, and saturated or partially-unsaturated C₃₋₇ carbocyclyl; or
      alternatively, R¹ and R² may be combined together with the carbon atoms to which they attach to form
   (3-1) a 5- to 7-membered saturated or partially-unsaturated carbocycle, wherein the carbocycle may be optionally substituted with 1 to 4 the same or different substituents selected from the group consisting of:
      (a) halogen atom,
      (b) hydroxy,
      (c) C₁₋₆ alkyl optionally substituted with 1 to 3 the same or different substituents selected from the group consisting of halogen atom, hydroxy, and C₁₋₆ alkoxy, and
      (d) C₁₋₆ alkoxy optionally substituted with 1 to 3 the same or different substituents selected from the group consisting of halogen atom, hydroxy, and C₁₋₆ alkoxy, or
   (3-2) a 5- to 7-membered saturated or partially-unsaturated heterocycle, wherein the heterocycle may be optionally substituted with 1 to 4 the same or different substituents selected from the group consisting of (a) to (d) in the above (3-1) of the present clause;
      M² is
   (4-1) a group of the following formula (2a) or (2b): 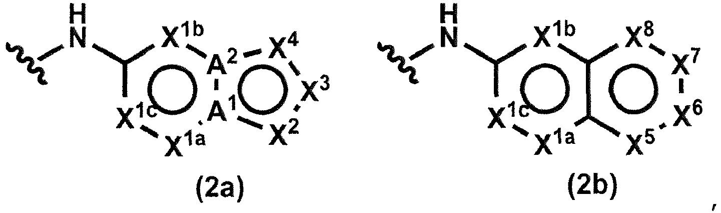 wherein
      X^{1a}, X^{1b}, X^{1c}, X⁵, X⁶, X⁷, and X⁸ are each independently N or CR³;
      X², X³, and X⁴ are each independently CR³, O, S, N, or NR⁴;
      A¹ and A² are each independently N or C;
      wherein X^{1a}, X^{1b}, X^{1c}, X², X³ X⁴, X⁵, X⁶ X⁷, X⁸, A¹, and A² are selected such that a ring containing them forms a 9- or 10-membered bicyclic aromatic heterocycle;
      R³ is
         (a) hydrogen atom,
         (b) halogen atom,
         (c) cyano,
         (d) hydroxy,
         (e) C₁₋₆ alkyl optionally substituted with 1 to 3 the same or different substituents selected from the group consisting of halogen atom, hydroxy, saturated or partially-unsaturated C₃₋₇ carbocyclyl, C₁₋₆ alkoxy, 4- to 7-membered saturated heterocyclyl optionally substituted with C₁₋₆ alkyl, and amino optionally substituted with 1 to 2 the same or different C₁₋₆ alkyl,
         (f) saturated or partially-unsaturated C₃₋₇ carbocyclyl optionally substituted with 1 to 4 the same or different substituents selected from the group consisting of halogen atom, hydroxy, C₁₋₆ alkyl, C₁₋₆ alkoxy, and amino optionally substituted with 1 to 2 the same or different C₁₋₆ alkyl,
         (g) C₁₋₆ alkoxy optionally substituted with 1 to 3 the same or different substituents selected from the group consisting of halogen atom, hydroxy, C₁₋₆ alkoxy, and amino optionally substituted with 1 to 2 the same or different C₁₋₆ alkyl,
         (h) amino optionally substituted with 1 to 2 the same or different substituents selected from the group consisting of C₁₋₆ alkyl optionally substituted with 1 to 3 the same or different substituents selected from the group consisting of halogen atom, hydroxy, and C₁₋₆ alkoxy; saturated or partially-unsaturated C₃₋₇ carbocyclyl; and C₂₋₇ alkylcarbonyl optionally substituted with 1 to 3 the same or different substituents selected from the group consisting of halogen atom, hydroxy, and C₁₋₆ alkoxy,
         (i) 5- or 6-membered heteroaryl optionally substituted with 1 to 4 the same or different substituents selected from the group consisting of halogen atom, cyano, and C₁₋₆ alkyl,
         (j) 4- to 7-membered saturated or partially-unsaturated heterocyclyl optionally substituted with 1 to 4 the same or different substituents selected from the group consisting of halogen atom, hydroxy, C₁₋₆ alkyl, and C₁₋₆ alkoxy, or
         (k) -C(O)NR^{x}R^{y}, wherein R^{x} and R^{y} are each independently hydrogen atom, C₁₋₆ alkyl, or saturated or partially-unsaturated C₃₋₇ carbocyclyl; or
         alternatively, R^{x} and RY may be combined together with the nitrogen atom to which they attach to form 4- to 7-membered saturated heterocyclyl;
      R⁴ is
         (a) hydrogen atom,
         (b) C₁₋₆ alkyl optionally substituted with 1 to 3 the same or different substituents selected from the group consisting of halogen atom, hydroxy, and C₁₋₆ alkoxy, or
         (c) saturated or partially-unsaturated C₃₋₇ carbocyclyl optionally substituted with 1 to 4 the same or different substituents selected from the group consisting of halogen atom, hydroxy, C₁₋₆ alkyl, and C₁₋₆ alkoxy,
      provided that when R³ and R⁴ exist plurally, each R³ and R⁴ may be the same or different,
   (4-2) a group of the following formula (2c): 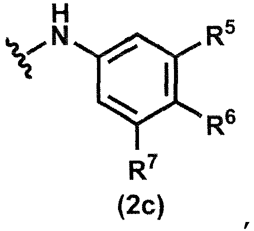 wherein R⁵, R⁶, and R⁷ are each independently
      (a) hydrogen atom,
      (b) halogen atom,
      (c) cyano,
      (d) C₁₋₆ alkyl optionally substituted with 1 to 3 the same or different substituents selected from the group consisting of halogen atom, hydroxy, saturated or partially-unsaturated C₃₋₇ carbocyclyl, and C₁₋₆ alkoxy,
      (e) saturated or partially-unsaturated C₃₋₇ carbocyclyl optionally substituted with 1 to 4 the same or different substituents selected from the group consisting of halogen atom, hydroxy, C₁₋₆ alkyl, and C₁₋₆ alkoxy,
      (f) C₁₋₆ alkoxy optionally substituted with 1 to 3 the same or different substituents selected from the group consisting of halogen atom, hydroxy, and C₁₋₆ alkoxy,
      (g) amino optionally substituted with 1 to 2 the same or different substituents selected from the group consisting of C₁₋₆ alkyl optionally substituted with 1 to 3 the same or different halogen atoms; saturated or partially-unsaturated C₃₋₇ carbocyclyl; and C₂₋₇ alkylcarbonyl,
      (h) 5- or 6-membered heteroaryl optionally substituted with 1 to 4 the same or different substituents selected from the group consisting of halogen atom, cyano, and C₁₋₆ alkyl,
      (i) 4- to 7-membered saturated or partially-unsaturated heterocyclyl optionally substituted with 1 to 4 the same or different substituents selected from the group consisting of halogen atom, hydroxy, C₁₋₆ alkyl, and C₁₋₆ alkoxy,
      (j) -C(O)NR^{x}R^{y}, wherein R^{x} and RY are each independently hydrogen atom, C₁₋₆ alkyl, or saturated or partially-unsaturated C₃₋₇ carbocyclyl; or
         alternatively, R^{x} and R^{y} may be combined together with the nitrogen atom to which they attach to form 4- to 7-membered saturated heterocyclyl,
      (k) C₂₋₇ alkylcarbonyl, or
      (1) C₂₋₇ alkoxycarbonyl, and
      either of the following condition (X) or (Y) is met:
      (X) at least one of R⁵, R⁶, and R⁷ is cyano, 5- or 6-membered heteroaryl (wherein the heteroaryl may be optionally substituted with 1 to 4 the same or different substituents selected from the group consisting of halogen atom, cyano, and C₁₋₆ alkyl), 4- to 7-membered saturated or partially-unsaturated heterocyclyl (wherein the heterocyclyl may be optionally substituted with 1 to 4 the same or different substituents selected from the group consisting of halogen atom, hydroxy, C₁₋₆ alkyl, and C₁₋₆ alkoxy), or - C(O)NR^{x}R^{y} (wherein R^{x} and R^{y} are each independently hydrogen atom, C₁₋₆ alkyl, or saturated or partially-unsaturated C₃₋₇ carbocyclyl; or alternatively, R^{x} and R^{y} may be combined together with the nitrogen atom to which they attach to form 4- to 7-membered saturated heterocyclyl); or
      (Y) R⁵ and R⁶ may be combined together with the carbon atom to which they attach to form a 5- to 7-membered saturated or partially-unsaturated carbocycle or heterocycle (wherein the carbocycle and heterocycle may be optionally substituted with 1 to 4 the same or different substituents selected from the group consisting of halogen atom, oxo, C₁₋₆ alkyl, C₁₋₆ alkoxy, and C₂₋₇ alkoxycarbonyl),
      wherein a group of formula (2c) may further be optionally substituted with a fluorine atom at a substitutable carbon atom of the ring,
   (4-3) a group of the following formula (2d), (2e), (2f), or (2g): 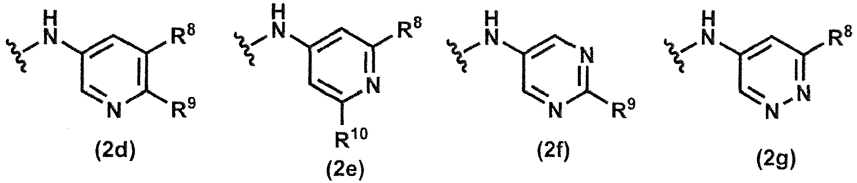 wherein R⁶, R⁹, and R¹⁰ are each independently
      (a) hydrogen atom,
      (b) halogen atom,
      (c) cyano,
      (d) C₁₋₆ alkyl optionally substituted with 1 to 3 the same or different substituents selected from the group consisting of halogen atom; hydroxy; saturated or partially-unsaturated C₃₋₇ carbocyclyl; C₁₋₆ alkoxy optionally substituted with hydroxy or C₁₋₆ alkoxy; 4- to 7-membered saturated or partially-unsaturated heterocyclyl optionally substituted with C₁₋₆ alkoxy or C₁₋₆ alkyl; 5- or 6-membered heteroaryl optionally substituted with C₁₋₆ alkyl; and amino (wherein the amino may be optionally substituted with 1 to 2 the same or different substituents selected from the group consisting of C₁₋₆ alkyl optionally substituted with 1 to 3 the same or different substituents selected from the group consisting of halogen atom, hydroxy, and C₁₋₆ alkoxy; saturated or partially-unsaturated C₃₋₇ carbocyclyl; 4- to 7-membered saturated heterocyclyl optionally substituted with C₁₋₆ alkoxy; and C₂₋₇ alkylcarbonyl optionally substituted with 1 to 3 the same or different substituents selected from the group consisting of halogen atom, hydroxy, and C₁₋₆ alkoxy);
      (e) saturated or partially-unsaturated C₃₋₇ carbocyclyl optionally substituted with 1 to 4 the same or different substituents selected from the group consisting of halogen atom, hydroxy, C₁₋₆ alkyl, C₁₋₆ alkoxy, and amino optionally substituted with 1 to 2 the same or different C₁₋₆ alkyl,
      (f) C₁₋₆ alkoxy optionally substituted with 1 to 3 the same or different substituents selected from the group consisting of halogen atom, hydroxy, C₁₋₆ alkoxy, and amino optionally substituted with 1 to 2 the same or different C₁₋₆ alkyl,
      (g) amino optionally substituted with 1 to 2 the same or different substituents selected from the group consisting of C₁₋₆ alkyl optionally substituted with 1 to 3 the same or different substituents selected from the group consisting of halogen atom, hydroxy, and C₁₋₆ alkoxy; saturated or partially-unsaturated C₃₋₇ carbocyclyl; and C₂₋₇ alkylcarbonyl optionally substituted with 1 to 3 the same or different substituents selected from the group consisting of halogen atom, hydroxy, and C₁₋₆ alkoxy,
      (h) 5- or 6-membered heteroaryl optionally substituted with 1 to 4 the same or different substituents selected from the group consisting of halogen atom, cyano, C₁₋₆ alkyl, C₁₋₆ alkoxy, and C₂₋₇ alkoxycarbonyl,
      (i) 4- to 7-membered saturated or partially-unsaturated heterocyclyl optionally substituted with 1 to 4 the same or different substituents selected from the group consisting of C₁₋₆ alkyl optionally substituted with 1 to 3 the same or different substituents selected from the group consisting of halogen atom, hydroxy, and C₁₋₆ alkoxy; C₁₋₆ alkoxy; 4- to 7-membered saturated or partially-unsaturated heterocyclyl; C₂₋₇ alkylcarbonyl optionally substituted with 1 to 3 the same or different substituents selected from the group consisting of halogen atom, hydroxy, and C₁₋₆ alkoxy; and oxo,
      (j) 4- to 7-membered saturated or partially-unsaturated heterocyclyl-oxy optionally substituted with 1 to 4 C₁₋₆ alkyl,
      (k) -C(O)NR^{x}R^{y}, wherein R^{x} and R^{y} are each independently hydrogen atom, C₁₋₆ alkyl, or saturated or partially-unsaturated C₃₋₇ carbocyclyl; or
         alternatively, R^{x} and R^{y} may be combined with the nitrogen atom to which they attach to form 4- to 7-membered saturated heterocyclyl,
      (l) -C(O)OR^{z}, wherein R^{z} is C₁₋₆ alkyl, or
      (m) ethenyl optionally substituted with one 6-membered saturated heterocyclyl group;
      wherein R⁸ and R⁹ may be combined together with the carbon atoms to which they attach to form a 5- to 7-membered saturated or partially-unsaturated carbocycle or heterocycle, wherein the carbocycle and heterocycle may be optionally substituted with 1 to 4 the same or different substituents selected from the group consisting of halogen atom and C₁₋₆ alky,
      wherein both of R⁸ and R⁹ in formula (2d) are not hydrogen atoms at the same time, and the group of formula (2e) may further be optionally substituted with a fluorine atom at a substitutable carbon atom of the ring,
   (4-4) a group of the following formula (2h): 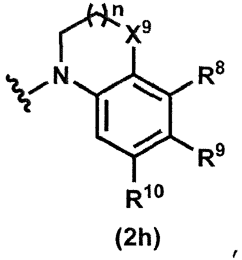 wherein R⁸, R⁹, and R¹⁰ are the same as those defined in the above (4-3);
      n is 0, 1, or 2;
      X⁹ is CH₂ or 0;
      wherein the group of formula (2h) may further be optionally substituted with a fluorine atom at a substitutable carbon atom of the ring,
   (4-5) a group of the following formula (2i), (2j), or (2k) : 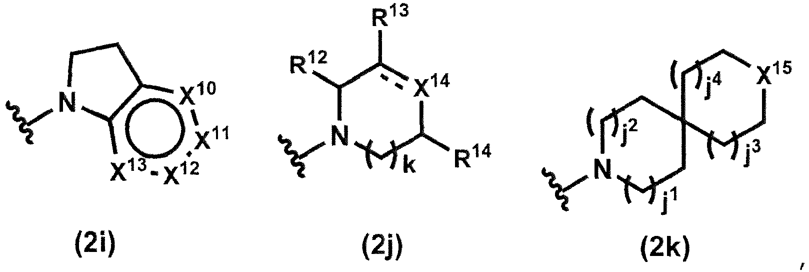 wherein X¹⁰, X¹¹, X¹², and X¹³ are each independently N or CR¹¹;
      wherein X¹⁰, X¹¹, X¹², and X¹³ are selected such that a 6-membered ring comprising them forms an aromatic heterocycle;
      X¹⁴ is CR¹⁵, CHR¹⁵, NR¹⁶, or O;
      provided that when X¹⁴ is CR¹⁵, a bond comprising a broken line in formula (2j) denotes a double bond, and that when X¹⁴ is CHR¹⁵, NR¹⁶, or O, a bond comprising a broken line in formula (2j) denotes a single bond;
      X¹⁵ is NR¹⁷ or O;
      R¹¹ is
      (a) hydrogen atom,
      (b) halogen atom,
      (c) 5- or 6-membered heteroaryl,
      (d) 5- or 6-membered heteroaryl-methyl, or
      (e) 4- to 7-membered saturated or partially-unsaturated heterocyclyl optionally substituted with 1 to 4 the same or different substituents selected from the group consisting of C₁₋₆ alkyl, C₁₋₆ alkoxy, C₂₋₇ alkylcarbonyl, and C₂₋₇ alkoxycarbonyl,
      provided that when R¹¹ exists plurally, each R¹¹ may be the same or different;
      R¹², R¹³, and R¹⁴ are each independently
      (a) hydrogen atom, or
      (b) methyl,
      wherein R¹² and R¹⁴, or R¹³ and R¹⁴ may be combined together with the carbon atoms to which they attach to form a bridged structure;
      R¹⁵ is
      (a) phenyl,
      (b) benzyl,
      (c) 5- to 10-membered heteroaryl optionally substituted with 1 to 2 the same or different substituents selected from the group consisting of fluorine atom and methoxy,
      (d) hydroxy,
      (e) phenyloxy, or
      (f) phenylamino;
      R¹⁶ is
      (a) phenyl optionally substituted with 1 to 2 the same or different substituents selected from the group consisting of fluorine atom and methoxy,
      (b) 5- or 6-membered heteroaryl optionally substituted with 1 to 2 the same or different substituents selected from the group consisting of methyl, methoxy, fluorine atom, trifluoromethyl, and difluoromethoxy,
      (c) 5- or 6-membered heteroarylmethyl optionally substituted with 1 to 2 methyl,
      (d) 5- or 6-membered saturated or partially-unsaturated carbocyclyl, or
      (e) 6-membered saturated heterocyclyl,
      R¹⁷ is
      (a) pyridyl,
      (b) 6-membered saturated heterocyclyl, or
      (c) methoxypropyl;
      k is 0, 1, or 2;
      j¹, j², j³, and j⁴ are each independently 0 or 1,
   (4-6) a group of the following formula (21): 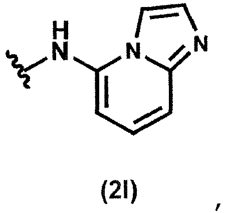 or
   (4-7) a group of the following formula (2m) or (2n): 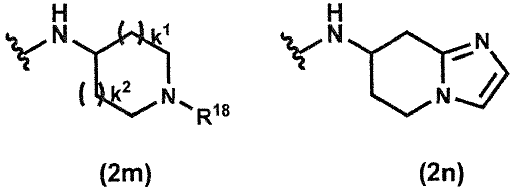 wherein R¹⁸ is
      (a) phenyl, or
      (b) benzyl;
      k¹ and k² are each independently 0 or 1;
      wherein the nitrogen-containing saturated ring in formula (2m) may be optionally substituted with oxo, provided that the compound according to Formula (1) is not the following compounds: 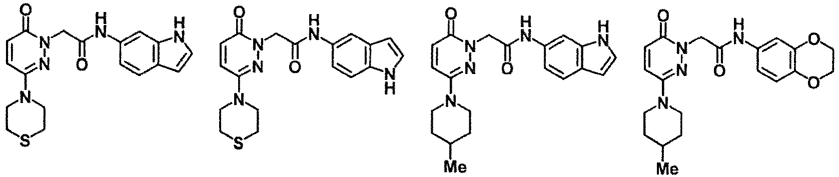 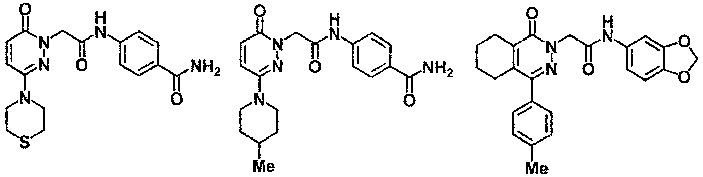 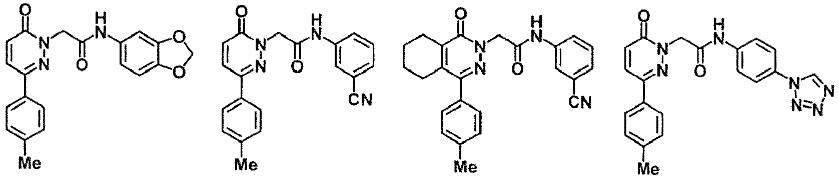 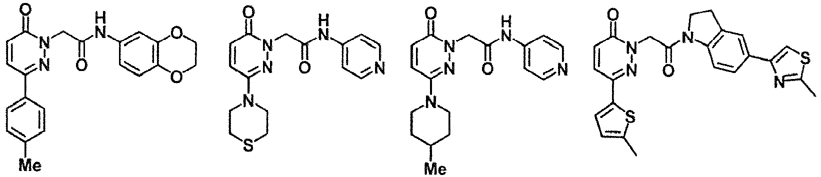 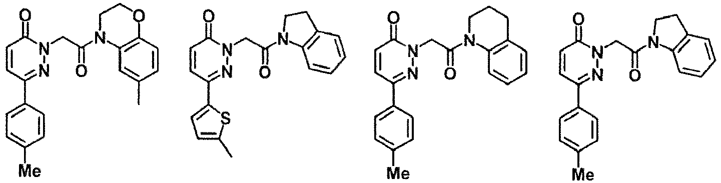 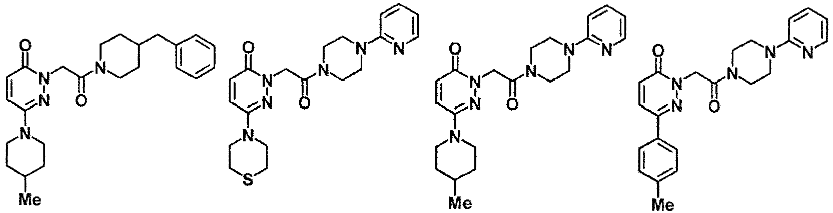 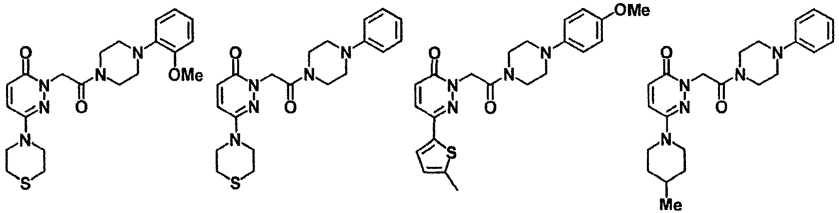 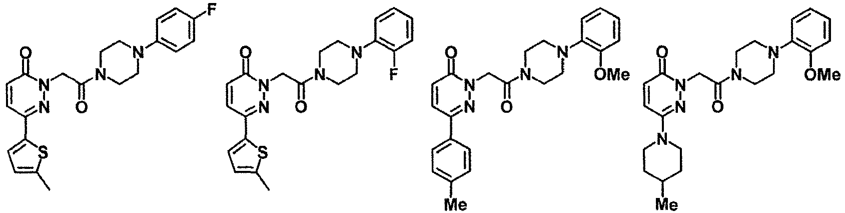 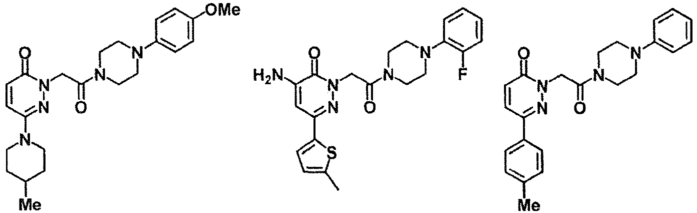 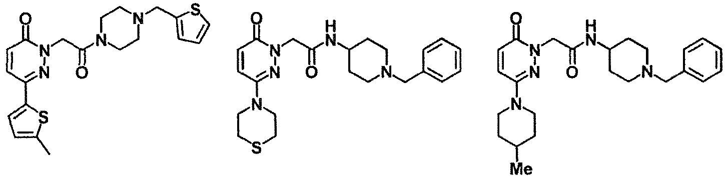
[2] The compound according to [1], or a pharmaceutically acceptable salt thereof, wherein
   R¹ and R² are each independently
   (1) hydrogen atom,
   (2) C₁₋₆ alkyl optionally substituted with 1 to 3 the same or different substituents selected from the group consisting of halogen atom, hydroxy, saturated or partially-unsaturated C₃₋₇ carbocyclyl, and C₁₋₆ alkoxy,
   (3) C₁₋₆ alkoxy optionally substituted with 1 to 3 the same or different substituents selected from the group consisting of halogen atom, hydroxy, and C₁₋₆ alkoxy, or
   (4) amino optionally substituted with 1 to 2 the same or different substituents selected from the group consisting of C₁₋₆ alkyl optionally substituted with 1 to 3 the same or different halogen atoms, and saturated or partially-unsaturated C₃₋₇ carbocyclyl; or
   alternatively, R¹ and R² may be combined together with the carbon atoms to which they attach to form a 5- to 7-membered saturated or partially-unsaturated carbocycle.
[3] The compound according to [1] or [2], or a pharmaceutically acceptable salt thereof, wherein M² is
   (1) a group of any one of the following formulae (11) - (37): 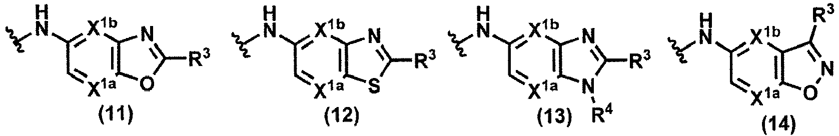 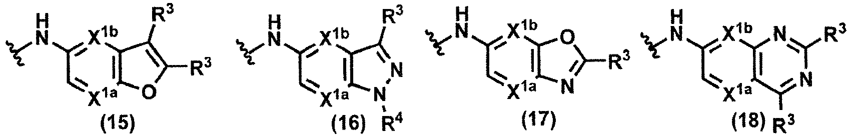 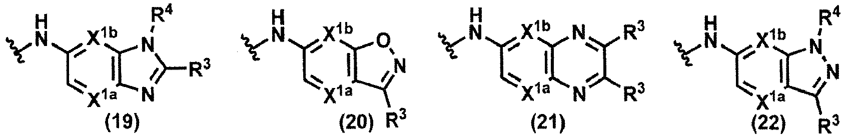 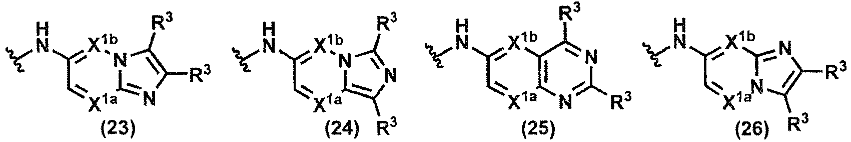 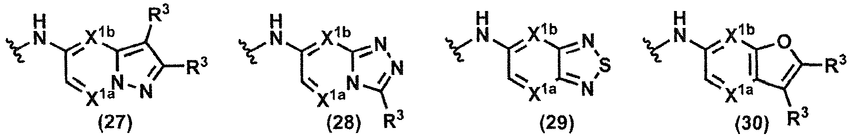 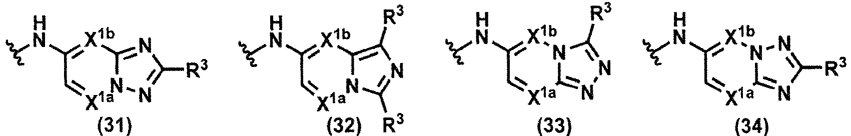 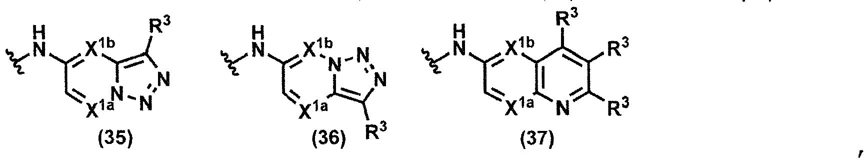 wherein X^{1a}, X^{1b}, R³, and R⁴ are the same as those defined in the above [1],
   (2) 4-cyanophenylamino,
   (3) a group of the following formula (2c'): 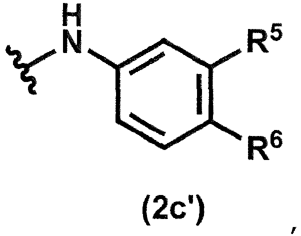 wherein R⁵ and R⁶ are each independently
      (a) hydrogen atom,
      (b) halogen atom,
      (c) cyano,
      (d) amino optionally substituted with 1 to 2 the same or different substituents selected from the group consisting of C₁₋₆ alkyl optionally substituted with 1 to 3 the same or different halogen atoms; saturated or partially-unsaturated C₃₋₇ carbocyclyl; and C₂₋₇ alkylcarbonyl,
      (e) 5- or 6-membered heteroaryl optionally substituted with 1 to 4 the same or different substituents selected from the group consisting of halogen atom, cyano, and C₁₋₆ alkyl,
      (f) 4- to 7-membered saturated or partially-unsaturated heterocyclyl optionally substituted with 1 to 4 the same or different substituents selected from the group consisting of halogen atom, hydroxy, C₁₋₆ alkyl, and C₁₋₆ alkoxy, or
      (g) -C(O)NR^{x}R^{y}, wherein R^{x} and R^{y} are each independently hydrogen atom, C₁₋₆ alkyl, or saturated or partially-unsaturated C₃₋₇ carbocyclyl; or
         alternatively, R^{x} and R^{y} may be combined together with the nitrogen atom to which they attach to form 4- to 7-membered saturated heterocyclyl, and
      either of the following condition (X') or (Y') is met:
      (X') at least one of R⁵ and R⁶ is cyano, 5- or 6-membered heteroaryl (wherein the heteroaryl may be optionally substituted with 1 to 4 the same or different substituents selected from the group consisting of halogen atom, cyano, and C₁₋₆ alkyl), 4- to 7-membered saturated or partially-unsaturated heterocyclyl (wherein the heterocyclyl may be optionally substituted with 1 to 4 the same or different substituents selected from the group consisting of halogen atom, hydroxy, C₁₋₆ alkyl, and C₁₋₆ alkoxy), or -C(O)NR^{x}R^{y} (wherein R^{x} and R^{y} are each independently hydrogen atom, C₁₋₆ alkyl, or saturated or partially-unsaturated C₃₋₇ carbocyclyl; or alternatively, R^{x} and R^{y} may be combined together with the nitrogen atom to which they attach to form 4- to 7-membered saturated heterocyclyl); or
      (Y') R⁵ and R⁶ may be combined together with the carbon atoms to which they attach to form a 5- to 7-membered saturated or partially-unsaturated carbocycle or heterocycle (wherein the carbocycle and heterocycle may be optionally substituted with 1 to 4 the same or different substituents selected from the group consisting of halogen atom, oxo, C_{1- 6} alkyl, C₁₋₆ alkoxy, and C₂₋₇ alkoxycarbonyl) ,
         wherein the group of formula (2c') may further be optionally substituted with a fluorine atom at a substitutable carbon atom of the ring,
   (4) a group of the following formula (2d), (2e), (2f), or (2g): 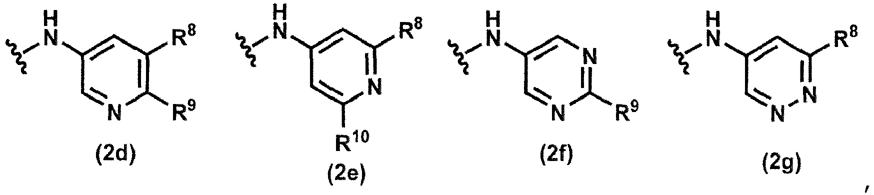 wherein R⁸, R⁹, and R¹⁰ are each independently
      (a) hydrogen atom,
      (b) halogen atom,
      (c) cyano,
      (d) C₁₋₆ alkyl optionally substituted with 1 to 3 the same or different substituents selected from the group consisting of halogen atom; hydroxy; C₁₋₆ alkoxy optionally substituted with hydroxy or C₁₋₆ alkoxy; 4- to 7-membered saturated or partially-unsaturated heterocyclyl optionally substituted with C₁₋₆ alkyl or C₁₋₆ alkoxy; 5- or 6-membered heteroaryl optionally substituted with C₁₋₆ alkyl; and amino, wherein the amino may be optionally substituted with 1 to 2 the same or different substituents selected from the group consisting of C₁₋₆ alkyl optionally substituted with 1 to 3 the same or different substituents selected from the group consisting of halogen atom, hydroxy, and C₁₋₆ alkoxy; saturated or partially-unsaturated C₃₋₇ carbocyclyl; 4- to 7-membered saturated heterocyclyl optionally substituted with C₁₋₆ alkoxy; and C₂₋₇ alkylcarbonyl optionally substituted with 1 to 3 the same or different substituents selected from the group consisting of halogen atom, hydroxy, and C₁₋₆ alkoxy,
      (e) C₁₋₆ alkoxy optionally substituted with 1 to 3 the same or different substituents selected from the group consisting of halogen atom, hydroxy, and C₁₋₆ alkoxy,
      (f) amino optionally substituted with 1 to 2 the same or different substituents selected from the group consisting of C₁₋₆ alkyl optionally substituted with halogen atom; saturated or partially-unsaturated C₃₋₇ carbocyclyl; and C₂₋₇ alkylcarbonyl optionally substituted with 1 to 3 the same or different substituents selected from the group consisting of halogen atom, hydroxy, and C₁₋₆ alkoxy,
      (g) 5- or 6-membered heteroaryl optionally substituted with 1 to 4 the same or different substituents selected from the group consisting of halogen atom, cyano, C₁₋₆ alkyl, C₁₋₆ alkoxy, and C₂₋₇ alkoxycarbonyl,
      (h) 4- to 7-membered saturated or partially-unsaturated heterocyclyl optionally substituted with 1 to 4 the same or different substituents selected from the group consisting of C₁₋₆ alkyl optionally substituted with 1 to 3 the same or different substituents selected from the group consisting of halogen atom, hydroxy, and C₁₋₆ alkoxy; C₁₋₆ alkoxy; 4- to 7-membered saturated or partially-unsaturated heterocyclyl; C₂₋₇ alkylcarbonyl optionally substituted with 1 to 3 the same or different substituents selected from the group consisting of halogen atom, hydroxy, and C₁₋₆ alkoxy; and oxo,
      (i) 4- to 7-membered saturated or partially-unsaturated heterocyclyl-oxy optionally substituted with 1 to 4 C₁₋₆ alkyl,
      (j) -C(O)NR^{x}R^{y}, wherein R^{x} and R^{y} are each independently hydrogen atom, C₁₋₆ alkyl, or saturated or partially-unsaturated C₃₋₇ carbocyclyl; or
         alternatively, R^{x} and R^{y} may be combined together with the nitrogen atom to which they attach to form 4- to 7-membered saturated heterocyclyl,
      (k) -C(O)OR^{z}, wherein R^{z} is C₁₋₆ alkyl, or
      (l) ethenyl optionally substituted with one 6-membered saturated heterocyclyl group;
      wherein R⁸ and R⁹ may be combined together with the carbon atoms to which they attach to form a 5- to 7-membered saturated or partially-unsaturated carbocycle or heterocycle, wherein the carbocycle and heterocycle may be optionally substituted with 1 to 4 the same or different substituents selected from the group consisting of halogen atom and C₁₋₆ alkyl;
      wherein both of R⁸ and R⁹ of formula (2d) are not hydrogen atoms at the same time, and a group of formula (2e) may further be optionally substituted with a fluorine atom at a substitutable carbon atom of the ring, or
   (5) a group of the following formula (2h'): 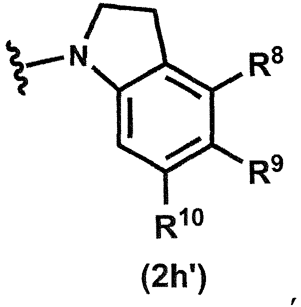 wherein R⁸, R⁹, and R¹⁰ are the same as those defined in the above (4) of the present clause.
[4] The compound according to [3], or a pharmaceutically acceptable salt thereof, wherein
   M² is
   (1) a group of any one of the following formulae (11), (12), (18), (26), (31), and (34): 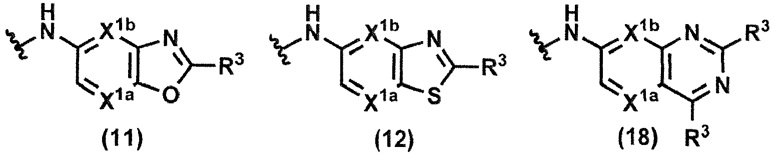 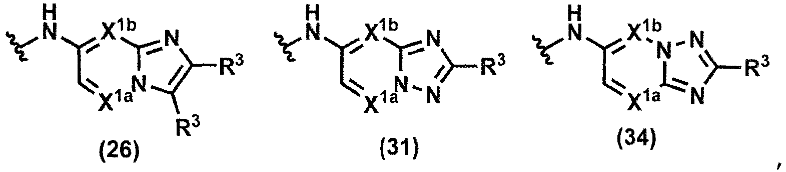 wherein X^{1a}, X^{1b}, and R³ are the same as those defined in the above [1],
   (2) 4-cyanophenylamino,
   (3) a group of the following formula (2h"): 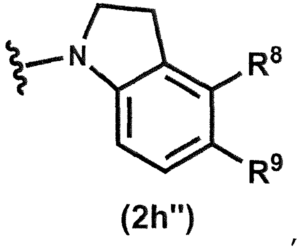 wherein R⁸ and R⁹ are the same as those defined in the above [3].
[5] The compound according to any one of [1] to [4], or a pharmaceutically acceptable salt thereof, wherein
   M¹ is
   (1) saturated or partially-unsaturated C₄₋₁₂ carbocyclyl optionally substituted with 1 to 4 the same or different substituents selected from the group consisting of halogen atom and C₁₋₆ alkyl optionally substituted with 1 to 3 the same or different substituents selected from the group consisting of halogen atom, hydroxy, and C₁₋₆ alkoxy, or
   (2) 4- to 12-membered saturated or partially-unsaturated heterocyclyl optionally substituted with 1 to 4 the same or different substituents selected from the group consisting of halogen atom and C₁₋₆ alkyl optionally substituted with 1 to 3 the same or different substituents selected from the group consisting of halogen atom, hydroxy, and C₁₋₆ alkoxy.
[6] The compound according to [5], or a pharmaceutically acceptable salt thereof, wherein
   M¹ is a group of the following formula (3): 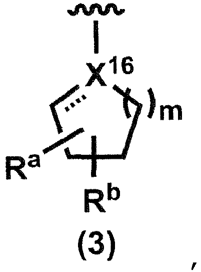 wherein X¹⁶ is N, C, or CH;
   a bond comprising a broken line denotes a single or double bond;
   m is 0, 1, 2, or 3;
   R^{a} and R^{b} are each independently
   (1-1) hydrogen atom,
   (1-2) halogen atom, or
   (1-3) C₁₋₆ alkyl optionally substituted with 1 to 3 the same or different substituents selected from the group consisting of halogen atom, hydroxy, and C₁₋₆ alkoxy; or
      alternatively, R^{a} and R^{b} may be combined together with the carbon atom(s) to which they attach to form a 3- to 6-membered saturated carbocycle, wherein the carbocycle may be optionally substituted with 1 to 4 the same or different substituents selected from the group consisting of:
      (a) halogen atom,
      (b) hydroxy,
      (c) C₁₋₆ alkyl optionally substituted with 1 to 3 the same or different substituents selected from the group consisting of halogen atom, hydroxy, and C₁₋₆ alkoxy, and
      (d) C₁₋₆ alkoxy optionally substituted with 1 to 3 the same or different substituents selected from the group consisting of halogen atom, hydroxy, and C₁₋₆ alkoxy.
[7] The compound according to any one of [1] to [6], or a pharmaceutically acceptable salt thereof, wherein Formula (1) is formula (1"): 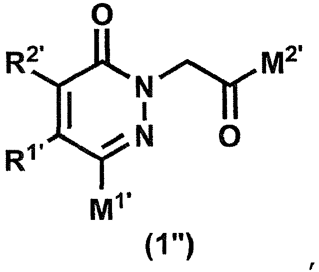 wherein M¹' is a group of any one of the following formulae (38) - (52): 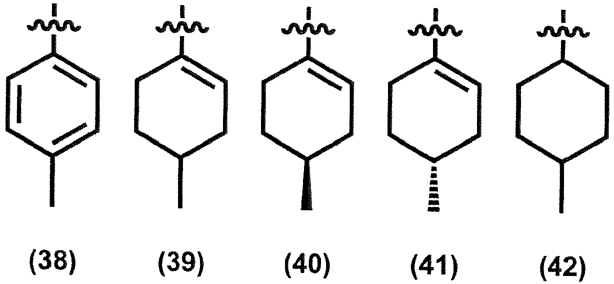 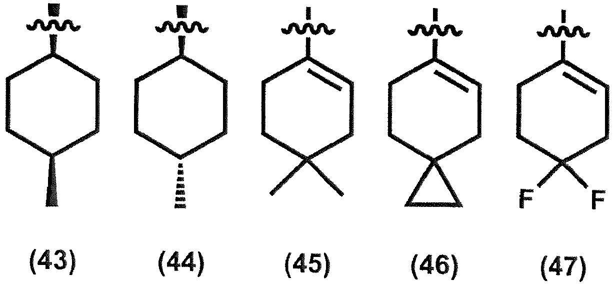 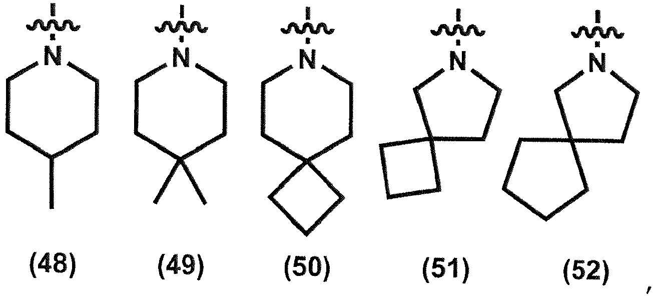 R¹' and R²' are each independently
   (2-1) hydrogen atom,
   (2-2) halogen atom,
   (2-3) cyano,
   (2-4) methyl, or
   (2-5) methoxy, and
      M²' is
      (1) a group of any one of the following formulae (53) - (58): 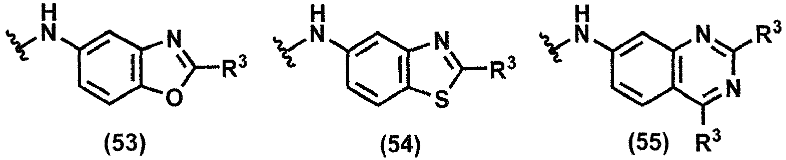 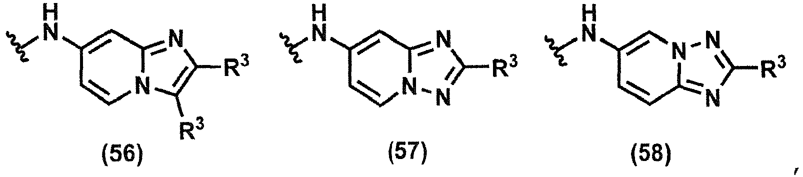 wherein R³, where R³s are each independent when existing plurally, is
         (a) hydrogen atom,
         (b) halogen atom,
         (c) cyano,
         (d) C₁₋₆ alkyl optionally substituted with 1 to 3 the same or different substituents selected from the group consisting of halogen atom, hydroxy, saturated or partially-unsaturated C₃₋₇ carbocyclyl, C₁₋₆ alkoxy, 4- to 7-membered saturated heterocyclyl optionally substituted with C₁₋₆ alkoxy, and amino optionally substituted with 1 to 2 the same or different C₁₋₆ alkyl,
         (e) C₁₋₆ alkoxy optionally substituted with 1 to 3 the same or different substituents selected from the group consisting of halogen atom, hydroxy, C₁₋₆ alkoxy, and amino optionally substituted with 1 to 2 the same or different C₁₋₆ alkyl, or
         (f) amino optionally substituted with 1 to 2 the same or different substituents selected from the group consisting of C₁₋₆ alkyl optionally substituted with 1 to 3 the same or different substituents selected from the group consisting of halogen atom, hydroxy, and C₁₋₆ alkoxy; saturated or partially-unsaturated C₃₋₇ carbocyclyl; and C₂₋₇ alkylcarbonyl optionally substituted with 1 to 3 the same or different substituents selected from the group consisting of halogen atom, hydroxy, and C₁₋₆ alkoxy,
      (2) 4-cyanophenylamino, or
      (3) a group of the following formula (2h'''): 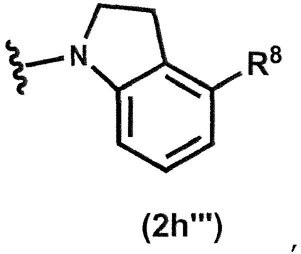 wherein R⁸ is
         (a) C₁₋₆ alkyl optionally substituted with 1 to 3 the same or different substituents selected from the group consisting of halogen atom; hydroxy; C₁₋₆ alkoxy optionally substituted with hydroxy or C₁₋₆ alkoxy; 4- to 7-membered saturated or partially-unsaturated heterocyclyl optionally substituted with C₁₋₆ alkyl or C₁₋₆ alkoxy; 5- or 6-membered heteroaryl optionally substituted with C₁₋₆ alkyl; and amino, wherein the amino may be optionally substituted with 1 to 2 the same or different substituents selected from the group consisting of C₁₋₆ alkyl optionally substituted with 1 to 3 the same or different substituents selected from the group consisting of halogen atom, hydroxy, and C₁₋₆ alkoxy; saturated or partially-unsaturated C₃₋₇ carbocyclyl; 4- to 7-membered saturated heterocyclyl optionally substituted with C₁₋₆ alkoxy; and C₂₋₇ alkylcarbonyl optionally substituted with 1 to 3 the same or different substituents selected from the group consisting of halogen atom, hydroxy, and C₁₋₆ alkoxy,
         (b) C₁₋₆ alkoxy optionally substituted with 1 to 3 the same or different substituents selected from the group consisting of halogen atom, hydroxy, and C₁₋₆ alkoxy,
         (c) amino optionally substituted with 1 to 2 the same or different substituents selected from the group consisting of C₁₋₆ alkyl optionally substituted with halogen atoms; saturated or partially-unsaturated C₃₋₇ carbocyclyl; and C₂₋₇ alkylcarbonyl optionally substituted with 1 to 3 the same or different substituents selected from the group consisting of halogen atom, hydroxy, and C₁₋₆ alkoxy,
         (d) 5- or 6-membered heteroaryl optionally substituted with 1 to 4 the same or different substituents selected from the group consisting of halogen atom, cyano, C₁₋₆ alkyl, C₁₋₆ alkoxy, and C₂₋₇ alkoxycarbonyl,
         (e) 4- to 7-membered saturated or partially-unsaturated heterocyclyl optionally substituted with 1 to 4 the same or different substituents selected from the group consisting of C₁₋₆ alkyl optionally substituted with 1 to 3 the same or different substituents selected from the group consisting of halogen atom, hydroxy, and C₁₋₆ alkoxy; C₁₋₆ alkoxy; 4- to 7-membered saturated or partially-unsaturated heterocyclyl; C₂₋₇ alkylcarbonyl optionally substituted with 1 to 3 the same or different substituents selected from the group consisting of halogen atom, hydroxy, and C₁₋₆ alkoxy; and oxo; or
         (f) 4- to 7-membered saturated or partially-unsaturated heterocyclyl-oxy optionally substituted with 1 to 4 C₁₋₆ alkyl.
[8] The compound according to [7], or a pharmaceutically acceptable salt thereof, wherein
   M¹' is a group of the following formula (38): 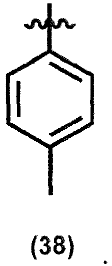
[9] The compound according to [7], or a pharmaceutically acceptable salt thereof, wherein
   M¹' is a group of the following formula (39), (40), (41), or (45) : 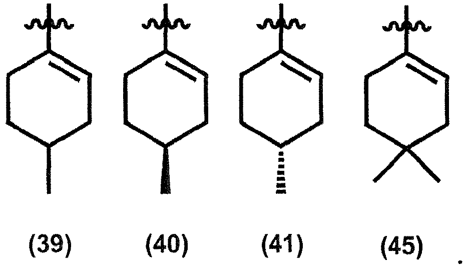
[10] The compound according to [7], or a pharmaceutically acceptable salt thereof, wherein
   M¹' is a group of the following formula (48), (50), or (51): 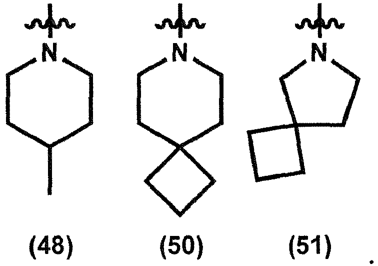
[11] The compound according to any one of [7] to [10], or a pharmaceutically acceptable salt thereof, wherein M²' is a group of any one of the following formulae (53) - (58) : 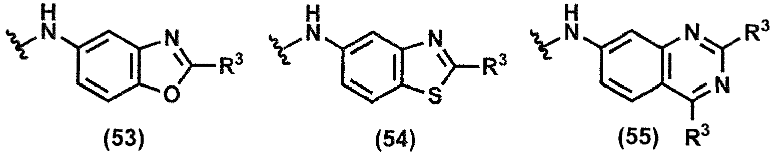 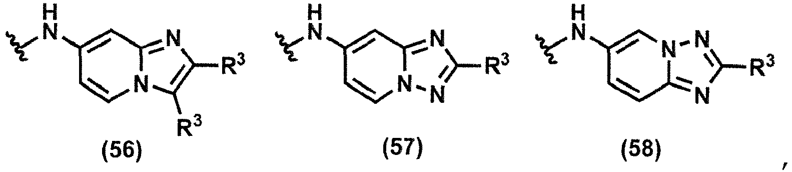 wherein R³ is hydrogen atom, halogen atom, cyano, C₁₋₆ alkyl, C₁₋₆ alkoxy, or amino optionally substituted with 1 to 2 the same or different C₁₋₆ alkyl.
[12] The compound according to any one of [7] to [10], or a pharmaceutically acceptable salt thereof, wherein M²' is the following formula (57) or (58): 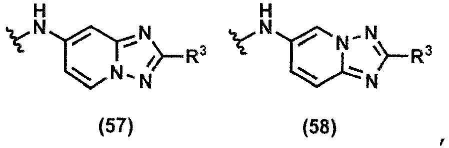 wherein R³ is hydrogen atom, halogen atom, cyano, C₁₋₆ alkyl, C₁₋₆ alkoxy, or amino optionally substituted with 1 to 2 the same or different C₁₋₆ alkyl.
[13] The compound according to any one of [7] to [10], or a pharmaceutically acceptable salt thereof, wherein M²' is 4-cyanophenylamino.
[14] The compound according to any one of [7] to [10], or a pharmaceutically acceptable salt thereof, wherein
   M²' is
   (3) a group of the following formula (2h'''): 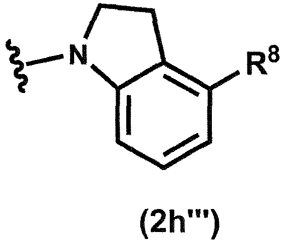 wherein R⁸ is
   (a) 5- or 6-membered heteroaryl optionally substituted with 1 to 4 the same or different substituents selected from the group consisting of halogen atom, cyano, and C₁₋₆ alkyl, or
   (b) 4- to 7-membered saturated or partially-unsaturated heterocyclyl optionally substituted with 1 to 4 the same or different substituents selected from the group consisting of C₁₋₆ alkyl optionally substituted with 1 to 3 the same or different substituents selected from the group consisting of halogen atom, hydroxy, and C₁₋₆ alkoxy; C₁₋₆ alkoxy; and oxo.
[15] The compound according to [1], or a pharmaceutically acceptable salt thereof, wherein the compound is selected from the group consisting of:
   N-(4-cyanophenyl)-2-[3-(4-methylpiperidin-1-yl)-6-oxopyridazin-1(6H)-yl]acetamide (Example 1),
   N-(1,3-benzooxazol-5-yl)-2-[3-(4-methylpiperidin-1-yl)-6-oxopyridazin-1(6H)-yl]acetamide (Example 50),
   2-[3-(6-azaspiro[3.4]octan-6-yl)-6-oxopyridazin-1(6H)-yl]-N-(quinazolin-7-yl)acetamide (Example 236),
   N-[2-(dimethylamino)-1,3-benzooxazol-5-yll-2-[3-(4-methylcyclohex-1-en)-6-oxopyridazin-1(6H)-yl]acetamide (Example 244),
   2-[3-(4,4-dimethylcyclohex-1-en-1-yl)-6-oxopyridazin-1(6H)-yl]-N-([1,2,4]triazolo[1,5-a]pyridin-6-yl)acetamide (Example 428),
   2-[3-(4,4-dimethylcyclohex-1-en-1-yl)-6-oxopyridazin-1(6H)-yl]-N-([1,2,4]triazolo[1,5-a]pyridin-7-yl)acetamide (Example 429),
   N-(1,3-benzooxazol-5-yl)-2-[3-(4,4-dimethylcyclohex-1-en-1-yl)-6-oxopyridazin-1(6H)-y1]acetamide (Example 445),
   2-[6-oxo-3-(spiro[2.5]oct-5-en-6-yl)pyridazin-1(6H)-yl]-N-([1,2,4]triazolo[1,5-a]pyridin-7-yl)acetamide (Example 512),
   2-{2-oxo-2-[4-(pyridazin-4-yl)-2.3-dihydro-1H-indol-1-yl]ethyl}-6-(spiro[2.5]oct-5-en-6-yl)pyridazin-3(2H)one (Example 526),
   N-(1,3-benzooxazol-5-yl)-2-[3-(4-methylcyclohex-1-en-1-yl)-6-oxopyridazin-1(6H)-yl)acetamide (Example 531),
   2-{3-[(4S)-4-methylcyclohex-1-en-1-yl]-6-oxopyridazin-1(6H)-yl}-N-([1,2,4]triazolo[1,5-a]pyridin-7-yl)acetamide (Example 537),
   2-{3-[(4R)-4-methylcyclohex-1-en-1-yl]-6-oxopyridazin-1(6H)-yl}-N-([1,2,4]triazolo[1,5-a]pyridin-7-yl)acetamide (Example 538),
   2-{3-[(4S)-4-methylcyclohex-1-en-1-yl]-6-oxopyridazin-1(6H)-yl}-N-([1,2,4]triazolo[1,5-a]pyridin-6-yl)acetamide (Example 539), and
   2-{3-[(4R)-4-methylcyclohex-1-en-1-yl]-6-oxopyridazin-1(6H)-yl}-N-([1,2,4]triazolo[1,5-a]pyridin-6-yl)acetamide (Example 540).
[16] A pharmaceutical composition comprising a compound according to any one of [1] to [15], or a pharmaceutically acceptable salt thereof, as an active ingredient.
[17] A medicament for activating Nav1.1, comprising a compound according to any one of [1] to [15], or a pharmaceutically acceptable salt thereof, as an active ingredient.
[18] A medicament for treating and/or preventing a central nervous system disease, comprising a compound according to any one of [1] to [15], or a pharmaceutically acceptable salt thereof, as an active ingredient.
[19] A medicament for treating and/or preventing a disease involving Nav1.1, comprising a compound according to any one of [1] to [15], or a pharmaceutically acceptable salt thereof, as an active ingredient.
[20] A medicament for treating and/or preventing a disease involving reduced function of Nav1.1 comprising a compound according to any one of [1] to [15] or a pharmaceutically acceptable salt thereof, as an active ingredient.
[21] The medicament according to claim 19 or 20, wherein the disease involving Nav1.1 is a central nervous system disease.
[22] The medicament according to [18] or [21], wherein the central nervous system disease is at least one selected from the group consisting of febrile seizure (FS); generalised epilepsy with febrile seizure plus (GEFS+); epilepsy (specifically, focal epilepsy, generalized epilepsy); epileptic syndrome (such as Dravet syndrome, intractable childhood epilepsy with generalized tonic-clonic seizure (ICE-GTC), epilepsy with myoclonic-atonic seizure (Doose syndrome), West syndrome, Lennox-Gastaut syndrome (Rasmussen's encephalitis and Lennox-Gastaut syndrome), infantile spasm, sever infantile multifocal epilepsy (SIMFE), severe myoclonic epilepsy, borderline (SMEB), and benign familial neonatal-infantile seizure (BFNIS)); schizophrenia; autism spectrum disorder (ASD); and attention deficit hyperactivity disorder (ADHD).
[23] Use of a compound according to any one of [1] to [15], or a pharmaceutically acceptable salt thereof, in the manufacture of a medicament for treating and/or preventing a disease involving Nav1.1.
[24] A compound according to any one of [1] to [15], or a pharmaceutically acceptable salt thereof, for use in treating and/or preventing a disease involving Nav1.1.
[25] A method for treating and/or preventing a disease involving Nav1.1, comprising administering to a patient in need thereof a therapeutically effective amount of a compound according to any one of [1] to [15] or a pharmaceutically acceptable salt thereof.
[26] A combination medicament comprising a compound according to any one of [1] to [15], or a pharmaceutically acceptable salt thereof, and one or more drugs selected from drugs classified as antiepileptic agents, antidepressant agents, antiparkinsonian agents, antischizophrenic agents, or therapeutic agents for ADHD.
[27] A compound according to any one of [1] to [15], or a pharmaceutically acceptable salt thereof, or a pharmaceutical composition or medicament according to any one of [16] to [22], for use in treating and/or preventing a central nervous system disease, for combination use with one or more drugs selected from drugs classified as antiepileptic agents, antidepressant agents, antiparkinsonian agents, antischizophrenic agents, or therapeutic agents for ADHD.

### EFFECT OF THE INVENTION

The present compound can have a significant effect on the activation of Nav1.1. Furthermore, in one embodiment, the present compound can have a selective activity to Nav1.1, compared with the activity to different subtypes of voltage-dependent sodium channels such as Nav1.5. Thus, it is expected that the present compound is useful as a medicament for treating and/or preventing diseases involving Nav1.1 and various central nervous system diseases.

### DESCRIPTION OF EMBODIMENTS

Hereinafter, the present invention is explained in detail. The present specification may denote the number of carbon atoms in definitions of "substituents" as, for example, "C₁₋₆" . Specifically, the term "C₁₋₆ alkyl" is synonymous with alkyl having 1 to 6 carbon atoms.

Examples of the term "halogen atom" include fluorine atom, chlorine atom, bromine atom, and iodine atom.

The term "C₁₋₆ alkyl" means a straight- or branched-chain saturated hydrocarbon group having 1 to 6 carbon atoms. It is preferably "C₁₋₄ alkyl". Examples of the term "C₁₋₆ alkyl" include methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, and 2-ethylbutyl.

The term "C₂₋₇ alkylcarbonyl" means a carbonyl group substituted with the above "C₁₋₆ alkyl". For example, it is preferably "C₂₋₄ alkylcarbonyl". Examples of the term "C₂₋₇ alkylcarbonyl" include methylcarbonyl, ethylcarbonyl, normal-propylcarbonyl, and isopropylcarbonyl.

The term "C₂₋₆ alkenyl" means a straight- or branched-chain unsaturated hydrocarbon group having 1 to 3 carbon-carbon double bonds and 2 to 6 carbon atoms. It is preferably "C₂₋₄ alkenyl". Examples of the term "C₂₋₆ alkenyl" include ethenyl, propenyl, butenyl, pentenyl, and hexenyl.

The term "saturated or partially-unsaturated C₃₋₇ carbocyclyl" means a 3- to 7-membered monocyclic or polycyclic saturated or partially-unsaturated hydrocarbon group. It is preferably "saturated or partially-unsaturated C₅₋₇ carbocyclyl". Examples of the term "saturated or partially-unsaturated C₃₋₇ carbocyclyl" include cyclopropyl, cyclobutyl, cyclopentyl, cyclohexyl, cyclopentenyl, and cyclohexenyl.

The term "saturated or partially-unsaturated C₄₋₁₂ carbocyclyl" means a 4- to 12-membered monocyclic or polycyclic saturated or partially-unsaturated hydrocarbon group. It is preferably "saturated or partially-unsaturated C₄₋₆ carbocyclyl". Examples of the term "saturated or partially-unsaturated C₄₋₁₂ carbocyclyl" include cyclooctyl, cyclodecyl, and cyclododecyl, besides those listed as examples of the above "saturated or partially-unsaturated C₃₋₇ carbocyclyl".

The above "saturated or partially-unsaturated C₄₋₁₂ carbocyclyl" includes saturated or partially-unsaturated bicyclic groups and saturated or partially-unsaturated spiro groups. Examples include groups of the following formulae:

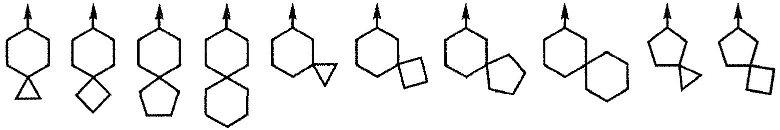

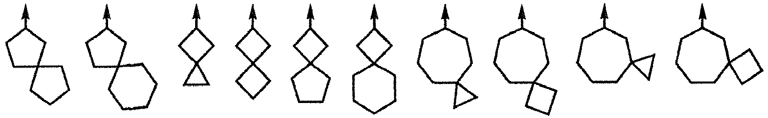

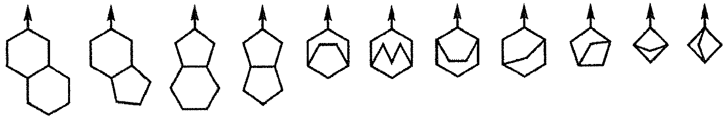

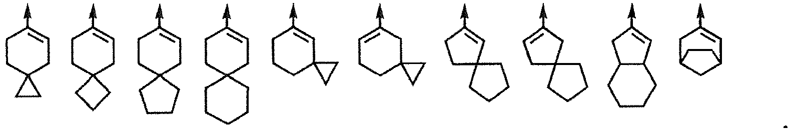

The term "5- or 6-membered saturated or partially-unsaturated carbocyclyl" means a 5- or 6-membered monocyclic saturated or partially-unsaturated hydrocarbon group. Examples of the term "5- or 6-membered saturated or partially-unsaturated carbocyclyl" include cyclopentyl, cyclohexyl, cyclopentenyl, and cyclohexenyl.

The term "5- to 7-membered saturated or partially-unsaturated carbocycle" means a monocyclic or bicyclic saturated or partially-unsaturated hydrocarbon group having 5 to 7 carbon atoms, and includes structures having partially-unsaturated bond(s), structures having bridged structure(s), and structures forming spiro ring(s). Examples of the term "5- to 7-membered saturated or partially-unsaturated carbocycle" include cyclopentane, cyclohexane, cycloheptane, cyclopentene, cyclohexene, cycloheptene, cyclohexadiene, and cycloheptadiene.

The term "3- to 6-membered saturated carbocycle" means a saturated hydrocarbon ring having 3 to 6 carbon atoms, and includes structures forming spiro ring(s). Examples of the term "3- to 6-membered saturated carbocycle" include cyclopropane, cyclobutane, cyclopentane, and cyclohexane.

The "C₁₋₆ alkyl" part of the term "C₁₋₆ alkoxy" is synonymous with the above "C₁₋₆ alkyl". This term is preferably "C₁₋₄ alkoxy". Examples of the term "C₁₋₆ alkoxy" include, for example, methoxy, ethoxy, propoxy, isopropoxy, butoxy, isobutoxy, sec-butoxy, and tert-butoxy.

The term "C₂₋₇ alkoxycarbonyl" means a carbonyl group substituted with the above "C₁₋₆ alkoxy". For example, it is preferably "C₂₋₅ alkoxycarbonyl". Examples of the term "C₂₋₇ alkoxycarbonyl" include methoxycarbonyl, ethoxycarbonyl, propoxycarbonyl, isopropoxycarbonyl, butoxycarbonyl, isobutoxycarbonyl, sec-butoxycarbonyl, and tert-butoxycarbonyl.

The term "5- or 6-membered heteroaryl" means a 5- or 6-membered aromatic group which comprises one or more (for example, 1 to 4) the same or different heteroatoms selected from nitrogen atom, sulfur atom, and oxygen atom, and which may be optionally substituted with oxo. Examples of the term "5- or 6-membered heteroaryl" include groups of the following formulae:

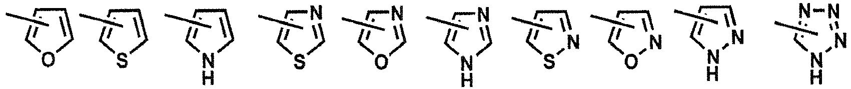

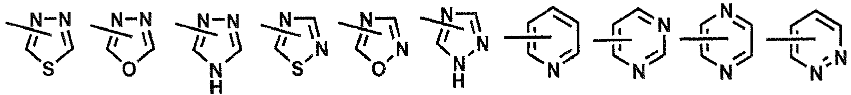

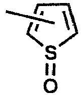

It is preferably imidazolyl, oxazolyl, thiazolyl, pyrazolyl, isoxazolyl, isothiazolyl, triazolyl, oxadiazolyl, pyridyl, pyrimidyl, pyrazyl, or pyridazyl; and it is more preferably imidazolyl, oxazolyl, thiazolyl, pyrazolyl, triazolyl, oxadiazolyl, pyridyl, pyrimidyl, pyrazyl, or pyridazyl.

The term "5- to 10-membered heteroaryl" includes, for example, a 5- to 10-membered monocyclic or 9- or 10-membered bicyclic aromatic heterocyclyl group. The "5- to 10-membered heteroaryl" group comprises one or more (for example, 1 to 4) the same or different heteroatoms selected from nitrogen atom, sulfur atom, and oxygen atom, and may be optionally substituted with oxo. The bicyclic heteroaryl group also includes fused structures of the above monocyclic heteroaryl group with an aromatic ring (such as benzene and pyridine) or non-aromatic ring (such as cyclohexane and piperidine). Examples of the term "5- to 10-membered heteroaryl" include groups of the following formulae: 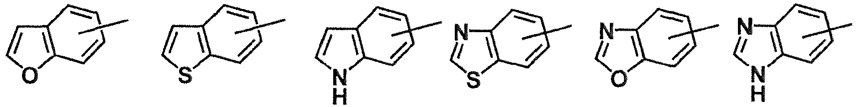 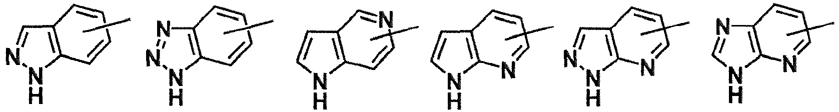 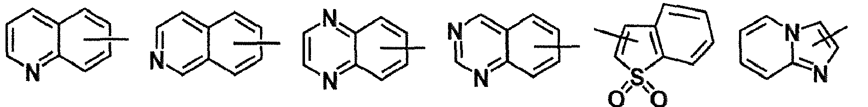 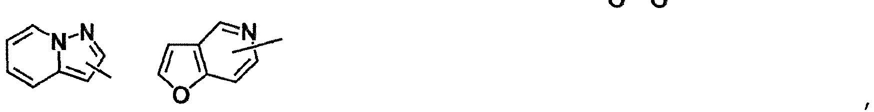 besides those listed as examples of the above "5- or 6-membered heteoaryl".

In the present specification, a bond across a ring means that a "group" having the bond is attached at a substitutable position of the ring to a group. For example, a heteroaryl group of the following formula: 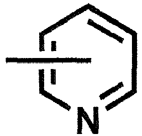 denotes 2-pyridyl, 3-pyridyl, or 4-pyridyl.

The term "4- to 7-membered saturated or partially-unsaturated heterocyclyl" includes, for example, a 4- to 7-membered monocyclic or polycyclic saturated or partially-unsaturated heterocyclyl group comprising 1 to 2 the same or different atoms selected from nitrogen atom, oxygen atom, and sulfur atom. It is preferably "5- to 7-membered saturated or partially-unsaturated heterocyclyl". Examples of the term "5- to 7-membered saturated or partially-unsaturated heterocyclyl" include pyranyl, dihydropyranyl, tetrahydropyranyl, tetrahydrofuryl, dihydropyrrolyl, dihydrofuranyl, pyrrolidinyl, imidazolidinyl, piperidinyl, piperazinyl, dioxanyl, azepanyl, morpholinyl, and thiomorpholinyl. Examples of the term "4- to 7-membered saturated or partially-unsaturated heterocyclyl" include azetidinyl and oxetanyl, besides those listed as examples of the above "5- to 7-membered saturated or partially-unsaturated heterocyclyl". Among them, examples of the term "4- to 7-membered saturated heterocyclyl" include azetidinyl, oxetanyl, tetrahydropyranyl, tetrahydrofuryl, pyrrolidinyl, imidazolidinyl, piperazinyl, dioxanyl, azepanyl, morpholinyl, and thiomorpholinyl. Each group may be attached to a group via any of carbon atom(s) and nitrogen atom(s) that constitute a ring.

The term "4- to 12-membered saturated or partially-unsaturated heterocyclyl" includes, for example, a 4- to 12-membered monocyclic or polycyclic saturated or partially-unsaturated heterocyclyl group comprising 1 to 3 the same or different atoms selected from nitrogen atom, oxygen atom, and sulfur atom. It is preferably a 4- to 10-membered saturated or partially-unsaturated heterocyclyl group. Examples of the group include azocanyl, 1,4-oxazocanyl, 1,5-oxazocanyl, 1,4-diazocanyl, 1,5-diazocanyl, besides those listed as examples of the above "4- to 7-membered saturated or partially-unsaturated heterocyclyl". Each group may be attached to a group via any of carbon atom(s) and nitrogen atom(s) that constitute a ring.

The term "4- to 7-membered saturated or partially-unsaturated heterocyclyl" or "4- to 12-membered saturated or partially-unsaturated heterocyclyl" includes a saturated or partially-unsaturated bicyclic group and a saturated or partially-unsaturated spiro group. Examples of the term "4-to 7-membered saturated or partially-unsaturated heterocyclyl" include groups of the following formulae:

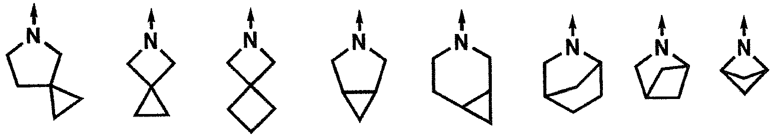

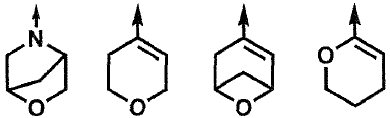

Examples of the term "4- to 12-membered saturated or partially-unsaturated heterocyclyl" include groups of the following formulae:

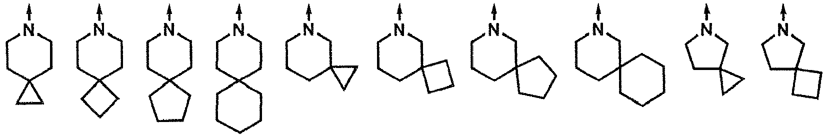

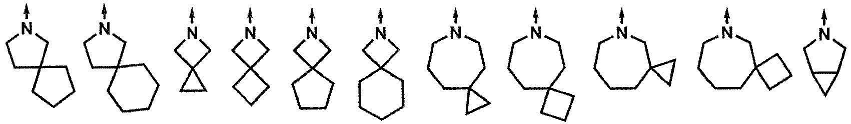

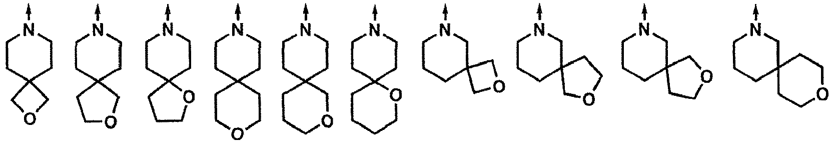

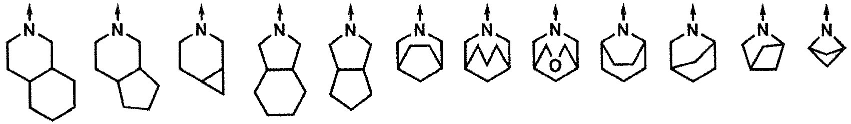

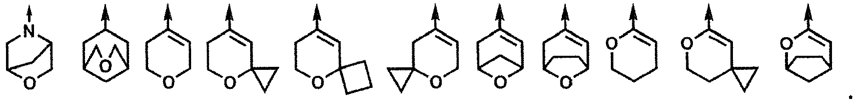

The term "nitrogen-containing saturated ring" includes a saturated heterocycle comprising one or more nitrogen atoms as ring components. Examples of the term "nitrogen-containing saturated ring" include azetidine, pyrrolidine, and piperidine.

The term "9- or 10-membered bicyclic aromatic heterocycle" means a bicyclic aromatic heterocycle which consists of 9 or 10 atoms and comprises 1 to 3 the same or different heteroatoms selected from the group consisting of oxygen atom, nitrogen atom, and sulfur atom, and which may be optionally substituted with oxo. The oxygen atom and sulfur atom of carbonyl, sulfinyl, sulfonyl, and thiocarbonyl which compose the bicyclic aromatic heterocycle do not count as ring members (i.e., the ring size) of the 9- or 10-membered ring nor as heteroatom(s) which compose the ring. Examples of the term "9- or 10-membered bicyclic aromatic heterocycle" include quinoline, isoquinoline, naphthyridine, quinazoline, quinoxaline, benzofuran, benzothiophene, indole, benzooxazole, benzoisooxazole, benzoimidazole, benzooxadiazole, benzothiadiazole, indolizine, benzofuran, indazole, pyrazolopyridine, imidazopyridine, triazolopyridine, imidazopyrimidine, imidazopyridazine, thiazolopyridine, pyrazolopyrimidine, triazolopyridazine, and furopyridine.

The term "3- to 6-membered saturated heterocycle" means a monocyclic or bicyclic saturated heterocycle which consists of 3 to 6 atoms and comprises 1 or 2 the same or different heteroatoms selected from the group consisting of oxygen atom, nitrogen atom, and sulfur atom. The saturated heterocycle includes structures forming spiro ring(s). The saturated heterocycle may be optionally substituted with oxo, and may comprise 1 or 2 carbonyl, thiocarbonyl, sulfinyl, or sulfonyl groups. The oxygen atom and sulfur atom of carbonyl, thiocarbonyl, sulfinyl, and sulfonyl do not count as ring members (i.e., the ring size) of the 3- to 6-membered ring nor as heteroatom(s) which compose the ring. Preferably, the "3- to 6-membered saturated heterocycle" includes "5- or 6-membered saturated heterocycle". Examples of the "5- or 6-membered saturated heterocycle" include, but not limited to, pyrrolidine, piperidine, piperazine, morpholine, tetrahydrofuran, and tetrahydropyran. Examples of the "3-to 6-membered saturated heterocycle" include aziridine and azetidine, besides those listed as examples of the above "5- or 6-membered saturated heterocycle". Examples of the "6-membered saturated heterocycle" include piperidine, morpholine, and tetrahydropyran.

The term "4- to 7-membered saturated or partially-unsaturated heterocyclyl-oxy" means an oxy group substituted with the above "4- to 7-membered saturated or partially-unsaturated heterocyclyl".

Examples of a pyridazinone ring comprising a 5- to 7-membered saturated or partially-unsaturated carbocycle, wherein the carbocycle is formed by combination of R¹ and R² together with the carbon atoms to which they attach, include rings of the following formulae:

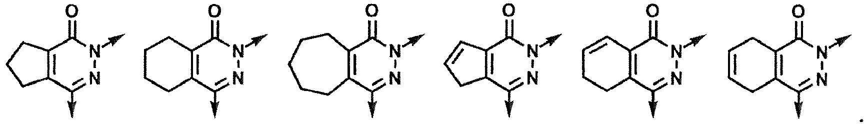

Examples of a pyridazinone ring comprising a 5- to 7-membered saturated or partially-unsaturated heterocycle, wherein the heterocycle is formed by combination of R¹ and R² together with the carbon atoms to which they attach, include rings of the following formulae:

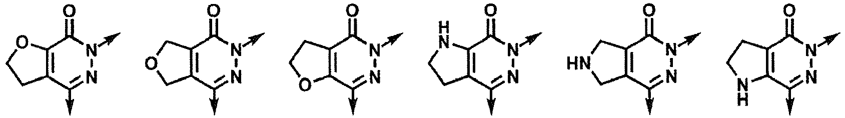

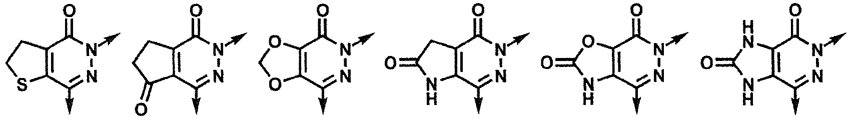

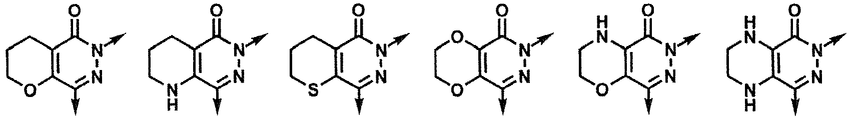

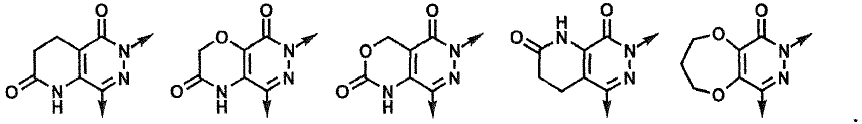

Examples of the group of formula (2c) comprising a 5-to 7-membered saturated or partially-unsaturated carbocycle or heterocycle, wherein the carbocycle and heterocycle are formed by combination of R⁵ and R⁶ together with the carbon atoms to which they attach, include groups of the following formulae:

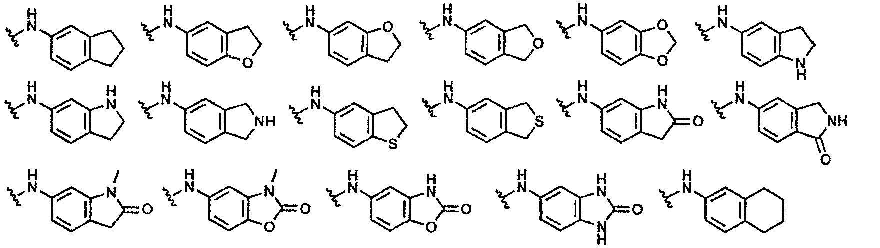

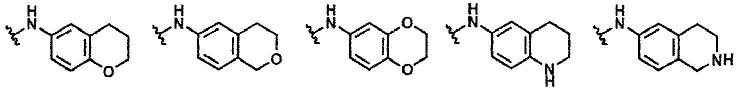

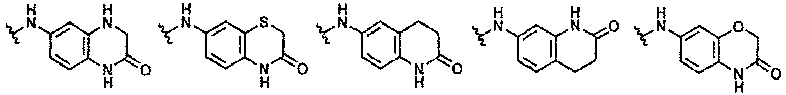

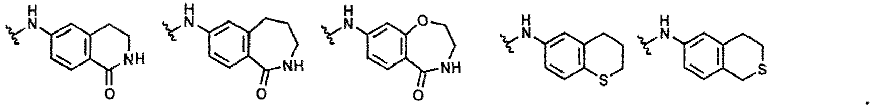

Examples of the group of formula (3) comprising a 3- to 6-membered saturated carbocycle or 3- to 6-membered saturated heterocycle, wherein the carbocycle and heterocycle are formed by combination of R^{a} and R^{b} together with the carbon atom(s) to which they attach, include groups of the following formulae:

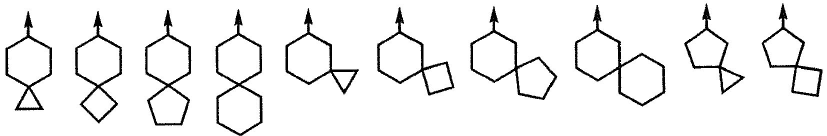

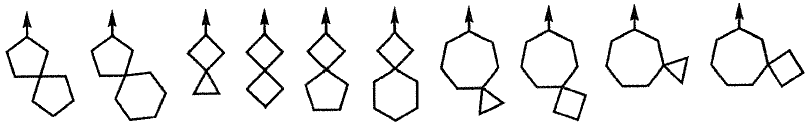

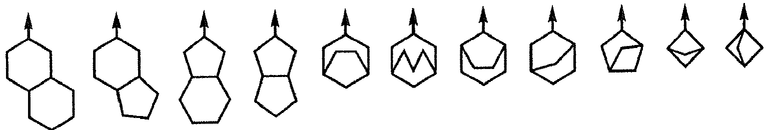

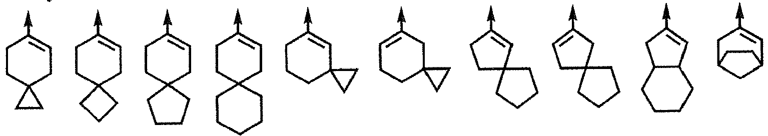

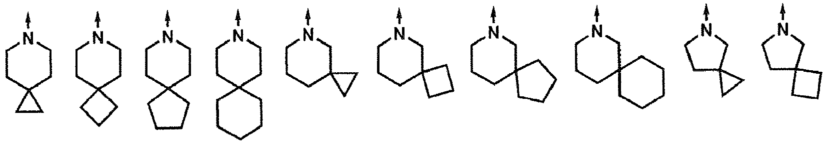

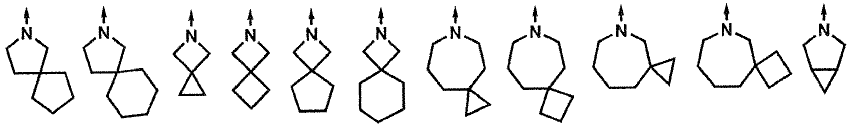

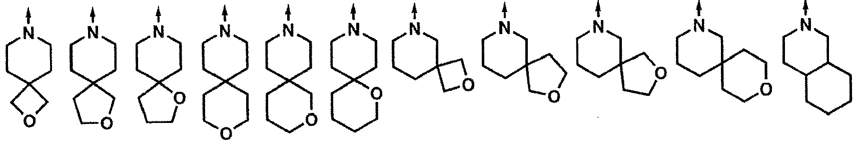

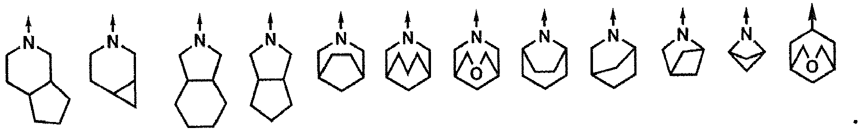

Examples of the group of formula (2a) or (2b) comprising a 9- or 10-membered bicyclic aromatic heterocycle include groups of the following formulae:

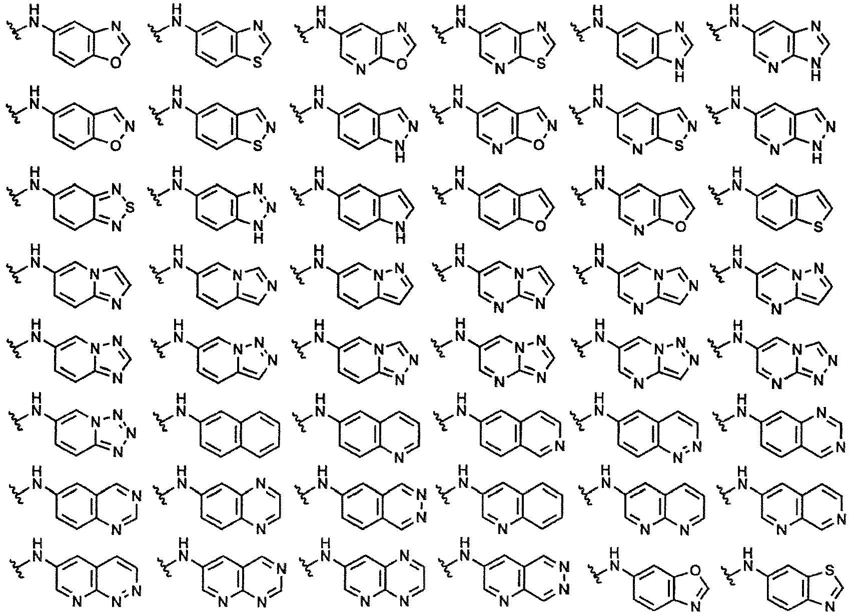

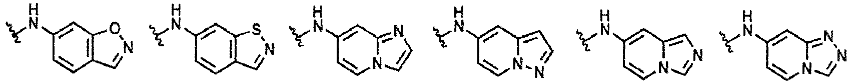

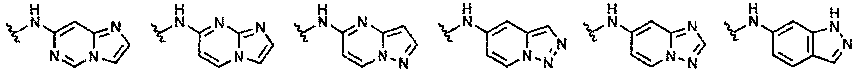

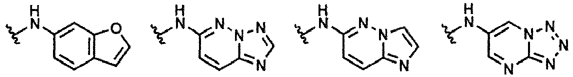

Among the present compounds of Formula (1), preferred embodiments of R¹, R², M¹, and M² are shown below, but the technical scope of the present invention shall not be limited to the scope of the following exemplary embodiments. Preferred embodiments shown below may be optionally combined with each other as long as they are not contradict.

R¹ and R² preferably include, each independently,
(1) hydrogen atom,
(2) C₁₋₆ alkyl optionally substituted with 1 to 3 the same or different substituents selected from the group consisting of halogen atom, hydroxy, saturated or partially-unsaturated C₃₋₇ carbocyclyl, and C₁₋₆ alkoxy,
(3) C₁₋₆ alkoxy optionally substituted with 1 to 3 the same or different substituents selected from the group consisting of halogen atom, hydroxy, and C₁₋₆ alkoxy, or
(4) amino optionally substituted with 1 to 2 the same or different substituents selected from the group consisting of C₁₋₆ alkyl optionally substituted with 1 to 3 the same or different halogen atoms; saturated or partially-unsaturated C₃₋₇ carbocyclyl; and C₂₋₇ alkylcarbonyl.

Another preferred embodiment of R¹ and R² includes the case when they are combined together with the carbon atoms to which they attach to form a 5- or 7-membered saturated or partially-unsaturated carbocycle.

M¹ preferably includes
(1) saturated or partially-unsaturated C₄₋₁₂ carbocyclyl optionally substituted with 1 to 4 the same or different substituents selected from the group consisting of halogen atom and C₁₋₆ alkyl, or
(2) 4- to 12-membered saturated or partially-unsaturated heterocyclyl optionally substituted with 1 to 4 the same or different substituents selected from the group consisting of halogen atom and C₁₋₆ alkyl.

M¹ is further preferably a group of the following formula (3'): 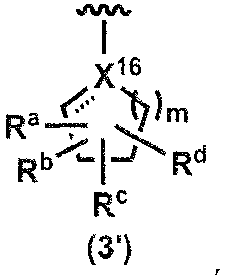 wherein X¹⁶ is N, C, or CH;
a bond comprising a broken line denotes a single bond or a double bond;
m is 0, 1, 2, or 3;
R^{a}, R^{b}, R^{c}, and R^{d} are each independently
(1-1) hydrogen atom,
(1-2) halogen atom,
(1-3) hydroxy,
(1-4) C₁₋₆ alkyl optionally substituted with 1 to 3 the same or different substituents selected from the group consisting of halogen atom, hydroxy, and C₁₋₆ alkoxy,
(1-5) C₁₋₆ alkoxy optionally substituted with 1 to 3 the same or different substituents selected from the group consisting of halogen atom, hydroxy, and C₁₋₆ alkoxy, or
(1-6) amino-carbonyl optionally substituted with C₁₋₆ alkyl which may be optionally substituted with 1 to 3 the same or different halogen atoms;
   wherein R^{a} and R^{b} may be combined together with the carbon atom(s) to which they attach to form
(2-1) a 3- to 6-membered saturated carbocycle, wherein the carbocycle may be optionally substituted with 1 to 4 the same or different substituents selected from the group consisting of:
   (a) halogen atom,
   (b) hydroxy,
   (c) C₁₋₆ alkyl optionally substituted with 1 to 3 the same or different substituents selected from the group consisting of halogen atom, hydroxy, and C₁₋₆ alkoxy, and
   (d) C₁₋₆ alkoxy optionally substituted with 1 to 3 the same or different substituents selected from the group consisting of halogen atom, hydroxy, and C₁₋₆ alkoxy, or
(2-2) a 3- to 6-membered saturated heterocycle, wherein the heterocycle may be optionally substituted with 1 to 4 the same or different substituents selected from the group consisting of (a) to (d) of the above (2-1) in the present clause.

M¹ is more preferably a group of formula (3') wherein X¹⁶ is C or N, m is 1 or 2, R^{a} and R^{b} are each independently hydrogen atom, halogen atom, or C₁₋₆ alkyl optionally substituted with 1 to 3 the same or different halogen atoms, and R^{c} and R^{d} are hydrogen atoms.

Another preferred embodiment of M¹ includes groups of the following formulae (3a), (3b), (3c), (3d), (3e), (3f), (3g), (3h), (3i), (3j), (3k), (3m), (3n), (3p), (3q), (3r), (3s), (3t), (3u), (3v), (3w), (3x), (3y), (3z), (3a'), (3b'), (3c'), (3d'), (3e'), and (3f'):

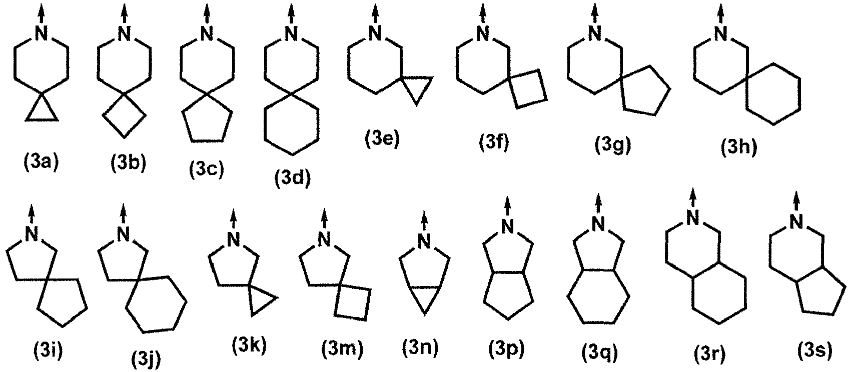

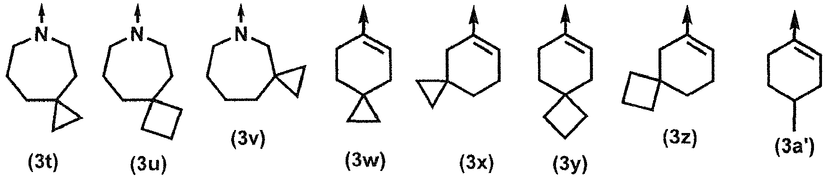

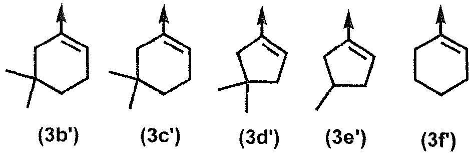

More preferably, M¹ is a group of formula (3a), (3b), (3c), (3d), (3e), (3f), (3g), (3h), (3i), (3j), (3k), (3m), (3n), (3w), (3x), (3y), (3z), (3a'), (3b'), (3c'), (3d'), (3e'), or (3f').

One embodiment of M¹ includes a group of the following formula (3''): 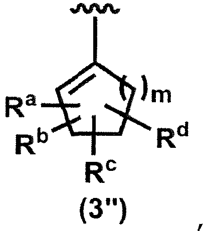 wherein m is 0, 1, 2, or 3;
R^{a}, R^{b}, R^{c}, and R^{d} are each independently
(1-1) hydrogen atom,
(1-2) halogen atom,
(1-3) hydroxy,
(1-4) C₁₋₆ alkyl optionally substituted with 1 to 3 the same or different substituents selected from the group consisting of halogen atom, hydroxy, and C₁₋₆ alkoxy,
(1-5) C₁₋₆ alkoxy optionally substituted with 1 to 3 the same or different substituents selected from the group consisting of halogen atom, hydroxy, and C₁₋₆ alkoxy, or
(1-6) amino-carbonyl optionally substituted with C₁₋₆ alkyl which may be optionally substituted with 1 to 3 the same or different halogen atoms; wherein R^{a} and R^{b} may be combined together with the carbon atom(s) to which they attach to form
(2-1) a 3- to 6-membered saturated carbocycle, wherein the carbocycle may be optionally substituted with 1 to 4 the same or different substituents selected from the group consisting of:
   (a) halogen atom,
   (b) hydroxy,
   (c) C₁₋₆ alkyl optionally substituted with 1 to 3 the same or different substituents selected from the group consisting of halogen atom, hydroxy, and C₁₋₆ alkoxy, and
   (d) C₁₋₆ alkoxy optionally substituted with 1 to 3 the same or different substituents selected from the group consisting of halogen atom, hydroxy, and C₁₋₆ alkoxy, or
(2-2) a 3- to 6-membered saturated heterocycle, wherein the heterocycle may be optionally substituted with 1 to 4 the same or different substituents selected from the group consisting of (a) to (d) of the above (2-1) in the present clause.

Another embodiment of M¹ includes a group of the following formula (3'''): 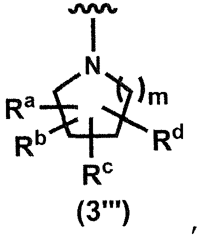 wherein m is 0, 1, 2, or 3;
R^{a}, R^{b}, R^{c}, and R^{d} are each independently
(1-1) hydrogen atom,
(1-2) halogen atom,
(1-3) hydroxy,
(1-4) C₁₋₆ alkyl optionally substituted with 1 to 3 the same or different substituents selected from the group consisting of halogen atom, hydroxy, and C₁₋₆ alkoxy,
(1-5) C₁₋₆ alkoxy optionally substituted with 1 to 3 the same or different substituents selected from the group consisting of halogen atom, hydroxy, and C₁₋₆ alkoxy, or
(1-6) amino-carbonyl optionally substituted with C₁₋₆ alkyl which may be optionally substituted with 1 to 3 the same or different halogen atoms;
   wherein R^{a} and R^{b} may be combined together with the carbon atom(s) to which they attach to form
(2-1) a 3- to 6-membered saturated carbocycle, wherein the carbocycle may be optionally substituted with 1 to 4 the same or different substituents selected from the group consisting of:
   (a) halogen atom,
   (b) hydroxy,
   (c) C₁₋₆ alkyl optionally substituted with 1 to 3 the same or different substituents selected from the group consisting of halogen atom, hydroxy, and C₁₋₆ alkoxy, and
   (d) C₁₋₆ alkoxy optionally substituted with 1 to 3 the same or different substituents selected from the group consisting of halogen atom, hydroxy, and C₁₋₆ alkoxy, or
(2-2) a 3- to 6-membered saturated heterocycle, wherein the heterocycle may be optionally substituted with 1 to 4 the same or different substituents selected from the group consisting of (a) to (d) of the above (2-1) in the present clause.

Preferably, M² includes the following groups:
(1) a group of any one of the following formulae (11) to (37): 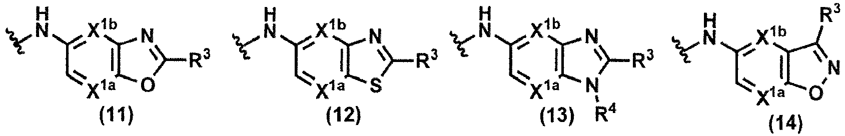 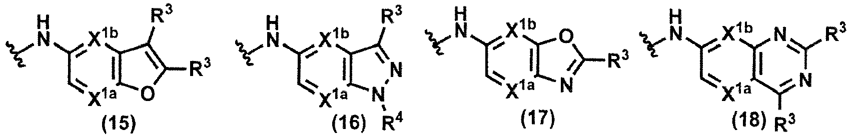 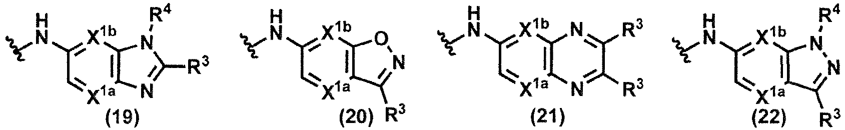 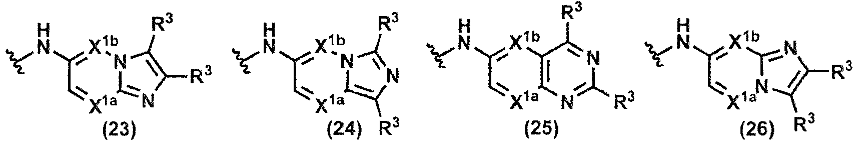 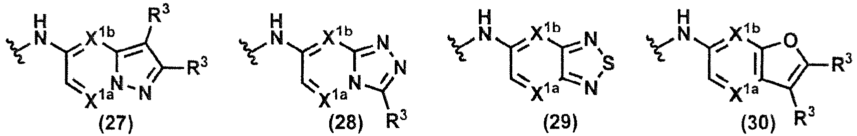 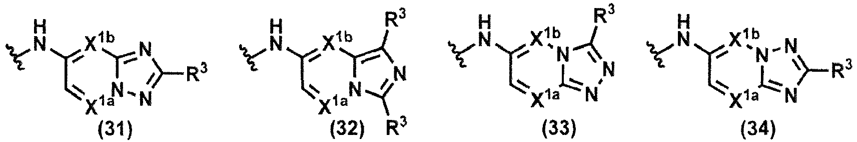 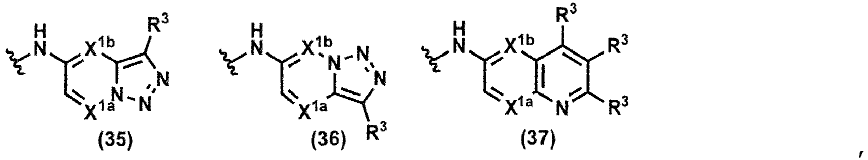 wherein X^{1a} and X^{1b} are each independently N or CR³;
   R⁴ is
   (a) hydrogen atom,
   (b) C₁₋₆ alkyl optionally substituted with 1 to 3 the same or different substituents selected from the group consisting of halogen atom, hydroxy, and C₁₋₆ alkoxy, or
   (c) saturated or partially-unsaturated C₃₋₇ carbocyclyl optionally substituted with 1 to 4 the same or different substituents selected from the group consisting of halogen atom, hydroxy, C₁₋₆ alkyl, and C₁₋₆ alkoxy;
   R³ is
   (a) hydrogen atom,
   (b) halogen atom,
   (c) cyano,
   (d) hydroxy,
   (e) C₁₋₆ alkyl optionally substituted with 1 to 3 the same or different substituents selected from the group consisting of halogen atom, hydroxy, saturated or partially-unsaturated C₃₋₇ carbocyclyl, and C₁₋₆ alkoxy,
   (f) saturated or partially-unsaturated C₃₋₇ carbocyclyl optionally substituted with 1 to 4 the same or different substituents selected from the group consisting of halogen atom, hydroxy, C₁₋₆ alkyl, and C₁₋₆ alkoxy,
   (g) C₁₋₆ alkoxy optionally substituted with 1 to 3 the same or different substituents selected from the group consisting of halogen atom, hydroxy, and C₁₋₆ alkoxy,
   (h) amino optionally substituted with 1 to 2 the same or different substituents selected from the group consisting of C₁₋₆ alkyl optionally substituted with 1 to 3 the same or different halogen atoms; saturated or partially-unsaturated C₃₋₇ carbocyclyl; and C₂₋₇ alkylcarbonyl,
   (i) 5- to 6-membered heteroaryl optionally substituted with 1 to 4 the same or different substituents selected from the group consisting of halogen atom, cyano, and C₁₋₆ alkyl,
   (j) 5- to 6-membered saturated heterocyclyl optionally substituted with 1 to 4 the same or different substituents selected from the group consisting of halogen atom, hydroxy, C₁₋₆ alkyl, and C₁₋₆ alkoxy, or
   (k) -C(O)NR^{x}R^{y}, wherein R^{x} and R^{y} are each independently hydrogen atom, C₁₋₆ alkyl, or saturated or partially-unsaturated C₃₋₇ carbocyclyl; or
   alternatively, R^{x} and R^{y} may be combined together with the nitrogen atom to which they attach to form 4- to 7-membered saturated heterocyclyl,
   provided that when R³ exist plurally, each R³ may be the same or different with each other,
(2) 4-cyanophenylamino,
(3) a group of the following formula (2c''): 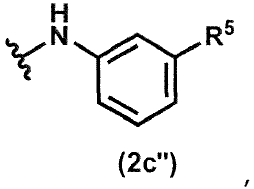 wherein R⁵ is 5-membered heteroaryl optionally substituted with 1 to 2 the same or different substituents selected from the group consisting of halogen atom and C₁₋₆ alkyl,
(4) a group of the following formula (2c'''): 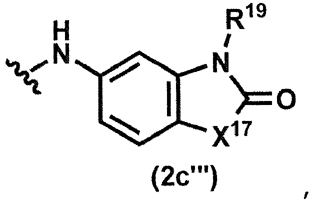 wherein X¹⁷ is O or CH₂; and
   R¹⁹ is hydrogen atom or C₁₋₆ alkyl, or
(5) a group of the following formula (2d), (2e), or (2j') : 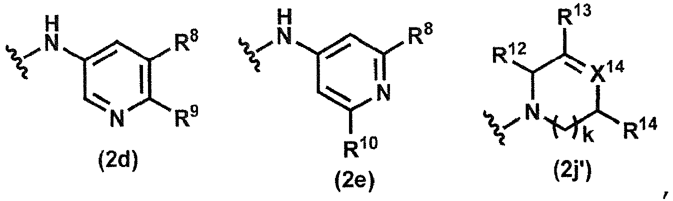 wherein R⁸, R⁹, and R¹⁰ are each independently
   (a) hydrogen atom,
   (b) halogen atom,
   (c) cyano,
   (d) C₁₋₆ alkyl optionally substituted with 1 to 3 the same or different halogen atoms,
   (e) C₁₋₆ alkoxy optionally substituted with 1 to 3 the same or different halogen atoms,
   (f) amino optionally substituted with 1 to 2 the same or different C₁₋₆ alkyl,
   (g) 6-membered saturated heterocyclyl, or
   (h) 5- or 6-membered heteroaryl optionally substituted with 1 to 4 the same or different substituents selected from the group consisting of halogen atom and C₁₋₆ alkyl;
   X¹⁴ is CR²⁰;
   R¹², R¹³, and R¹⁴ are each independently
   (a) hydrogen atom, or
   (b) methyl;
   R²⁰ is
   (a) phenyl optionally substituted with 1 to 2 the same or different substituents selected from the group consisting of fluorine atom and methoxy, or
   (b) 5- or 6-membered heteroaryl optionally substituted with 1 to 2 the same or different substituents selected from the group consisting of methyl, methoxy, fluorine atom, trifluoromethyl, and difluoromethoxy.

More preferably, M² is a group of the following formula (2a') or (2b'): 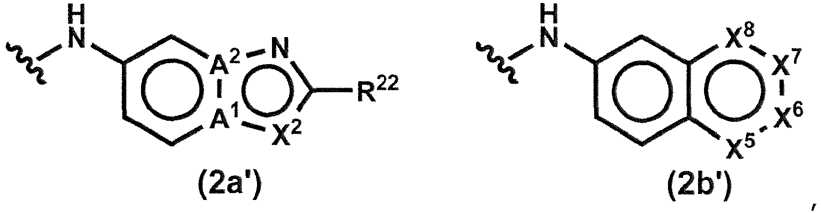 wherein X², X⁵, X⁶, X⁷, and X⁸ are each independently N, CR²¹, or O,
A¹ and A² are each independently N or C,
wherein X², X⁵, X⁶, X⁷, X⁸, A¹, and A² are selected such that a ring comprising them forms a 9- or 10-membered bicyclic aromatic heterocycle; and
R²¹ and R²² are each independently
(1) hydrogen atom,
(2) halogen atom,
(3) cyano,
(4) C₁₋₆ alkyl optionally substituted with 1 to 3 the same or different substituents selected from the group consisting of halogen atom, hydroxy, saturated or partially-unsaturated C₃₋₇ carbocyclyl, and C₁₋₆ alkoxy,
(5) saturated or partially-unsaturated C₃₋₇ carbocyclyl optionally substituted with 1 to 4 the same or different substituents selected from the group consisting of halogen atom, hydroxy, C₁₋₆ alkyl, and C₁₋₆ alkoxy,
(6) C₁₋₆ alkoxy optionally substituted with 1 to 3 the same or different substituents selected from the group consisting of halogen atom, hydroxy, and C₁₋₆ alkoxy, or
(7) amino optionally substituted with 1 to 2 the same or different substituents selected from the group consisting of C₁₋₆ alkyl optionally substituted with 1 to 3 the same or different halogen atoms; saturated or partially-unsaturated C₃₋₇ carbocyclyl; and C₂₋₇ alkylcarbonyl.

One embodiment of M² includes a group of any one of the following formulae (11) to (37): 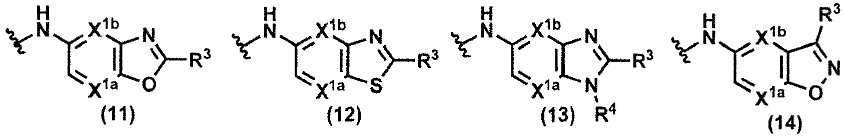 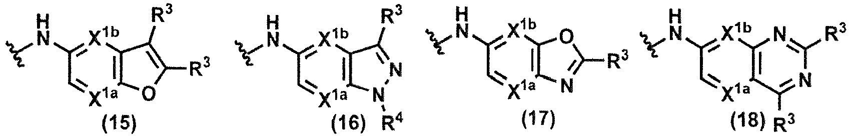 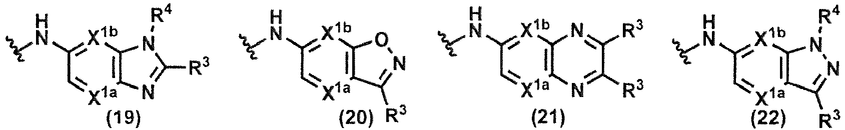 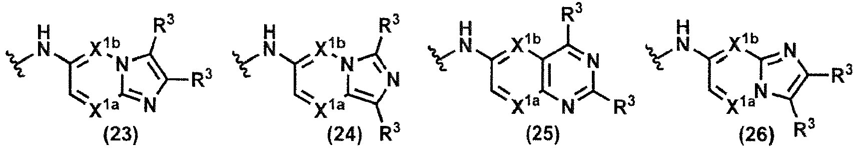 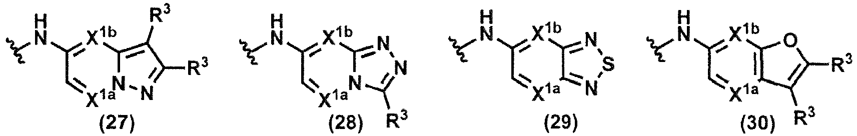 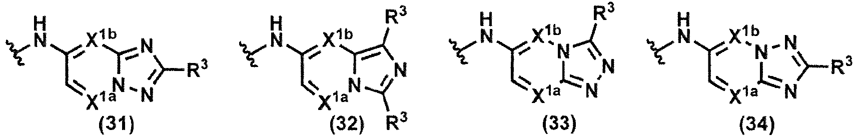 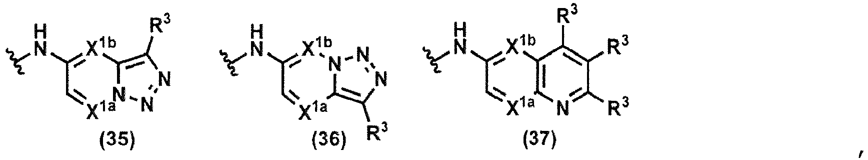 wherein X^{1a} and X^{1b} are each independently N or CR³;
R⁴ is
(a) hydrogen atom,
(b) C₁₋₆ alkyl optionally substituted with 1 to 3 the same or different substituents selected from the group consisting of halogen atom, hydroxy, and C₁₋₆ alkoxy, or
(c) saturated or partially-unsaturated C₃₋₇ carbocyclyl optionally substituted with 1 to 4 the same or different substituents selected from the group consisting of halogen atom, hydroxy, C₁₋₆ alkyl, and C₁₋₆ alkoxy;
R³ is
(a) hydrogen atom,
(b) halogen atom,
(c) cyano,
(d) hydroxy,
(e) C₁₋₆ alkyl optionally substituted with 1 to 3 the same or different substituents selected from the group consisting of halogen atom, hydroxy, saturated or partially-unsaturated C₃₋₇ carbocyclyl, and C₁₋₆ alkoxy,
(f) saturated or partially-unsaturated C₃₋₇ carbocyclyl optionally substituted with 1 to 4 the same or different substituents selected from the group consisting of halogen atom, hydroxy, C₁₋₆ alkyl, and C₁₋₆ alkoxy,
(g) C₁₋₆ alkoxy optionally substituted with 1 to 3 the same or different substituents selected from the group consisting of halogen atom, hydroxy, and C₁₋₆ alkoxy,
(h) amino optionally substituted with 1 to 2 the same or different substituents selected from the group consisting of C₁₋₆ alkyl optionally substituted with 1 to 3 the same or different halogen atoms; saturated or partially-unsaturated C₃₋₇ carbocyclyl; and C₂₋₇ alkylcarbonyl,
(i) 5- or 6-membered heteroaryl optionally substituted with 1 to 4 the same or different substituents selected from the group consisting of halogen atom, cyano, and C₁₋₆ alkyl,
(j) 5- or 6-membered saturated heterocyclyl optionally substituted with 1 to 4 the same or different substituents selected from the group consisting of halogen atom, hydroxy, C₁₋₆ alkyl, and C₁₋₆ alkoxy, or
(k) -C(O)NR^{x}R^{y}, wherein R^{x} and R^{y} are each independently hydrogen atom, C₁₋₆ alkyl, or saturated or partially-unsaturated C₃₋₇ carbocyclyl; or
alternatively, R^{x} and R^{y} may be combined together with the nitrogen atom to which they attach to form 4- to 7-membered saturated heterocyclyl,
provided that when R³ exist plurally, each R³ may be the same or different with each other.

Another embodiment of M² is 4-cyanophenylamino.

Another embodiment of M² includes a group of the following formula (2h'): 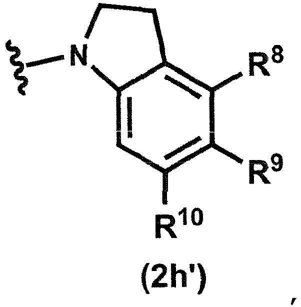 wherein R⁸, R⁹, and R¹⁰ are each independently
(a) hydrogen atom,
(b) halogen atom,
(c) cyano,
(d) C₁₋₆ alkyl optionally substituted with 1 to 3 the same or different substituents selected from the group consisting of halogen atom; hydroxy; C₁₋₆ alkoxy optionally substituted with hydroxy or C₁₋₆ alkoxy; 4- to 7-membered saturated or partially-unsaturated heterocyclyl optionally substituted with C₁₋₆ alkyl or C₁₋₆ alkoxy; 5- or 6-membered heteroaryl optionally substituted with C₁₋₆ alkyl; and amino, wherein the amino may be optionally substituted with 1 to 2 the same or different substituents selected from the group consisting of C₁₋₆ alkyl optionally substituted with 1 to 3 the same or different substituents selected from the group consisting of halogen atom, hydroxy, and C₁₋₆ alkoxy; saturated or partially-unsaturated C₃₋₇ carbocyclyl; 4- to 7-membered saturated heterocyclyl optionally substituted with C₁₋₆ alkoxy; and C₂₋₇ alkylcarbonyl optionally substituted with 1 to 3 the same or different substituents selected from the group consisting of halogen atom, hydroxy, and C₁₋₆ alkoxy,
(e) C₁₋₆ alkoxy optionally substituted with 1 to 3 the same or different substituents selected from the group consisting of halogen atom, hydroxy, and C₁₋₆ alkoxy,
(f) amino optionally substituted with 1 to 2 the same or different substituents selected from the group consisting of C₁₋₆ alkyl optionally substituted with halogen atom; saturated or partially-unsaturated C₃₋₇ carbocyclyl; and C₂₋₇ alkylcarbonyl optionally substituted with 1 to 3 the same or different substituents selected from the group consisting of halogen atom, hydroxy, and C₁₋₆ alkoxy,
(g) 5- or 6-membered heteroaryl optionally substituted with 1 to 4 the same or different substituents selected from the group consisting of halogen atom, cyano, C₁₋₆ alkyl, C₁₋₆ alkoxy, and C₂₋₇ alkoxycarbonyl,
(h) 4- to 7-membered saturated or partially-unsaturated heterocyclyl optionally substituted with 1 to 4 the same or different substituents selected from the group consisting of C₁₋₆ alkyl optionally substituted with 1 to 3 the same or different substituents selected from the group consisting of halogen atom, hydroxy, and C₁₋₆ alkoxy; C₁₋₆ alkoxy; 4- to 7-membered saturated or partially-unsaturated heterocyclyl; C₂₋₇ alkylcarbonyl optionally substituted with 1 to 3 the same or different substituents selected from the group consisting of halogen atom, hydroxy, and C₁₋₆ alkoxy; and oxo,
(i) 4- to 7-membered saturated or partially-unsaturated heterocyclyl-oxy optionally substituted with 1 to 4 C₁₋₆ alkyl,
(j) -C(O)NR^{x}R^{y}, wherein R^{x} and R^{y} are each independently hydrogen atom, C₁₋₆ alkyl, or saturated or partially-unsaturated C₃₋₇ carbocyclyl; or
   alternatively, R^{x} and R^{y} may be combined together with the nitrogen atom to which they attach to form 4- to 7-membered saturated heterocyclyl,
(k) -C(O)OR^{Z}, wherein R^{Z} is C₁₋₆ alkyl, or
(l) ethenyl optionally substituted with one 6-membered saturated heterocyclyl group;
wherein R⁸ and R⁹ may be combined together with the carbon atoms to which they attach to form a 5- to 7-membered saturated or partially-unsaturated carbocycle or heterocycle, wherein the carbocycle and heterocycle may be optionally substituted with 1 to 4 the same or different substituents selected from the group consisting of halogen atom and C₁₋₆ alkyl.

Examples of the term "pharmaceutically acceptable salt" include inorganic acid salts such as hydrochloride, hydrobromide, sulfate, phosphate, and nitrate; and organic acid salts such as acetate, propionate, oxalate, succinate, lactate, malate, tartrate, citrate, maleate, fumarate, methanesulfonate, p-toluenesulfonate, benzenesulfonate, and ascorbate.

The present compound encompasses any crystalline forms thereof.

A compound of Formula (1) may have at least one asymmetric carbon atom. Thus, the present compound encompasses racemates of a compound of Formula (1), as well as optical isomers thereof. A compound of Formula (1) encompasses deuterated compounds in which any one or more ¹H in the compound are replaced with ²H (D).

Hereinafter, methods for preparing a compound of Formula (1) in the present invention are exemplified, but the present invention is not limited to such examples.

### Preparation Process

The present compound may be prepared by the following processes and methods which are combined with common synthetic methods.

Compounds in reaction schemes include those in the salt form, and such salts include, for example, those described in the above "pharmaceutically acceptable salt". Note that these reactions are merely illustrative, and other methods may be optionally applied for preparing the present compound based on the knowledge of a person skilled in synthetic organic chemistry.

In each of the preparation process described below, when there is a functional group that needs protection, the functional group may be protected as necessary and deprotected after the completion of a reaction or a series of reactions to afford a targeted product, even if the use of the protective group is not specifically indicated.

As protective groups herein, common protective groups, for example those described in literatures (T. W. Greene and P. G. M. Wuts, "Protective Groups in Organic Synthesis", 3rd Ed., John Wiley and Sons, inc., New York (1999) etc.), may be used. More specifically, protective groups of an amino group include, for example, tert-butoxycarbonyl, benzyloxycarbonyl, p-toluenesulfonyl, o-nitrobenzenesulfonyl, and tetrahydropyranyl. Protective groups of a hydroxy group include, for example, trialkylsilyl, acetyl, benzyl, tetrahydropyranyl, and methoxymethyl. Protective groups of an aldehyde group include, for example, dialkylacetal and cyclic alkylacetal. Protective groups of a carboxyl group include, for example, tert-butyl ester, orthoester, and amide.

The introduction and deprotection of protective groups can be performed by methods commonly used in organic synthetic chemistry (for example, methods described in T. W. Greene and P. G. M. Wuts, "Protective Groups in Organic Synthesis", 3rd Ed., John Wiley and Sons, inc., New York (1999) etc.) or corresponding methods thereof.

A compound of Formula (1) is prepared by forming bonds at the positions of a, b, and c: 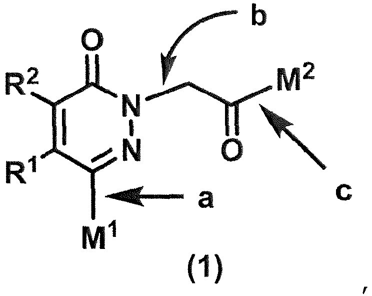 wherein M¹, M², R¹, and R² are the same as those defined in the above [1].

Processes for forming bonds at the positions a, b, and c are illustrated in the following Preparation Processes 1 to 5, but the sequence of forming bonds may be optionally modified.

### Preparation Process 1

A compound of Formula (1) can be prepared, for example, by the following process: 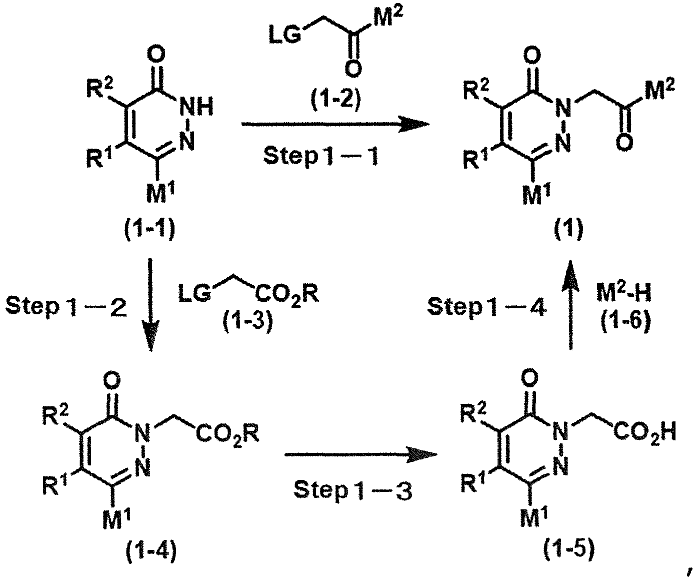 wherein M¹, M², R¹, and R² are the same as those defined in the above [1]; R is C₁₋₆ alkyl; and LG is a leaving group (for example, iodine atom, bromine atom, chlorine atom, and substituted sulfonyl (such as methanesulfonyl and p-toluenesulfonyl)).

### Step 1-1: Preparation step of Compound (1)

A compound of Formula (1) is prepared by reacting Compound (1-1) with Compound (1-2) in the presence of a base in an appropriate inert solvent. For Compound (1-1), a product synthesized in Preparation Process 4 or 5 described below, or a commercial product may be used. For Compound (1-2), a commercial product or a product synthesized by common methods or corresponding methods thereof may be used.

Examples of the base herein include inorganic bases such as potassium carbonate, sodium carbonate, cesium carbonate, potassium bicarbonate, sodium bicarbonate, dipotassium phosphate, potassium phosphate, disodium phosphate, sodium phosphate, potassium hydroxide, sodium hydroxide, and sodium hydride; metal alkoxides such as sodium methoxide and potassium tert-butoxide; and organic bases such as triethylamine, diisopropylethylamine, and pyridine.

Examples of the inert solvent herein include aprotic polar solvents such as dimethylformamide, N-methyl-2-pyrrolidinone, dimethylsulfoxide, acetonitrile, acetone, and methyl ethyl ketone; ether solvents such as diethyl ether, tetrahydrofuran, and 1,4-dioxane; halogenated hydrocarbon solvents such as chloroform and dichloromethane; aromatic hydrocarbon solvents such as benzene and toluene; and mixed solvents thereof.

The reaction temperature herein is selected from, but not limited to, usually the range of -10°C to 200°C, preferably the range of 0°C to 40°C. The reaction time herein is usually 10 minutes to 48 hours, but it depends on conditions including reaction temperature, materials, and solvents which are used.

### Step 1-2: Preparation step of Compound (1-4)

Compound (1-4) is prepared by reacting Compound (1-1) with Compound (1-3) according to the method described in Step 1-1. For Compound (1-3), a commercial product, or a product synthesized by common methods or corresponding methods thereof may be used.

### Step 1-3: Preparation step of Compound (1-5)

Compound (1-5) is prepared by hydrolyzing Compound (1-4) by common methods (for example, Protective Groups in Organic Synthesis 3rd Edition (John Wiley & Sons, Inc.), Comprehensive Organic Transformation, R. C. Laroque et al, VCH publisher Inc., 1989 etc.) or corresponding methods thereof.

### Step 1-4: Preparation step of Compound (1)

A compound of formula (1) is also prepared by reacting Compound (1-5) with Compound (1-6) in the presence or absence of a base in an appropriate inert solvent using a condensing agent. For Compound (1-6), a commercial product, or a product synthesized by common methods or corresponding methods thereof may be used.

Examples of the condensing agent include dicyclohexylcarbodiimide (DCC), diisopropylcarbodiimide (DIPC), 1-ethyl-3-(3-dimethylaminopropyl)-carbodiimide (WSC), benzotriazol-1-yl-tris(dimethylamino)phosphonium hexafluorophosphate (BOP), diphenylphosphoryl azide (DPPA), N,N-carbonyldiimidazole (CDI), benzotriazol-1-yl-N,N,N',N'-tetramethyluronium hexafluorophosphate (HBTU), and 7-azabenzotriazol-1-yl-N,N,N',N'-tetramethyluronium hexafluorophosphate (HATU). If necessary, additives such as N-hydroxysuccinimide (HOSu), 1-hydroxybenzotriazole (HOBt), and 3-hydroxy-4-oxo-3,4-dihydro-1,2,3-benzotriazine (HOOBt) may be added to the reaction.

Examples of the base herein include organic bases such as triethylamine, diisopropylethylamine, and pyridine; inorganic bases such as potassium carbonate, sodium carbonate, cesium carbonate, potassium bicarbonate, sodium bicarbonate, dipotassium phosphate, potassium phosphate, disodium phosphate, sodium phosphate, potassium hydroxide, sodium hydroxide, and sodium hydride; and metal alkoxides such as sodium methoxide and potassium tert-butoxide.

Examples of the inert solvent herein include aprotic polar solvents such as dimethylformamide, N-methyl-2-pyrrolidinone, dimethylsulfoxide, acetonitrile, acetone, and methyl ethyl ketone; ether solvents such as diethyl ether, tetrahydrofuran, and 1,4-dioxane; halogenated hydrocarbon solvents such as chloroform and dichloromethane; aromatic hydrocarbon solvents such as benzene and toluene; and mixed solvents thereof.

The reaction temperature herein is selected from, but not limited to, usually the range from -10°C to 200°C, preferably the range from 0°C to 40°C. Reaction time is usually 10 minutes to 48 hours, but it depends on conditions including reaction temperature, materials, and solvents which are used.

The present step can also be proceeded, for example, by activating a carbonyl group with acid anhydride, mixed acid anhydride, or acid halide, and then reacting with Compound (1-6) .

### Preparation Process 2

Among compounds of Formula (1), a compound of formula (2-4) is prepared, for example, by the following process: 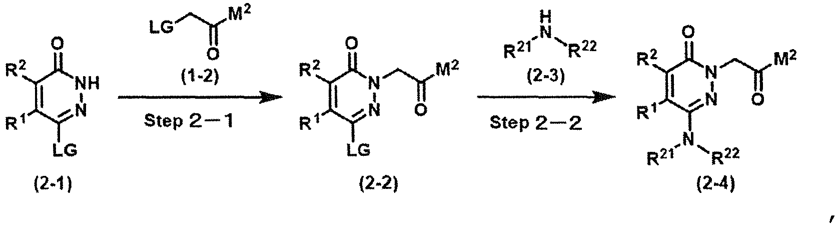 wherein R¹, R², and M² are the same as those defined in the above [1]; LG is a leaving group (for example, iodine atom, bromine atom, chlorine atom, and substituted sulfonyl (such as methanesulfonyl and p-toluenesulfonyl)); R²¹ and R²² are each independently optionally-substituted C₁₋₆ alkyl; or alternatively, R²¹ and R²² may be combined together with the nitrogen atom to which they attach to form an optionally-substituted 4- to 12-membered saturated heterocycle.

### Step 2-1: Preparation step of Compound (2-2)

Compound (2-2) is prepared from Compound (2-1) and Compound (1-2) according to the method described in Step 1-1. For Compound (2-1), a product synthesized by common methods (for example, those described in Tetrahedron, 2015, 71, 4859, Bioorganic & Medicinal Chemistry Letters, 2015, 25, 1030, etc.) or corresponding methods thereof, or a commercial product may be used.

### Step 2-2: Preparation step of Compound (2-4)

Compound (2-4) can be prepared by reacting Compound (2-2) with Compound (2-3) in the presence of a base in an appropriate inert solvent. For Compound (2-3), a commercial product, or a product synthesized by common methods or corresponding methods thereof may be used.

Examples of the base herein include organic bases such as triethylamine, diisopropylethylamine, and pyridine; inorganic bases such as potassium carbonate, sodium carbonate, cesium carbonate, potassium bicarbonate, sodium bicarbonate, dipotassium hydrogen phosphate, potassium phosphate, disodium hydrogen phosphate, sodium phosphate, potassium hydroxide, sodium hydroxide, and sodium hydride; and metal alkoxides such as sodium methoxide and potassium tert-butoxide.

Examples of the inert solvent herein include aprotic polar solvents such as dimethylformamide, N-methyl-2-pyrrolidinone, dimethylsulfoxide, acetonitrile, acetone, and methyl ethyl ketone; halogenated hydrocarbon solvents such as chloroform and dichloromethane; aromatic hydrocarbon solvents such as benzene and toluene; and mixed solvents thereof.

The reaction temperature herein is selected from, but not limited to, usually the range of 20°C to 200°C, preferably the range of 50°C to 170°C. The present step may be conducted under microwave irradiation, if necessary. The reaction time herein is usually 10 minutes to 48 hours, but it depends on conditions including reaction temperature, materials, and solvents which are used.

### Preparation Process 3

Among compounds of Formula (1), compounds of formulae (3-2) and (3-3) are prepared, for example, by the following process: 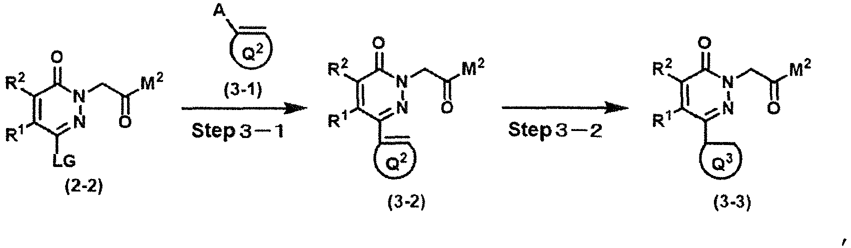 wherein R¹, R², and M² are the same as those described in the above [1]; LG is a leaving group (for example, iodine atom, bromine atom, chlorine atom, and substituted sulfonyl (such as methanesulfonyl and p-toluenesulfonyl)); A is boronate, boronate ester, BF₃K, or BF₃Na; Q² is optionally-substituted 4- to 12-membered partially-unsaturated heterocyclyl or saturated or partially-unsaturated C₄₋₁₂ carbocyclyl; and Q³ is optionally-substituted saturated or partially-unsaturated C₄₋₁₂ carbocyclyl, or optionally-substituted 4- to 12-membered saturated heterocyclyl.

### Step 3-1: Preparation step of Compound (3-2)

Compound (3-2) is prepared by reacting Compound (2-2) with Compound (3-1) in the presence of a palladium catalyst, a phosphine ligand, and a base in an appropriate inert solvent. For Compound (3-1), a commercial product, or a product synthesized by common methods or corresponding methods may be used.

Examples of the palladium catalyst herein include tetrakis(triphenylphosphine)palladium (0), bis(dibenzylideneacetone)palladium (0), tris(dibenzylideneacetone)dipalladium (0), bis(tri-tert-butylphosphine)palladium (0), palladium (0) acetate, [1,1-bis(diphenylphosphino)ferrocene]palladium (II) dichloride, bis(di-tert-butyl(4-dimethylaminophenyl)phosphine)dichloropalladium (II).

Phosphine ligands include, for example, o-tolylphosphine, 2-dicyclohexylphosphino-2',6'-dimethoxybiphenyl (S-Phos), 2-(dicyclohexylphosphino)-2',4',6'-triisopropylbiphenyl (X-Phos), 1,1'-bis(diphenylphosphino)ferrocene (DPPF), 1,2-bis(diphenylphosphino)ethane (DPPE), 1,3-bis(diphenylphosphino)propane (DPPP), 1,4-bis(diphenylphosphino)butane (DPPB), 2,2'-bis(diphenylphosphino)-1,1'-binaphthyl (BINAP), 4,5-bis(diphenylphosphino)-9,9-dimethylxanthene (XANT-Phos), and bis(2-(diphenylphosphino)phenyl) ether (DPE-Phos).

Examples of the base herein include sodium carbonate, potassium carbonate, cesium carbonate, potassium phosphate, sodium hydroxide, and potassium hydroxide.

Examples of the inert solvent herein include 1,4-dioxane, THF, 1,2-dimethoxyethane, water, and mixed solvents thereof.

The reaction temperature herein is selected from, but not limited to, usually the range of 50°C to 200°C, preferably the range of 80°C to 150°C. The present step can be conducted under microwave irradiation, if necessary. Reaction time is usually 30 minutes to 48 hours.

### Step 3-2: Preparation step of Compound (3-3)

Compound (3-3) is prepared by catalytic reduction of Compound (3-2) with a metal catalyst in an appropriate inert solvent under hydrogen atmosphere.

Examples of the metal catalyst herein include palladium/carbon, palladium hydroxide/carbon, Raney nickel, platinum oxide/carbon, and rhodium/carbon. The amount of a metal catalyst is usually 0.1% to 1000% by weight to Compound (3-2), and preferably 1% to 100% by weight.

Examples of the inert solvent herein include ethers such as tetrahydrofuran; and esters such as ethyl acetate.

The hydrogen pressure herein is usually 1 to 100 atm, and preferably 1 to 5 atm.

The reaction temperature herein is selected from, but not limited to, usually the range of 0°C to 120°C, preferably the range of 20°C to 80°C. Reaction time is usually 30 minutes to 72 hours.

### Preparation Process 4

Among compounds of formula (1-1), a compound of formula (4-3) is prepared, for example, by the following process: 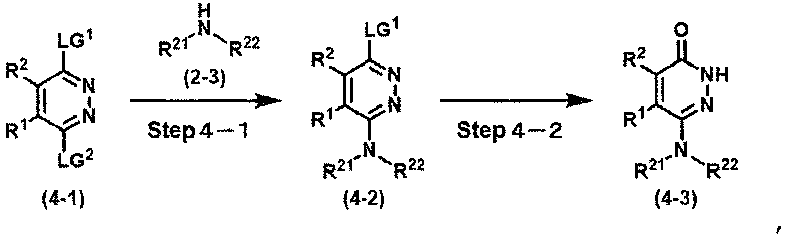 wherein R¹ and R² are the same as those defined in the above [1]; LG¹ and LG² are each independently a leaving group (for example, iodine atom, bromine atom, chlorine atom, and substituted sulfonyl (such as methanesulfonyl and p-toluenesulfonyl)); R²¹ and R²² are each independently optionally-substituted C₁₋₆ alkyl, optionally-substituted C₃₋₁₀ cycloalkyl, or optionally-substituted C₃₋₁₀ cycloalkyl-C₁₋₄ alkyl; or alternatively, R²¹ and R²² may be combined together with the nitrogen atom to which they attach to form an optionally-substituted 4- to 12-membered saturated heterocycle.

### Step 4-1: Preparation step of Compound (4-2)

Compound (4-2) is prepared from Compound (4-1) and Compound (2-3) according to the method described in Step 2-2. As Compound (4-1) and Compound (2-3), a commercial product, or a product synthesized by common methods (for example, WO 2004/006922, ACS Medicinal Chemistry Letters, 2012, 3, 903. etc.) or corresponding methods thereof may be used. The amount of Compound (2-3) used herein is usually 1.0 equivalent to 1.5 equivalent, and preferably 1.05 equivalent to 1.2 equivalent, to the amount of Compound (4-2) .

### Step 4-2: Preparation step of Compound (4-3)

Compound (4-3) is prepared from Compound (4-2) according to common methods (for example, Bioorganic & Medicinal Chemistry Letters, 2013, 23, 2007., WO 2012/114268, etc.) or corresponding methods thereof.

### Preparation Process 5

Among compounds of formula (1-1), a compound of formula (5-4) is prepared, for example, by the following process: 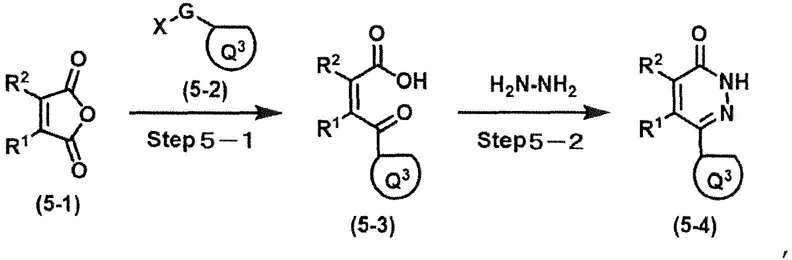 wherein R¹ and R² are the same as those defined in the above [1]; Q³ is optionally-substituted saturated or partially-unsaturated C₄₋₁₂ carbocyclyl, or optionally-substituted 4-to 12-membered saturated heterocyclyl; G is a metal species such as magnesium and zinc; and X is a halogen atom.

### Step 5-1: Preparation step of Compound (5-3)

Compound (5-3) is prepared by reacting Compound (5-1) with Organometallic Compound (5-2) such as a Grignard reagent according to common methods (for example, Organic Letters, 2015, 17, 5517., Organic & Biomolecular Chemistry, 2014, 12, 2049., etc.) or corresponding methods thereof. As Compound (5-1) and Compound (5-2), commercial products, or products synthesized by common methods (for example, Organic Letters, 2008, 10, 4815., Journal of Organic Chemistry, 2015, 80, 12182., etc.) or corresponding methods thereof may be used.

### Step 5-2: Preparation step of Compound (5-4)

Compound (5-4) is prepared by reacting Compound (5-2) with hydrazine according to common methods (for example, Journal of Medicinal Chemistry, 1993, 36, 4052., WO 2007/020343, etc.) or corresponding methods thereof.

The above preparation processes can be optionally combined to provide the present compound which has desired substituents at desired positions. Isolation and purification of intermediates or products in the above preparation processes can be carried out by optional combination of methods which are commonly used in organic synthetic chemistry, such as filtration, extraction, washing, drying, concentration, crystallization, and various types of chromatography. Intermediates can also be subjected to a subsequent reaction without any particular purification.

Some material compounds or intermediates in the above preparation processes may exist in a salt form such as hydrochloride according to, for example, reaction conditions, and they can be used as they are or in a free form. When material compounds or intermediates are obtained in a salt form and the material compounds or intermediates need to be used or obtained in a free form, they can be converted into a free form by dissolving or suspending them in an appropriate solvent, followed by neutralization with a base such as a sodium bicarbonate solution.

Some compounds of Formula (1) or pharmaceutically acceptable salts thereof may have isomers including tautomers such as keto-enol forms, regioisomers, geometric isomers, or optical isomers. All possible isomers including them, and mixtures of such isomers in any ratio, are also encompassed in the present invention.

The optical isomers can also be separated by common separation processes such as methods using an optically active column or fractional crystallization at an appropriate step of the above preparation processes. An optically active material can be used as a starting material.

In the case where a salt of a compound of Formula (1) is needed, when the compound of Formula (1) is obtained in a salt form, the salt can be obtained by purification of the obtained salt, and when the compound of Formula (1) is obtained in a free form, the salt can be formed by dissolving or suspending the compound of Formula (1) in an appropriate solvent, followed by addition of an acid or base. Compound (1) or a pharmaceutically acceptable salt thereof may exist in a form of hydrate or solvate (e.g., ethanolate) with various types of solvents such as water and ethanol, and such hydrates and solvents are also encompassed in the present invention.

The present compound may be useful as a medicament for activating Navl.1 because it exhibits the activation effect of Nav1.1.

The present compound have the activation effect of Nav1.1 and thus, may be useful as a medicament for treating and/or preventing diseases involving Nav1.1, especially diseases involving reduced function of Nav1.1, for example as a medicament for treating and/or preventing central nervous system diseases (for example, febrile seizure; generalized epilepsy with febrile seizure plus; epilepsy (specifically, focal epilepsy, generalized epilepsy); epileptic syndrome (such as Dravet syndrome, intractable childhood epilepsy with generalized tonic-clonic seizure, epilepsy with myoclonic-atonic seizure, West syndrome, Lennox-Gastaut syndrome, infantile spasms, sever infantile multifocal epilepsy, severe myoclonic epilepsy, borderline; and benign familial neonatal-infantile seizure); schizophrenia; autism spectrum disorder; and attention deficit hyperactivity disorder).

In addition, the present compound is expected as a medicament for treating and/or preventing the above epileptic syndrome or epilepsy (especially, intractable epilepsy) wherein symptoms cannot be adequately suppressed with multiple drugs, especially three or more existing antiepileptic agents.

One embodiment of the present invention has a selective pharmacological activity especially to Nav1.1, and less to other subtypes of Nav, such as Nav1.5, and thus, the possibility of cardiotoxicity is expected to be reduced to provide high safety.

The term "preventing" used herein refers to the act of administering the present compound to a healthy person who has not developed a disease, and is intended, for example, to prevent the onset of a disease. The term "treating" used herein refers to the act of administering the present compound to a person, i.e., a patient, who has been diagnosed by a doctor as being affected with a disease.

The present compound may be administered directly via an appropriate route of administration, or administered in an appropriate dosage form after formulation.

As a route of administration, it is preferable to use the most effective route for treatment, and examples of the route of administration include oral; and parenteral administration such as intravenous administration, application, inhalation, and eye drop. The route of administration is preferably oral administration.

Examples of the dosage form herein include a tablet, a capsule, a powder, a granule, a liquid, a suspension, an injection, a patch, and a poultice. The dosage form is preferably a tablet.

Formulation into a dosage form or a pharmaceutical composition can be carried out according to common methods using pharmaceutically acceptable additives.

As a pharmaceutically acceptable additive, an excipient, a disintegrant, a binder, a fluidizer, a lubricant, a coating, a solubilizer, a solubilizing adjuvant, a thickener, a dispersant, a stabilizing agent, a sweetening agent, a flavor, and the like can be used, depending on a purpose. Specifically, examples of the pharmaceutically acceptable additive herein include lactose, mannitol, crystalline cellulose, low-substituted hydroxypropylcellulose, corn starch, partially-pregelatinized starch, carmellose calcium, croscarmellose sodium, hydroxypropylcellulose, hydroxypropylmethylcellulose, polyvinyl alcohol, magnesium stearate, sodium stearyl fumarate, polyethylene glycol, propylene glycol, titanium oxide, and talc.

The amount and the frequency of administration of these dosage forms or pharmaceutical compositions can be optionally determined depending on the mode of administration, a disease of a patient or symptoms thereof, the age or weight of a patient, and the like. The amount of an active ingredient (herein, also referred to as "therapeutically effective amount") per day can usually be administered to an adult in several portions in a day, preferably in one to three portions in a day, wherein the amount ranges from about 0.0001 to about 5000 mg, more preferably from about 0.001 to about 1000 mg, further preferably from about 0.1 to about 500 mg, especially preferably from about 1 to about 300 mg.

The present compound may be used in combination with another agent (hereinafter, also referred to as an "agent for combination use") in order to enhance the effect of the present compound and/or reduce side effects. Examples of such agent include antiepileptic agents, antipsychotic agents, antidepressant agents, mood-stabilizing agents, antianxiety agents, psychostimulant drugs, antiemetic agents, sleep-introducing agents, anticonvulsant agents, antiparkinsonian agents, antischizophrenic agents, and therapeutic agents for ADHD. Specifically, the present compound can also be combined with agents such as signal enhancing agents of GABA including valproic acid; positive allosteric modulators of GABAA receptors including clobazam; T-type voltage-dependent calcium channel inhibitors including ethosuximide; SV2A ligands including levetiracetam; medicaments of partial seizure including carbamazepine; calcium channel α2δ (alpha 2 delta) ligands including pregabalin; voltage-dependent potassium channel activators including retigabine; and AMPA receptor antagonist including perampanel. The present compound may also be used in combination with multi-acting receptor-targeted antipsychotic agents (MARTA) including clozapine; serotonin-dopamine antagonists (SDA) including risperidon; dopamine receptor partial agonists (DPA) including aripiprazole; selective serotonin reuptake inhibitors (SSRI) including fluvoxamine; serotonin noradrenaline reuptake inhibitors (SNRI) including duloxetine; noradrenergic and specific serotonergic antidepressant agents (NaSSA) including mirtazapine; mood-stabilizing agents including lithium carbonate; serotonin 1A receptor agonists including tandospirone; histamine H1-receptor antagonists including hydroxyzine; central nervous system stimulants including methylphenidate; and selective noradrenaline reuptake inhibitors including atomoxetine.

The timing to administer the present compound and an agent for combination use is not limited, and they may be administered to a subject of treatment concurrently or with a time lag. The present compound may be formulated as a combination medicament with an agent for combination use. The dose or mixing ratio of such agent can be optionally selected depending on a subject to be administered, a route of administration, a targeted disease, symptoms, and combination thereof, on the basis of the doses in the clinical use. For example, when the subject to be administered is a human, an agent for combination use may be used in 0.01 to 100 parts by weight to 1 part by weight of the present compound.

### EXAMPLES

The present invention is explained in more detail in the following by referring to Reference examples, Examples, and Tests; however, the present invention is not limited thereto. The names of compounds in the following Reference examples and Examples do not necessarily conform to the IUPAC nomenclature.

In the present specification, the abbreviations shown below may be used.
CDCl₃: deuterated chloroform
DMSO-d₆: deuterated dimethylsulfoxide
Rt: retention time
min: minute(s)
HATU: O-(7-aza-1H-benzotriazol-1-yl)-N,N,N',N'-tetramethyluronium hexafluorophosphate
THF: tetrahydrofuran
TFA: trifluoroacetic acid
DMF: N,N-dimethylformamide
Boc: tert-butoxycarbonyl

Physicochemical data of each compound in Examples and Reference examples were obtained with the following apparatus:
¹H-NMR: JEOL JNM-AL400; JEOL JNM-ECS400; Brucker AVANCE 400 Spectrometer

Symbols used in NMR are defined as follows: s for singlet, d for doublet, dd for doublet of doublets, t for triplet, td for triplet of doublets, q for quartet, m for multiplet, br for broad singlet or multiplet, and J for coupling constant.

LC/MS data of each compound in Examples and Reference examples were obtained with any one of the following apparatuses:

### Method A

Detection apparatus: ACQUITY (registered trademark) SQ deteceter (Waters Corporation)
HPLC: ACQUITY UPLC (registered trademark) SYSTEM
Column: Waters ACQUITY UPLC (registered trademark) BEH C18
(1.7 um, 2.1 mm x 30 mm)

### Method B

Detection apparatus: Shimadzu LCMS-2020
Column: Phenomenex Kinetex (C18, 1.7 um, 2.1 mm x 50 mm)

### Method C

Detection apparatus: Agilent 6110 Quadropole LC/MS
HPLC: Agilent 1200 series
Column: XBridge C18 (3.5 um, 4.6 mm x 50 mm)

### Method D

Detection apparatus: ACQUITY (registered trademark) SQ deteceter (Waters Corporation)
HPLC: ACQUITY UPLC (registered trademark) SYSTEM
Column: Waters ACQUITY UPLC (registered trademark) BEH C18 (1.7 um, 2.1 mm x 30 mm)

High performance liquid chromatograph-mass spectrometer; Measurement conditions for LC/MS are shown below, observed values of mass spectrometry [MS(m/z)] are shown in MH⁺, and retention times are shown in Rt (minutes). In each observed value, measurement conditions used for the measurement are described as any one of A to D.

### Method A

Solvents: Solution A; 0.06% formic acid/H₂O, Solution B; 0.06% formic acid/acetonitrile
Gradient condition: 0.0 to 1.3 minutes (linear gradient of B from 2% to 96%)
Flow rate: 0.8 mL/min; Detection UV: 220 nm and 254 nm; Temperature: 40°C

### Method B

Solvents: Solution A; 0.05% TFA/H₂O, Solution B; acetonitrile Gradient condition: 0.0 to 1.7 minutes (linear gradient of B from 10% to 99%)
Flow rate: 0.5 mL/min; Detection UV: 220 nm; Temperature: 40°C

### Method C

Solvents: Solution A; 10 mM NH₄HCO₃/H₂O, Solution B; acetonitrile
Gradient condition: 0.0 to 0.2 minutes (5% B), 0.2 to 1.5 minutes (linear gradient of B from 5% to 95%), 1.5 to 2.8 minutes (95% B)
Flow rate: 1.8 mL/min; Detection UV: 214 nm and 254 nm; Temperature 50°C

### Method D

Solvents: Solution A; 0.05% formic acid/H₂O, Solution B; acetonitrile
Gradient condition: 0.0 to 1.3 minutes (linear gradient of B from 10% to 95%), 1.3 to 1.5 minutes (10% B)
Flow rate: 0.8 mL/min; Detection UV: 220 nm and 254 nm; Temperature: 40°C

### Reference example 1

### 2-Chloro-N-(4-cyanophenyl)acetamide

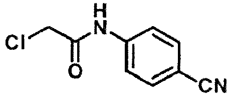

To a suspension of 4-aminobenzonitrile (25.2 g) and potassium carbonate (35.4 g) in acetone (200 ml) was added dropwise 2-chloroacetyl chloride (28.9 g) at 0°C, and the mixture was heated under reflux for 2 hours. After cooling to room temperature, the reaction mixture was poured slowly into water (400 ml), resulting in precipitation of a solid. The precipitated solid was filtered, washed with water, and dried under reduced pressure to obtain the titled compound (35.0 g).
¹H-NMR (400 MHz, DMSO-d₆) δ: 10.71 (s, 1H), 7.80 (d, J = 9.2 Hz, 2H), 7.76 (d, J = 9.2 Hz, 2H), 4.30 (s, 2H).

### Reference example 2

### 6-(4-Methylpiperidin-1-yl)pyridazin-3(2H)-one

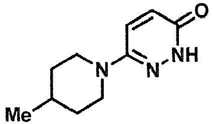

To a solution of 3,6-dichloropyridazine (34.3 g) in dimethylformamide (288 mL) were added 4-methylpiperidine (27.4 g) and triethylamine (48.1 mL). The reaction mixture was stirred at 80°C for 8 hours, and concentrated under reduced pressure. After the addition of saturated aqueous sodium bicarbonate (200 mL) and water (200 mL) thereto, the mixture was extracted twice with ethyl acetate. The obtained organic layers were combined, dried over sodium sulfate, and concentrated. The residue was dissolved in acetic acid (460 mL), and the mixture was heated under reflux for 37 hours. The reaction mixture was cooled to room temperature, and then solvent was concentrated under reduced pressure. To the residue were added 10% aqueous sodium hydroxide (300 mL) and diethyl ether (150 mL), resulting in precipitation of a solid. The precipitated solid was filtered, washed sequentially with water, diethyl ether, and ethyl acetate, and dried under reduced pressure to obtain the titled compound (31.6 g).
¹H-NMR (400 MHz, CDCl₃) δ: 7.17 (d, J = 10.5 Hz, 1H), 6.83 (d, J = 10.1 Hz, 1H), 3.73 (dt, J = 12.9, 2.4 Hz, 2H), 2.71 (td, J = 12.6, 2.6 Hz, 2H), 1.72-1.65 (m, 2H), 1.59-1.47 (m, 1H), 1.25 (dd, J = 12.3, 4.1 Hz, 1H), 1.19 (dd, J = 12.1, 4.3 Hz, 1H), 0.95 (d, J = 6.4 Hz, 3H).

### Reference example 3

### 2-(3-Chloro-6-oxopyridazin-1(6H)-yl)-N-(4-cyanophenyl)acetamide

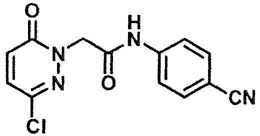

To a solution of the compound of Reference example 1 (16.7 g) in dimethylformamide (240 mL) were added potassium carbonate (23.7 g) and 6-chloropyridazin-3(2H)-one (14.8 g). After stirring at room temperature for 6 hours, water (360 mL) was added to the mixture, resulting in precipitation of a solid, and the precipitated solid was filtered. After washing with water, the solid was dried under reduced pressure to obtain the titled compound (18.4 g).
¹H-NMR (400 MHz, DMSO-d₆) δ: 10.78 (s, 1H), 7.79 (dt, J = 8.8, 2.1 Hz, 2H), 7.73 (dt, J = 9.0, 2.1 Hz, 2H), 7.64 (d, J = 9.6 Hz, 1H), 7.11 (d, J = 10.1 Hz, 1H), 4.90 (s, 2H).

### Reference example 4

### 2-(4,4-Difluorocyclohex-1-en-1-yl)-4,4,5,5-tetramethyl-1,3,2-dioxaborolane

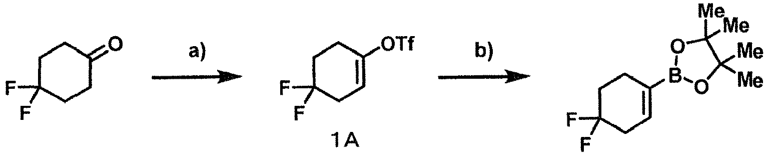

a) To a solution of 4,4-difluorocyclohexanone (25.0 g) in 1,2-dichloroethane (373 mL) were added 2-chloropyridine (26.5 g) and trifluoromethanesulfonic anhydride (63.1 g), and the mixture was stirred at 50°C for 6 hours. After cooling to 0°C, hexane (750 mL) was added thereto, resulting in precipitation of a solid, and the precipitated solid was filtered. The eluent was concentrated under reduced pressure to obtain Compound 1A (46.1 g).
   ¹H-NMR (400 MHz, CDCl₃) δ: 5.67-5.63 (m, 1H), 2.69 (td, J = 13.5, 2.9 Hz, 2H), 2.60-2.55 (m, 2H), 2.24-2.14 (m, 2H).
b) To a solution of Compound 1A (46.1 g) and bis(pinacolato)diboron (52.8 g) in 1,4-dioxane (577 mL) were added potassium acetate (42.5 g) and 1,1'-bis(diphenylphosphino)ferrocene palladium dichloride (6.34 g), and the mixture was heated under reflux for 2 hours. After cooling to room temperature, the mixture was filtered through Celite, and the eluent was concentrated under reduced pressure. After the addition of ethyl acetate (1.5 L), the organic layer was washed with water (300 mL) and brine (200 mL). The mixture was dried over anhydrous sodium sulfate, filtered, and concentrated under reduced pressure. The residue was purified by silica gel chromatography (solvent; hexane:ethyl acetate = 100:0, then 90:10) to obtain the titled compound (39.8 g).
   ¹H-NMR (400 MHz, CDCl₃) δ: 6.37-6.35 (m, 1H), 2.60-2.50 (m, 2H), 2.41-2.36 (m, 2H), 2.00-1.89 (m, 2H), 1.24 (s, 12H).

### Reference example 5

### 6-(4,4-Dimethylcyclohexyl)pyridazin-3(2H)-one

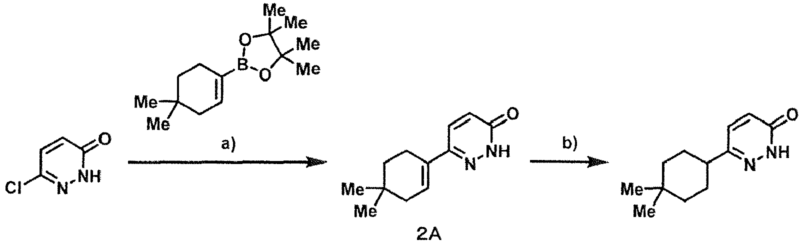

a) 6-Chloropyridazin-3(2H)-one (497 mg), 2-(4,4-dimethylcyclohex-1-en-1-yl)-4,4,5,5-tetramethyl-1,3,2-dioxaborolane (900 mg), and 2 mol/L aqueous sodium carbonate (4.76 mL) were suspended in 1,2-dimethoxyethane (17 mL). To the mixture was added 1,1'-bis(diphenylphosphino)ferrocene palladium dichloride (279 mg). The mixture was stirred under a nitrogen atmosphere at 80°C for 6 hours. After cooling to room temperature, water was added thereto, and the mixture was extracted with ethyl acetate. The organic layer was dried over anhydrous sodium sulfate, filtered, and concentrated under reduced pressure. The residue was purified by silica gel column chromatography (solvent; chloroform:methanol = 99:1, then 93:7) to obtain Compound 2A (88 mg).
   ¹H-NMR (400 MHz, DMSO-d₆) δ: 7.80 (d, J = 10.1 Hz, 1H), 6.80 (d, J = 9.6 Hz, 1H), 6.43-6.40 (m, 1H), 2.35-2.30 (m, 2H), 1.98 (dt, J = 4.1, 1.8 Hz, 2H), 1.42 (t, J = 6.4 Hz, 2H), 0.91 (s, 6H).
b) A suspension of Compound 2A (88 mg) and 10% palladium/carbon (20 mg) in methanol (20 mL) was stirred under a hydrogen atmosphere at room temperature for 10 hours. Pd/C was filterd through Celite, and washed with methanol. The eluent was concentrated to obtain the titled compound (89 mg).
   ¹H-NMR (400MHz, CDCl₃) δ: 10.66 (s, 1H), 7.19 (d, J = 10.1 Hz, 1H), 6.89 (d, J = 9.6 Hz, 1H), 2.42 (tt, J = 11.8, 4.0 Hz, 1H), 1.71-1.67 (m, 2H), 1.63-1.55 (m, 2H), 1.52-1.45 (m, 2H), 1.27 (td, J = 12.3, 4.0 Hz, 2H), 0.93 (s, 3H), 0.92 (s, 3H) .

### Reference exmple 6

### [3-(4-Methylpiperidin-1-yl)-6-oxopyridazin-1(6H)-yl]acetic acid

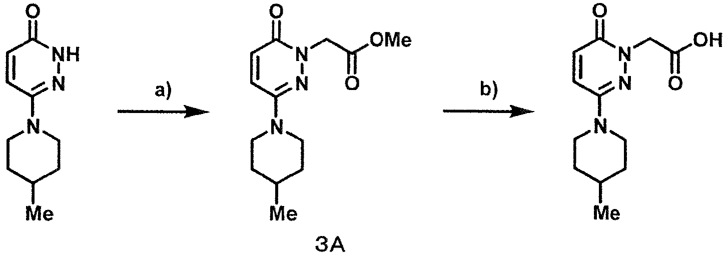

a) The compound of Reference example 2 (2.5 g), methyl bromoacetate (2.8 g), and potassium carbonate (3.6 g) in dimethylformamide (26 mL) were stirred at room temperature for 2.5 hours. After the addition of saturated aqueous ammonium chloride, the mixture was extracted with ethyl acetate. The organic layer was dried over anhydrous sodium sulfate, filtered, and concentrated under reduced pressure. The residue was purified by silica gel column chromatography (solvent; hexane:ethyl acetate = 30:70) to obtain Compound 3A (3.2 g).
   ¹H-NMR (400 MHz, CDCl₃) δ: 7.16 (d, J = 10.1 Hz, 1H), 6.84 (d, J = 10.1 Hz, 1H), 4.75 (s, 2H), 3.77-3.74 (m, 2H), 3.76 (s, 3H), 2.71 (td, J = 12.5, 2.4 Hz, 2H), 1.69 (d, J = 12.5 Hz, 2H), 1.59-1.48 (m, 1H), 1.28-1.18 (m, 2H), 0.96 (d, J = 6.4 Hz, 3H).
b) Compound 3A (3.2 g) was dissolved in a mixed solvent of methanol (20 mL) and THF (20 mL), and 2 mol/L aqueous sodium hydroxide (30 mL) was added thereto with ice cooling. Then, the mixture was stirred at room temperature for 30 minutes. The organic solvent of the reaction mixture was removed under reduced pressure, and to the resulting aqueous layer was added 1 mol/L hydrochloric acid to adjust pH to 3, resulting in precipitation of a solid. The precipitated solid was filtered, washed with water, and dried under reduced pressure to obtain the titled compound (3.0 g).
   ¹H-NMR (400 MHz, CD₃OD) δ: 7.50 (d, J = 10.1 Hz, 1H), 6.86 (d, J = 10.1 Hz, 1H), 4.72 (s, 2H), 3.92-3.88 (br, 2H), 2.76 (td, J = 12.7, 2.6 Hz, 2H), 1.73-1.68 (br, 2H), 1.64-1.51 (m, 1H), 1.23 (ddd, J = 24.4, 12.7, 3.9 Hz, 2H), 0.97 (d, J = 6.4 Hz, 3H).

### Reference example 7

### 4-(4-Methylphenyl)-5,6,7,8-tetrahydrophthalazin-1(2H)-one

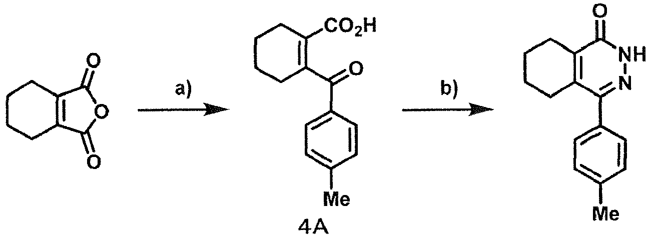

a) To a solution of 4,5,6,7-tetrahydroisobenzofuran-1,3-dione (5.0 g) in tetrahydrofuran (329 mL) was added dropwise a solution of p-tolylmagnesium bromide in tetrahydrofuran (32.9 mL), and the mixture was stirred at room temperature for 23 hours. To the reaction mixture was added 1 mol/L hydrochloric acid, resulting in precipitation of a solid, and the precipitated solid was filtered and washed with water. The resulting solid was purified by silica gel column chromatography (solvent; chloroform:methanol = 9:1) to obtain Compound 4A (10.7 g).
   ¹H-NMR (400 MHz, CDCl₃) δ: 7.40-7.31 (m, 2H), 7.15 (d, J = 8.5 Hz, 2H), 2.42-2.20 (m, 6H), 1.98-1.43 (m, 5H).
b) To a solution of Compound 4A (7.61 g) in ethanol (156 mL) was added hydrazine monohydrate (3.03 mL), and the mixture was heated under reflux for 5 hours. After cooling to room temperature, saturated aqueous sodium bicarbonate was added thereto, and ethanol was removed by concentration under reduced pressure, resulting in precipitation of a solid. The precipitated solid was filtered, washed with water, and dried under reduced pressure to obtain the titled compound (6.22 g).
   ¹H-NMR (400 MHz, CDCl₃) δ: 10.42 (s, 1H), 7.26-7.25 (m, 2H), 7.23-7.21 (m, 2H), 2.64-2.62 (m, 2H), 2.40-2.37 (m, 5H), 1.79-1.77 (m, 2H), 1.68-1.65 (m, 2H).

### Reference example 8

### 6-(2-Azaspiro[4.4]nonan-2-yl)-4-methylpyridazin-3(2H)-one

### Reference example 9

### 6-(2-Azaspiro[4.4]nonan-2-yl)-5-methylpyridazin-3(2H)-one

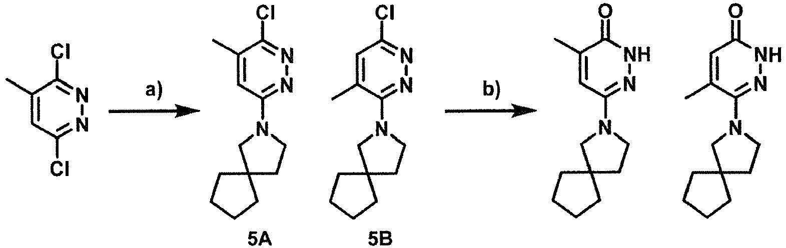

a) To a solution of 3,6-dichloro-4-methylpyridazine (0.40 g) in dimethylformamide (4 mL) was added 2-azaspiro[4.4]nonane (0.31 g) and triethylamine (1.03 mL). The reaction mixture was stirred at 80°C for 6 hours. After the addition of saturated aqueous sodium bicarbonate (20 mL) and water (20 mL), the mixture was extracted twice with ethyl acetate. The combined organic layer was dried over sodium sulfate and concentrated. The residue was purified by silica gel column chromatography (solvent; hexane:ethyl acetate = 90:10, and then 30:70) to obtain Compound 5A (250 mg) and Compound 5B (260 mg).
b) Compound 5A was dissolved in acetic acid (3 mL), and the reaction mixture was subjected to microwave irradiation and stirred at 200°C for 2 hours. The reaction mixture was cooled to room temperature, and toluene was added thereto. The mixture was concentrated under reduced pressure and purified by silica gel column chromatography (solvent; chloroform:methanol = 100:0, then 98:2) to obtain 6-(2-azaspiro[4.4]nonan-2-yl)-4-methylpyridazin-3(2H)-one (220 mg) .
   LC-MS: [M+H]⁺ / Rt (min) 234.2 / 0.832 (Method A)

Similarly, 6-(2-azaspiro[4.4]nonan-2-yl)-5-methylpyridazin-3(2H)-one (195 mg) was obtained from Compound 5B.
LC-MS: [M+H]⁺ / Rt (min) 234.2 / 0.839 (Method A)

### Reference example 10

### 6-(6-Azaspiro[3.4]octan-6-yl)-4-methoxypyridazin-3(2H)-one

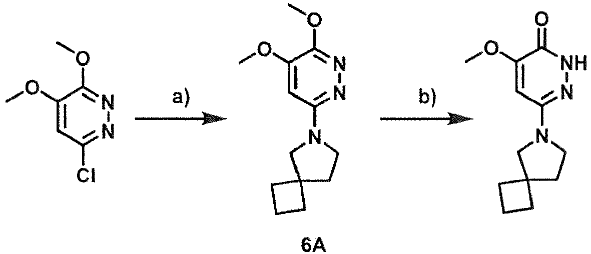

a) To a solution of 6-chloro-3,4-dimethoxypyridazine (0.25 g) in toluene (2.5 mL) were added 6-azaspiro[3.4]octane (0.23 g), potassium tert-butoxide (0.14 g), and 2,2'-bis(biphenylphosphino)-1,1'-binaphthalene (6.4 mg). The reaction mixture was subjected to microwave irradiation, and stirred at 80°C for 1 hour. After the reaction mixture was cooled to room temperature, water (20 mL) was added thereto, and the mixture was extracted twice with ethyl acetate. The combined organic layer was dried over sodium sulfate, and then concentrated. The residue was purified by silica gel column chromatography (solvent; hexane:ethyl acetate = 90:10,
   then 15:85) to obtain Compound 6A (44 mg).
   LC-MS: [M+H]⁺ / Rt (min) 250.2 / 0.506 (Method A)
b) Compound 6A (80 mg) was dissolved in dioxane (500 µL) and concentrated hydrochloric acid (2 mL). The reaction mixture was subjected to microwave irradiation, and stirred at 100°C for 2 hours. The reaction mixture was cooled to room temperature, and then concentrated under reduced pressure. The residue was poured into water, resulting in precipitation of a solid, and the precipitated solid was filtered to obtain the titled compound (53 mg).
   LC-MS: [M+H]⁺ / Rt (min) 236.2 / 0.527 (Method A)

### Reference example 11

### 1,1-Difluoro-4,4-dimethyl-6-azaspiro[2.5]octane hydrochloride

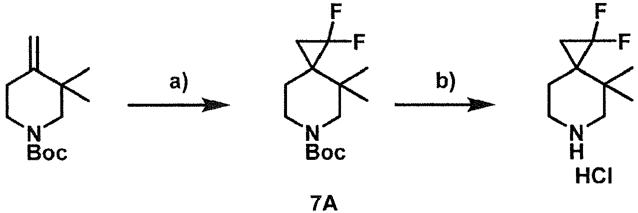

a) To a solution of tert-butyl 3,3-dimethyl-4-methylidenepiperidine-1-carboxylate (4.6 g) in tetrahydrofuran (29.2 mL) were added (trifluoromethyl)trimethylsilane (10.54 ml) and sodium iodide (1.53 g), and the mixture was heated under reflux for 33 hours. To the reaction mixture was added heptane (10 ml), and the mixture was concentrated under reduced pressure. The residue was dissolved in ethyl acetate. The organic layer was washed with water, saturated aqueous sodium thiosulfate, and brine, dried over anhydrous magnesium sulfate, and concentrated under reduced pressure. The residue was purified with silica gel column chromatography (solvent; hexane:ethyl acetate = 95:5, and then 75:25) to obtain Compound 7A (4.2 g).
   ¹H-NMR (400 MHz, CDCl₃) δ: 3.87 (br s, 1H), 3.43 (br s, 1H), 3.06-2.99 (m, 1H), 2.87-2.83 (m, 1H), 1.97-1.90 (m, 1H), 1.46 (s, 9H), 1.44-1.40 (m, 1H), 1.31-1.23 (m, 1H), 1.06 (s, 3H), 0.92-0.86 (m, 4H).
b) Compound 7A (4.2 g) was dissolved in cyclopentyl methyl ether (10 ml). To the mixture was added a solution of hydrogen chloride in cyclopentyl methyl ether (18.5 mL), and the mixture was stirred for 5 hours, resulting in precipitation of a solid. The precipitated solid was filtered, and dried to obtain the titled compound (3.71 g). ¹H-NMR (400 MHz, DMSO-d₆) δ: 9.59 (s, 1H), 8.96 (s, 1H), 3.16-3.12 (m, 1H), 2.94-2.91 (m, 1H), 2.88-2.70 (m, 2H), 2.09-2.02 (m, 1H), 1.70-1.64 (m, 1H), 1.46-1.40 (m, 1H), 1.32-1.26 (m, 1H), 1.17 (s, 3H), 0.89 (s, 3H).

### Reference example 12

### 7-Chloro-2-methyl-1,3-benzoxazole-5-amine

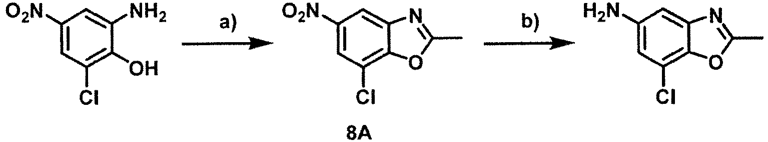

a) To a solution of 2-amino-6-chloro-4-nitrophenol (0.5 g) in DMF (15 mL) were added triethyl orthoacetate (1.72 g) and p-toluenesulfonic acid (0.23 g), and the mixture was stirred at 70°C for 4 hours. To the reaction mixture was added saturated aqueous sodium bicarbonate, and the mixture was extracted with ethyl acetate. The organic layer was washed with brine, dried over anhydrous sodium sulfate, and concentrated under reduced pressure. The residue was purified by silica gel column chromatography (solvents; hexane:ethyl acetate = 80:20) to obtain Compound 8A (0.37 g).
   ¹H-NMR (400 MHz, CDCl₃) δ: 8.46 (d, J = 1.8 Hz, 1H), 8.30 (d,
   J = 2.4 Hz, 1H), 2.75 (s, 3H).
b) Compound 8A (62.5 mg) was dissolved in a mixed solvent of methanol (4 ml) and water (1 ml), and reduced iron (164 mg) and ammonium chloride (157 mg) were added thereto. The mixture was stirred at 70°C for 2 hours. Insoluble substances were filtered out through Celite, and the organic layer was concentrated. The residue was dissolved again in ethyl acetate. The mixture was washed with water and brine, and dried over anhydrous sodium sulfate, and concentrated under reduced pressure to obtain the titled compound (43.7 mg) .
   ¹H-NMR (400 MHz, CDCl₃) δ: 6.81 (d, J = 2.1 Hz, 1H), 6.66 (d, J = 1.8 Hz, 1H), 3.69 (br s, 2H), 2.61 (s, 3H).

### Reference example 13

### [1,2,4]Triazolo[1,5-a]pyridine-7-amine 2hydrochloride

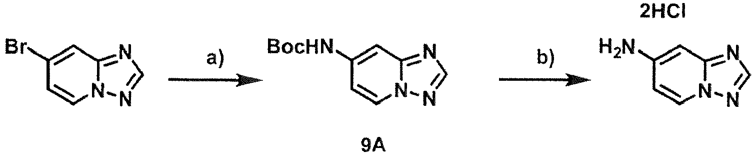

a) A solution of 7-bromo[1,2,4]triazolo[1,5-a]pyridine (975 mg), tert-butyl carbamate (865 mg), sodium t-butoxide (710 mg), [(2-di-tert-butylphosphino-2',4',6'-triisopropylbiphenyl)-2-(2'-amino-1,1'-biphenyl)]palladium(II) methanesulfonate (196 mg) in toluene (33 mL) was stirred at 100°C for 2 hours. To the reaction mixture was added water, and the mixture was extracted with a mixed solvent of chloroform and ethanol (3:1). The organic layer was washed with brine, dried over anhydrous sodium sulfate, and concentrated under reduced pressure. The residue was purified by amino silica gel column chromatography (solvent; hexane:ethyl acetate = 75:25, and then ethyl acetate) to obtain the titled compound 9A (780 mg) .
   ¹H-NMR (400 MHz, CDCl₃) δ: 8.43 (d, J = 7.3 Hz, 1H), 8.23 (s, 1H), 7.70 (d, J = 2.4 Hz, 1H), 7.18 (dd, J = 7.3, 2.4 Hz, 1H), 6.79 (s, 1H), 1.52 (s, 9H).
b) Compound 9A (780 mg) was suspended in ethyl acetate (3 ml), and a solution of hydrogen chloride in dioxane (16 ml) was added thereto. The mixture was stirred at 40°C for 3 hours. The reaction mixture was concentrated under reduced pressure, and the following steps were repeated twice: t toluene was added thereto, and the mixture was concentrated under reduced pressure. To the residue was added ethyl acetate, and the mixture was stirred, and then filtered to obtain the titled compound (499 mg).
   ¹H-NMR (400 MHz, CD₃OD) δ: 8.71 (s, 1H), 8.54 (d, J = 7.3 Hz, 1H), 6.90 (dd, J = 7.3, 2.1 Hz, 1H), 6.71 (d, J = 2.1 Hz, 1H) .

### Reference example 14

### 4-(Morpholin-4-yl)-2,3-dihydro-1H-indole 2hydrochloride

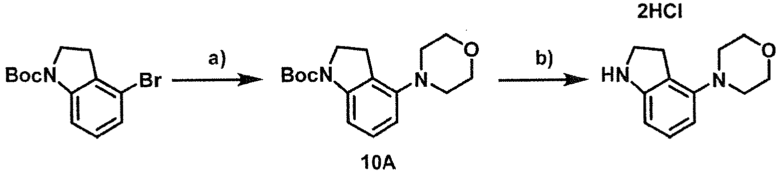

a) A solution of tert-butyl 4-bromo-2,3-dihydro-1H-indole-1-carboxylate (1.55 g), morpholine (1.81 g), tris(dibenzylideneacetone)dipalladium (0) (0.48 g), (R)-(+)-2,2'-bis(diphenylphosphino)-1, 1-binaphthyl (0.324 g), and sodium t-butoxide (0.999 g) in toluene (17.3 mL) was stirred at 100°C for 2 hours. Insoluble substances were filtered out through Celite, and the organic layer was concentrated. To the residue was added saturated aqueous ammonium chloride, and the mixture was extracted with ethyl acetate. The organic layer was washed with brine, dried over anhydrous sodium sulfate, and concentrated under reduced pressure. The residue was purified by silica gel column chromatography (solvent; hexane:ethyl acetate = 80:20) to obtain Compound 10A (1.07 g).
   LC-MS: [M+H]⁺/ Rt (min) 305.2 / 1.984 (Method B)
b) Compound 10A (1.07 g) was dissolved in ethyl acetate (15 ml), and a solution of hydrogen chloride in ethyl acetate (15 ml) was added thereto. The mixture was stirred at room temperature for 20 hours, and concentrated under reduced pressure. To the residue was added ethyl acetate, and the mixture was filtered to obtain the titled compound (0.92 g). ¹H-NMR (400 MHz, CD₃OD) δ: 7.48 (t, J = 8.2 Hz, 1H), 7.27 (t, J = 7.9 Hz, 2H), 3.94-3.92 (m, 4H), 3.87 (t, J = 7.6 Hz, 2H), 3.41 (t, J = 7.3 Hz, 2H), 3.26-3.24 (m, 4H).

### Reference example 15

### 4-(Pyridazin-4-yl)-2,3-dihydro-1H-indole hydrochloride

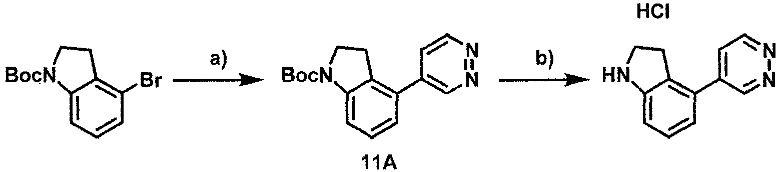

a) A solution of tert-butyl 4-bromo-2,3-dihydro-1H-indole-1-carboxylate (2.62 g), 4-(4,4,5,5-tetramethyl-1,3,2-dioxaborolan-2-yl)pyridazine (2.17 g), bis(di-tert-butyl(4-dimethylaminophenyl)phosphine)dichloropalladium (II) (0.62 g), 2 M aqueous potassium acetate (13 ml) in acetonitrile (35 mL) was stirred at 90°C for 4 hours. To the reaction mixture was added water, and the mixture was extracted with ethyl acetate. The organic layer was washed with brine, dried over anhydrous sodium sulfate, and concentrated under reduced pressure. The residue was purified by silica gel column chromatography (solvent; ethyl acetate, and then ethyl acetate:methanol = 95:5) to obtain Compound 11A (2.30 g).
   LC-MS: [M+H]⁺/ Rt (min) 298.2 / 0.891 (Method A)
b) Compound 11A (2.30 g) was dissolved in chloroform (35 ml) . After the addition of a solution of hydrogen chloride in ethyl acetate (35 ml), the mixture was stirred at room temperature for 3 hours. The reaction mixture was concentrated under reduced pressure, and the following steps were repeated twice: toluene was added to the mixture, and the mixture was concentrated under reduced pressure. The titled compound was obtained in 1.8 g.
   LC-MS: [M+H]⁺/ Rt (min) 198.2 / 0.333 (Method A)

### Reference example 16

### [3-(4-Methylcyclohex-1-en-1-yl)-6-oxopyridazin-1(6H)-yl]acetic acid

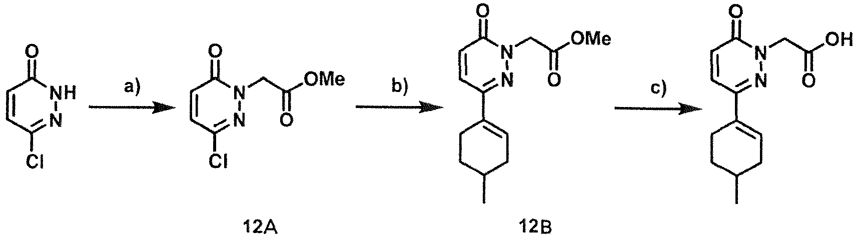

a) According to the method of a) in Reference example 6, Compound 12A (49.4 g) was obtained using 6-chloropyridazin-3(2H)-one (44.0 g).
   ¹H-NMR (400 MHz, DMSO-d₆) δ: 7.64 (d, J = 9.8 Hz, 1H), 7.13 (d, J = 9.8 Hz, 1H), 4.85 (s, 2H), 3.69 (s, 3H).
b) A solution of Compound 12A (2.15 g), 4,4,5,5-tetramethyl-2-(4-methylcyclohex-1-en-1-yl)-1,3,2-dioxaborolane (3.06 g) and 2 mol/L aqueous potassium acetate (15.92 mL) was suspended in acetonitrile (140 mL), and bis(di-tert-butyl(4-dimethylaminophenyl)phosphine)dichloropalladium (II) (301 mg) was added thereto. The mixture was stirred under a nitrogen atmosphere at 90°C for 5 hours. After cooling to room temperature, water was added thereto, and the mixture was extracted with ethyl acetate. The organic layer was dried over anhydrous sodium sulfate, filtered, and concentrated under reduced pressure. The residue was purified by silica gel column chromatography (solvent; hexane:ethyl acetate = 90:10, and then 70:30) to obtain Compound 12B (2.40 g).
   LC-MS: [M+H]⁺/ Rt (min) 263.2 / 0.935 (Method A)
c) According to the method of b) in Reference example 6, the titled compound (1.5 g) was obtained using Compound 12B (2.40 g).
   LC-MS: [M+H]⁺/ Rt (min) 249.2 / 0.835 (Method A)

### Reference example 17

### 2-(Trifluoromethyl)imidazo[1,2-a]pyridine-7-amine

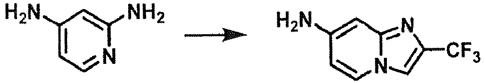

Pyridine-2,4-diamine (300 mg) and sodium bicarbonate (462 mg) was suspended in ethanol (9.1 mL), and 3-chloro-1,1,1, trifluoropropan-2-one was added thereto. The mixture was heated under reflux for 4 hours. After cooling to room temperature, water was added to the reaction mixture, and the mixture was extracted with a mixed solvent of chloroform and ethanol (3:1). The organic layer was dried over anhydrous sodium sulfate and concentrated under reduced pressure. The residue was purified by amino silica gel column chromatography (solvent; hexane:ethyl acetate = 50:50, and then ethyl acetate) to obtain the titled compound (250 mg) .
LC-MS: [M+H]⁺/ Rt (min) 202.1 / 0.344 (Method A)

### Reference example 18

### 7-Fluoro-1,3-benzoxazole-5-amine

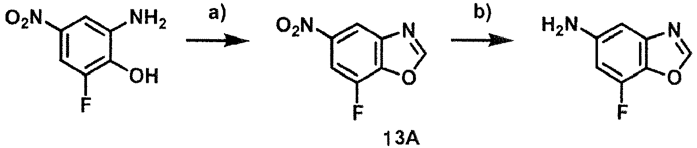

a) 2-Amino-6-fluoro-4-nitrophenol (507 mg) and triethoxymethane (0.98 mL) were dissolved in chloroform (14.7 mL) and acetic acid (0.68 mL). The mixture was heated under reflux for 5 hours. After cooling to room temperature, the mixture was concentrated under reduced pressure. The residue was purified by silica gel column chromatography (solvent; hexane:ethyl acetate = 83:17, and then 75:25) to obtain Compound 13A (330 mg).
   LC-MS: [M+H]⁺ / Rt (min) 183.0 / 0.749 (Method A)
b) Compound 13A (330 mg) was dissolved in methanol (9.0 mL) and palladium/carbon (96 mg) was added thereto. The mixture was stirred at room temperature for 3 hours. Insoluble substances were filtered through Celite, and the organic layer was concentrated to obtain the titled compound (268 mg) .
   LC-MS: [M+H]⁺ / Rt (min) 153.0 / 0.445 (Method A)

### Reference example 19

### 2-(Difluoromethyl)-1,3-benzoxazole-5-amine

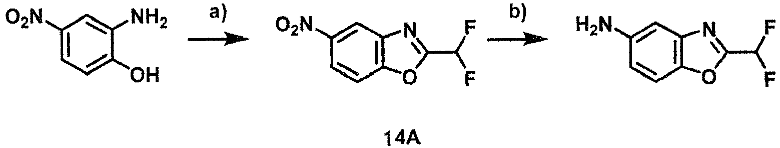

a) 2-Amino-4-nitrophenol (300 mg), triethylamine (1.36 mL), triphenylphosphine (1.28 g), and difluoroacetic acid (0.12 mL) were dissolved in carbon tetrachloride (6.5 mL), and the mixture was heated under reflux for 3 hours. After cooling to room temperature, to the reaction mixture was added water, and the mixture was extracted with a mixed solvent of chloroform and methanol (10:1). The organic layer was dried over anhydrous sodium sulfate, and concentrated under reduced pressure. The residue was purified by silica gel column chromatography (solvate; hexane:ethyl acetate = 75:25, and then ethyl acetate) to obtain Compound 14A (331 mg). LC-MS: [M+H]⁺ / Rt (min) 215.1 / 0.850 (Method A) b) According to the method of b) in Reference example 18, the titled compound was obtained using Compound 14A (330 mg) .
   LC-MS: [M+H]⁺ / Rt (min) 185.1 / 0.505 (Method A)

### Reference example 20

### 7-Fluoro-2-methoxy-1,3-benzoxazole-5-amine

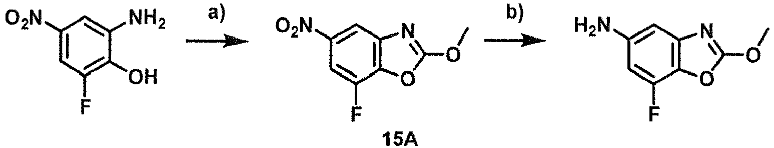

a) According to the method of a) in Reference example 18, Compound 15A (318 mg) was obtained by using 2-amino-6-fluoro-4-nitrophenol (303 mg) and tetramethyl orthocarbonate (0.47 mL) .
   LC-MS: [M+H]⁺ / Rt (min) 213.1 / 0.843 (Method A) b) According to the method of b) in Reference example 18, the titled compound (248 mg) was obtained by using Compound 120A (318 mg).
   LC-MS: [M+H]⁺ / Rt (min) 183.0 / 0.517 (Method A)

### Reference example 21

### 2-Methyl[1,3]thiazolo[5,4-b]pyridine-6-amine

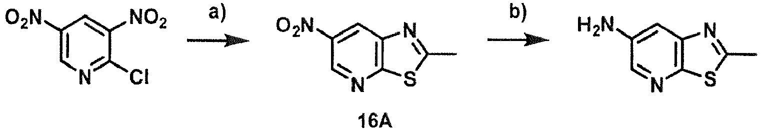

a) 2-Chloro-3,5-dinitropyridine (300 mg) was dissolved in sulfolane (9.8 mL), and thioacetamide (1.48 g) was added thereto. The mixture was heated under reflux at 110°C for 3 hours. After cooling to room temperature, water was added to the reaction mixture, and the mixture was extracted with ethyl acetate. The organic layer was dried over anhydrous sodium sulfate, and concentrated under reduced pressure. The residue was purified by silica gel column chromatography (solvent; hexane:ethyl acetate = 90:10, and then 75:25) to obtain Compound 16A (0.23 g).
   LC-MS: [M+H]⁺ / Rt (min) 196.1 / 0.667 (Method A)
b) Compound 16A (177 mg) was dissolved in ethanol (3.8 mL), and water (1.3 mL), ammonium chloride (485 mg), and reduced iron (253 mg) were added thereto. The mixture was heated under reflux for 3 hours. Insoluble substances were filtered through Celite, and the organic layer was concentrated under reduced pressure. To the residue was added saturated aqueous sodium bicarbonate, and the mixture was extracted with chloroform. The organic layer was dried over anhydrous sodium sulfate, and concentrated under reduced pressure to obtain the titled compound (97 mg).
   LC-MS: [M+H]⁺ / Rt (min) 166.0 / 0.423 (Method A)

### Reference example 22

### 2-(5-Amino-1,3-benzoxazol-2-yl)propan-2-ol

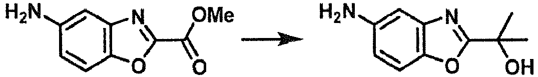

Methyl 5-amino-1,3-benzoxazole-2-carboxylate (200 mg) and cerium (III) chloride (1.03 g) were dissolved in tetrahydrofuran (6.9 mL). While stirring at 0°C, 3 mol/L methylmagnesium bromide (1.39 ml) was added thereto, and the mixture was stirred at 0°C for 2 hours. To the reaction mixture was added saturated aqueous ammonium chloride, and the mixture was extracted with chloroform. The organic layer was washed with saturated aqueous sodium bicarbonate and brine, dried over anhydrous sodium sulfate, and concentrated under reduced pressure. The residue was purified by silica gel column chromatography (solvate; ethyl acetate, and then ethyl acetate:methanol = 96:4) to obtain the titled compound (10.0 mg).
LC-MS: [M+H]⁺ / Rt (min) 193.1 / 0.317 (Method A)

### Reference example 23

### 1-(5-Methoxy-2-methylpyridin-3-yl)piperazine 3hydrochloride

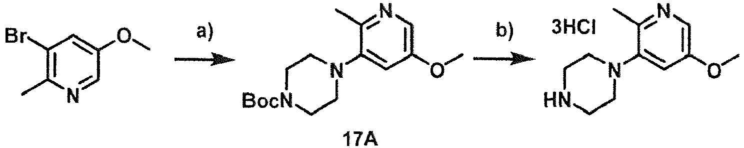

a) 1-Boc-piperazine (277 mg), tris(dibenzylideneacetone)dipalladium (0) (91 mg), 4,5-bis(diphenylphosphino)-9,9-dimethylxanthene (172 mg), and sodium t-butoxide (190 mg) were suspended in toluene (10 ml) . 3-Bromo-5-methoxy-2-methylpyridine (200 mg) was added thereto, and the mixture was stirred at 70°C for 1 hour. After cooling to room temperature, ethyl acetate was added to the reaction mixture. Insoluble substances were filtered through Celite, and the organic layer was concentrated under reduced pressure. The residue was purified by silica gel column chromatography (solvent; hexane:ethyl acetate = 70:30, and then 50:50) to obtain Compound 17A (296 g).
   ¹H-NMR (400 MHz, CDCl₃) δ: 7.94 (d, J = 2.4 Hz, 1H), 6.86 (d, J = 2.4 Hz, 1H), 3.84 (s, 3H), 3.60-3.57 (m, 4H), 2.86-2.84 (m, 4H), 2.48 (s, 3H), 1.49 (s, 9H)
b) Compound 17A (296 mg) was dissolved in methanol (5 mL), and 2 mol/L solution of hydrogen chloride in methanol (9.6 mL) was added thereto while stirring at 0°C. The mixture was stirred at 50°C for 3 hours. After cooling to room temperature, the precipitated solid was filtered, and the resulting solid was dried under reduced pressure to obtain the titled compound (271.7 mg).
   ¹H-NMR (400 MHz, CD₃OD) δ: 8.16 (d, J = 2.4 Hz, 1H), 7.83 (d, J = 2.4 Hz, 1H), 4.03 (s, 3H), 3.47-3.45 (m, 4H), 3.37-3.35 (m, 4H), 2.68 (s, 3H) .

### Reference example 24

### (2R,6S)-2,6-Dimethyl-1-(pyridin-3-yl)piperazine 3hydrochloride

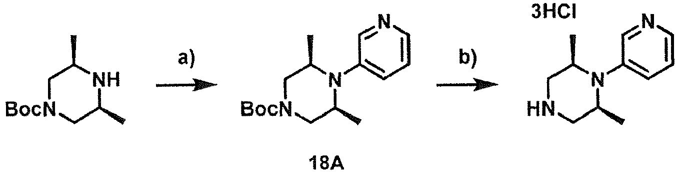

a) 0.5 mol/L Potassium hexamethyldisilazide (2.57 ml), tert-butyl (3R, 5S)-3,5-dimethylpiperazine-1-carboxylate (250 mg), and 3-bromopyridine (184 mg) were suspended in 1,4-dioxane (10 ml), and the mixture was stirred at 100°C for 8 hours. After cooling to room temperature, water was added thereto, and the mixture was extracted with ethyl acetate. The organic layer was washed with brine, dried over anhydrous sodium sulfate, filtered, and then concentrated under reduced pressure. The residue was purified by silica gel column chromatography (solvent; hexane:ethyl acetate = 80:20, and then 40:60) to obtain Compound 18A (114 mg).
   LC-MS: [M+H]⁺ / Rt (min) 292.0 / 0.689 (Method A)
b) According to the method of b) in Reference example 15, the titled compound (75 mg) was obtained by using Compound 18A (114 mg).
   LC-MS: [M+H]⁺ / Rt (min) 192.2 / 0.149 (Method A)

### Reference example 25

### 4-[(Morpholin-4-yl)methyl]-2,3-dihydro-1H-indole

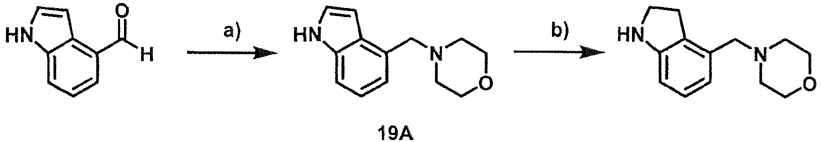

a) 2,3-Dihydro-1H-indole-4-carbaldehyde (147 mg) was dissolved in dichloromethane (2 ml), and morpholine (88 mg) and sodium triacetoxyborohydride (322 mg) were added thereto. The mixture was stirred at room temperature for 4 hours. To the reaction mixture was added saturated aqueous sodium bicarbonate, and the mixture was extracted with ethyl acetate. The organic layer was washed with brine, dried over anhydrous sodium sulfate, filtered, and then concentrated under reduced pressure to obtain Compound 19A (209 mg).
   LC-MS: [M+H]⁺ / Rt (min) 217.2 / 0.292 (Method A)
b) Compound 19A (209 mg) were dissolved in acetic acid (3 ml), and sodium cyanoborohydride (182 mg) was added thereto, and the mixture was stirred at room temperature for 4 hours. The mixture was concentrated under reduced pressure, and the following steps were repeated twice: toluene was added thereto, and the mixture was concentrated under reduced pressure. To the residue was added saturated aqueous sodium bicarbonate, and the mixture was extracted with ethyl acetate. The organic layer was washed with brine, dried over anhydrous sodium sulfate, filtered, and then concentrated under reduced pressure to obtain the titled compound (256 mg).
   LC-MS: [M+H]⁺ / Rt (min) 219.2 / 0.131 (Method A)

### Reference example 26

### 4-[(1H-Imidazol-1-yl)methyl]-2,3-dihydro-1H-indole hydrochloride

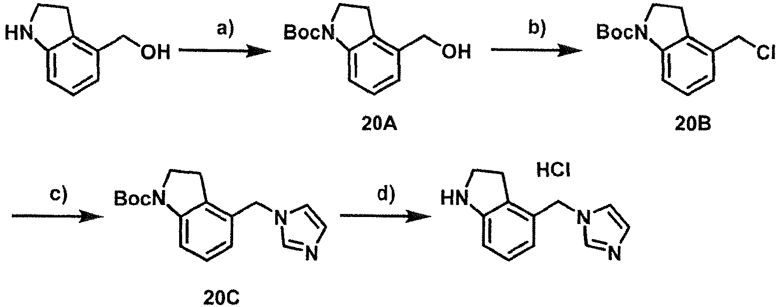

a) (2,3-Dihydro-1H-indol-4-yl)methanol (0.92 g) was dissolved in tetrahydrofuran (2 ml), and di-tert-butyl dicarbonate (1.48 g) was added thereto. The mixture was stirred at room temperature overnight. The reaction mixture was concentrated under reduced pressure, resulting in precipitation of a solid, and the resulting residue was purified by silica gel column chromatography (solvent; hexane, and then hexane:ethyl acetate = 70:30) to obtain Compound 20A (1.54 g).
   LC-MS: [M+H-tBu]⁺ / Rt (min) 194.1 / 0.866 (Method A)
b) Compound 20A (1.54 g) was dissolved in dichloromethane (20 ml), and thionyl chloride (0.57 ml) was added thereto while stirring at 0°C. The mixture was warmed to room temperature, and stirred overnight. The reaction mixture was concentrated under reduced pressure, and the following steps were repeated twice: toluene was added thereto, and the mixture was concentrated under reduced pressure to obtain Compound 20B (1.65 g).
   LC-MS: [M+H-tBu]⁺ / Rt (min) 212.1 / 1.169 (Method A)
c) Compound 20B (200 mg) was dissolved in dimethylformamide (3 ml), and imidazole (2.5 g) was added thereto. The mixture was stirred at 80°C for 2 hours. Water was added thereto, and the mixture was extracted with ethyl acetate. The organic layer was washed with water and brine, dried over anhydrous sodium sulfate, filtered, and concentrated under reduced pressure. The residue was purified by silica gel column chromatography (solvent; ethyl acetate, and then ethyl acetate:methanol = 95:5) to obtain Compound 20C (80 mg).
   LC-MS: [M+H]⁺ / Rt (min) 300.3 / 0.683 (Method A)
d) Compound 20C (80 mg) were dissolved in chloroform (3 ml), and a solution of hydrogen chloride in ethyl acetate (0.54 mL) were added thereto. The reaction mixture was stirred at room temperature for 1 hour, and concentrated under reduced pressure. The following steps were repeated twice: to the residue was added toluene, and the mixture was concentrated to obtain the titled compound (53 mg).
   LC-MS: [M+H]⁺ / Rt (min) 200.1 / 0.115 (Method A)

### Reference example 27

### Methyl (3-chloro-5-methyl-6-oxopyridazin-1(6H)-yl)acetate

### Reference example 28

### Methyl (3-chloro-4-methyl-6-oxopyridazin-1(6H)-yl)acetate

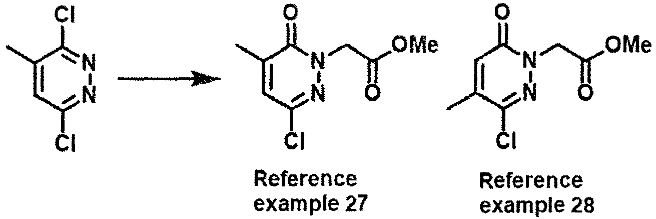

3,6-Dichloro-4-methylpyridazine (650 mg) was dissolved in acetic acid (6 mL), and the reaction mixture was subjected to microwave irradiation, and stirred at 200°C for 2 hours. After cooling to room temperature, the following steps were repeated three times: toluene was added thereto, and the mixture was concentrated under reduced pressure. The residue was dissolved in dimethylformamide (3 mL), and methyl bromoacetate (855 mg) and potassium carbonate (1.10 g) were added thereto. The mixture was stirred at room temperatuer overnight. After the addition of saturated aqueous ammonium chloride, the mixture was extracted with ethyl acetate. The organic layer was dried over anhydrous sodium sulfate, filtered, and concentrated under reduced pressure. The residue was purified by silica gel column chromatography (solvent; hexane:ethyl acetate = 90:10, and then 25:75) to obtain the compound of Reference example 27 (374 mg) and the compound of Reference example 28 (190 mg), respectively. Reference example 27 LC-MS: [M+H]⁺ / Rt (min) 217.1 / 0.610

### (Method A)

Reference example 28 LC-MS: [M+H]⁺ / Rt (min) 217.1 / 0.598

### (Method A)

### Reference example 29

### 4,4,5,5-Tetramethyl-2-(4-methylcyclopent-1-en-1-yl)-1,3,2-dioxaborolane

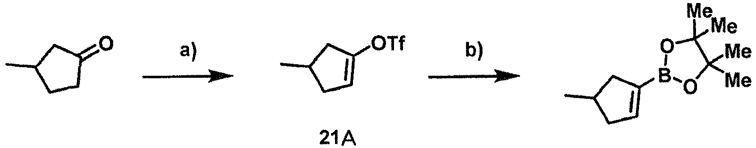

a) A solution of 4,4-dimethyl-2-cyclopenten-1-one (750 mg) in tetrahydrofuran (40 mL) was stirred at -78°C, and 1 mol/L solution of lithium tri-sec-butylborohydride in tetrahydrofuran (7.8 ml) was added thereto. The mixture was stirred at -78°C for 1 hour. To the reaction mixture was added a solution of 2-[N,N-bis(trifluoromethylsulfonyl)amino]pyridine (2.80 g) in tetrahydrofuran (10 ml), and the mixture was stirred at room temperature for 1 hour. To the reaction mixture was added water, and the mixture was extracted with ethyl acetate. The organic layer was washed with 2 mol/L aqueous sodium hydroxide (30 mL) and brine, dried over anhydrous sodium sulfate, filtered, and concentrated under reduced pressure. The residue was purified by silica gel column chromatography (solvent; hexane:ethyl acetate = 99:1) to obtain Compound 21A (310 mg).
   ¹H-NMR (400 MHz, CDCl₃) δ: 5.57-5.55 (m, 1H), 2.78-2.70 (m, 1H), 2.63-2.49 (m, 2H), 2.22-2.15 (m, 1H), 2.02-1.95 (m, 1H), 1.11 (d, J = 6.8 Hz, 3H).
b) According to the method of b) in Reference example 4, the titled compound (150 mg) was obtained by using Compound 21A (300 mg).
   ¹H-NMR (400 MHz, CDCl₃) δ: 6.48-6.43 (1H, m), 2.67-2.52 (2H, m), 2.42-2.28 (1H, m), 2.08-1.95 (2H, m), 1.33-1.21 (12H, m), 1.04-0.99 (3H, m).

### Reference example 30

### 2-(1,1-Difluorospiro[2.5]oct-5-en-6-yl)-4,4,5,5-tetramethyl-1,3,2-dioxaborolane

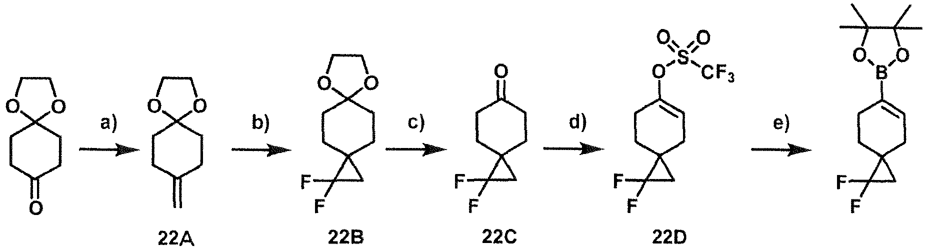

a) To a suspension of methyltriphenylphosphonium bromide (121.0 g) in toluene (570 mL) was added potassium tert-butoxide (37.9 g), and the mixture was stirred at room temperature for 1 hour. To the reaction mixture was added a solution of 1,4-dioxaspiro[4.5]decan-8-one (24.0 g) in toluene (1000 ml), and the mixture was stirred at room temperature for 2 hours. To the reaction mixture was added sandy magnesium chloride (64.4 g), and the mixture was stirred at 60°C for 2 hours. To the reaction mixture was added acetone (13.54 ml), and the mixture was stirred at 60°C for 2 hours, and then at room temperature overnight. The reaction mixture was filtered, and the solid was washed with heptane (400 ml). The eluent was concentrated under reduced pressure. To the residue was added hexane, and the mixture was stirred for a while. Insoluble substances were filtered, and the eluent was concentrated under reduced pressure. The residue was purified by silica gel column chromatography (solvate; hexane, and then hexane:ethyl acetate = 85:15) to obtain Compound 22A (20.8 g).
   ¹H-NMR (400 MHz, CDCl₃) δ: 4.65 (s, 2H), 3.96 (s, 4H), 2.27 (t, J = 6.5 Hz, 4H), 1.69 (t, J = 6.5 Hz, 4H).
b) According to the method of a) in Reference example 11, Compound 22B (24.0 g) was obtained by using Compound 22A (20.8 g).
   ¹H-NMR (400 MHz, CDCl₃) δ: 3.96 (s, 4H), 1.74-1.66 (m, 8H), 1.05 (t, J = 8.2 Hz, 2H).
c) Compound 22B (23.8 g) was dissolved in a mixed solvent of acetone (180 ml) and water (120 ml). p-Toluenesulfonic acid monohydrate (2.22 g) was added thereto, and the mixture was heated under reflux for 4 hours. After cooling to room temperature, saturated aqueous sodium bicarbonate was added thereto, and the acetone layer was removed under reduced pressure. The aqueous layer was extracted twice with ethyl acetate. The combined organic layer was dried over anhydrous magnesium sulfate, filtered, and concentrated under reduced pressure. The residue was purified by silica gel column chromatography (solvent; hexane:ethyl acetate = 90:10, and then hexane:ethyl acetate = 75:25) to obtain Compound 22C (15.2 g).
   ¹H-NMR (400 MHz, CDCl₃) δ: 2.48-2.37 (m, 4H), 1.98-1.89 (m, 4H), 1.25-1.21 (m, 2H).
d) A solution of Compound 22C (4.77 g) and 2,6-lutidine in dichloromethane (48 ml) was stirred at 0°C, and trifluoromethanesulfonic anhydride (10.57 ml) was added thereto. The mixture was warmed, and heated under reflux for 3 hours. After cooling to room temperature, saturated aqueous sodium bicarbonate was added thereto, and the dichloromethane layer was removed under reduced pressure. The aqueous layer was extracted twice with ethyl acetate. The combined organic layer was washed sequentially with 1 mol/l aqueous hydrochloric acid, water, and brine, dried over anhydrous magnesium sulfate, filtered, and concentrated under reduced pressure. The residue was purified by silica gel column chromatography (solvent; hexane:ethyl acetate = 95:5, and then hexane:ethyl acetate = 77:23) to obtain Compound 22D (6.61 g).
   ¹H-NMR (400 MHz, CDCl₃) δ: 5.78 (t, J = 3.7 Hz, 1H), 2.45-2.35 (m, 3H), 2.19-2.14 (m, 1H), 1.86-1.81 (m, 2H), 1.21-1.11 (m, 2H).
e) To a solution of Compound 22D (6.61 g), triphenylphosphine (593 mg), potassium phenoxide (2.99 g), 4,4,4',4',5,5,5',5'-octamethyl-2,2'-bi(1,3,2-dioxaborolane) (6.03 g) in toluene (113 ml) was added bis(triphenylphosphine)palladium(II) dichloride (794 mg), and the mixture was stirred at 50°C for 4 hours. To the reaction mixture were added potassium phenoxide (1.14 g) and tetrakis(triphenylphosphine)palladium(0) (784 mg), and the mixture was stirred at 50°C for 1.5 hours. After cooling to room temperature, water and ethyl acetate were added thereto, and the mixture was filtered through Celite. The organic layer was washed with 1 M aqueous sodium carbonate and brine, dried over anhydrous magnesium sulfate, filtered, and concentrated under reduced pressure. The residue was purified by silica gel column chromatography (solvent; hexane, and then hexane:ethyl acetate = 80:20), and purified again by silica gel column chromatography (solvent; hexane:toluene = 50:50, and then hexane:toluene:ethyl acetate = 45:50:5) to obtain the titled compound (3.72 g).
   ¹H-NMR (400 MHz, CDCl₃) δ: 6.53-6.50 (m, 1H), 2.36-2.14 (m, 3H), 2.05-2.00 (m, 1H), 1.67-1.55 (m, 2H), 1.25 (s, 12H), 1.09-0.97 (m, 2H).

### Reference example 31

### 8-Fluoro-[1,2,4]triazolo[1,5-a]pyridine-7-amine

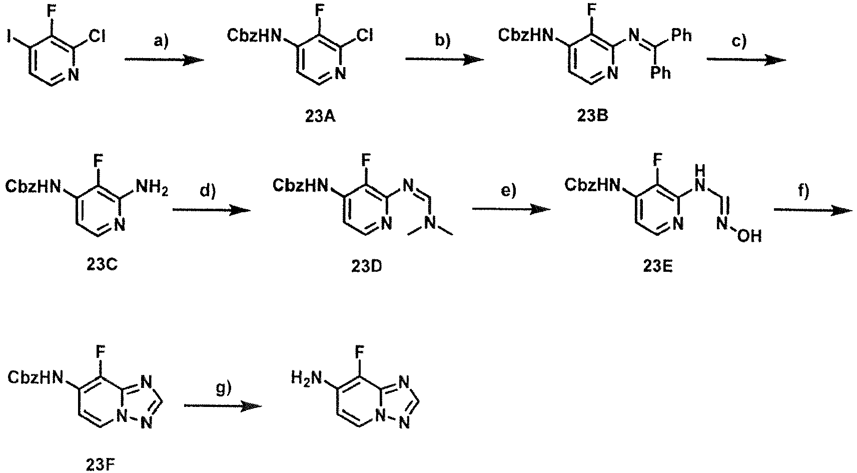

a) A solution of 2-chloro-3-fluoro-4-iodopyridine (3.1 g), benzyl carbamate (2.28 g), 4,5-bis(diphenylphosphino)-9,9-dimethylxanthene (1.74 g), and tris(dibenzylideneacetone)dipalladium (0) (1.32 g) in toluene (80 mL) was stirred at 100°C for 8 hours. After cooling to room temperature, water and ethyl acetate were added thereto, and the mixture was filtered through Celite. The aqueous layer was extracted with ethyl acetate. The organic layer was dried over anhydrous sodium sulfate, and concentrated under reduced pressure. The residue was purified by silica gel column chromatography (solvent; hexane:ethyl acetate = 87:13, and then hexane:ethyl acetate = 67:33) to obtain Compound 23A (1.8 g).
   LC-MS: [M+H]⁺ / Rt (min) 281.1 / 0.948 (Method A)
b) A solution of Compound 23A (1.25 g), benzophenone imine (1.12 mL), sodium t-butoxide (642 mg), and [(2-di-tert-butylphosphino-2',4',6'-triisopropylbiphenyl)-2-(2'-amino-1,1'-biphenyl)]palladium (II) methanesulfonate (354 mg) in toluene (18 ml) was stirred at 150°C for 5 hours under microwave irradiation. After cooling to room temperature, water and ethyl acetate were added thereto, and the mixture was filtered through Celite. The aqueous layer was extracted with ethyl acetate. The organic layer was dried over anhydrous sodium sulfate, and concentrated under reduced pressure. The residue was purified by amino silica gel column chromatography (solvent; hexane:ethyl acetate = 87:13, and then hexane:ethyl acetate = 67:33) to obtain Compound 23B (490 mg).
   LC-MS: [M+H]⁺ / Rt (min) 426.3 / 1.117 (Method A)
c) Compound 23B (780 mg) was dissolved in tetrahydrofuran (5.3 ml), and 1 mol/L aqueous hydrochloric acid (5.3 ml) was added thereto. The mixture was stirred at room temperature for 4 hours. To the reaction mixture was added saturated sodium bicarbonate, and the mixture was extracted with a mixed solvent of chloroform and ethanol (3:1). The organic layer was dried oved anhydrous sodium sulfate, and concentrated under reduced pressure. The residue was purified by amino silica gel column chromatography (solvent; hexane:ethyl acetate = 87:13, and then ethyl acetate) to obtain Compound 23C (179 mg).
   LC-MS: [M+H]⁺ / Rt (min) 262.1 / 0.542 (Method A)
d) Compound 23C (179 mg) was dissolved in 2-propanol (2.7 ml), and N,N-dimethylformamide dimethyl acetal (0.119 ml) was added thereto. The mixture was stirred at 100°C for 3 hours. To the reaction mixture was added toluene (2.7 mL), and the mixture was concentrated under reduced pressure to obtain Compound 23D (212 mg).
   LC-MS: [M+H]⁺ / Rt (min) 317.2 / 0.598 (Method A)
e) Compound 23D (178 mg) was dissolved in 2-propanol (2.7 ml), and 50% aqueous hydroxyamine (0.05 ml) was added thereto.
   The mixture was stirred at 60°C for 6 hours. To the reaction mixture was added water, and the mixture was extracted with chloroform. The organic layer was dried over anhydrous sodium sulfate, and concentrated under reduced pressure. The residue was purified by silica gel column chromatography (solvent; hexane:ethyl acetate = 67:33, and then hexane:ethyl acetate = 30:70) to obtain Compound 23E (146 mg) .
   LC-MS: [M+H]⁺ / Rt (min) 305.2 / 0.782 (Method A)
f) Compound 23E (171 mg) was dissolved in tetrahydrofuran (5.6 ml), and trifluoroacetic anhydride (0.119 ml) was added thereto. The mixture was stirred at room temperature for 21 hours. To the reaction mixture was added saturated sodium bicarbonate, and the mixture was extracted with chloroform. The organic layer was dried over anhydrous sodium sulfate, and concentrated under reduced pressure. The residue was purified by silica gel column chromatography (solvent; hexane:ethyl acetate = 83:17, and then hexane:ethyl acetate = 50:50) to obtain Compound 23F (82 mg).
   LC-MS: [M+H]⁺ / Rt (min) 287.2 / 0.742 (Method A)
g) Compound 23F (90 mg) was dissolved in methanol (1.6 ml), and 10% palladium/carbon (17 mg) was added thereto. The mixture was stirred under a hydrogen atmosphere at room temperature for 4 hours. Palladium/carbon was filtered through Celite, and the eluent was washed with methanol. The eluent was concentrated to obtain the titled compound (47 mg).
   LC-MS: [M+H]⁺ / Rt (min) 153.0 / 0.294 (Method A)

### Example 1

### N-(4-Cyanophenyl)-2-[3-(4-methylpiperidin-1-yl)-6-oxopyridazin-1(6H)-yl]acetamide

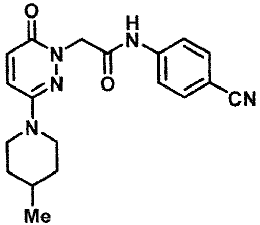

To a solution of the compound of Reference example 2 (1.0 g) in dimethylformamide (14 mL) were added potassium carbonate (1.43 g) and the compound of Reference example 1 (1.0 g). After stirring at room temperature for 24 hours, water was added thereto, and the mixture was extracted with ethyl acetate. The organic layer was dried over anhydrous sodium sulfate, filtered, and concentrated under reduced pressure. The residue was purified by silica gel column chromatography (solvent; hexane:ethyl acetate = 2:3, and then ethyl acetate) to obtain the titled compound (1.21 g). ¹H-NMR (400 MHz, CDCl₃) δ: 9.79 (s, 1H), 7.63 (dt, J = 9.0, 2.0 Hz, 2H), 7.55 (dt, J = 8.5, 1.8 Hz, 2H), 7.24 (d, J = 9.8 Hz, 1H), 6.93 (d, J = 10.4 Hz, 1H), 4.84 (s, 2H), 3.84 (d, J = 13.4 Hz, 2H), 2.77 (td, J = 12.8, 2.4 Hz, 2H), 1.75-1.68 (m, 2H), 1.58-1.53 (m, 1H), 1.25 (dd, J = 12.5, 4.0 Hz, 1H), 1.19 (dd, J = 12.2, 4.3 Hz, 1H), 0.97 (d, J = 6.7 Hz, 3H) .

### Example 2

### N-(4-Cyanophenyl)-2-[3-(4,4-dimethylpiperidin-1-yl)-6-oxopyridazin-1(6H)-yl]acetamide

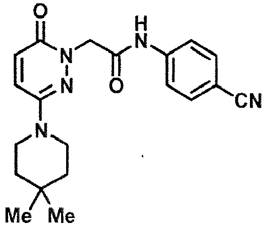

A solution of the compound of Reference example 3 (60 mg), 4,4-dimethylpiperidine hydrochloride (93 mg), and diisopropylethylamine (1 mL) in dimethylacetamide (0.5 mL) was stirred at 150°C for 11 hours. After the completion of the reaction, water was added thereto, and the mixture was extracted with ethyl acetate. The organic layer was dried over anhydrous sodium sulfate, filtered, and concentrated under reduced pressure. The residue was purified by reversed-phase HPLC (eluent; 0.035% trifluoroacetic acid in acetonitrile/water), and then by amino silica gel column chromatography (solvent; chloroform:methanol = 99:1, and then 93:7) to obtain the titled compound (22 mg).
¹H-NMR (400 MHz, CDCl₃) δ: 9.78 (s, 1H), 7.65 (d, J = 8.5 Hz, 2H), 7.57 (d, J = 8.5 Hz, 2H), 7.25 (d, J = 9.8 Hz, 1H), 6.94 (d, J = 10.4 Hz, 1H), 4.84 (s, 2H), 3.32-3.28 (m, 4H), 1.46-1.42 (m, 4H), 0.98 (s, 6H).

### Examples 3 to 36

According to the method of Example 1 or 2 and common reaction conditions, the compounds of Examples 3 to 36 were obtained by using corresponding material compounds.

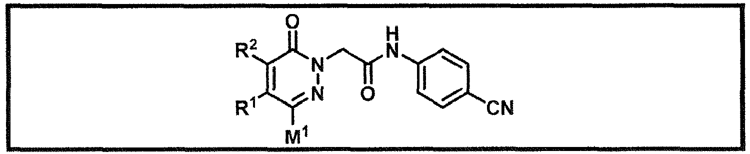

| Example | M¹ | R¹ | R² | LC-MS: [M+H]⁺ / Rt (min) (Method) |
|---|---|---|---|---|
| 3 | 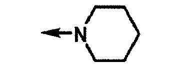 | H | H | 338.3 / 0.810 (Method A) |
| 4 | 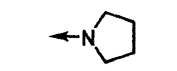 | H | H | 324.2 / 0.730 (Method A) |
| 5 | 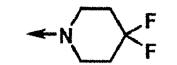 | H | H | 374.2 / 0.778 (Method A) |
| 6 | 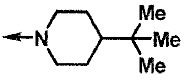 | H | H | 394.4 / 1.05 (Method A) |
| 7 | 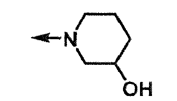 | H | H | 354.3 / 0.586 (Method A) |
| 8 | 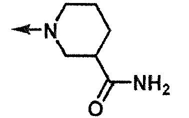 | H | H | 381.3 / 0.552 (Method A) |
| 9 | 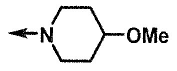 | H | H | 368.3 / 0.689 (Method A) |
| 10 | 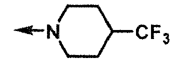 | H | H | 406.3 / 0.864 (Method A) |
| 11 | 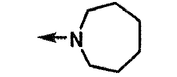 | H | H | 352.3 / 0.860 (Method A) |
| 12 | 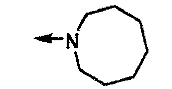 | H | H | 366.4 / 0.872 (Method A) |
| 13 | 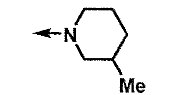 | H | H | 352.3 / 0.874 (Method A) |
| 14 | 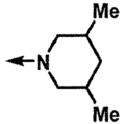 | H | H | 366.3 / 0.945 (Method A) |
| 15 | 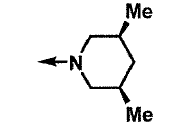 | H | H | 366.4 / 0.951 (Method A) |
| 16 | 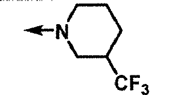 | H | H | 406.3 / 0.875 (Method A) |
| 17 | 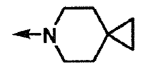 | H | H | 364.3 / 0.885 (Method A) |
| 18 | 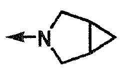 | H | H | 336.2 / 0.795 (Method A) |
| 19 | 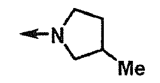 | H | H | 338.2 / 0.836 (Method A) |
| 20 | 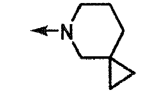 | H | H | 364.3 / 0.907 (Method A) |
| 21 | 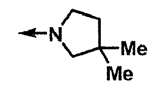 | H | H | 352.3 / 0.895 (Method A) |
| 22 | 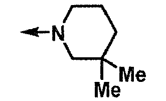 | H | H | 366.3 / 0.970 **(Method A)** |
| 23 | 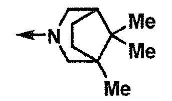 | H | H | 406.4 / 1.079 (Method A) |
| 24 | 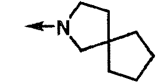 | H | H | 378.3 / 0.979 (Method A) |
| 25 | 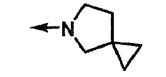 | H | H | 350.2 / 0.938 (Method A) |
| 26 | 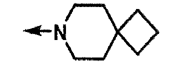 | H | H | 378.3 / 1.001 (Method A) |
| 27 | 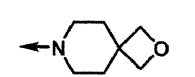 | H | H | 380.3 / 0.698 (Method A) |
| 28 | 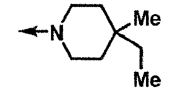 | H | H | 380.3 / 1.029 (Method A) |
| 29 | 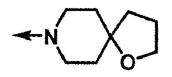 | H | H | 394.3 / 0.790 (Method A) |
| 30 | 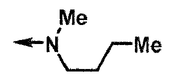 | H | H | 340.3 / 0.890 (Method A) |
| 31 | 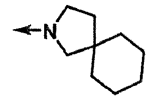 | H | H | 392.3 / 1.033 (Method A) |
| 32 | 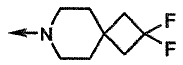 | H | H | 414.3 / 0.908 (Method A) |
| 33 | 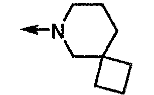 | H | H | 378.3 / 1.004 (Method A) |
| 34 | 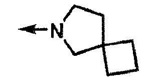 | H | H | 364.3 / 0.938 (Method A) |
| 35 | 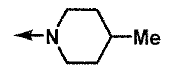 | Me | Me | 380.3 / 1.05 (Method A) |
| 36 | 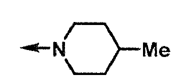 | - | (CH2)₄- | 406.3 / 1.14 (Method A) |

### Example 37

### N-(4-Cyanophenyl)-2-[3-(4,4-difluorocyclohex-1-en-l-yl)-6-oxopyridazin-1(6H)-yl]acetamide

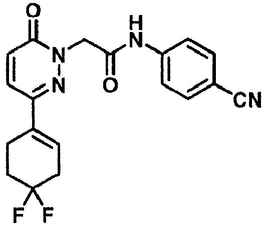

The compound of Reference example 3 (1.58 g), the compound of Reference example 4 (1.74 g), and 2 mol/L aqueous sodium carbonate (6.85 mL) were suspended in 1,2-dimethoxyethane (25 mL), and 1,1'-bis(diphenylphosphino)ferrocene palladium dichloride (401 mg) was added thereto. The mixture was stirred under a nitrogen atmosphere at 80°C for 4 hours. After cooling to room temperature, water was added thereto, and the mixture was extracted with ethyl acetate. The organic layer was dried over anhydrous sodium sulfate, filtered, and concentrated under reduced pressure. The residue was purified by silica gel column chromatography (solvent; chloroform:methanol = 99:1, and then 93:7), and recrystallized with ethanol (60 mL)-acetonitrile (20 mL) to obtain the titled compound (1.64 g).
¹H-NMR (400 MHz, CDCl₃) δ: 9.37 (s, 1H), 7.65-7.58 (m, 3H), 7.54 (d, J = 8.7 Hz, 2H), 7.04 (d, J = 9.6 Hz, 1H), 6.22 (s, 1H), 4.99 (s, 2H), 2.82-2.71 (m, 4H), 2.20-2.10 (m, 2H).

### Example 38

### N-(4-Cyanophenyl)-2-[3-(4,4-dimethylcyclohexyl)-6-oxopyridazin-1(6H)-yl]acetamide

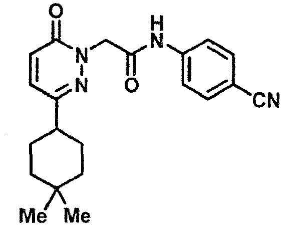

To a solution of the compound of Reference example 5 (40 mg) in dimethylformamide (2 mL) were added potassium carbonate (54 mg) and the compound of Reference example 1 (45 mg). After cooling to room temperature for 6 hours, water was added thereto, and the mixture was extracted with ethyl acetate. The organic layer was dried over anhydrous sodium sulfate, filtered, and concentrated under reduced pressure. The residue was purified by silica gel column chromatography (solvent; hexane:ethyl acetate = 1:1, and then 1:4) to obtain the titled compound (60 mg).
¹H-NMR (400 MHz, CDCl₃) δ: 9.54 (s, 1H), 7.62 (dt, J = 9.0, 2.2 Hz, 2H), 7. 54 (dt, J = 9.1, 2. 0 Hz, 2H), 7.27 (d, J = 9.5 Hz, 1H), 7.00 (d, J = 9.6 Hz, 1H), 4.95 (s, 2H), 2.48 (tt, J = 11.9, 3.7 Hz, 1H), 1.73-1.69 (m, 2H), 1.64-1.57 (td, J = 12.8, 3.63 Hz, 2H), 1.55-1.46 (m, 2H), 1.28 (td, J = 13.2, 4.1 Hz, 2H), 0.94 (s, 3H), 0.93 (s, 3H).

### Examples 39 to 49

According to the methods of Example 37 and 38 and common reaction conditions, the compounds of Examples 39 to 49 were obtained by using corresponding material compounds.

| 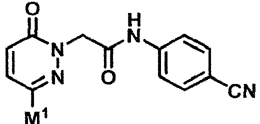 | | |
|---|---|---|
| Example | M¹ | LC-MS: [M+H] ⁺ / Rt (min) (Method) |
| 39 | 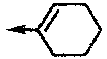 | 335.2 / 0.901 (Method A) |
| 40 | 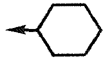 | 337.2 / 0.920 (Method A) |
| 41 | 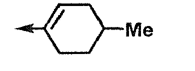 | 349.3 / 0.978 (Method A) |
| 42 | 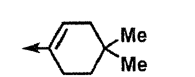 | 363.3 / 1.03 (Method A) |
| 43 | 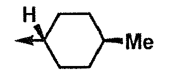 | 351.2 / 0.968 (Method A) |
| 44 | 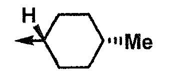 | 351.2 / 0.985 (Method A) |
| 45 | 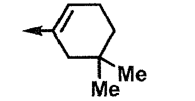 | 363.3 / 1.046 (Method A) |
| 46 | 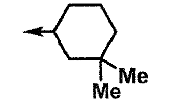 | 365.3 / 1.058 (Method A) |
| 47 | 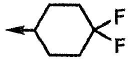 | 373.2 / 0.858 (Method A) |
| 48 | 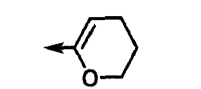 | 337.2 / 0.777 (Method A) |
| 49 | 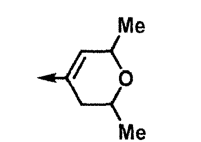 | 365.2 / 0.817 (Method A) |

### Example 50

### N-(1,3-Benzooxazol-5-yl)-2-[3-(methylpiperidin-1-yl)-6-oxopyridazin-1(6H)-yl]acetamide

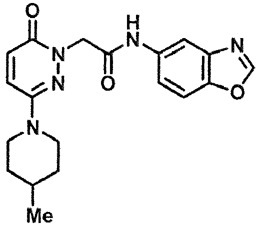

To a suspension of the compound of Reference example 6 (50 mg), 1,3-benzoxazole-5-amine (32 mg), and HATU (91 mg) in acetonitrile (1.5 mL) was added N,N-diisopropylethylamine (0.34 mL), and the mixture was stirred at room temperature for 2 hours. After the addition of saturated aqueous ammonium chloride, the mixture was extracted with ethyl acetate. The organic layer was dried over anhydrous sodium sulfate, filtered, and concentrated under reduced pressure. The residue was purified by silica gel column chromatography solvent; ethyl acetate:methanol = 100:0, and then 92:8) to obtain the titled compound (38 mg).
¹H-NMR (400 MHz, CDCl₃) δ: 9.35 (s, 1H), 8.09 (s, 1H), 8.06 (s, 1H), 7.48 (s, 2H), 7.23 (d, J = 9.8 Hz, 1H), 6.94 (d, J = 9.8 Hz, 1H), 4.86 (s, 2H), 3.86-3.83 (m, 2H), 2.80-2.74 (m, 2H), 1.73-1.70 (m, 2H), 1.61-1.53 (m, 1H), 1.28-1.18 (m, 2H), 0.97 (d, J = 6.4 Hz, 3H).

### Examples 51 to 99

According to the method of Example 50 and common reaction conditions, the compounds of Examples 51 to 99 were obtained by using corresponding material compounds.

| 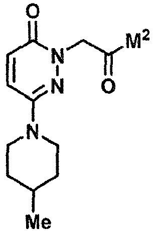 | | |
|---|---|---|
| Example | M² | Analytical data |
| 51 | 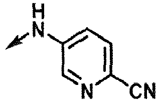 | ¹H-NMR (400 MHz, CDCl₃) δ: 10.14 (br, 1H), 8.63 (s, 1H), 8.29 (d, J = 8.5 Hz, 1H) , 7. 61 (d, J = 8.5 Hz, 1H) , 7.28-7.25 (m, 1H), 6.95 (d, J = 10.1 Hz, 1H), 4.87 (s, 2H), 3.87-3.84 (m, 2H) , 2.82-2.75 (m, 2H), 1. 74-1.71 (m, 2H), 1.60-1.54 (m, 1H), 1.28-1.17 (m, 2H), 0. 97 (d, J = 6.4 Hz, 3H) . |
| 52 | 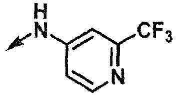 | ¹H-NMR (400 MHz, CDCl₃) δ: 10.21 (s, 1H), 8.50 (d, J = 5.5 Hz, 1H), 7.83 (s, 1H), 7.60 (d, J = 5.9 Hz, 1H) , 7.26 (d, *J* = 10.0 Hz, 1H), 6.95 (d, *J* = 9.6 Hz, 1H), 4.87 (s, 2H), 3.82 (d, *J* = 12.8 Hz, 2H), 2.76 (td, *J* = 12.7, 2.3 Hz, 2H), 1.70 (d, *J* = 12.3 Hz, 2H), 1.59-1.50 (m, 1H), 1.24 (dd, *J* = 12.1, 3.4 Hz, 1H), 1.18 (dd, *J* = 12.1, 3.4 Hz, 1H), 0.95 (d, *J* = 6.4 Hz, 3H). |
| 53 | 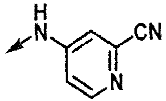 | ¹H-NMR (400 MHz, DMSO-d₆) δ: 11.01 (s, 1H), 8.59 (d, *J* = 5.5 Hz, 1H), 8.08 (d, *J* = 1.8 Hz, 1H), 7.76 (dd, *J* = 5.8, 2.1 Hz, 1H), 7.57 (d, *J* = 10.4 Hz, 1H), 6.85 (d, J = 9.8 Hz, 1H), 4.75 (s, 2H), 3.81 (d, *J* = 12.8 Hz, 2H), 2.66 (t, J = 11.3 Hz, 2H), 1.63 (d, *J* = 12.2 Hz, 2H), 1.55-1.46 (m, 1H), 1.15 (dd, J = 11.9, 4.0 Hz, 1H), 1.09 (dd, J = 12.5, 3.4 Hz, 1H), 0.90 (d, J = 6.1 Hz, 3H). |
| 54 | 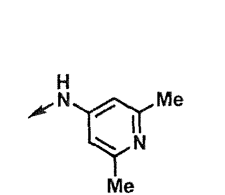 | ¹H-NMR (400 MHz, CDCl₃) δ: 7.34 (s, 2H), 7.26 (d, J = 10.3 Hz, 1H), 7.16 (br, 1H), 6.90 (d, J = 10.4 Hz, 1H) , 4.91 (s, 2H), 3.81 (d, J = 13.4 Hz, 2H), 2.75 (td, J = 12.8, 2.4 Hz, 2H), 2.54 (s, 6H), 1.70 (d, J = 12.8 Hz, 2H), 1.58-1.51 (m, 1H), 1.23 (dd, J = 12.2, 3.7 Hz, 1H), 1.17 (dd, J = 12.5, 3.4 Hz, 1H), 0.95 (d, J = 6.1 Hz, 3H) . |
| 55 | 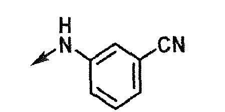 | LC-MS: [M+H]⁺ / Rt (min) 352.2 / 1.83 (Method B) |
| 56 | 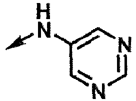 | LC-MS: [M+H]⁺ / Rt (min) 329.2 / 1.60 (Method B) |
| 57 | 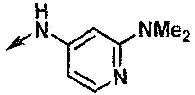 | LC-MS: [M+H]⁺ / Rt (min) 371.2 / 1.55 (Method B) |
| 58 | 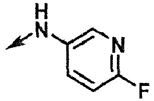 | LC-MS: [M+H]⁺ / Rt (min) 346.2 / 1.74 (Method B) |
| 59 | 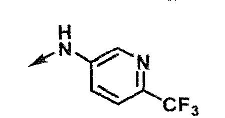 | ¹H-NMR (400 MHz, CDCl₃) δ: 9.95 (br, 1H), 8.64 (s, 1H), 8.30-8.28 (m, 1H), 7.60-7.56 (m, 1H), 7.26 (d, J = 10.1 Hz, 1H), 6.95 (d, J = 10.1, 1H), 4.87 (s, 2H), 3.86-3.83 (m, 2H) , 2.80-2.75 (m, 2H) , 1.72 (d, J = 12.3 Hz, 2H), 1.58-1.53 (m, 1H), 1.28-1.17 (m, 2H), 0.97 (d, J = 6.4 Hz, 3H). |
| 60 | 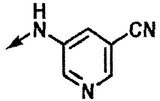 | ¹H-NMR (400 MHz, CDCl₃) δ: 10.06 (br, 1H), 8.70 (d, J = 2.1 Hz, 1H), 8.56 (d, J = 2.1 Hz, 1H), 8.52 (t, J = 2.1 Hz, 1H), 7.26 (d, J = 9.6 Hz, 1H), 6.95 (d, J = 9.6 Hz, 1H), 4.86 (s, 2H), 3.85 (d, J = 12.8 Hz, 2H), 2.78 (t, J = 12.8 Hz, 2H), 1.72 (d, J = 12.3 Hz, 2H), 1.60-1.55 (m, 1H), 1.28-1.18 (m, 2H), 0.97 (d, J = 6.4 Hz, 3H) . |
| 61 | 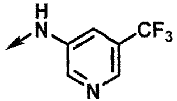 | 1H-NMR (400 MHz, CDCl₃) δ: 9.88 (br, 1H), 8.74 (s, 1H), 8.56 (s, 1H), 8.40 (s, 1H), 7.24 (d, J = 9.9 Hz, 1H), 6.94 (d, J = 9.9 Hz, 1H), 4.85 (s, 2H), 3.85-3.82 (m, 2H), 2.80-2.73 (m, 2H), 1.73-1.69 (m, 2H), 1.58-1.53 (m, 1H), 1.26-1.15 (m, 2H), 0.95 (d, J = 6.4 Hz, 3H). |
| 62 | 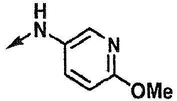 | LC-MS: [M+H]⁺ / Rt (min) 358.2 / 1.73 (Method B) |
| 63 | 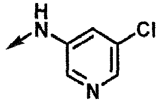 | ¹H-NMR (400 MHz, CDCl₃) δ: 9.76 (br, 1H), 8.41 (d, J = 1.8 Hz, 1H), 8.25 (d, J = 1.8 Hz, 1H), 8.22 (d, J = 1.8 Hz, 1H), 7.25 (d, J = 9.8 Hz, 1H), 6.94 (d, J = 9.8 Hz, 1H), 4.85 (s, 2H), 3.86-3.82 (m, 2H), 2.81-2.74 (m, 2H), 1.73-1.70 (m, 2H), 1.61-1.53 (m, 1H), 1.27-1.17 (m, 2H), 0.97 (d, J = 6.7 Hz, 3H). |
| 64 | 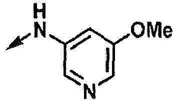 | LC-MS: [M+H]+ / Rt (min) 358.2 / 1.55 (Method B) |
| 65 | 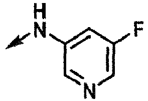 | LC-MS: [M+H]+ / Rt (min) 346.2 / 1.73 (Method B) |
| 66 | 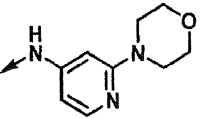 | ¹H-NMR (400 MHz, CDCl₃) δ: 9.37 (br, 1H), 8.04 (d, J = 5.5 Hz, 1H), 7.23 (d, J = 9.8 Hz, 1H) , 7.08 (d, J = 1.5 Hz, 1H), 6.92 (d, J = 9.8 Hz, 1H), 6.63 (dd, J = 5.5, 1.5 Hz, 1H), 4.81 (s, 2H), 3.85-3.78 (m, 6H) , 3.49-3.47 (m, 4H), 2.80-2.73 (m, 2H), 1.73-1.70 (m, 2H), 1.58-1.53 (m, 1H), 1.27-1.17 (m, 2H), 0.97 (d, J = 6.7 Hz, 3H). |
| 67 | 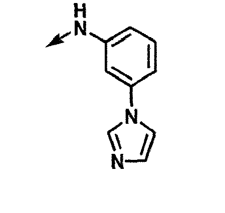 | ¹H-NMR (400 MHz, CDCl₃) δ: 9.57 (br, 1H) , 7.84-7.82 (m, 2H), 7.36-7.34 (m, 2H), 7.26 (d, J = 2. 7 Hz, 1H), 7.24 (d, J = 10.1 Hz, 1H), 7.18 (s, 1H), 7.10-7.06 (m, 1H), 6.94 (d, J = 10.1 Hz, 1H), 4.85 (s, 2H), 3.86-3.83 (m, 2H), 2.81-2.74 (m, 2H), 1.73-1.70 (m, 2H), 1.62-1.54 (m, 1H), 1.30-1.18 (m, 2H), 0.97 (d, J = 6.4 Hz, 3H). |
| 68 | 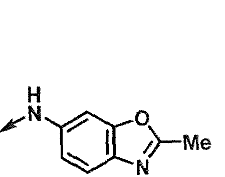 . | ¹H-NMR (400 MHz, CDCl₃) δ: 9.39 (br, 1H), 8.10 (d, J = 2.0 Hz, 1H), 7.50 (d, J = 8.6 Hz, 1H), 7.23 (d, J = 10.1 Hz, 1H), 7.12 (dd, J = 8.6, 2.0 Hz, 1H), 6.93 (d, J = 10.1 Hz, 1H), 4.85 (s, 2H), 3.86-3.83 (m, 2H), 2.81-2.74 (m, 2H), 2.61 (s, 3H), 1.73-1.70 (m, 2H), 1.60-1.52 (m, 1H), 1.28-1.18 (m, 2H), 0.97 (d, J = 6.4 Hz, 3H). |
| 69 | 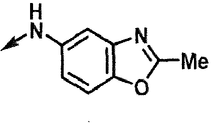 | ¹H-NMR (400 MHz, CDCl₃) δ: 9.23 (br, 1H) , 7.90 (s, 1H), 7.40-7.34 (m, 2H), 7.22 (d, J = 9.9 Hz, 1H), 6.93 (d, J = 9.9 Hz, 1H), 4.85 (s, 2H), 3.86-3.83 (m, 2H), 2.80-2.74 (m, 2H), 2.61 (s, 3H), 1.73-1.70 (m, 2H), 1.60-1.52 (m, 1H), 1.28-1.18 (m, 2H), 0.97 (d, J = 6.4 Hz, 3H). |
| 70 | 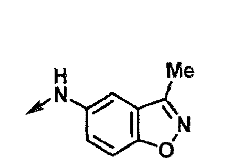 | ¹H-NMR (400 MHz, CDCl₃) δ: 9.50 (br, 1H), 8.08 (s, 1H), 7.43-7.42 (m, 2H), 7.24 (d, J = 10.1 Hz, 1H), 6.94 (d, J = 10.1 Hz, 1H), 4.87 (s, 2H), 3.87-3.83 (m, 2H), 2.781-2.74 (m, 2H), 2.53 (s, 3H), 1.74-1.70 (m, 2H), 1. 60-1. 53 (m, 1H), 1.28-1.18 (m, 2H), 0.97 (d, J = 6.4 Hz, 3H). |
| 71 | 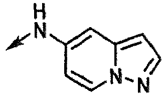 | ¹H-NMR (400 MHz, CDCl₃) δ: 9.59 (br, 1H), 8.32 (d, J = 7.8 Hz, 1H), 7.99 (d, J = 1.8 Hz, 1H), 7.87 (d, J = 2.3 Hz, 1H), 7.24 (d, J = 10.3 Hz, 1H), 6.94 (d, J = 10. 3 Hz, 1H), 6.72 (dd, J = 7.8, 2.3 Hz, 1H), 6.38 (d, J = 1.8 Hz, 1H), 4.85 (s, 2H), 3.87-3.83 (m, 2H), 2.81-2.74 (m, 2H), 1.74-1.70 (m, 2H), 1.61-1.53 (m, 1H), 1.28-1.18 (m, 2H), 0.97 (d, J = 6.9 Hz, 3H). |
| 72 | 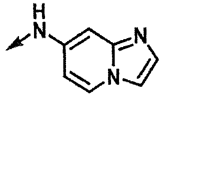 | ¹H-NMR (400 MHz, CDCl₃) δ: 9.72 (br, 1H), 7.95 (d, J = 7.3 Hz, 1H), 7.87 (br, 1H), 7.52 (br s, 1H), 7.43 (s, 1H), 7.23 (d, J = 10.1 Hz, 1H), 6.99 (dd, J = 7.3, 2.4 Hz, 1H), 6.92 (d, J = 10.1 Hz, 1H), 4.86 (s, 2H), 3.85-3.82 (m, 2H), 2.80-2.73 (m, 2H), 1.73-1.70 (m, 2H), 1.61-1.52 (m, 1H), 1.27-1.17 (m, 2H), 0.96 (d, J = 6.7 Hz, 3H). |
| 73 | 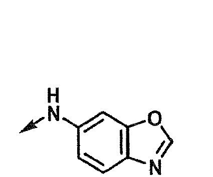 | ¹H-NMR (400 MHz, CDCl₃) δ: 9.52 (br, 1H), 8.24 (d, J = 1.8 Hz, 1H), 8.04 (s, 1H), 7.65 (d, J = 8.5 Hz, 1H), 7.23 (d, J = 10.4 Hz, 1H), 7.19 (dd, J = 8.5, 1.8 Hz, 1H), 6.94 (d, J = 10.4 Hz, 1H), 4.86 (s, 2H), 3.87-3.83 (m, 2H), 2.81-2.74 (m, 2H), 1.73-1.71 (m, 2H), 1.61-1.52 (m, 1H), 1.28-1.19 (m, 2H), 0.97 (d, J = 6.7 Hz, 3H). |
| 74 | 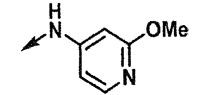 | LC-MS: [M+H]+ / Rt (min) 358.2 / 1.59 (Method B) |
| 75 | 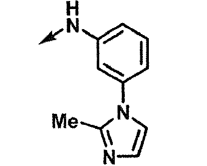 | LC-MS: [M+H]+ / Rt (min) 407.2 / 1.57 (Method B) |
| 76 | 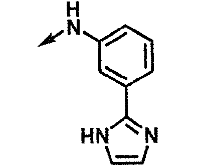 | LC-MS: [M+H]+ / Rt (min) 393.2 / 1.55 (Method B) |
| 77 | 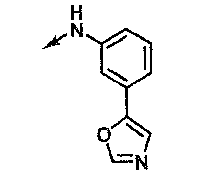 | LC-MS: [M+H]+ / Rt (min) 394.2 / 1.83 (Method B) |
| 78 | 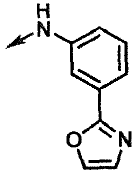 | LC-MS: [M+H]+ / Rt (min) 394.2 / 1.85 (Method B) |
| 79 | 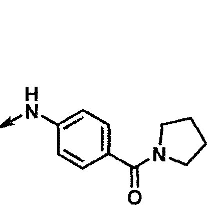 | ¹H-NMR (400 MHz, CDCl₃) δ: 9.36 (br, 1H), 7.55 (d, J = 8.5 Hz, 2H), 7.48 (d, J = 8.5 Hz, 2H), 7.22 (d, J = 10.1 Hz, 1H), 6.92 (d, J = 10.1 Hz, 1H), 4.83 (s, 2H), 3.85-3.82 (m, 2H), 3.63-3.60 (m, 2H), 3.44-3.41 (m, 2H), 2.79-2.73 (m, 2H), 1.95-1.90 (m, 2H), 1.88-1.84 (m, 2H), 1.73-1.69 (br m, 2H), 1.60-1.51 (br m, 1H), 1.27-1.18 (m, 2H), 0.96 (d, J = 6.4 Hz, 3H) . |
| 80 | 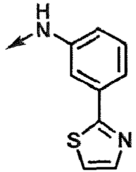 | ¹H-NMR (400 MHz, CDCl₃) δ: 9.21 (br, 1H), 8.06-8.05 (m, 1H), 7.85-7.84 (m, 1H), 7.72-7.68 (m, 2H), 7.40-7.36 (m, 1H), 7.33 (d, J = 3.1 Hz, 1H), 7.23 (d, J = 9.8 Hz, 1H), 6.94 (d, J = 9.8 Hz, 1H), 4.85 (s, 2H), 3.86-3.83 (m, 2H), 2.81-2.74 (m, 2H), 1.73-1.71 (m, 2H), 1.62-1.52 (m, 1H), 1.28-1.18 (m, 2H), 0.97 (d, J = 6.7 Hz, 3H). |
| 81 | 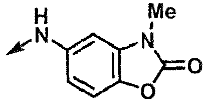 | ¹H-NMR (400 MHz, CDCl₃) δ: 9.40 (br, 1H), 7.63 (d, J = 2.1 Hz, 1H), 7.24 (d, J = 10.1 Hz, 1H), 7.07 (d, *J* = 8.5 Hz, 1H), 6.93 (d, J = 10.1 Hz, 1H), 6.92 (dd, J = 8.5, 2.1 Hz, 1H), 4.84 (s, 2H), 3.86-3.83 (m, 2H), 3.37 (s, 3H), 2.81-2.74 (m, 2H), 1.73-1.71 (br, 2H), 1.62-1.53 (m, 1H), 1.28-1.17 (m, 2H), 0.97 (d, J = 6.7 Hz, 3H). |
| 82 | 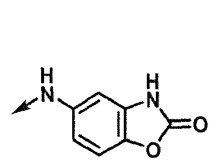 | ¹H-NMR (400 MHz, CD3OD) δ: 7.60 (br, 1H), 7.52 (d, J = 10.1 Hz, 1H), 7.15 (d, J = 8.7 Hz, 1H), 7.10 (d, J = 8.7 Hz, 1H), 6.89 (d, J = 10.1 Hz, 1H), 4.82 (s, 2H), 3.93-3.90 (m, 2H), 2.81-2.73 (m, 2H), 1.71-1.70 (m, 2H), 1.56 (br, 1H), 1.28-1.18 (m, 2H), 0.96 (d, J = 6.4 Hz, 3H). |
| 83 | 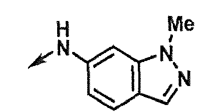 | ¹H-NMR (400 MHz, CDCl₃) δ: 9.43 (br, 1H), 8.09 (s, 1H), 7.87 (s, 1H), 7.58 (d, J = 8.5 Hz, 1H), 7.23 (d, J = 10.1 Hz, 1H), 6.94 (d, J = 10.1 Hz, 1H), 6.89 (dd, J = 8.5, 1.2 Hz, 1H), 4.87 (s, 2H), 4.02 (s, 3H), 3.86-3.83 (m, 2H), 2.81-2.74 (m, 2H), 1.73-1.70 (m, 2H), 1.62-1.52 (m, 1H), 1.28-1.18 (m, 2H), 0.97 (d, J = 6.7 Hz, 3H). |
| 84 | 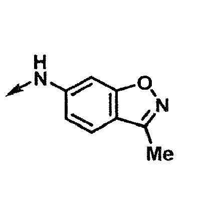 | ¹H-NMR (400 MHz, CDCl₃) δ: 9.71 (br, 1H), 8.08 (s, 1H), 7.47 (d, J = 8.7 Hz, 1H), 7.24 (d, J = 10.3 Hz, 1H), 7.20 (d, J = 8.7 Hz, 1H), 6.94 (d, J=10.3Hz, 1H), 4.86 (s, 2H), 3.86-3.83 (m, 2H), 2.81-2.74 (m, 2H), 2.53 (s, 3H), 1.73-1.70 (m, 2H), 1.60-1.53 (m, 1H), 1.28-1.17 (m, 2H), 0.97 (d, J = 6.4 Hz, 3H). |
| 85 | 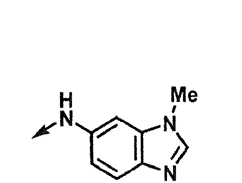 | ¹H-NMR (400 MHz, CDCl₃) δ: 9.37 (br, 1H), 8.08 (s, 1H), 7.77-7.75 (br, 1H), 7.65-7.62 (m, 1H), 7.23 (d, J = 10.1 Hz, 1H), 6.99-6.97 (br, 1H), 6.94 (d, J = 10.1 Hz, 1H), 4.88 (s, 2H), 3.86-3.83 (m, 2H), 3.76 (br, 3H), 2.80-2.74 (m, 2H), 1.73-1.70 (br, 2H), 1.60-1.52 (m, 1H), 1.28-1.18 (m, 2H), 0. 97 (d, J = 6. 9 Hz, 3H) . |
| 86 | 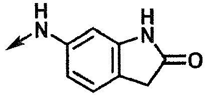 | ¹H-NMR (400 MHz, CD3OD) δ: 7.52 (d, J = 10.1 Hz, 1H), 7.39 (d, J = 1.8 Hz, 1H), 7.17 (d, J = 7.9 Hz, 1H), 7.03 (dd, J = 7.9, 1.8 Hz, 1H), 6.89 (d, J = 10.1 Hz, 1H), 4.82 (s, 2H), 3.93-3.90 (m, 2H), 3.47 (s, 2H), 2.80-2.73 (m, 2H), 1.72-1.69 (m, 2H), 1.61-1.52 (m, 1H), 1.28-1.18 (m, 2H), 0.96 (d, J = 6.1 Hz, 3H). |
| 87 | 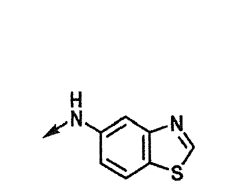 | ¹H-NMR (400 MHz, CDCl₃) δ: 9.43 (br, 1H), 8.98 (s, 1H), 8.38 (d, J = 1.8 Hz, 1H), 7.84 (d, J = 8.5 Hz, 1H), 7.61 (dd, J = 8.5, 1.8 Hz, 1H), 7.23 (d, J = 10.1 Hz, 1H), 6.95 (d, J = 10.1 Hz, 1H), 4.88 (s, 2H), 3.87-3.83 (m, 2H), 2.81-2.74 (m, 2H), 1.73-1.70 (m, 2H), 1.62-1.53 (m, 1H), 1.28-1.18 (m, 2H), 0.97 (d, J = 6.1 Hz, 3H). |
| 88 | 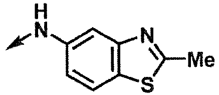 | ¹H-NMR (400 MHz, CDCl₃) δ: 9.30 (br, 1H), 8.18 (d, J = 1.8 Hz, 1H), 7.70 (d, J = 8.6 Hz, 1H), 7.51 (dd, J = 8.6, 1.8 Hz, 1H), 7.22 (d, J = 10.1 Hz, 1H), 6.93 (d, J = 10.1 Hz, 1H), 4.86 (s, 2H), 3.86-3.83 (m, 2H), 2.81 (s, 3H) , 2.80-2.73 (m, 2H) , 1.73-1.70 (m, 2H), 1.61-1.52 (m, 1H), 1.28-1.18 (m, 2H), 0.96 (d, J = 6.7 Hz, 3H). |
| 89 | 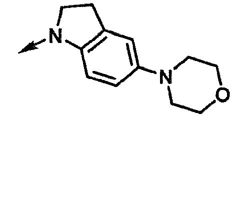 | ¹H-NMR (DMSO-d₆) δ: 7.83 (d, J = 9.2 Hz, 1H), 7.56 (d, J = 9.8 Hz, 1H), 6.89 (s, 1H), 6.85 (d, J = 9.8 Hz, 1H), 6.73 (dd, J = 8.5, 2.4 Hz, 1H), 4.82 (s, 2H), 4.18-4.10 (m, 2H), 3.83-3.77 (m, 2H), 3.74-3.68 (m, 4H), 3.33-3.27 (m, 1H), 3.19-3.10 (m, 2H), 3.07-3.00 (m, 4H), 2.70-2.60 (m, 2H), 1.68-1.59 (m, 2H), 1.56-1.45 (br, 1H), 1.20-1.08 (m, 2H), 0.91 (3H, d, J = 6.7 Hz). |
| 90 | 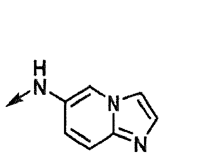 | ¹H-NMR (400 MHz, CD3OD) δ: 9.21 (s, 1H), 7.89 (s, 1H), 7.62-7.53 (m, 3H), 7.35 (d, J = 9.5 Hz, 1H), 6.90 (d, J = 9.5 Hz, 1H), 4.87 (s, 2H), 3.94-3.90 (m, 2H), 2.80-2.74 (m, 2H), 1.72-1.68 (m, 2H), 1.57 (s, 1H), 1.28-1.17 (m, 2H), 0.96 (d, J = 6.7 Hz, 3H). |
| 91 | 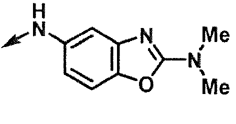 | ¹H-NMR (400 MHz, CDCl₃) δ: 8.97 (br, 1H), 7.52 (s, 1H), 7.20 (d, J = 9.8 Hz, 1H), 7.14-7.14 (br, 2H), 6.91 (d, J = 9.8 Hz, 1H), 4.83 (s, 2H), 3.85-3.82 (m, 2H), 3.18 (s, 6H), 2.79-2.72 (m, 2H), 1.72-1.69 (m, 2H), 1.61-1.51 (m, 1H), 1.28-1.18 (m, 2H), 0.97 (d, J = 6.7 Hz, 3H). |
| 92 | 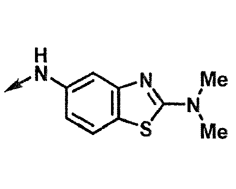 | ¹H-NMR (400 MHz, CDCl₃) δ: 9.38 (br, 1H), 7.63 (d, J = 2.4 Hz, 1H), 7.42 (dd, J = 8.5, 2.4 Hz, 1H), 7.24 (d, J = 10.1 Hz, 1H), 7.20 (d, J = 8.5 Hz, 1H), 6.93 (d, J = 10.1 Hz, 1H), 4.82 (s, 2H), 3.86-3.83 (m, 2H), 3.28 (s, 3H), 2.81-2.74 (m, 2H), 2.46 (s, 3H), 1.73-1.70 (m, 2H), 1.61-1.52 (m, 1H), 1.28-1.18 (m, 2H), 0.97 (d, J = 6.1 Hz, 3H). |
| 93 | 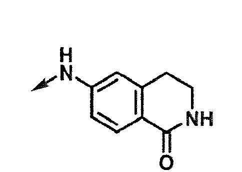 | ¹H-NMR (400 MHz, CDCl₃) δ: 9.48 (br, 1H), 7.99 (d, J = 8.5 Hz, 1H), 7.63 (d, J = 1.8 Hz, 1H), 7.31 (dd, J = 8.5, 1.8 Hz, 1H), 7.23 (d, J = 9.9 Hz, 1H), 6.93 (d, J = 9.9 Hz, 1H), 5.77 (s, 1H), 4.83 (s, 2H), 3.86-3.82 (m, 2H), 3.55-3.51 (m, 2H), 2.98-2.95 (m, 2H), 2.81-2.74 (m, 2H), 1.73-1.71 (m, 2H), 1.61-1.54 (m, 1H), 1.28-1.17 (m, 2H), 0.97 (d, J = 6.9 Hz, 3H). |
| 94 | 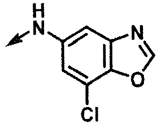 | ¹H-NMR (400 MHz, CDCl₃) δ: 9.53 (br, 1H), 8.09 (s, 1H), 7.95 (d, J = 1.8 Hz, 1H), 7.62 (d, J = 1.8 Hz, 1H), 7.24 (d, J = 10.1 Hz, 1H), 6.95 (d, *J* = 10.1 Hz, 1H), 4.85 (s, 2H), 3.87-3.83 (m, 2H), 2.81-2.74 (m, 2H), 1.74-1.71 (m, 2H), 1.62-1.50 (m, 1H), 1.28-1.18 (m, 2H), 0.97 (d, J = 6.9 Hz, 3H). |
| 95 | 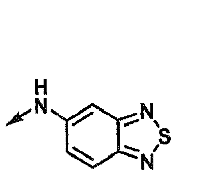 | ¹H-NMR (CDCl₃) δ: 9.80 (s, 1H), 8.50 (d, J = 2.0 Hz, 1H), 7.88 (d, J = 9.9 Hz, 1H), 7.50 (dd, J = 9.1, 2.0 Hz, 1H), 7.25 (d, *J* = 9.1 Hz, 1H), 6.96 (d, J = 9.9 Hz, 1H), 4.89 (s, 2H), 3.87-3.84 (m, 2H), 2.82-2.75 (m, 2H), 1.74-1.71 (m, 2H), 1.61-1.53 (m, 1H), 1.28-1.18 (m, 2H), 0.97 (d, J = 6.9 Hz, 3H). |
| 96 | 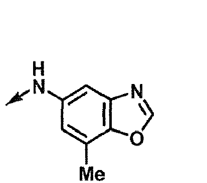 | ¹H-NMR (400 MHz, CDCl₃) δ: 9.25 (br, 1H), 8.04 (s, 1H), 7.89 (s, 1H), 7.28 (br, 1H), 7.22 (d, *J* = 10.1 Hz, 1H), 6.94 (d, *J* = 10.1 Hz, 1H), 4.85 (s, 2H), 3.86-3.83 (m, 2H), 2.81-2.74 (m, 2H), 2.49 (s, 3H), 1.73-1.70 (br, 2H), 1.61-1.53 (m, 1H), 1.28-1.18 (m, 2H), 0.97 (d, J = 6.7 Hz, 3H) . |
| 97 | 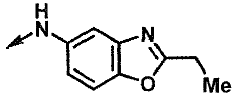 | ¹H-NMR (400 MHz, CDCl₃) δ: 9.20 (br, 1H), 7.92 (d, *J* = 1.2 Hz, 1H), 7.40-7.35 (m, 2H), 7.22 (d, J = 10.4 Hz, 1H), 6.93 (d, J = 10.4 Hz, 1H), 4.85 (s, 2H), 3.86-3.83 (br, 2H), 2.94 (q, J = 7.7 Hz, 2H), 2.80-2.74 (m, 2H), 1.73-1.70 (br, 2H), 1.58-1.53 (m, 1H), 1.43 (t, J = 7.7 Hz, 3H), 1.28-1.18 (m, 2H), 0.97 (d, J = 6.7 Hz, 3H). |
| 98 | 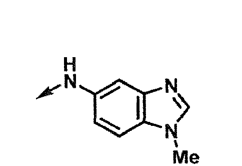 | ¹H-NMR (400 MHz, CDCl₃) δ: 9.08 (s, 1H), 7.93 (s, 1H), 7.83 (s, 1H), 7.49 (d, J = 8.5 Hz, 1H), 7.29-7..26 (m, 1H), 7.20 (d, J = 9.8 Hz, 1H), 6.92 (d, J = 9.8 Hz, 1H), 4.86 (s, 2H), 3.85-3.82 (m, 2H), 3.80 (s, 3H), 2.79-2.73 (m, 2H), 1.73-1.70 (m, 2H), 1.60-1.51 (m, 1H), 1.28-1.18 (m, 2H), 0. 96 (d, J = 6.7 Hz, 3H) . |
| 99 | 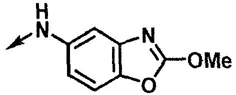 3H) | ¹H-NMR (400 MHz, CDCl₃) δ: 9.19 (br, 1H), 7.79 (s, 1H), 7.24 (s, 1H), 7.23 (s, 1H), 7.22 (d, J = 10.4 Hz, 1H), 6.92 (d, J = 10.4 Hz, 1H), 4.84 (s, 2H), 4.20 (s, 3H), 3.85-3.82 (m, 2H), 2.80-2.73 (m, 2H), 1.73-1.70 (m, 2H), 1.61-1.52 (m, 1H), 1.28-1.17 (m, 2H), 0.97 (d, J = 6.7 Hz, 3H) . |

### Examples 100 to 135

According to the methods of Examples 2, 37, 38, and 50 and corresponding reaction conditions, the compounds of Examples 100 to 135 were obtained by using corresponding material compounds.

| Example | Chemical structure | Analytical data |
|---|---|---|
| 100 | 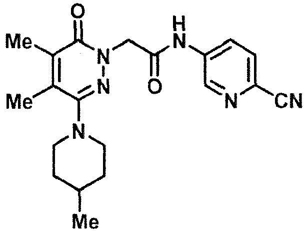 | ¹H-NMR (DMSO-d6) δ: 10.97 (br, 1H), 8.85 (d, J = 2.7 Hz, 1H), 8.22 (dd, J = 8.5, 2.5 Hz, 1H), 7.99 (d, J = 8.2 Hz, 1H), 4.81 (s, 2H), 3.20-3.13 (m, 2H), 2.59-2.52 (m, 2H), 2.14 (3H, s), 2.03 (3H, s), 1.72-1.64 (m, 2H), 1.53-1.41 (br, 1H), 1.31-1.19 (m, 2H), 0.94 (3H, d, J = 6.4 Hz). |
| 101 | 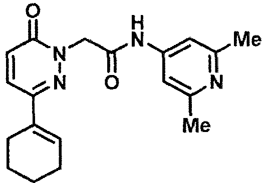 | LC-MS: [M+H]+ / Rt (min) 339.2 / 0.638 (Method A) |
| 102 | 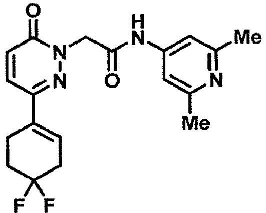 | LC-MS: [M+H]+ / Rt (min) 375.3 / 0.595 (Method A) |
| 103 | 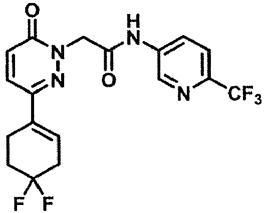 | ¹H-NMR (400 MHz, CDCl₃) δ: 9.51 (s, 1H), 8.60 (d, J = 2.3 Hz, 1H), 8.26 (dd, J = 8.7, 2.3 Hz, 1H), 7.63 (d, J = 10.1 Hz, 1H), 7.54 (d, J = 8.2 Hz, 1H), 7.03 (d, J = 9.6 Hz, 1H), 6.21 (br, 1H), 5.00 (s, 2H), 2.79-2.68 (m, 4H), 2.19-2.06 (m, 2H). |
| 104 | 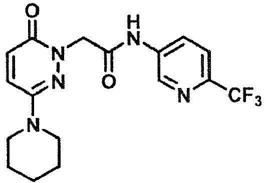 | LC-MS: [M+H]+ / Rt (min) 382.2 / 0.860 (Method A) |
| 105 | 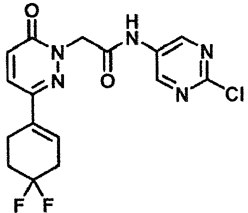 | LC-MS: [M+H]+ / Rt (min) 382.2 / 0.771 (Method A) |
| 106 | 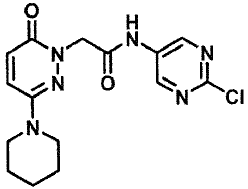 | LC-MS: [M+H]+ / Rt (min) 349.2 / 0.734 (Method A) |
| 107 | 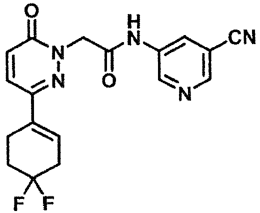 | ¹H-NMR (400 MHz, CDCl₃) δ: 9.59 (s, 1H), 8.66 (d, J = 2.4 Hz, 1H), 8.54 (d, J = 1.8 Hz, 1H), 8.44 (t, J = 2.1 Hz, 1H), 7.64 (d, J = 9.8 Hz, 1H), 7.04 (d, J = 9.8 Hz, 1H), 6.22 (1H, bs), 5.00 (s, 2H), 2.79-2.70 (m, 4H), 2.19-2.09 (m, 2H). |
| 108 | 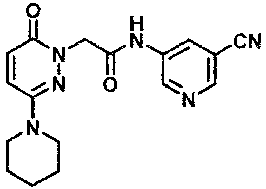 | LC-MS: [M+H]+ / Rt (min) 339.2 / 0.713 (Method A) |
| 109 | 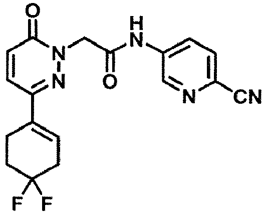 | ¹H-NMR (400 MHz, DMSO-d₆) δ: 11.03 (s, 1H), 8.85 (d, J = 2.7 Hz, 1H), 8.21 (dd, J = 8.7, 1.8 Hz, 1H), 7.99 (d, J = 8.7 Hz, 1H), 7.93 (d, J = 10.1 Hz, 1H), 6.97 (d, J = 9.6 Hz, 1H), 6.45 (br, 1H), 4.94 (s, 2H), 2.78 (t, J = 13.7 Hz, 2H), 2.63-2.57 (m, 2H), 2.18-2.07 (m, 2H). |
| 110 | 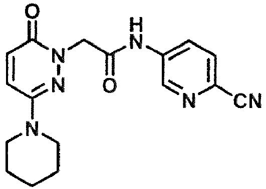 | LC-MS: [M+H]+ / Rt (min) 339.2 / 0.743 (Method A) |
| 111 | 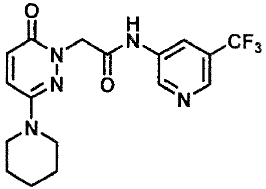 | LC-MS: [M+H]+ / Rt (min) 382.3 / 0.840 (Method A) |
| 112 | 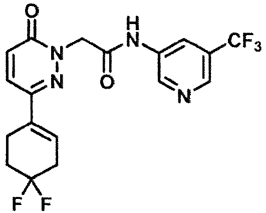 | ¹H-NMR (400 MHz, CDCl₃) δ: 9.45 (s, 1H), 8.64 (s, 1H), 8.49 (s, 1H), 8.31 (s, 1H), 7.58 (dd, J = 10.1, 2.1 Hz, 1H), 6.99 (dd, J = 9.8, 1.8 Hz, 1H), 6.17 (br, 1H), 4.96 (s, 2H), 2.74-2. 64 (m, 4H), 2.13-2.03 (m, 2H). |
| 113 | 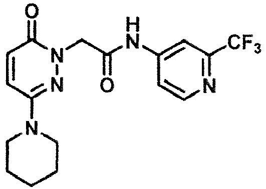 | LC-MS: [M+H]+ / Rt (min) 382.3 / 0.845 (Method A) |
| 114 | 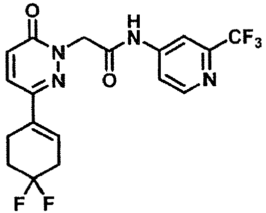 | ¹H-NMR (400 MHz, CDCl₃) δ: 9.66 (s, 1H), 8.51 (d, J = 5.9 Hz, 1H), 7.78 (d, J = 1.8 Hz, 1H), 7.64 (d, J = 9.6 Hz, 1H), 7.57 (dd, J = 5.3, 2.1 Hz, 1H), 7.04 (d, J = 9.6 Hz, 1H), 6.22 (br, 1H), 4.99 (s, 2H), 2.79-2.70 (m, 4H), 2.18-2.08 (m, 2H). |
| 115 | 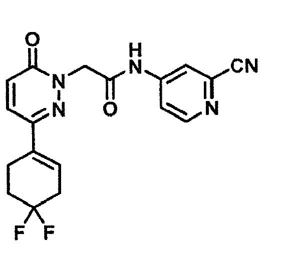 | ¹H-NMR (400 MHz, DMSO-d₆) δ: 11.12 (s, 1H), 8.60 (d, J = 5.9 Hz, 1H), 8.08 (d, J = 2.3 Hz, 1H), 7.94 (d, J = 10.1 Hz, 1H), 7.76 (dd, J = 5.5, 2.3 Hz, 1H), 6.99 (d, J = 10. 1 Hz, 1H), 6.45 (br, 1H), 4.94 (s, 2H), 2.78 (t, J = 13.7 Hz, 2H), 2.63-2.57 (m, 2H), 2.18-2.08 (m, 2H). |
| 116 | 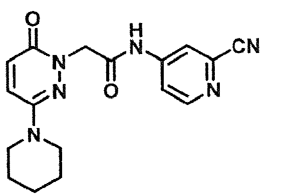 | LC-MS: [M+H] + / Rt (min) 339.2 / 0.743 (Method A) |
| 117 | 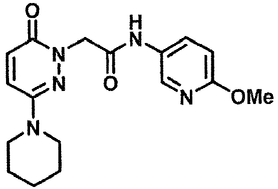 | LC-MS: [M+H] + / Rt (min) 344.3 / 0.721 (Method A) |
| 118 | 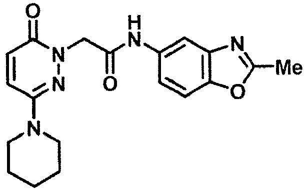 | LC-MS: [M+H] + / Rt (min) 368.3 / 0.747 (Method A) |
| 119 | 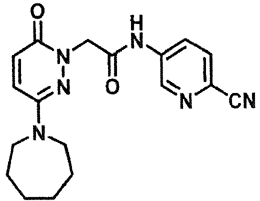 | ¹H-NMR (400 MHz, CDCl₃) δ: 10.29 (s, 1H), 8.64 (d, J = 1.8 Hz, 1H), 8.28 (dd, J = 8.7, 2.7 Hz, 1H), 7.59 (d, J = 8.7 Hz, 1H), 7.15 (d, J = 10.1 Hz, 1H), 6.95 (d, J = 10.1 Hz, 1H), 4.87 (s, 2H), 3.47 (t, J = 5.9 Hz, 4H), 1.73 (br, 4H), 1.56-1.53 (m, 4H). |
| 120 | 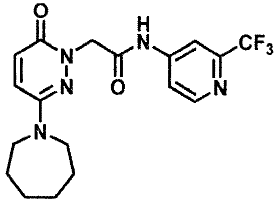 | ¹H-NMR (400 MHz, CDCl₃) δ: 10.31 (s, 1H), 8.54 (d, J = 5.5 Hz, 1H), 7.85 (d, J = 1.8 Hz, 1H), 7.66 (dd, J = 5.5, 1.8 Hz, 1H), 7.15 (d, J = 10.1 Hz, 1H), 6.95 (d, J = 10.1 Hz, 1H), 4.86 (s, 2H), 3.47 (t, J = 5.9 Hz, 4H), 1.73 (br, 4H), 1.56-1.53 (m, 4H). |
| 121 | 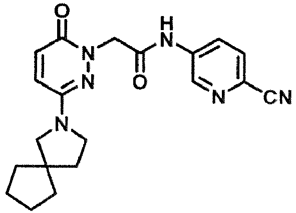 | ¹H-NMR (400 MHz, DMSO-d₆) δ: 10.91 (s, 1H), 8.85 (d, J = 2.4 Hz, 1H), 8.22 (dd, J = 8.5, 2.4 Hz, 1H), 7.99 (d, J = 9.2 Hz, 1H), 7.26 (d, J = 9.8 Hz, 1H), 6.85 (d, J = 10.4 Hz, 1H) , 4.75 (s, 2H), 3.36 (t, J = 6.7 Hz, 2H), 3.16 (s, 2H), 1.80 (t, J = 6.7 Hz, 2H), 1.68-1.48 (m, 8H). |
| 122 | 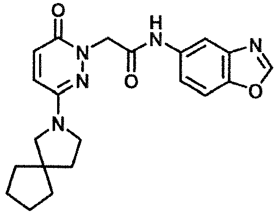 | ¹H-NMR (400 MHz, DMSO-d₆) δ: 10.35 (s, 1H), 8.70 (s, 1H), 8.12 (d, J = 2.4 Hz, 1H), 7.72 (d, J = 8.5 Hz, 1H), 7.51 (dd, J = 8.9, 2.1 Hz, 1H), 7.25 (d, J = 9.8 Hz, 1H), 6.85 (d, J = 9.8 Hz, 1H), 4.71 (s, 2H), 3.40-3.34 (m, 2H), 3.17 (s, 2H), 1.80 (t, J = 7.0 Hz, 2H), 1.68-1.49 (m, 8H). |
| 123 | 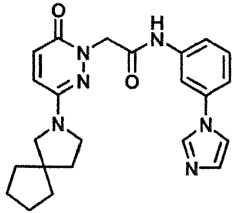 | ¹H-NMR (400 MHz, DMSO-d₆) δ: 10.43 (s, 1H), 8.14 (s, 1H), 7.89 (s, 1H), 7.62 (s, 1H), 7.49-7.44 (m, 2H), 7.35-7.31 (m, 1H), 7.24 (d, J = 9.6 Hz, 1H), 7.11 (s, 1H), 6.85 (d, J = 10.1 Hz, 1H), 4.71 (s, 2H), 3.38-3.34 (m, 2H), 3.16 (s, 2H), 1.80 (t, J = 6.9 Hz, 2H), 1.64-1.53 (m, 8H) . |
| 124 | 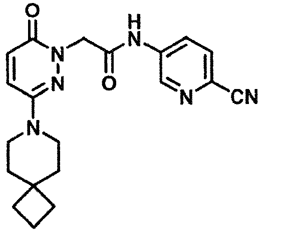 | ¹H-NMR (400 MHz, DMSO-d₆) δ: 10.92 (s, 1H), 8.85 (d, J = 2.3 Hz, 1H), 8.22 (dd, J = 8.2, 2.3 Hz, 1H), 7.99 (d, J = 8.7 Hz, 1H), 7.58 (d, J = 10.1 Hz, 1H), 6.85 (d, J = 10.1 Hz, 1H), 4.76 (s, 2H), 3.17-3.15 (m, 4H), 1.88-1.82 (m, 2H), 1.76-1.72 (m, 4H), 1.59-1.56 (m, 4H). |
| 125 | 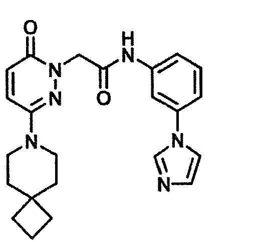 | ¹H-NMR (400 MHz, DMSO-d₆) δ: 10.45 (s, 1H), 8.14 (s, 1H), 7.89 (s, 1H), 7.62 (s, 1H), 7.57 (d, J = 10.1 Hz, 1H), 7.49-7.44 (m, 2H), 7.34-7.31 (m, 1H), 7.10 (s, 1H), 6.85 (d, J = 10.1 Hz, 1H), 4.72 (s, 2H), 3.16 (m, 4H), 1.89-1.82 (m, 2H), 1.77-1.72 (m, 4H), 1.58 (m, 4H). |
| 126 | 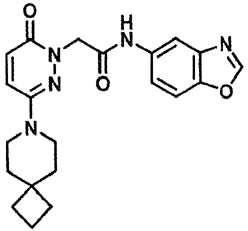 | ¹H-NMR (400 MHz, DMSO-d₆) δ: 10.37 (s, 1H), 8.71 (s, 1H), 8.11 (d, J = 1.8 Hz, 1H), 7.72 (d, J = 8.5 Hz, 1H), 7.57 (d, J = 9.8 Hz, 1H), 7.51 (dd, J = 8.9, 2.1 Hz, 1H), 6.85 (d, J = 9.8 Hz, 1H), 4.72 (s, 2H), 3.18-3.15 (m, 4H), 1.90-1.82 (m, 2H), 1.77-1.72 (m, 4H), 1.60-1.56 (m, 4H). |
| 127 | 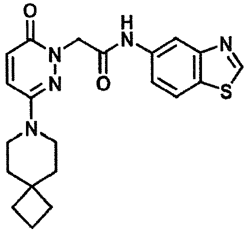 | ¹H-NMR (400 MHz, DMSO-d₆) δ: 10.45 (s, 1H), 9.37 (s, 1H), 8.44 (d, J = 1.8 Hz, 1H), 8.09 (d, J = 9.2 Hz, 1H), 7. 61-7.56 (m, 2H), 6.86 (d, J = 10.4 Hz, 1H), 4.75 (s, 2H), 3.19-3.14 (m, 4H), 1.90-1.82 (m, 2H), 1.77-1.72 (m, 4H), 1.60-1.56 (m, 4H). |
| 128 | 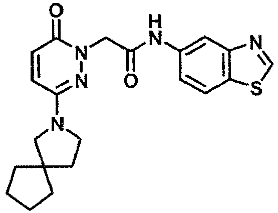 | ¹H-NMR (400 MHz, DMSO-d₆) δ: 10.43 (s, 1H), 9.37 (s, 1H), 8.45 (d, J = 1.8 Hz, 1H), 8.09 (d, J = 8.7 Hz, 1H), 7.60 (dd, J = 8.7, 1.8 Hz, 1H), 7.26 (d, J = 10.1 Hz, 1H), 6.86 (d, J = 9.6 Hz, 1H), 4.74 (s, 2H), 3.40-3.36 (m, 2H), 3.18 (s, 2H), 1.83-1.78 (m, 2H), 1.66-1.52 (m, 8H). |
| 129 | 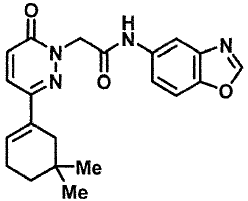 | ¹H-NMR (400 MHz, DMSO-d₆) δ: 10.47 (s, 1H), 8.70 (s, 1H) , 8.10 (d, J = 1.8 Hz, 1H), 7.89 (d, J = 9.8 Hz, 1H), 7.72 (d, J = 9.2 Hz, 1H), 7.51 (dd, J = 9.2, 1.8 Hz, 1H), 6.93 (d, J = 9.8 Hz, 1H), 6.56-6.52 (m, 1H), 4.88 (s, 2H), 2.27-2.20 (m, 2H) , 2.14 (s, 2H), 0.92 (s, 6H). |
| 130 | 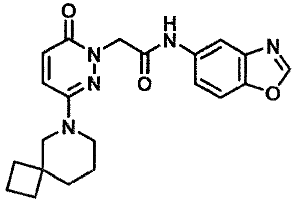 | ¹H-NMR (400 MHz, DMSO-d₆) δ: 10.36 (s, 1H), 8.69 (s, 1H), 8.10 (d, J = 1.8 Hz, 1H), 7.71 (d, J = 9.2 Hz, 1H), 7.61 (d, J = 10.6 Hz, 1H), 7.51 (dd, J = 9.2, 1.8 Hz, 1H), 6.84 (d, J = 9.8 Hz, 1H), 4.72 (s, 2H), 3.15-3.10 (m, 5H), 1.94-1.46 (m, 11H) . |
| 131 | 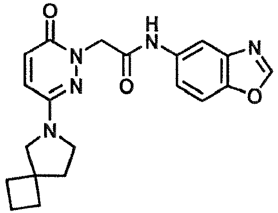 | ¹H-NMR (400 MHZ, DMSO-d₆) δ: 10.33 (s, 1H), 8.70 (d, J = 1.2 Hz, 1H), 8.11 (s, 1H), 7.71 (d, J = 8.7 Hz, 1H), 7.51 (d, J = 9.1 Hz, 1H), 7.23 (d, J = 10.1 Hz, 1H), 6.84 (d, J = 10.1 Hz, 1H), 4.70 (s, 2H), 3.33-3.26 (m, 3H), 2.03-1.80 (m, 9H) . |
| 132 | 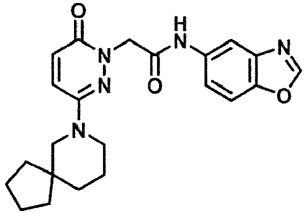 | ¹H-NMR (400 MHz, DMSO-d₆) δ: 10.37 (s, 1H), 8.71 (s, 1H), 8.11 (d, J = 1.8 Hz, 1H), 7.72 (d, J = 8.7 Hz, 1H), 7.56 (d, J = 10.1 Hz, 1H), 7.52 (dd, J = 8.9, 1.8 Hz, 1H), 6.84 (d, J = 10.1 Hz, 1H), 4.72 (s, 2H), 3.21-3.18 (m, 2H), 3.00 (s, 2H), 1.63-1.52 (m, 6H), 1.49-1.43 (m, 4H), 1.33-1.27 (m, 2H) . |
| 133 | 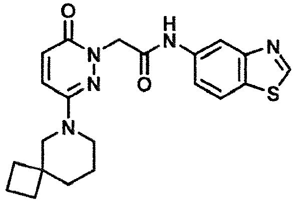 | ¹H-NMR (400 MHz, CDCl₃) δ: 9.42 (br, 1H), 8.98 (s, 1H), 8.39 (d, J = 2.0 Hz, 1H), 7.85 (d, J = 8.7 Hz, 1H), 7.62 (dd, J = 8.7, 2.0 Hz, 1H), 7.26 (d, J = 9.8 Hz, 1H), 6.95 (d, J = 9.8 Hz, 1H), 4.89 (s, 2H), 3.21 (br s, 2H), 3.18 (s, 2H), 1.94-1.88 (m, 2H), 1.79-1.73 (m, 4H), 1.60-1.59 (m, 4H). |
| 134 | 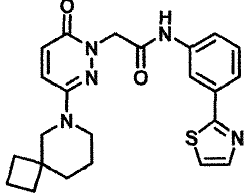 | LC-MS: [M+H]+ / Rt (min) 436.2 / 2.00 (Method B) |
| 135 | 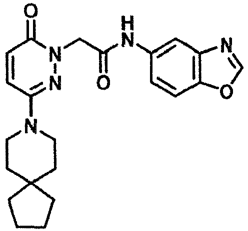 | ¹H-NMR (400 MHz, DMSO-d₆) δ: 10.38 (s, 1H) , 8.71 (s, 1H), 8.12 (d, J = 1.8 Hz, 1H) , 7.72 (d, J = 8.5 Hz, 1H), 7.58 (d, J = 10.4 Hz, 1H), 7.52 (dd, J = 8.5, 1.8 Hz, 1H), 6.86 (d, J = 10.4 Hz, 1H) , 4.73 (s, 2H), 3.25-3.21 (m, 4H), 1.60-1.56 (m, 4H), 1.48-1.40 (m, 8H) . |

### Examples 136 to 159

According to the methods of Example 37 or 50 and common reaction conditions, the compounds of Examples 136 to 159 were obtained by using corresponding material compounds.

| 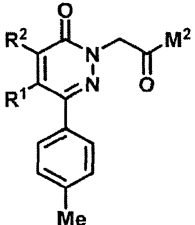 | | | | |
|---|---|---|---|---|
| Example | M² | R¹ | R² | Analytical data |
| 136 | 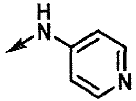 | H | H | LC-MS:[M+H]⁺ / Rt (min) 322.2 / 0.613 (Method A) |
| 137 | 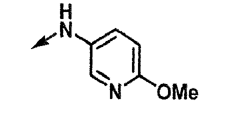 | H | H | LC-MS:[M+H]⁺ / Rt (min) 322.2 / 0.613 (Method A) |
| 138 | 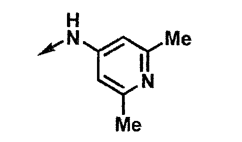 | H | H | LC-MS:[M+H]⁺ / Rt (min) 322.2 / 0.613 (Method A) |
| 139 | 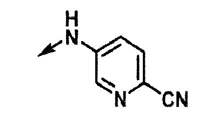 | H | H | LC-MS:[M+H]⁺ / Rt (min) 322.2 / 0.613 (Method A) |
| 140 | 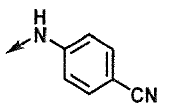 | H | H | LC-MS:[M+H]⁺ / Rt (min) 322.2 / 0.613 (Method A) |
| 141 | 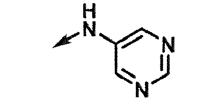 | H | H | LC-MS:[M+H]⁺ / Rt (min) 322.2 / 0.613 (Method A) |
| 142 | 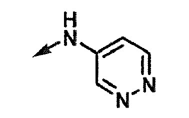 | H | H | LC-MS:[M+H]⁺ / Rt (min) 322.2 / 0.613 (Method A) |
| 143 | 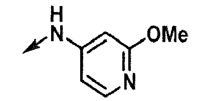 | H | H | LC-MS: [M+H] ⁺ / Rt (min) 322.2 / 0.613 (Method A) |
| 144 | 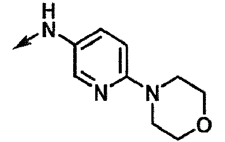 | H | H | LC-MS : [M+H] ⁺ / Rt (min) 322.2 / 0.613 (Method A) |
| 145 | 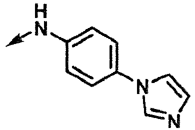 | H | H | LC-MS:[M+H]⁺ / Rt (min) 322.2 / 0.613 (Method A) |
| 146 | 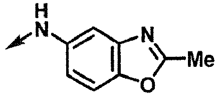 | H | H | LC-MS: [M+H]⁺ / Rt (min) 322.2 / 0.613 (Method A) |
| 147 | 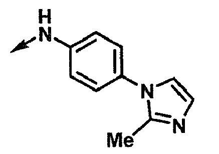 | H | H | LC-MS:[M+H]⁺ / Rt (min) 322.2 / 0.613 (Method A) |
| 148 | 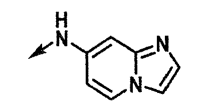 | H | H | LC-MS:[M+H]⁺ / Rt (min) 322.2 / 0.613 (Method A) |
| 149 | 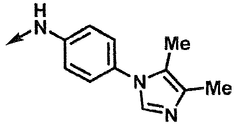 | H | H | LC-MS:[M+H]⁺ / Rt (min) 322.2 / 0.613 (Method A) |
| 150 | 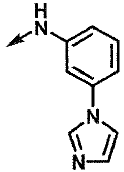 | H | H | LC-MS:[M+H] ⁺ / Rt (min) 322.2 / 0.613 (Method A) |
| 151 | 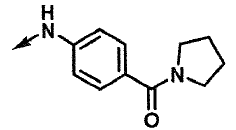 | H | H | LC-MS: [M+H] ⁺ / Rt (min) 322.2 / 0.613 (Method A) |
| 152 | 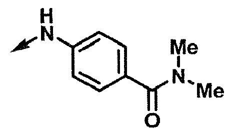 | H | H | LC-MS:[M+H]⁺ / Rt (min) 322.2 / 0.613 (Method A) |
| 153 | 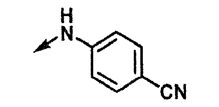 | Me | Me | LC-MS:[M+H]⁺ / Rt (min) 322.2 / 0.613 (Method A) |
| 154 | 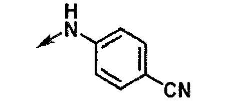 | Me | H | LC-MS: [M+H]⁺ / Rt (min) 322.2 / 0.613 (Method A) |
| 155 | 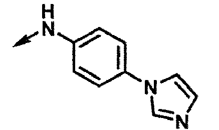 | - (CH₂) ₄- | | LC-MS:[M+H]⁺ / Rt (min) 322.2 / 0.613 (Method A) |
| 156 | 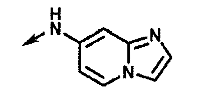 | Me | Me | LC-MS:[M+H]⁺ / Rt (min) 388.2 / 1.584 (Method B) |
| 157 | 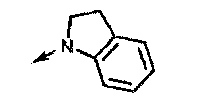 | H | H | LC-MS: [M+H]⁺ / Rt (min) 346.2 / 0.966 (Method A) |
| 158 | 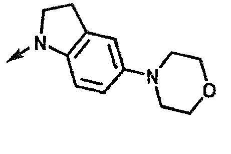 | H | H | ¹H-NMR (400 MHz, DMSO-d₆) δ: 8.09 (d, J = 9.8 Hz, 1H), 7.83-7.78 (m, 3H) , 7.31 (d, J = 7.9 Hz, 2H), 7.10 (d, J = 9.8 Hz, 1H), 6.91 (d, J = 1.8 Hz, 1H), 6.73 (dd, J = 8.5, 2.4 Hz, 1H), 5.13 (s, 2H), 4.22 (t, J = 8.2 Hz, 2H), 3.72 (t, J = 4.6 Hz, 4H), 3.18 (t, J = 8.2 Hz, 2H), 3.04 (t, J = 4.9 Hz, 4H), 2.36 (s, 3H) . |
| 159 | 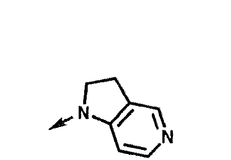 | H | H | ¹H-NMR (400MHz, DMSO-d₆) δ: 8.43 (s, 1H), 8.32 (d, J = 4.9 Hz, 1H) , 8.11 (d, J = 9.8 Hz, 1H), 7.81-7.78 (m, 3H), 7.31 (d, J = 8.5 Hz, 2H), 7.12 (d, J = 9.8 Hz, 1H), 5.21 (s, 2H), 4.32 (t, J = 8.5 Hz, 2H), 3.31-3.25 (m, 2H), 2.36 (s, 3H). |

### Examples 160 to 192

According to the method of Example 1, 2, or 50 and common reaction conditions, the compounds of Examples 160 to 192 were obtained by using corresponding material compounds.

| Example | Chemical structure | Analytical data |
|---|---|---|
| 160 | 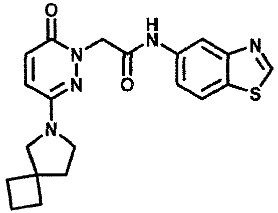 | ¹H-NMR (400 MHz, CDCl₃) δ: 9.50 (br s, 1H), 8.98 (s, 1H), 8.38 (d, J = 2.3 Hz, 1H), 7.84 (d, J = 8.7 Hz, 1H), 7.62 (dd, J = 8.7, 2.3 Hz, 1H), 6.99 (d, J = 9.9 Hz, 1H), 6. 95 (d, J = 9.9 Hz, 1H), 4.88 (s, 2H) , 3.39 (t, J = 6.9 Hz, 2H), 3.35 (s, 2H), 2.07-1.91 (m, 8H). |
| 161 | 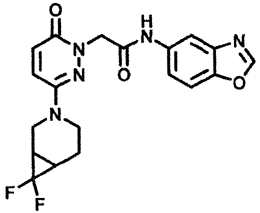 | ¹H-NMR (400 MHz, CDCl₃) δ: 9.35 (s, 1H), 8.06-8.00 (m, 2H), 7.43-7.37 (m, 2H), 7.13 (d, J = 10.0 Hz, 1H), 6.93 (d, J = 10.0 Hz, 1H), 4.85 (s, 2H), 3.81 (dd, J = 14.4, 7.0 Hz, 1H), 3.48-2.40 (m, 1H), 3.28-3.12 (m, 2H), 2.00-1.92 (m, 1H), 1.86-1.68 (m, 3H) . |
| 162 | 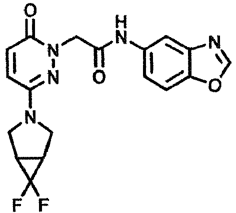 | ¹H-NMR (400 MHz, CDCl₃) δ: 9.52 (s, 1H), 8.01 (d, J = 12.2 Hz, 2H), 7.42-7.35 (m, 2H), 6.92 (s, 2H), 4.85 (s, 2H), 3.75-3.58 (m, 4H), 2.37 (d, J = 11.6 Hz, 2H). |
| 163 | 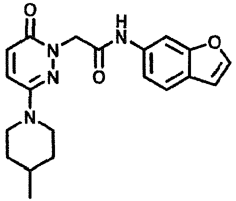 | ¹H-NMR (400 MHz, CDCl₃) δ: 9.27 (br s, 1H), 8.04 (br s, 1H), 7.57 (d, J = 1.8 Hz, 1H), 7.46 (d, J = 8.5 Hz, 1H), 7.22 (d, J = 9.8 Hz, 1H), 7.14 (dd, J = 8.5, 1.8 Hz, 1H) , 6.93 (d, J = 9.8 Hz, 1H), 6.70-6.69 (br m, 1H), 4.85 (s, 2H), 3.86-3.83 (m, 2H), 2.80-2.73 (m, 2H), 1.73-1.70 (m, 2H), 1.59-1.51 (m, 1H), 1.28-1.18 (m, 2H), 0.97 (d, J = 6.7 Hz, 3H). |
| 164 | 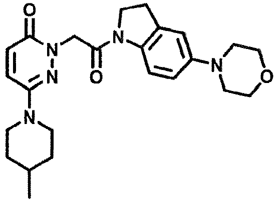 | LC-MS: [M+H]⁺ / Rt (min) 438.4 / 0.858 (Method A) |
| 165 | 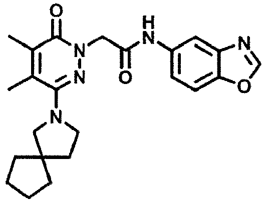 | ¹H-NMR (400MHz, DMSO-d₆) δ: 10.36 (s, 1H), 8.70 (s, 1H) , 8.11 (d, J = 2.4 Hz, 1H), 7.71 (d, J = 8.5 Hz, 1H), 7.50 (dd, J = 8.9, 2.1 Hz, 1H), 4.72 (s, 2H), 3.32-3.28 (m, 2H), 3.06 (s, 2H), 2.16 (s, 3H), 2.04 (s, 3H), 1.73 (t, J = 7.0 Hz, 2H), 1.63-1.46 (m, 8H). |
| 166 | 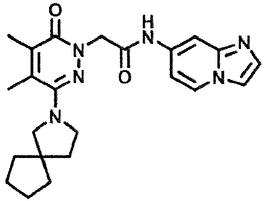 | ¹H-NMR (400MHz, DMSO-d₆) δ: 10.44 (s, 1H), 8.44 (d, J = 7.3 Hz, 1H) , 7.90 (s, 1H) , 7.81 (s, 1H), 7.44 (d, J = 1.2 Hz, 1H), 6.94 (dd, J = 7.3, 1.8 Hz, 1H) , 4.73 (s, 2H) , 3.32-3.29 (m, 2H), 3.07 (s, 2H), 2.16 (s, 3H), 2.04 (s, 3H), 1.73 (t, J = 7.0 Hz, 2H), 1.63-1.46 (m, 8H). |
| 167 | 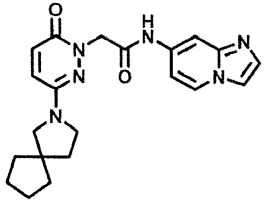 | ¹H-NMR (400 MHz, CDCl₃) δ: 9.67 (s, 1H), 7.99 (d, J = 7.3 Hz, 1H), 7.86 (d, J = 1.8 Hz, 1H), 7.55 (s, 1H), 7.47 (s, 1H) , 7.04 (dd, J = 7.3, 1.8 Hz, 1H), 7.00 (d, J = 10.1 Hz, 1H), 6.95 (d, J = 10.1 Hz, 1H), 4.85 (s, 2H), 3.44 (t, J = 6.9 Hz, 2H), 3.23 (s, 2H), 1.87 (t, J = 6.9 Hz, 2H) , 1.71-1.56 (m, 8H) . |
| 168 | 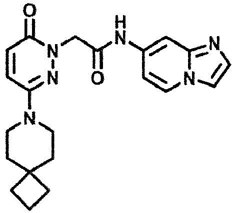 | ¹H-NMR (400 MHz, CDCl₃) δ : 9.54 (br s, 1H), 7.99 (d, J = 7.3 Hz, 1H), 7.86 (d, J = 1.8 Hz, 1H) , 7.56 (s, 1H) , 7.47 (s, 1H), 7.24 (d, J = 10.1 Hz, 1H), 7.02 (dd, J = 7.3, 1.8 Hz, 1H), 6.93 (d, J = 10.1 Hz, 1H), 4.85 (s, 2H), 3.25-3.23 (m, 4H) , 1.94-1.88 (m, 2H), 1.81-1.78 (m, 4H), 1.66-1.64 (m, 4H). |
| 169 | 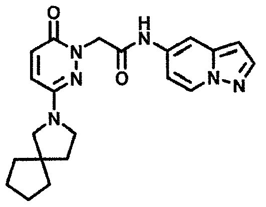 | ¹H-NMR (400 MHz, CDCl₃) δ: 9.69 (br s, 1H), 8.32 (d, J = 7.5 Hz, 1H), 7.99 (d, J = 2.4 Hz, 1H), 7.87 (d, J = 2.3 Hz, 1H), 7.00 (d, J = 10.1 Hz, 1H) , 6.95 (d, J = 10.1 Hz, 1H), 6.72 (dd, J = 7.5, 2.3 Hz, 1H), 6.39 (d, J = 2.4 Hz, 1H), 4.84 (s, 2H), 3.44 (t, J = 7.0 Hz, 2H) , 3.22 (s, 2H) , 1.87 (t, J = 7.0 Hz, 2H) , 1.71-1.66 (m, 4H), 1.62-1.56 (m, 4H). |
| 170 | 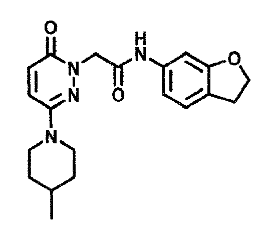 | ¹H-NMR (400 MHz, CDCl₃) δ : 8.99 (br s, 1H), 7.20 (d, J = 9.8 Hz, 1H), 7.10-7.10 (br m, 1H), 7.07 (d, J = 7.9 Hz, 1H), 6.92-6.89 (m, 2H), 4.80 (s, 2H), 4.55 (t, J = 8.7 Hz, 2H), 3.84-3.81 (m, 2H), 3.14 (t, J = 8.7 Hz, 2H), 2.79-2.72 (m, 2H), 1.72-1.69 (m, 2H), 1.58-1.53 (m, 1H), 1.28-1.17 (m, 2H) , 0.96 (d, J = 6.7 Hz, 3H). |
| 171 | 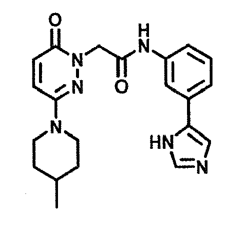 | ¹H-NMR (400 MHz, CDCl₃) δ : 7.67 (s, 1H), 7.63 (br s, 1H), 7.46-7.44 (br m, 1H) , 7.37 (br s, 1H), 7.28 (s, 1H), 7.24 (br s, 1H) , 7.22 (d, J = 9.8 Hz, 1H), 6.91 (d, J = 9.8 Hz, 1H), 4.88 (s, 2H) , 3.85-3.83 (br m, 2H) , 2.81-2.74 (m, 2H), 1.73-1.70 (m, 2H) , 1.66-1.49 (m, 1H) , 1.29-1.19 (m, 2H) , 0.97 (d, J = 6.7 Hz, 3H) . |
| 172 | 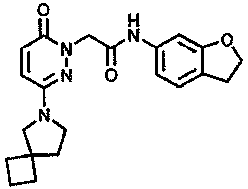 | ¹H-NMR (400 MHz, CDCl₃) δ : 9.08 (br s, 1H), 7.10 (d, J = 1.2 Hz, 1H), 7.06 (d, J = 7.9 Hz, 1H), 6.97-6.90 (m, 3H), 4.80 (s, 2H), 4.55 (t, J = 8.7 Hz, 2H), 3.37 (t, J = 6.7 Hz, 2H), 3.34 (s, 2H), 3.13 (t, J = 8.7 Hz, 2H), 2.04-1.91 (m, 8H) . |
| 173 | 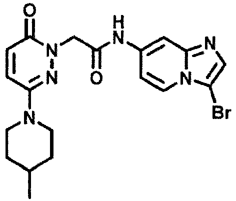 | ¹H-NMR (400 MHz, CDCl₃) δ : 9.74 (br s, 1H) , 7.95-7.93 (m, 2H), 7.51 (s, 1H), 7.25 (d, J = 10.1 Hz, 1H) , 7.08 (dd, J = 7.9, 1.8 Hz, 1H) , 6.94 (d, J = 10.1 Hz, 1H), 4.86 (s, 2H) , 3.87-3.83 (m, 2H), 2.81-2.74 (m, 2H), 1.73-1.70 (m, 2H), 1.62-1.54 (m, 1H), 1.28-1.18 (m, 2H), 0.97 (d, J = 6.7 Hz, 3H) . |
| 174 | 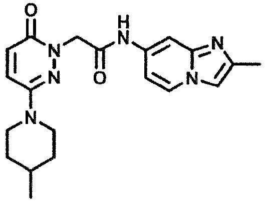 | ¹H-NMR (400 MHz, CDCl₃) δ : 9.46 (br s, 1H), 7.89 (d, J = 7.3 Hz, 1H), 7.72 (d, J = 2.4 Hz, 1H), 7.23 (d, J = 10.4 Hz, 1H) , 7.21 (br s, 1H) , 6.99-6. 96 (m, 1H) , 6.93 (d, J = 10.4 Hz, 1H), 4.84 (s, 2H) , 3.86-3.83 (m, 2H), 2.81-2.74 (m, 2H), 2.41 (s, 3H), 1.73-1.70 (m, 2H), 1.63-1.53 (m, 1H) , 1. 28-1.18 (m, 2H), 0.97 (d, J = 6.7 Hz, 3H) . |
| 175 | 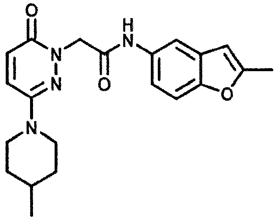 | ¹H-NMR (400 MHz, CDCl₃) δ : 9.04 (br s, 1H), 7.78 (d, J = 2.1 Hz, 1H), 7.28 (d, J = 8.5 Hz, 1H), 7.21 (d, J = 9.8 Hz, 1H), 7.18 (dd, J = 8.5, 2.1 Hz, 1H), 6.92 (d, J = 9.8 Hz, 1H), 6.31 (s, 1H), 4.84 (s, 2H), 3.85-3.82 (m, 2H), 2.80-2.73 (m, 2H) , 2.42 (s, 3H) , 1.73-1.69 (m, 2H) , 1.61-1.53 (m, 1H) , 1.28-1.18 (m, 2H) , 0.97 (d, J = 6.7 Hz, 3H). |
| 176 | 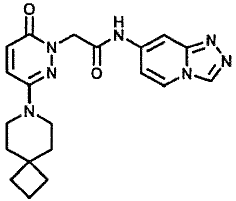 | ¹H-NMR (400 MHz, CD₃OD) δ : 9.05 (s, 1H), 8.43 (d, J = 7.3 Hz, 1H), 8.20 (d, J = 1.8 Hz, 1H), 7.54 (d, J = 9.8 Hz, 1H), 7.10 (dd, J = 7.3, 1.8 Hz, 1H), 6.90 (d, J = 9.8 Hz, 1H), 4.87 (s, 2H), 3.29-3.26 (m, 4H), 1.97-1.89 (m, 2H), 1.84-1.79 (m, 4H), 1.67-1.65 (m, 4H) . |
| 177 | 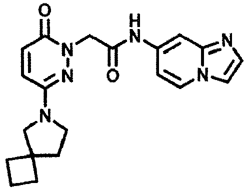 | ¹H-NMR (400 MHz, CDCl₃) δ : 9.62 (br s, 1H) , 7.99 (d, J = 7.3 Hz, 1H), 7.85-7.84 (br m, 1H) , 7.55 (d, J = 1.5 Hz, 1H) , 7.47 (s, 1H), 7.03 (dd, J = 7.3, 1.5 Hz, 1H) , 6.99 (d, J = 9.8 Hz, 1H), 6.94 (d, J = 9.8 Hz, 1H), 4.85 (s, 2H) , 3.39 (t, J = 6.7 Hz, 2H), 3.35 (s, 2H), 2.06-1.91 (m, 8H). |
| 178 | 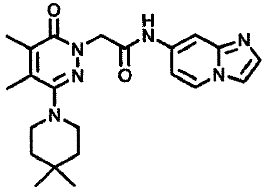 | LC-MS: [M+H]⁺ / Rt (min) 409.3 / 1.71 (Method B) |
| 179 | 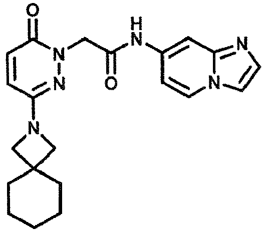 | LC-MS: [M+H]⁺ / Rt (min) 393.4 / 0.628 (Method A) |
| 180 | 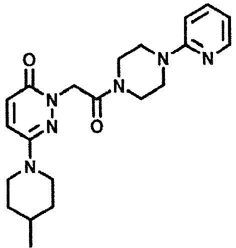 | LC-MS: [M+H]⁺ / Rt (min) 397.1 / 0.609 (Method A) |
| 181 | 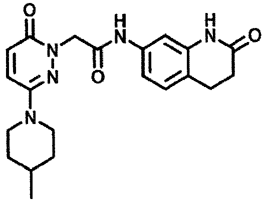 | ¹H-NMR (400 MHz, CDCl₃) δ : 9.26 (brs, 1H) , 7.50 (s, 1H) , 7.25 (d, J = 2.0 Hz, 1H), 7.22 (d, J = 9.8 Hz, 1H), 7.05 (d, J = 8.1 Hz, 1H), 6.93 (d, J = 9.8 Hz, 1H) , 6.92 (dd, J = 8.1, 2.0 Hz, 1H), 4.82 (s, 2H), 3.85-3.82 (m, 2H), 2.91-2.89 (m, 2H), 2.76 (td, J = 12.8, 2.4 Hz, 2H), 2.61-2.58 (m, 2H), 1.73-1.69 (m, 2H), 1.60-1.53 (m, 1H), 1.27-1.17 (m, 2H), 0.97 (d, J = 6.1 Hz, 3H). |
| 182 | 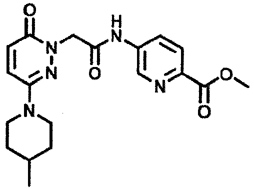 | LC-MS: [M+H]⁺ / Rt (min) 386.2 / 1.695 (Method B) |
| 183 | 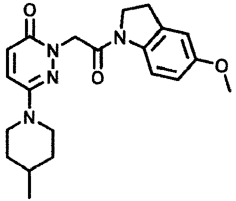 | ¹H-NMR (400 MHz, CDCl₃) δ : 8.11 (d, J = 8.5 Hz, 1H), 7.18 (d, J = 9.8 Hz, 1H), 6.87 (d, J = 9.8 Hz, 1H) , 6.75 (br s, 1H), 6.70-6.68 (br m, 1H), 4.88 (s, 2H), 4.17-4.13 (m, 2H) , 3.77 (s, 3H) , 3.77-3.75 (m, 2H) , 3.25-3.20 (m, 2H) , 2.75-2.68 (m, 2H), 1.70-1.67 (m, 2H), 1.56-1.49 (m, 1H), 1.28-1.18 (m, 2H), 0.95 (d, J = 6.7 Hz, 3H). |
| 184 | 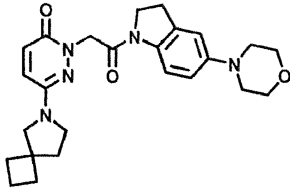 | ¹H-NMR (400 MHz, CDCl₃) δ : 8.10 (d, J = 8.5 Hz, 1H), 6.94 (d, J = 9.8 Hz, 1H), 6.88 (d, J = 9.8 Hz, 1H) , 6.78 (br s, 1H), 6.74-6.71 (br m, 1H), 4.88 (s, 2H), 4.16-4.11 (m, 2H), 3.86-3.83 (m, 4H), 3.37-3.33 (m, 2H), 3.30 (s, 2H), 3.24-3.20 (m, 2H) , 3.10-3.08 (m, 4H), 2.04-1.86 (m, 8H). |
| 185 | 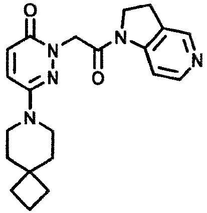 | ¹H-NMR (400 MHz, CDCl₃) δ : 8.41 (s, 1H), 8.38 (d, J = 5.5 Hz, 1H), 8.00 (br s, 1H), 7.21-7.19 (m, 1H), 6.88 (d, J = 9.8 Hz, 1H), 4.89 (br s, 2H), 4.21 (t, J = 8.5 Hz, 2H), 3.33-3.29 (m, 2H), 3.19-3.16 (m, 4H) , 1.92-1.87 (m, 2H) , 1.80-1.76 (m, 4H) , 1.65-1.62 (m, 4H) . |
| 186 | 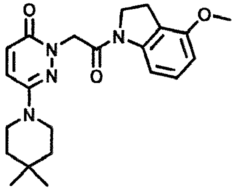 | ¹H-NMR (400 MHz, CDCl₃) δ : 7.82 (d, J = 8.5 Hz, 1H) , 7.19-7.12 (m, 2H), 6.88 (d, J = 9.8 Hz, 1H), 6.58 (d, J = 8.5 Hz, 1H), 4.88 (s, 2H), 4.17 (t, J = 8.5 Hz, 2H) , 3.84 (s, 3H) , 3.25-3.22 (m, 4H), 3.17 (t, J = 8.5 Hz, 2H), 1.44-1.42 (m, 4H), 0.96 (s, 6H). |
| 187 | 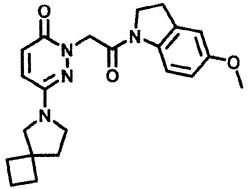 | ¹H-NMR (400 mHz, CDCl₃) δ : 8.12 (d, J = 8.9 Hz, 1H), 6.94 (d, J = 10.1 Hz, 1H), 6.89 (d, J = 10.1 Hz, 1H) , 6.75-6.75 (br m, 1H) , 6.69 (dd, J = 8.9, 2.4 Hz, 1H), 4.88 (s, 2H) , 4.15 (t, J = 8.5 Hz, 2H), 3.77 (s, 3H), 3.35 (t, J = 6.7 Hz, 2H), 3.31 (s, 2H), 3.23 (t, J = 8.5 Hz, 2H), 2.05-1.88 (m, 8H) . |
| 188 | 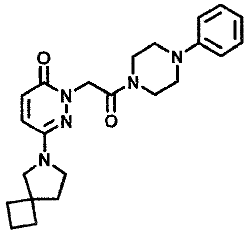 | ¹H-NMR (400 MHz, CDCl₃) δ : 7.31-7.28 (m, 2H), 6.94-6.89 (m, 4H), 6.86 (d, J = 10.4 Hz, 1H), 4.88 (s, 2H), 3.82-3.79 (m, 2H) , 3.67-3.65 (m, 2H), 3.35 (t, J = 6.7 Hz, 2H), 3.30 (s, 2H), 3.23-3.18 (m, 4H), 2.04-1.88 (m, 8H). |
| 189 | 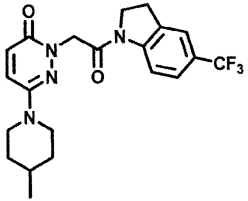 | ¹H-NMR (400 MHz, CDCl₃) δ : 8.28-8.26 (br m, 1H) , 7.45-7.43 (m, 2H), 7.20 (d, J = 10.4 Hz, 1H), 6.88 (d, J = 10.4 Hz, 1H), 4.91 (s, 2H), 4.25-4.21 (m, 2H), 3.78-3.75 (m, 2H), 3.33-3.29 (m, 2H), 2.76-2.69 (m, 2H), 1.71-1.67 (m, 2H), 1.57-1.48 (m, 1H), 1.28-1.18 (m, 2H), 0.96 (d, J = 6.7 Hz, 3H). |
| 190 | 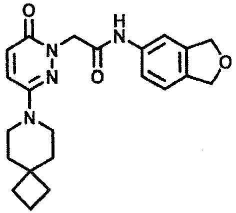 | ¹H-NMR (400 MHz, CDCl₃) δ : 9.23 (br s, 1H), 7.57 (s, 1H), 7.29-7.26 (m, 1H), 7.22 (d, J = 9.8 Hz, 1H) , 7.13 (d, J = 7.9 Hz, 1H), 6.92 (d, J = 9.8 Hz, 1H), 5.05 (s, 4H), 4.82 (s, 2H), 3.24-3.21 (m, 4H), 1.96-1.88 (m, 2H), 1.81-1.77 (m, 4H), 1.66-1.63 (m, 4H). |
| 191 | 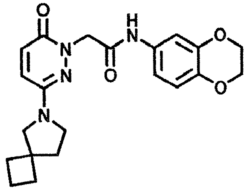 | ¹H-NMR (400 MHz, CDCl₃) δ : 8.96 (s, 1H), 7.18 (d, J = 2.4 Hz, 1H), 7.00-6.88 (m, 3H), 6.76 (d, J = 8.5 Hz, 1H), 4.79 (s, 2H), 4.23-4.20 (m, 4H) , 3.37 (t, J = 7.0 Hz, 2H), 3.33 (s, 2H), 2.07-1.90 (m, 8H). |
| 192 | 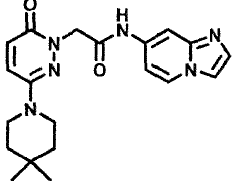 | ¹H-NMR (400 MHz, CDCl₃) δ : 9.62 (br s, 1H), 8.02 (d, J = 7.3 Hz, 1H), 7.90 (s, 1H) , 7.54 (s, 1H), 7.51 (s, 1H), 7.27-7.24 (m, 1H), 7.04 (d, J = 6.1 Hz, 1H), 6.94 (d, J = 10.4 Hz, 1H), 4.87 (s, 2H), 3.32-3.29 (m, 4H), 1.46-1.43 (m, 4H), 0.98 (s, 6H). |

### Examples 193 to 238

According to the method of Example 1, 2, or 50 and common reaction conditions, the compounds of Examples 193 to 238 were obtained by using corresponding material compounds.

| Example | Chemical structure | Analytical data |
|---|---|---|
| 193 | 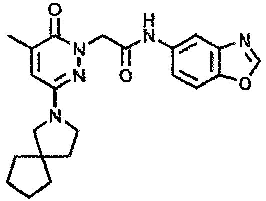 | ¹H-NMR (400MHz, DMSO-d₆) δ : 10.33 (s, 1H), 8.70 (s, 1H), 8.12 (d, J = 1.8 Hz, 1H), 7.72 (d, J = 8.5 Hz, 1H), 7.51 (dd, J = 8.8, 2.1 Hz, 1H), 7.16 (d, J = 1.2 Hz, 1H), 4.71 (s, 2H), 3.35 (t, J = 6.7 Hz, 2H), 3.16 (s, 2H), 2.08 (s, 3H), 1.80 (t, J = 7.0 Hz, 2H), 1.65-1.52 (m, 8H) . |
| 194 | 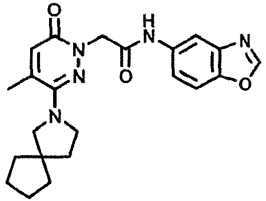 | ¹H-NMR (400MHz, DMSO-d₆) δ : 10.34 (s, 1H), 8.70 (s, 1H), 8.10 (d, J = 1.8 Hz, 1H), 7.71 (d, J = 8.6 Hz, 1H), 7.50 (dd, J = 8.6, 1.8 Hz, 1H), 6.71 (d, J = 1.2 Hz, 1H), 4.70 (s, 2H), 3.39 (t, J = 6.7 Hz, 2H), 3.14 (s, 2H), 2.25 (s, 3H), 1.74 (t, J = 7.0 Hz, 2H), 1.63-1.46 (m, 8H) . |
| 195 | 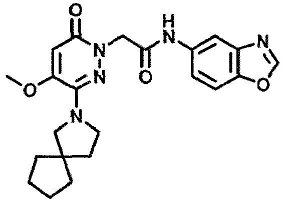 | ¹H-NMR (400MHz, DMSO-d₆) δ : 10.31 (s, 1H), 8.70 (s, 1H), 8.11 (d, J = 1.8 Hz, 1H), 7.71 (d, J = 8.7 Hz, 1H), 7.51 (dd, J = 8.7, 1.8 Hz, 1H), 6.20 (s, 1H) , 4.66 (s, 2H) , 3.83 (s, 3H), 3.45 (t, J = 7.1 Hz, 2H), 3.20 (s, 2H), 1.71 (t, J = 7.1 Hz, 2H), 1.63-1.48 (m, 8H) . |
| 196 | 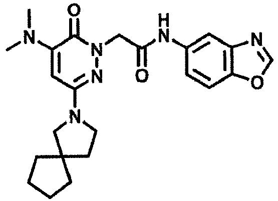 | ¹H-NMR (400MHz, DMSO-d₆) δ : 10.29 (s, 1H), 8.70 (s, 1H), 8.12 (d, J = 1.8 Hz, 1H), 7.71 (d, J = 8.5 Hz, 1H), 7.51 (dd, J = 8.8, 2.1 Hz, 1H), 5.83 (s, 1H), 4.66 (s, 2H), 3.35-3.31 (m, 2H), 3.14 (s, 2H), 3.04 (s, 6H), 1.77 (t, J = 6.7 Hz, 2H), 1.65-1.49 (m, 8H). |
| 197 | 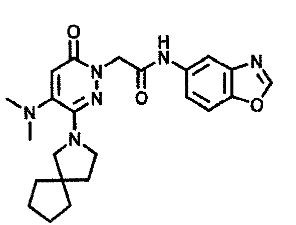 | ¹H-NMR (400MHz, DMSO-d₆) δ : 10.35 (s, 1H), 8.70 (s, 1H), 8.12 (d, J = 1.8 Hz, 1H), 7.71 (d, J = 9.1 Hz, 1H), 7.51 (dd, J = 8.8, 2.1 Hz, 1H), 5.98 (s, 1H), 4.67 (s, 2H), 3.24 (t, J = 7.3 Hz, 2H), 3.04 (s, 2H), 2.79 (s, 6H), 1.73 (t, J = 7.0 Hz, 2H), 1.63-1.47 (m, 8H). |
| 198 | 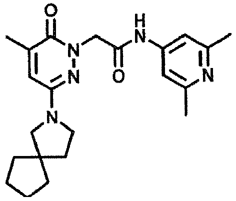 | ¹H-NMR (400MHz, DMSO-d₆) δ : 10.36 (s, 1H), 7.21 (s, 2H), 7.16-7.14 (m, 1H), 4.68 (s, 2H), 3.35-3.31 (m, 2H), 3.14 (s, 2H), 2.35 (s, 6H), 2.06 (s, 3H), 1.79 (t, J = 6.7 Hz, 2H), 1.66-1.49 (m, 8H). |
| 199 | 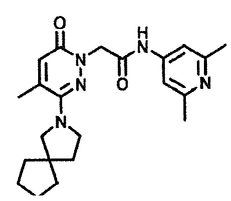 | ¹H-NMR (400MHz, DMSO-d₆) δ : 10.38 (s, 1H), 7.20 (s, 2H), 6.70 (d, J = 1.2 Hz, 1H), 4.68 (s, 2H), 3.38 (t, J = 7.0 Hz, 2H), 3.13 (s, 2H), 2.35 (s, 6H), 2.25 (s, 3H), 1.74 (t, J = 7.0 Hz, 2H), 1.63-1.47 (m, 8H). |
| 200 | 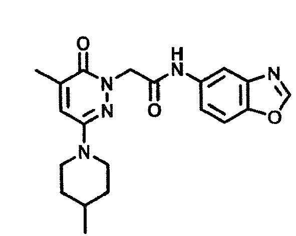 | ¹H-NMR (400MHz, DMSO-d₆) δ : 10.35 (s, 1H), 8.70 (s, 1H) , 8.11 (d, J = 1.8 Hz, 1H), 7.72 (d, J = 8.6 Hz, 1H), 7.53-7.47 (m, 2H), 4.73 (s, 2H), 3.81 (d, J = 13.4 Hz, 2H), 2.68-2.61 (m, 2H), 2.07 (s, 3H), 1.64 (d, J = 12.2 Hz, 2H), 1.57-1.45 (m, 1H), 1.19-1.09 (m, 2H), 0.91 (d, J = 6.2 Hz, 3H). |
| 201 | 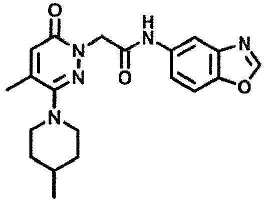 | ¹H-NMR (400MHz, DMSO-d₆) δ : 10.39 (s, 1H), 8.71 (s, 1H) , 8.11 (d, J = 1.8 Hz, 1H), 7.72 (d, J = 9.2 Hz, 1H), 7.51 (dd, J = 8.9, 2.1 Hz, 1H), 6.78 (d, J = 1.2 Hz, 1H), 4.75 (s, 2H), 3.23 (d, J = 12.8 Hz, 2H) , 2.63-2.52 (m, 2H), 2.20 (s, 3H), 1.67 (d, J = 10.4 Hz, 2H), 1.55-1.43 (m, 1H), 1.29-1.19 (m, 2H), 0.94 (d, J = 6.7 Hz, 3H). |
| 202 | 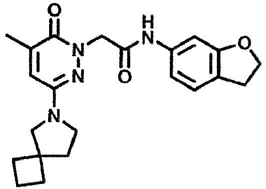 | ¹H-NMR (400 MHz, CDCl₃) δ : 9.11 (br s, 1H), 7.08-7.05 (br m, 2H), 6.93 (dd, J = 8.2, 2.0 Hz, 1H), 6.81 (d, J = 2.0 Hz, 1H), 4.80 (s, 2H), 4.55 (t, J = 8.7 Hz, 2H), 3.36 (t, J = 6.7 Hz, 2H), 3.32 (s, 2H), 3.13 (t, J = 8.7 Hz, 2H), 2.23 (d, J = 1.2 Hz, 3H), 2.06-1.90 (m, 8H). |
| 203 | 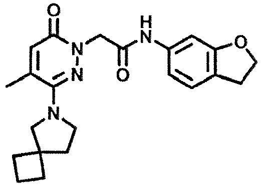 | ¹H-NMR (400 MHz, CDCl₃) δ : 9.17 (br s, 1H), 7.11 (br s, 1H) , 7.07-7.05 (br m, 1H) , 6.90 (dd, J = 7.9, 1.8 Hz, 1H), 6.71 (br s, 1H), 4.78 (s, 2H), 4.55 (t, J = 8.7 Hz, 2H), 3.38 (t, J = 6.7 Hz, 2H), 3.33 (s, 2H), 3.13 (t, J = 8.7 Hz, 2H), 2.28 (d, J = 1.2 Hz, 3H), 2.06-1.86 (m, 8H) . |
| 204 | 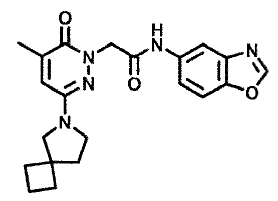 | ¹H-NMR (400MHz, DMSO-d₆) δ : 10.34 (s, 1H) , 8.71 (s, 1H) , 8.12 (d, J = 2.4 Hz, 1H), 7.72 (d, J = 8.5 Hz, 1H), 7.52 (dd, J = 8.9, 2.1 Hz, 1H), 7.15 (d, J = 1.2 Hz, 1H), 4.72 (s, 2H), 3.31-3.28 (m, 4H) , 2.08 (d, J = 1.2 Hz, 3H) , 2.03-1.80 (m, 8H) . |
| 205 | 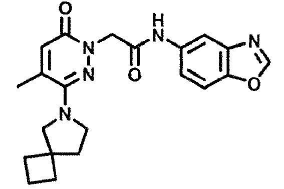 | ¹H-NMR (400MHz, DMSO-d₆) δ : 10.35 (s, 1H) , 8.71 (s, 1H), 8.11 (d, J = 1.8 Hz, 1H), 7.72 (d, J = 8.6 Hz, 1H), 7.51 (dd, J = 8.9, 2.1 Hz, 1H), 6.71 (s, 1H), 4.70 (s, 2H), 3.35-3.28 (m, 4H), 2.25 (s, 3H) , 2.03-1.76 (m, 8H). |
| 206 | 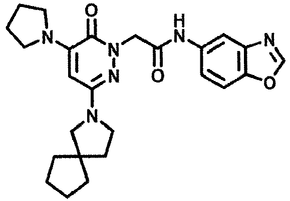 | ¹H-NMR (400MHz, DMSO-d₆) δ : 10.33 (s, 1H), 8.71 (s, 1H) , 8.12 (d, J = 1.8 Hz, 1H), 7.72 (d, J = 8.5 Hz, 1H), 7.51 (dd, J = 8.5, 1.8 Hz, 1H), 5.78 (s, 1H), 4.67 (s, 2H), 3.29-3.25 (m, 4H) , 3.10 (t, J = 7.3 Hz, 2H), 2.95 (s, 2H), 1.91-1.87 (m, 4H), 1.75 (t, J = 7.3 Hz, 2H), 1.61-1.51 (m, 8H). |
| 207 | 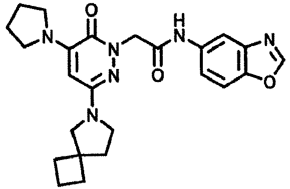 | ¹H-NMR (400MHz, DMSO-d₆) δ : 10.28 (s, 1H) , 8.70 (s, 1H) , 8.13 (d, J = 1.8 Hz, 1H), 7.71 (d, J = 8.6 Hz, 1H), 7.51 (dd, J = 8.9, 2.1 Hz, 1H), 5.52 (s, 1H), 4.63 (s, 2H), 3.57 (s, 4H), 3.29-3.23 (m, 4H), 2.01-1.79 (m, 12H). |
| 208 | 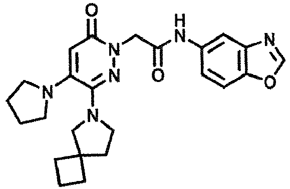 | ¹H-NMR (400MHz, DMSO-d₆) δ : 10.33 (s, 1H), 8.70 (s, 1H), 8.12 (d, J = 1.8 Hz, 1H), 7.71 (d, J = 8.6 Hz, 1H), 7.51 (dd, J = 8.9, 2.1 Hz, 1H), 5.78 (s, 1H), 4.66 (s, 2H), 3.26-3.22 (m, 4H), 3.08 (s, 2H), 3.04 (t, J = 7.2 Hz, 2H), 2.03-1.76 (m, 12H) . |
| 209 | 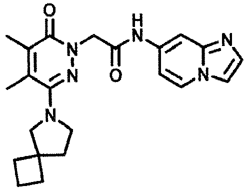 | ¹H-NMR (400MHz, DMSO-d₆) δ : 10.45 (s, 1H), 8.45 (d, J = 7.3 Hz, 1H) , 7.91 (d, J = 1.2 Hz, 1H), 7.82 (s, 1H), 7.45 (d, J = 1.2 Hz, 1H), 6.95 (dd, J = 7.3, 1.8 Hz, 1H), 4.74 (s, 2H), 3.26-3.21 (m, 4H), 2.16 (s, 3H), 2.05 (s, 3H), 2.01-1.78 (m, 8H). |
| 210 | 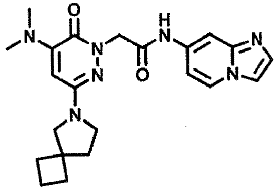 | LC-MS: [M+H]⁺ / Rt (min) 422.4 / 0.695 (Method A) |
| 211 | 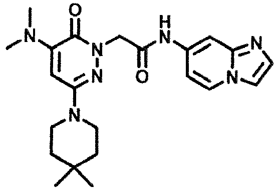 | ¹H-NMR (400 MHz, CDCl₃) δ : 9.42 (br s, 1H), 7.99 (d, J = 7.3 Hz, 1H), 7.71 (br m, 1H), 7.55 (br m, 1H), 7.46 (s, 1H), 7.16 (dd, J = 7.3, 1.8 Hz, 1H), 5.99 (s, 1H), 4.83 (s, 2H), 3.27-3.24 (m, 4H), 3.13 (s, 6H), 1.46-1.43 (m, 4H) , 0.97 (s, 6H) . |
| 212 | 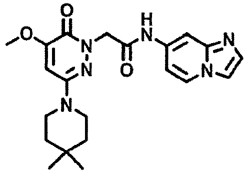 | LC-MS: [M+H]⁺ / Rt (min) 411.4 / 0.618 (Method A) |
| 213 | 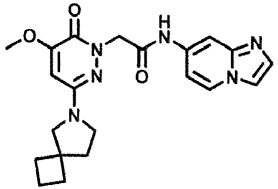 | LC-MS: [M+H]⁺ / Rt (min) 409.4 / 0.608 (Method A) |
| 214 | 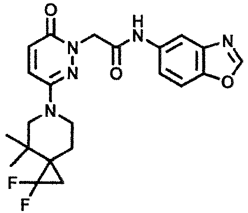 | ¹H-NMR (400 MHz, CDCl₃) δ : 9.37 (s, 1H), 8.04 (d, J = 9.2 Hz, 2H) , 7.42 (s, 2H) , 7.20 (d, J = 10.0 Hz, 1H), 6.94 (d, J = 10.0 Hz, 1H), 4.85 (s, 2H) , 3.70-3.65 (m, 1H), 3.21 (d, J = 12.6 Hz, 1H) , 3.00-2.95 (m, 1H), 2.84 (d, J = 12.6 Hz, 1H) , 2.08-2.04 (m, 1H), 1.48-1.32 (m, 2H), 1.11 (s, 3H), 0.96-0.84 (m, 1H), 0.92 (s, 3H). |
| 215 | 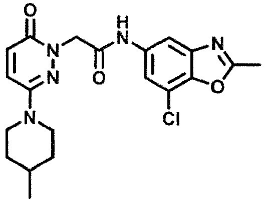 | ¹H-NMR (400 MHz, CDCl₃) δ : 9.41 (br s, 1H), 7.76 (d, J = 1.8 Hz, 1H), 7.53 (d, J = 1.8 Hz, 1H), 7.23 (d, J = 10.1 Hz, 1H), 6.94 (d, J = 10.1 Hz, 1H), 4.84 (s, 2H), 3.84 (d, J = 12.8 Hz, 2H), 2.81-2.74 (m, 2H), 2.65 (s, 3H), 1.74-1.70 (m, 2H), 1.59-1.53 (m, 1H), 1.30-1.18 (m, 2H), 0.97 (d, J = 6.7 Hz, 3H) . |
| 216 | 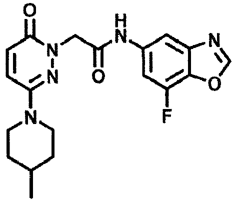 | ¹H-NMR (400 MHz, CDCl₃) δ : 9.53 (br s, 1H), 8.07 (s, 1H), 7.75 (d, J = 1.4 Hz, 1H), 7.53-7.50 (m, 1H), 7.24 (d, J = 10.4 Hz, 1H), 6.94 (d, J = 10.4 Hz, 1H), 4.85 (s, 2H), 3.87-3.83 (m, 2H), 2.80-2.74 (m, 2H), 1.74-1.70 (m, 2H), 1.61-1.50 (m, 1H), 1.31-1.18 (m, 2H), 0.97 (d, J = 6.7 Hz, 3H) . |
| 217 | 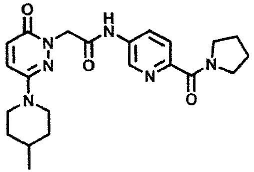 | ¹H-NMR (400 MHz , CDCl₃) δ : 9.65 (s, 1H), 8.64 (d, J = 2.7 Hz, 1H), 8.10 (dd, J = 8.5, 2.7 Hz, 1H), 7.84 (d, J = 8.5 Hz, 1H), 7.25 (d, J = 10.4 Hz, 1H), 6.95 (d, J = 10.4 Hz, 1H), 4.86 (s, 2H), 3.87-3.83 (m, 2H), 3.78-3.74 (m, 2H), 3.68-3.65 (m, 2H), 2.81-2.74 (m, 2H), 1.93-1.89 (m, 4H), 1.74-1.70 (m, 2H), 1.58-1.53 (m, 1H), 1.28-1.18 (m, 2H), 0.97 (d, J = 6.7 Hz, 3H). |
| 218 | 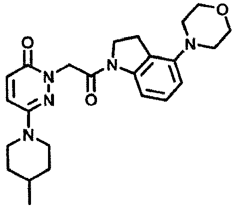 | ¹H-NMR (400 MHz, CDCl₃) δ : 7.94-7.92 (br m, 1H), 7.19-7.14 (m, 2H), 6.87 (d, J = 9.8 Hz, 1H), 6.68 (d, J = 7.9 Hz, 1H), 4.89 (s, 2H), 4.17-4.13 (m, 2H), 3.85-3.83 (m, 4H), 3.77-3.74 (m, 2H), 3.19-3.15 (m, 2H) , 2.99-2.97 (m, 4H), 2.75-2.68 (m, 2H), 1.70-1.67 (m, 2H), 1.60-1.49 (m, 1H), 1.28-1.18 (m, 2H) , 0.95 (d, J = 6.1 Hz, 3H). |
| 219 | 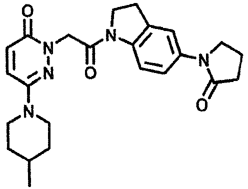 | ¹H-NMR (400 MHz, CDCl₃) δ : 8.15 (d, J = 9.0 Hz, 1H), 7.79 (s, 1H), 7.18 (d, J = 10.1 Hz, 1H), 7.09 (dd, J = 9.0, 2.1 Hz, 1H), 6.88 (d, J = 10.1 Hz, 1H), 4.89 (s, 2H), 4.19-4.14 (m, 2H) , 3.86-3.82 (m, 2H), 3.76 (br m, 2H), 3.29-3.25 (br m, 2H), 2.75-2.68 (br m, 2H), 2.62-2.58 (m, 2H), 2.18-2.11 (m, 2H), 1.70-1.67 (m, 2H), 1.56-1.49 (m, 1H), 1.28-1.18 (m, 2H), 0.96 (d, J = 6.7 Hz, 3H). |
| 220 | 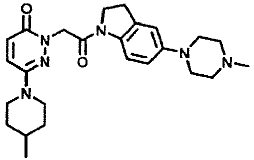 | ¹H-NMR (400 MHz, CDCl₃) δ : 8.07 (d, J = 9.1 Hz, 1H) , 7.17 (d, J = 10.1 Hz, 1H), 6.87 (d, J = 10.1 Hz, 1H), 6.79 (br s, 1H), 6.75 (d, J = 8.4 Hz, 1H), 4.87 (s, 2H), 4.13 (t, J = 8.4 Hz, 2H), 3.77-3.74 (br m, 2H), 3.21 (t, J = 8.2 Hz, 2H), 3.16-3.14 (m, 4H), 2.74-2.68 (m, 2H), 2.58 (d, J = 9.8 Hz, 4H), 2.35 (s, 3H), 1.71-1.66 (m, 2H), 1.57-1.47 (m, 1H), 1.27-1.17 (m, 2H) , 0.95 (d, J = 6.1 Hz, 3H). |
| 221 | 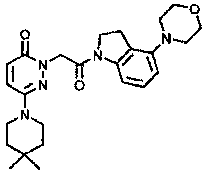 | ¹H-NMR (400 MHz, CDCl₃) δ : 7.93 (d, J = 7.9 Hz, 1H), 7.20-7.14 (m, 2H), 6.88 (d, J = 9.8 Hz, 1H), 6.69 (d, J = 7.9 Hz, 1H), 4.89 (s, 2H), 4.18-4.13 (m, 2H), 3.85-3.83 (m, 4H), 3.25-3.20 (m, 4H), 3.19-3.15 (m, 2H), 2.99-2.97 (m, 4H), 1.44-1.41 (m, 4H), 0.96 (s, 6H). |
| 222 | 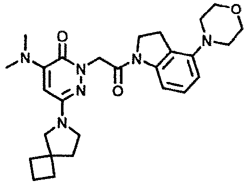 | LC-MS: [M+H]⁺ / Rt (min) 493.4 / 1.077 (Method A) |
| 223 | 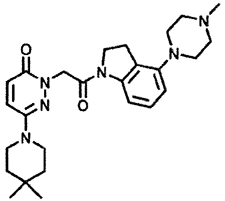 | ¹H-NMR (400 MHz, CDCl₃) δ : 7.91 (d, J = 7.9 Hz, 1H), 7.18 (d, J = 10.1 Hz, 1H), 7.14 (t, J = 7.9 Hz, 1H), 6.88 (d, J = 10.1 Hz, 1H), 6.69 (d, J = 7.9 Hz, 1H), 4.89 (s, 2H) , 4.14 (t, J = 8.2 Hz, 2H), 3.24-3.22 (m, 4H), 3.16 (t, J = 8.2 Hz, 2H), 3.03-3.00 (m, 4H), 2.57 (br s, 4H), 2.36 (s, 3H) , 1.44-1.41 (br m, 4H), 0.96 (s, 6H). |
| 224 | 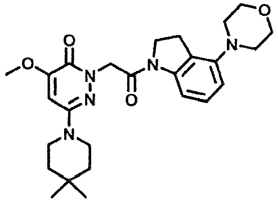 | ¹H-NMR (400MHz, DMSO-d₆) δ : 7.67 (d, J = 8.0 Hz, 1H), 7.11 (t, J = 7.6 Hz, 1H), 6.74 (s, 1H) , 6.67 (d, J = 7.6 Hz, 1H), 4.84 (s, 2H), 4.18-4.14 (m, , 2H), 3.82 (s, 3H), 3.73-3.71 (m, 4H), 3.24-3.21 (m, 4H), 3.12-3.07 (m, 2H), 2.93-2.90 (m, 4H), 1.38-1.35 (m, 4H), 0.93 (s, 6H). |
| 225 | 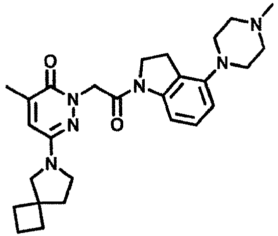 | ¹H-NMR (400 MHz, CDCl₃) δ : 7.89 (d, J = 7.9 Hz, 1H), 7.13-7.09 (m, 1H), 6.82 (br s, 1H), 6.66 (d, J = 7.9 Hz, 1H), 4.89 (s, 2H), 4.17-4.12 (m, 2H), 3.34 (t, J = 6.7 Hz, 2H), 3.30 (s, 2H), 3.12-3.08 (m, 2H), 3.02 (br s, 4H), 2.77 (br s, 4H), 2.51 (br s, 3H), 2.21 (d, J = 1.2 Hz, 3H), 2.05-1.86 (m, 8H). |
| 226 | 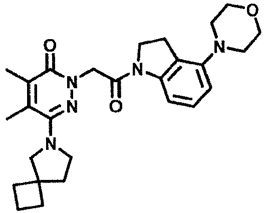 | ¹H-NMR (400MHz, DMSO-d₆) δ : 7.68 (d, J = 7.9 Hz, 1H), 7.12 (t, J = 7.9 Hz, 1H) , 6.67 (d, J = 7.9 Hz, 1H), 4.86 (s, 2H), 4.18 (t, J = 8.2 Hz, 2H), 3.73 (t, J = 4.6 Hz, 4H), 3.25-3.20 (m, 4H), 3.11 (t, J = 8.2 Hz, 2H), 2.92 (t, J = 4.3 Hz, 4H), 2.16 (s, 3H), 2.05 (s, 3H), 2.00-1.78 (m, 8H). |
| 227 | 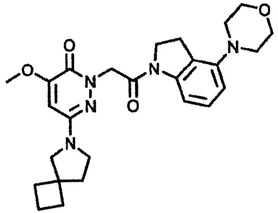 | ¹H-NMR (400MHz, DMSO-d₆) δ : 7.67 (d, J = 8.0 Hz, 1H), 7.11 (t, J = 8.0 Hz, 1H), 6. 66 (d, J = 8.0 Hz, 1H), 6.39 (s, 1H), 4.82 (s, 2H), 4.18-4.14 (m, 2H), 3.83 (s, 3H), 3.73-3.71 (m, 4H), 3.30-3.28 (m, 2H), 3.12-3.07 (m, 2H), 2.93-2.90 (m, 5H), 2.00-1.84 (m, 9H). |
| 228 | 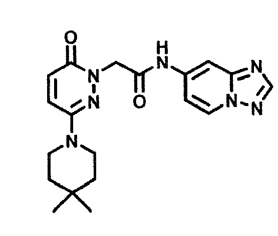 | ¹H-NMR (400 MHz, CDCl₃) δ : 10.26 (s, 1H), 8.39 (d, J = 7.3 Hz, 1H), 8.25 (s, 1H), 8.12 (d, J = 1.8 Hz, 1H), 7.28-7.25 (m, 1H), 7.22 (d, J = 7.3 Hz, 1H), 6.95 (d, J = 9.8 Hz, 1H), 4.91 (s, 2H), 3.35-3.25 (m, 4H), 1.48-1.38 (m, 4H), 0.98 (s, 6H). |
| 229 | 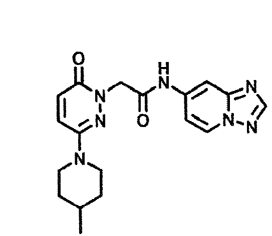 | ¹H-NMR (400 MHz, CDCl₃) δ : 10.01 (s, 1H), 8.42 (d, J = 7.3 Hz, 1H), 8.24 (s, 1H), 8.11 (d, J = 2.4 Hz, 1H), 7.35-7.21 (m, 1H), 7.12 (dd, J = 7.6, 2.1 Hz, 1H), 6.95 (d, J = 9.8 Hz, 1H), 4.87 (s, 2H), 3.85 (d, J = 13.4 Hz, 2H), 2.78 (td, J = 12.8, 2.4 Hz, 2H), 1.72 (d, J = 12.8 Hz, 2H), 1.27-1.17 (m, 3H), 0.97 (d, J = 6.1 Hz, 3H). |
| 230 | 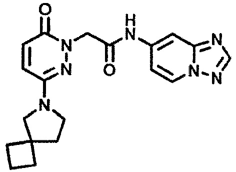 | ¹H-NMR (400 MHz, CDCl₃) δ : 10.11 (s, 1H), 8.44 (d, J = 7.3 Hz, 1H), 8.26 (s, 1H), 8.12 (d, J = 1.8 Hz, 1H), 7.15 (dd, J = 7.3, 2.4 Hz, 1H), 7.05 (d, J = 10.4 Hz, 1H), 6.95 (d, J = 10.4 Hz, 1H), 4.89 (s, 2H), 3.40 (t, J = 7.0 Hz, 2H), 3.36 (s, 2H), 2.10-1.90 (m, 8H). |
| 231 | 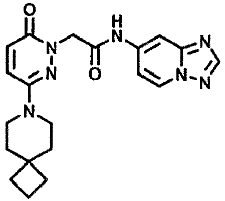 | ¹H-NMR (400 MHz, CDC1₃) δ : 10.07 (s, 1H), 8.40 (d, J = 7.3 Hz, 1H), 8.24 (s, 1H), 8.10 (d, J = 1.2 Hz, 1H), 7.30-7.24 (m, 1H) ,7.15-7.11 (m, 1H), 6. 95 (d, J = 9.8 Hz, 1H), 4.89 (s, 2H), 3.27-3.24 (m, 4H), 1.98-1.89 (m, 2H), 1.85-1.77 (m, 4H), 1.68-1.65 (m, 4H). |
| 232 | 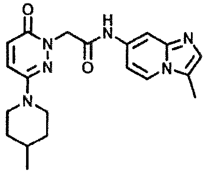 | LC-MS: [M+H]⁺ / Rt (min) 381.2 / 1.54 (Method B) |
| 233 | 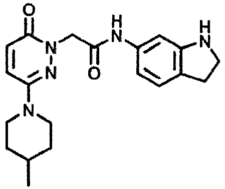 | ¹H-NMR (400 MHz, CDCl₃) δ : 8.87 (s, 1H), 7.20 (d, J = 10.1 Hz, 1H), 7.05 (d, J = 1.8 Hz, 1H), 6.98 (d, J = 7.6 Hz, 1H), 6.90 (d, J = 10.1 Hz, 1H), 6.64 (dd, J = 7.6, 1.8 Hz, 1H), 4.79 (s, 2H), 3.84-3.77 (m, 3H), 3.54 (t, J = 8.2 Hz, 2H), 2.95 (t, J = 8.2 Hz, 2H), 2.79-2.72 (m, 2H) , 1.72-1.69 (m, 2H), 1.58-1.51 (m, 1H), 1.27-1.17 (m, 2H), 0.96 (d, J = 6.7 Hz, 3H). |
| 234 | 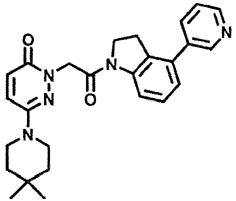 | ¹H-NMR (400MHz, DMSO-d₆) δ : 8.72 (s, 1H), 8.60 (d, J = 4.9 Hz, 1H), 8.07 (d, J = 7.9 Hz, 1H), 7.94 (d, J = 7.9 Hz, 1H), 7.59 (d, J = 9.8 Hz, 1H), 7.51 (dd, J = 7.9, 4.9 Hz, 1H), 7.32 (t, J = 7.9 Hz, 1H), 7.13 (d, J = 7.3 Hz, 1H), 6.87 (d, J = 9.8 Hz, 1H), 4.89 (s, 2H), 4.21 (t, J = 8.2 Hz, 2H), 3.29-3.23 (m, 6H), 1.39-1.36 (m, 4H) , 0.94 (s, 6H). |
| 235 | 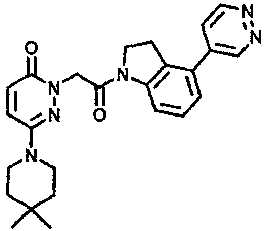 | ¹H-NMR (400MHz, DMSO-d₆) δ: 9.45 (dd, J = 2.4, 1.2 Hz, 1H), 9.32 (dd, J = 5.5, 1.2 Hz, 1H), 8.15 (d, J = 7.9 Hz, 1H), 7.87 (dd, J = 4.9, 2.4 Hz, 1H), 7.59 (d, J = 10.4 Hz, 1H), 7.38 (t, J = 7.9 Hz, 1H), 7.28-7.25 (m, 1H), 6.87 (d, J = 10.4 Hz, 1H), 4.91 (s, 2H), 4.23 (t, J = 8.2 Hz, 2H), 3.36 (t, J = 8.2 Hz, 2H), 3.26-3.23 (m, 4H), 1.37 (t, J = 5.8 Hz, 4H), 0.94 (s, 6H) . |
| 236 | 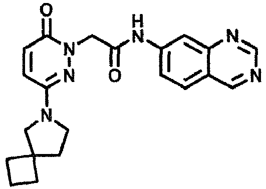 | ¹H-NMR (400 MHz, CDCl₃) δ: 10.18 (s, 1H), 9.15 (s, 2H), 8.23 (s, 1H), 7.75-7.65 (m, 2H), 7.01-6.92 (m, 2H), 4.93 (s, 2H), 3.41-3.35 (m, 2H), 2.08-1.87 (m, 10H) . |
| 237 | 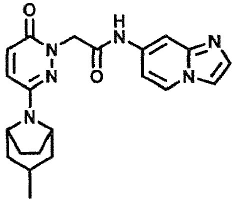 | ¹H-NMR (400 MHz, CD₃OD)δ: 8.32 (s, 1H), 7.97 (s, 1H), 7.70 (s, 1H), 7.52-7.37 (m, 2H), 7.06 (s, 1H), 6.89 (s, 1H), 4.95-4.85 (m, 2H), 4.27 (s, 2H), 2.15-1.90 (m, 3H), 1.90-1.70 (m, 3H), 1.55-1.40 (m, 3H), 1.24 (d, J = 6.0 Hz, 3H). |
| 238 | 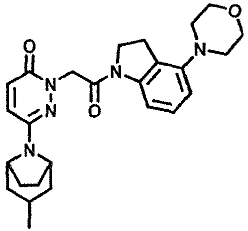 | ¹H-NMR (400 MHz, CDCl₃) δ: 7.98 (d, J = 7.3 Hz, 1H), 7.18 (t, J = 8.2 Hz, 1H), 7.06 (d, J = 9.8 Hz, 1H), 6.88 (d, J = 9.8 Hz, 1H), 6.74 (d, J = 7.3 Hz, 1H), 4.90 (s, 2H), 4.19-4.09 (m, 4H), 3.89 (t, J = 25.6 Hz, 4H), 3.24 (s, 2H), 3.04 (s, 4H), 2.05-1.90 (m, 2H), 1.78-1.70 (m, 2H), 1.30-1.22 (m, 3H), 0.90-0.85 (m, 2H), 0.83 (d, J = 6.7 Hz, 3H). |

### Examples 239 to 243

According to the method of Example 1, 37, or 50 and common reaction conditions, the compounds of Examples 239 to 243 were obtained by using corresponding material compounds.

| 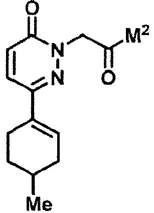 | | |
|---|---|---|
| Example | M² | Analytical data |
| 239 | 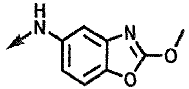 | ¹H-NMR, (400 MHz, CDCl₃) δ: 8.87 (s, 1H), 7.76 (s, 1H), 7.60 (d, J = 9.8 Hz, 1H), 7.24 (br s, 2H), 6.98 (d, J = 9.8 Hz, 1H), 6.37 (br s, 1H), 4.97 (s, 2H), 4.21 (s, 3H), 2.65-2.62 (br m, 1H), 2.37-2.30 (br m, 2H), 1.89-1.81 (br m, 2H), 1.76-1.67 (br m, 1H), 1.34-1.24 (m, 1H), 1.01 (d, J = 6.1 Hz, 3H). |
| 240 | 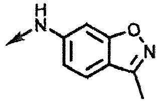 | ¹H-NMR, (400 MHz, CDCl₃) δ: 9.18 (s, 1H), 8.02-7.99 (m, H1), 7.59 (d, J = 9.8 Hz, 1H), 7.48 (d, J = 8.6 Hz, 1H), 7.26-7.22 (m, H1), 6.98 (d, J = 9.8 Hz, 1H), 6.38 (s, 1H), 4.98 (s, 2H), 2.68-2.59 (m, 1H), 2.52 (s, 3H), 2.43-2.31 (m, 2H), 1.92-1.82 (m, 2H), 1.8-1.70 (m, 1H), 1.39-1.27 (m, 1H), 1.02 (d, J = 6.1 Hz, 3H). |
| 241 | 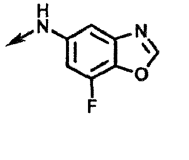 | ¹H-NMR, (400 MHz, CDCl₃) δ: 9.28 (s, 1H), 8.06 (s, 1H), 7.71 (s, 1H), 7.62 (d, J = 9.8 Hz, 1H), 7.50 (d, J = 11.6 Hz, 1H), 6.99 (d, J = 9.8 Hz, 1H), 6.38 (s, 1H), 4.99 (s, 2H), 2.73-2.57 (m, 1H), 2.38-2.27 (m, 2H), 1.90-1.78 (m, 2H), 1.75-1.57 (m, 2H), 1.01 (d, J = 6.7 Hz, 3H). |
| 242 | 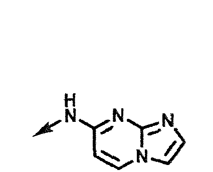 | ¹H-NMR (400 MHz, CDCl₃) δ: 9.38 (s, 1H), 8.33 (d, J = 7.3 Hz, 1H), 7.99 (d, J = 7.3 Hz, 1H), 7.68 (d, J = 1.2 Hz, 1H), 7.59 (d, J = 9.8 Hz, 1H), 7.44 (d, J = 1.2 Hz, 1H), 6.97 (d, J = 9.8 Hz, 1H), 6.38 (s, 1H), 5.14 (s, 2H), 2.65-2.55 (m, 1H), 2.37-2.25 (m, 2H), 1.91-1.80 (m, 2H), 1.78-1.65 (m, 1H), 1.35-1.20 (m, 1H), 1.00 (d, J = 6.1 Hz, 3H). |
| 243 | 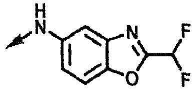 | ¹H-NMR (400 MHz, CDCl₃) δ: 9.34 (s, 1H), 8.11 (s, 1H), 7.62 (d, J = 9.8 Hz, 1H), 7.48-7.42 (m, 2H), 6.99 (d, J = 9.8 Hz, 1H), 6.76 (t, J = 52.4 Hz, 1H), 6.38 (s, 1H), 5.02 (s, 2H), 2.68-2.53 (m, 1H), 2.40-2.28 (m, 2H), 1.90-1.79 (m, 2H), 1.48-1.42 (m, 1H), 1.35-1.24 (m, 1H), 1.01 (d, J = 6.1 Hz, 3H) . |

### Example 244

### N-[2-(Dimethylamino)-1,3-benzooxazol-5-yl]-2-[3-(4-methylcyclohex-1-en-1-yl)-6-oxopyridazin-1(6H)-yl]acetamide

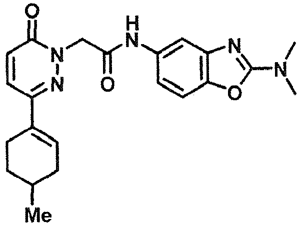

To a suspension of the compound of Reference example 16 (45 mg), N²,N²-dimethyl-1,3-benzoxazole-2,5-diamine (39 mg), and HATU (90 mg) in acetonitrile (1.8 mL) was added N,N-diisopropylethylamine (0.13 mL), and the mixture was stirred at room temperature for 2 hours. After the addition of saturated aqueous ammonium chloride, the mixture was extracted with ethyl acetate. The organic layer was dried over anhydrous sodium sulfate, filtered, and concentrated under reduced pressure. The residue was purified by silica gel column chromatography (solvent; ethyl acetate:methanol = 100:0, and then 96:4) to obtain the titled compound (75 mg) .
¹H-NMR (400 MHz, CDCl₃) δ: 8.68 (s, 1H), 7.60 (d, J = 9.8 Hz, 1H), 7.50 (s, 1H), 7.21-7.14 (m, 2H), 6.98 (d, J = 9.8 Hz, 1H), 6.38 (s, 1H), 4.97 (s, 2H), 3.20 (s, 6H), 2.70-2.60 (m, 1H), 2.41-2.30 (m, 2H), 1.92-1.82 (m, 2H), 1.80-1.67 (m, 1H), 1.35-1.25 (m, 1H), 1.03 (d, J = 6.1 Hz, 3H).

### Examples 245 to 424

According to the method of Example 1, 37, or 50 and common reaction conditions, the compounds of Examples 245 to 424 were obtained by using corresponding material compounds.

| 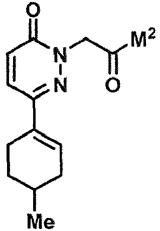 | | |
|---|---|---|
| Example | M² | Analytical data |
| 245 | 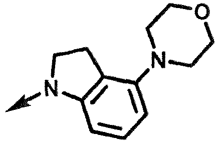 | ¹H-NMR (400 MHz, CDCl₃) δ: 7.91 (d, J =7.9 Hz, 1H), 7.55 (d, J = 9.8 Hz, 1H), 7.16 (t, J = 8.2 Hz, 1H), 6.92 (d, J = 9.8 Hz, 1H), 6.69 (d, J = 7.9 Hz, 1H), 6.33-6.32 (br m, 1H), 5.01 (d, J = 3. 7 Hz, 2H), 4.19 (t, J = 8.2 Hz, 2H), 3.87-3.84 (m, 4H), 3.19 (t, J = 8.2 Hz, 2H), 2.99-2.97 (m, 4H), 2.61-2.56 (m, 1H), 2.34-2.25 (m, 2H), 1.88-1.78 (m, 2H), 1.74-1.65 (m, 1H), 1.32-1.22 (m, 1H), 0.99 (d, J = 6.1 Hz, 3H). |
| 246 | 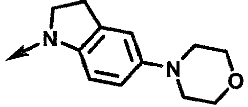 | LC-MS: [M+H]⁺ / Rt (min) 435.4 / 1.308 (Method A) |
| 247 | 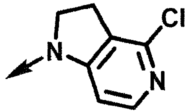 | LC-MS: [M+H]⁺ / Rt (min) 385.3 / 1.019 (Method A) |
| 248 | 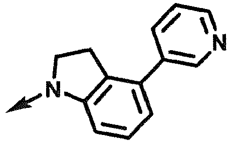 | ¹H-NMR (400 MHz, DMSO-d₆) δ: 8.73 (d, J = 1.8 Hz, 1H), 8.60 (d, J = 4.9 Hz, 1H), 8.06 (d, J = 7.9 Hz, 1H), 7.95 (d, J = 7.9 Hz, 1H), 7.90 (d, J = 10.4 Hz, 1H), 7.51 (dd, J = 7.9, 4.9 Hz, 1H), 7.32 (t, J = 7.6 Hz, 1H), 7.14 (d, J = 7.3 Hz, 1H), 6.96 (d, J = 9.8 Hz, 1H), 6.55 (s, 1H), 5.07 (s, 2H), 4.25 (t, J = 8.5 Hz, 2H), 3.32-3.27 (m, 2H), 2.67-2.54 (m, 2H), 2.37-2.19 (m, 2H), 1.87-1.62 (m, 3H), 0.98 (d, J = 6.7 Hz, 3H) . |
| 249 | 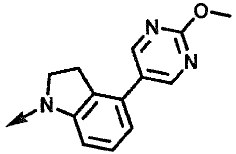 | ¹H-NMR (400 MHz, DMSO-d₆) δ: 8.79 (s, 2H), 8.05 (d, J = 7.9 Hz, 1H), 7.89 (d, J = 10.4 Hz, 1H), 7.31 (t, J = 7.9 Hz, 1H), 7.15 (d, J = 7.3 Hz, 1H), 6.95 (d, J = 9.8 Hz, 1H), 6.56-6.52 (m, 1H), 5.06 (s, 2H), 4.25 (t, J = 8.2 Hz, 2H), 3.98 (s, 3H), 3.34-3.29 (m, 2H), 2.56-2.51 (m, 1H), 2.36-2.18 (m, 2H), 1.87-1.60 (m, 3H), 1.29-1.19 (m, 1H), 0.97 (d, J = 6.7 Hz, 3H). |
| 250 | 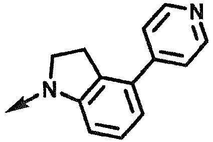 | ¹H-NMR (400 MHz, DMSO-d₆) δ: 8.67-8.65 (m, 2H), 8.09 (d, J = 7.9 Hz, 1H), 7.89 (d, J = 9.8 Hz, 1H), 7.55-7.53 (m, 2H), 7.33 (t, J = 7.9 Hz, 1H), 7.17 (d, J = 6.7 Hz, 1H), 6.96 (d, J = 10.4 Hz, 1H), 6.56-6.53 (m, 1H), 5.07 (s, 2H), 4.25 (t, J = 8.2 Hz, 2H), 3.35-3.31 (m, 1H), 2.56-2.51 (m, 1H), 2.37-2.18 (m, 2H), 1.87-1.61 (m, 3H), 1.29-1.18 (m, 1H), 1.16-1.12 (m, 1H), 0.97 (d, J = 6.1 Hz, 3H). |
| 251 | 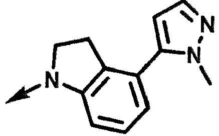 | LC-MS: [M+H]⁺ / Rt (min) 430.2 / 0.965 (Method A) |
| 252 | 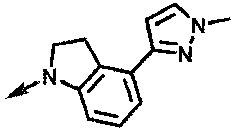 | ¹H-NMR (400 MHz, DMSO-d₆) δ: 7.95 (d, J = 7.9 Hz, 1H), 7.89 (d, J = 9.8 Hz, 1H), 7.78 (d, J = 2.4 Hz, 1H), 7.45 (d, J = 7.9 Hz, 1H), 7.20 (t, J = 7.9 Hz, 1H), 6.95 (d, J = 10.4 Hz, 1H), 6.67 (d, J = 2.4 Hz, 1H), 6.55-6.53 (m, 1H), 5.05 (s, 2H), 4.27 (t, J = 8.2 Hz, 2H), 3.91 (s, 3H), 3.46 (t, J = 8.5 Hz, 2H), 2.58-2.51 (m, 1H), 2.37-2.18 (m, 2H), 1.88-1.62 (m, 3H), 1.29-1.18 (m, 1H), 0.97 (d, J = 5.8 Hz, 3H) . |
| 253 | 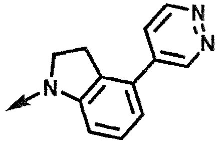 | ¹H-NMR (400 MHz, CDCl₃) δ: 9.32-9.26 (m, 2H), 8.34 (d, J = 8.5 Hz, 1H), 7.58-7.52 (m, 2H), 7.35 (t, J = 7.9 Hz, 1H), 7.11 (d, J = 7.7 Hz, 1H), 6. 93 (d, J = 9.5 Hz, 1H), 6.35-6.33 (m, 1H), 5.09-5.00 (m, 2H), 4.25 (t, J = 8.2 Hz, 2H), 3.36 (t, J = 8.2 Hz, 2H), 2.62-2.54 (m, 1H), 2.37-2.25 (m, 2H), 2.02-1.64 (m, 4H), 1.00 (d, J = 6.5 Hz, 3H). |
| 254 | 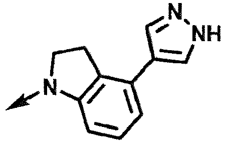 | LC-MS: [M+H]⁺ / Rt (min) 416.3 / 0.933 (Method A) |
| 255 | 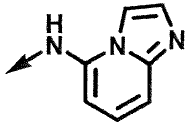 | ¹H-NMR (400 MHz, CDCl₃) δ: 10.20 (s, 1H), 7.72 (s, 1H), 7.66 (t, J = 4.0 Hz, 2H), 7.45 (t, J = 9.1 Hz, 2H), 7.22 (dd, J = 8.8, 7.6 Hz, 1H), 7.08 (d, J = 9.8 Hz, 1H), 6.41 (s, 1H), 5.09 (s, 2H), 2.68-2.59 (m, 1H), 2. 40-2.28 (m, 2H), 1.91-1.80 (m, 2H), 1.79-1.65 (m, 1H), 1.39-1.22 (m, 1H), 1.01 (d, J = 6.7 Hz, 3H). |
| 256 | 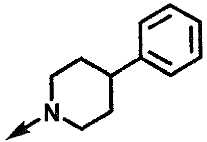 | LC-MS: [M+H]⁺ / Rt (min) 392.2 / 2.01 (Method B) |
| 257 | 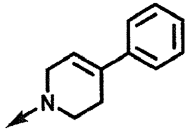 | ¹H-NMR (400 MHz, CDCl₃) δ: 7.53 (d, J = 9.8 Hz, 1H), 7.41-7.28 (m, 5H), 6.91 (d, J = 9.8 Hz, 1H), 6.31 (br s, 1H), 6.08-6.02 (br m, 1H), 5.05 (d, J = 3.7 Hz, 1H), 4.99 (d, J = 3.7 Hz, 1H), 4.27 (d, J = 2.8 Hz, 1H), 4.20 (d, J = 2.8 Hz, 1H), 3.87-3.84 (br m, 1H), 3.74-3.71 (br m, 1H), 2.66-2.56 (m, 3H), 2.34-2.26 (m, 2H), 1.87-1.78 (m, 2H), 1.73-1.65 (m, 1H), 1.32-1.22 (m, 1H), 0.99 (d, J = 6.7 Hz, 3H) . |
| 258 | 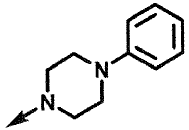 | ¹H-NMR (400 MHz, CDCl₃) δ: 7.53 (d, J = 9.8 Hz, 1H), 7.33-7.29 (m, 2H), 7.04-6.92 (m, 3H), 6.90 (d, J = 9.8 Hz, 1H), 6.32 (br s, 1H), 5.00 (br s, 2H), 3.84-3.72 (br m, 4H), 3.28-3.22 (br m, 4H), 2.62-2.56 (m, 1H), 2.35-2.25 (m, 2H), 1.87-1.79 (m, 2H), 1.74-1.65 (m, 1H), 1.33-1.22 (m, 1H), 1.00 (d, J = 6.1 Hz, 3H) . |
| 259 | 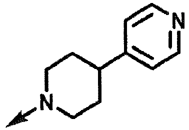 | ¹H-NMR (400 MHz, CDCl₃) δ: 8.60-8.56 (m, 2H), 7.54 (dd, J = 9.8, 1.2 Hz, 1H), 7.36-7.25 (br m, 2H), 6.90 (d, J = 9.8 Hz, 1H), 6.32 (br s, 1H), 5.08-5.02 (m, 1H), 4.96-4.90 (br m, 1H), 4.81-4.77 (br m, 1H), 4.01-3.98 (br m, 1H), 3.30-3.24 (br m, 1H), 2.92-2.79 (br m, 1H), 2.77-2.71 (br m, 1H), 2.61-2.57 (br m, 1H), 2.35-2.29 (br m, 2H), 2.00-1.91 (m, 2H), 1.87-1.70 (m, 5H), 1.33-1.24 (m, 1H), 1.00 (d, J = 6.1 Hz, 3H). |
| 260 | 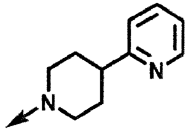 | ¹H-NMR (400 MHz, CDCl₃) δ: 8.59-8.57 (m, 1H), 7.82-7.78 (br m, 1H), 7.53 (d, J = 10.1 Hz, 1H), 7.30-7.27 (br m, 2H), 6.89 (d, J = 10.1 Hz, 1H), 6.32-6.30 (m, 1H), 5.05-4.93 (m, 2H), 4.77-4.74 (br m, 1H), 3.99-3.95 (br m, 1H), 3.34-3.27 (m, 1H), 3.19-3.11 (br m, 1H), 2.83-2.75 (m, 1H), 2.62-2.57 (br m, 1H), 2.35-2.28 (m, 2H), 2.14-2.11 (br m, 1H), 2.03-2.00 (br m, 1H), 1.92-1.77 (m, 4H), 1.75-1.66 (m, 1H), 1.33-1.23 (br m, 1H), 1.00 (d, J = 6.7 Hz, 3H) . |
| 261 | 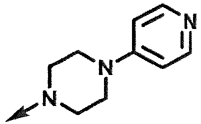 | ¹H-NMR (400 MHz, CD₃OD) δ: 8.17 (d, J = 7.0 Hz, 2H), 7.85 (d, J = 9.8 Hz, 1H), 7.02 (d, J = 7.0 Hz, 2H), 6.94 (d, J = 9.8 Hz, 1H), 6.52-6.50 (br m, 1H), 5.11 (s, 2H), 3.84-3.77 (br m, 4H), 3.73-3.71 (br m, 2H), 3. 66-3. 63 (br m, 2H), 2.65-2.58 (m, 1H), 2.40-2.26 (m, 2H), 1.91-1.81 (m, 2H), 1.72 (br s, 1H), 1.35-1.25 (m, 1H), 1.02 (d, J = 6.7 Hz, 3H) . |
| 262 | 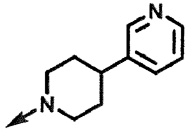 | ¹H-NMR (400 MHz, CD₃OD) δ: 8.62 (br s, 1H), 8.53 (br s, 1H), 8.14-8.08 (m, 1H), 7.85 (d, J = 9.8 Hz, 1H), 7.68-7.63 (m, 1H), 6.95 (d, J = 9.8 Hz, 1H), 6.52-6.50 (br m, 1H), 5.18 (dd, J = 15.9, 3.7 Hz, 1H), 5.05-5.00 (m, 1H), 4.68-4.64 (br m, 1H), 4.17-4.12 (m, 1H), 3.38-3.30 (br m, 2H), 3.10-3.03 (m, 1H), 2.88-2.81 (m, 1H), 2.66-2.60 (br m, 1H), 2.41-2.28 (m, 2H), 2.02-1.83 (m, 4H), 1.78-1.67 (m, 2H), 1.36-1.26 (m, 1H), 1.02 (d, J = 6.7 Hz, 3H). |
| 263 | 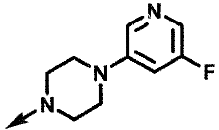 | LC-MS: [M+H]⁺ / Rt (min) 412.3 / 0.803 (Method A) |
| 264 | 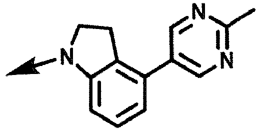 | ¹H-NMR (400 MHz, DMSO-d₆) δ: 8.87 (s, 2H), 8.08 (d, J = 7.9 Hz, 1H), 7.89 (d, J = 9.8 Hz, 1H), 7.33 (t, J = 7.9 Hz, 1H), 7.18 (d, J = 6.7 Hz, 1H), 6.96 (d, J = 9.8 Hz, 1H), 6.54 (s, 1H), 5.07 (s, 2H), 4.25 (t, J = 8.2 Hz, 2H), 3.36-3.33 (m, 2H), 2.70-2.64 (m, 4H), 2.37-2.18 (m, 2H), 1.88-1.62 (m, 3H), 1.29-1.20 (m, 1H), 0.98 (d, J = 6.7 Hz, 3H). |
| 265 | 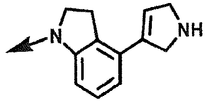 | LC-MS: [M+H]⁺ / Rt (min) 417.4 / 0.728 (Method A) |
| 266 | 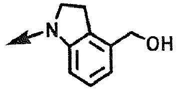 | LC-MS: [M+H]⁺ / Rt (min) 380.4 / 0.892 (Method A) |
| 267 | 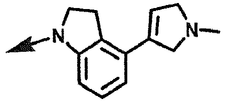 | LC-MS: [M+H]⁺ / Rt (min) 431.4 / 0.793 (Method A) |
| 268 | 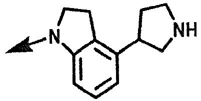 | LC-MS: [M+H]⁺ / Rt (min) 419.5 / 0.767 (Method A) |
| 269 | 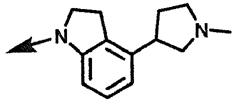 | ¹H-NMR (400 MHz, DMSO-d₆) δ: 7.88 (d, J = 9.8 Hz, 1H), 7.81 (d, J = 7.9 Hz, 1H), 7.12 (t, J = 7.6 Hz, 1H), 7.03 (d, J = 7.9 Hz, 1H), 6.94 (d, J = 9.8 Hz, 1H), 6.54-6.52 (m, 1H), 5.02 (s, 2H), 4.22 (t, J = 8.5 Hz, 2H), 3.39-3.32 (m, 3H), 3.19 (t, J = 8.2 Hz, 2H), 2.81 (t, J = 8.5 Hz, 1H), 2.67-2.42 (m, 5H), 2.36-2.18 (m, 3H), 1. 85-1. 61 (m, 4H), 1.28-1.17 (m, 1H), 0.97 (d, J = 6.7 Hz, 3H). |
| 270 | 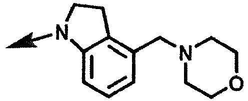 | ¹H-NMR (400 MHz, DMSO-d₆) δ: 7.88 (d, J = 9.8 Hz, 2H), 7.12 (t, J = 7.6 Hz, 1H), 6.98-6.93 (m, 2H), 6.55-6.52 (m, 1H), 5.03 (s, 2H), 4.24 (t, J = 8.5 Hz, 2H), 3.56 (t, J = 4.3 Hz, 4H), 3.42 (s, 2H), 3.23 (t, J = 8.2 Hz, 2H), 2.55-2.50 (m, 1H), 2.38-2.16 (m, 6H), 1.87-1.74 (m, 2H), 1.72-1.60 (m, 1H), 1.28-1.17 (m, 1H), 0.97 (d, J = 6.7 Hz, 3H) . |
| 271 | 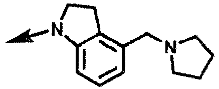 | LC-MS: [M+H]⁺ / Rt (min) 433.4 / 0.848 (Method A) |
| 272 | 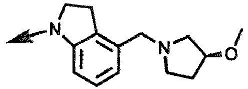 | ¹H-NMR (400 MHz, CDCl₃) δ: 8.09 (d, J = 7. 9 Hz, 1H), 7.55 (d, J = 10.4 Hz, 1H), 7.13 (t, J = 7. 9 Hz, 1H), 7. 00 (d, J = 7.3 Hz, 1H), 6.92 (d, J = 9.8 Hz, 1H), 6.33 (s, 1H), 5.05-4.97 (m, 2H), 4.19 (t, J = 8.2 Hz, 2H), 3.95-3.89 (m, 1H), 3.59-3.52 (m, 2H), 2.76-2.44 (m, 5H), 2.34-2.27 (m, 2H), 2.11-2.02 (m, 1H), 1.87-1.62 (m, 10H), 0.99 (d, J = 6.7 Hz, 3H). |
| 273 | 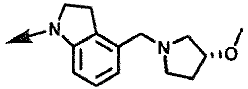 | ¹H-NMR (400 MHz, CDCl₃) δ: 8.09 (d, J = 7.9 Hz, 1H), 7.55 (d, J = 9.8 Hz, 1H), 7.15-7.11 (m, 1H), 7.02-6.98 (m, 1H), 6.92 (d, J = 9.8 Hz, 1H), 6.32 (s, 1H), 5.06-4.97 (m, 2H), 4.19 (t, J = 8.2 Hz, 2H), 3.94-3.89 (m, 1H), 3.60-3.53 (m, 3H), 2.76-2.45 (m, 6H), 2.35-2.27 (m, 2H), 1.86-1.57 (m, 9H), 0.99 (d, J = 6.7 Hz, 3H) . |
| 274 | 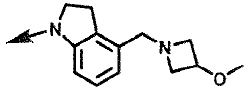 | ¹H-NMR (400 MHz, DMSO-d₆) δ: 7.87 (t, J = 9.4 Hz, 2H), 7.11 (t, J = 7.9 Hz, 1H), 6.96-6.93 (m, 2H), 6.53 (s, 1H), 5.02 (s, 2H), 4.23 (t, J = 8.2 Hz, 2H), 4.00-3.94 (m, 1H), 3.53 (s, 4H), 3.49-3.45 (m, 4H), 3.20-3.15 (m, 5H), 2.87-2.83 (m, 2H), 2.70-2.54 (m, 1H), 2.36-2.17 (m, 2H), 1.88-1.74 (m, 2H), 1.70-1.60 (m, 1H), 1.29-1.18 (m, 1H), 0.97 (d, J = 6.7 Hz, 3H). |
| 275 | 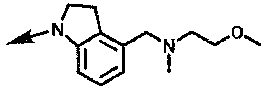 | ¹H-NMR (400 MHz, DMSO-d₆) δ: 7.90-7.86 (m, 2H), 7.11 (t, J = 7.6 Hz, 1H), 6.97-6.93 (m, 2H), 6.53 (s, 1H), 5.03 (s, 2H), 4.23 (t, J = 8.5 Hz, 2H), 3.46-3.42 (m, 4H), 3.23-3.19 (m, 5H), 2.53-2.51 (m, 3H), 2.36-2.17 (m, 2H), 2.14 (s, 3H), 1.86-1.74 (m, 2H), 1.72-1.60 (m, 1H), 1.28-1.17 (m, 1H), 0.97 (d, J = 6.1 Hz, 3H) . |
| 276 | 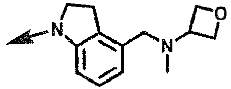 | ¹H-NMR (400 MHz, DMSO-d₆) δ: 7.91-7.87 (m, 2H), 7.13 (t, J = 7.6 Hz, 1H), 6.96 (t, J = 7.9 Hz, 2H), 6.54 (s, 1H), 5.03 (s, 2H), 4.51 (t, J = 6.4 Hz, 2H), 4.43 (t, J = 6.1 Hz, 2H), 4.25 (t, J = 8.5 Hz, 2H), 3.61-3.54 (m, 1H), 3.30-3.25 (m, 4H), 2.56-2.52 (m, 1H), 2.36-2.18 (m, 2H), 1.95 (s, 3H), 1.87-1.75 (m, 2H), 1.73-1.61 (m, 1H), 1.29-1.17 (m, 1H), 0.97 (d, J = 6.1 Hz, 3H) . |
| 277 | 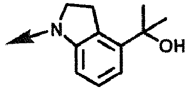 | ¹H-NMR (400 MHz, DMSO-d₆) δ: 7.93-7.87 (m, 2H), 7.12-7.07 (m, 2H), 6.94 (d, J = 10.4 Hz, 1H), 6.55-6.52 (m, 1H), 5.03 (s, 2H), 4.97 (s, 1H), 4.17 (t, J = 8.2 Hz, 2H), 3.42 (t, J = 8.5 Hz, 2H), 2.57-2.53 (m, 1H), 2.37-2.17 (m, 2H), 1.87-1.61 (m, 3H), 1.46 (s, 6H), 1.30-1.18 (m, 1H), 0.97 (d, J = 6.1 Hz, 3H). |
| 278 | 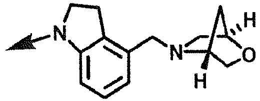 | ¹H-NMR (400 MHz, DMSO-d₆) δ: 7.89-7.85 (m, 2H), 7.11 (t, J = 7.6 Hz, 1H), 7.02 (d, J = 7.9 Hz, 1H), 6.94 (d, J = 9.8 Hz, 1H), 6.54 (s, 1H), 5.03 (s, 2H), 4.35 (s, 1H), 4.24 (t, J = 8.5 Hz, 2H), 3.91 (d, J = 7.3 Hz, 1H), 3.71-3.62 (m, 2H), 3.53-3.51 (m, 1H), 3.41 (s, 1H), 3.22 (t, J = 8.5 Hz, 2H), 2.72 (d, J = 8.5 Hz, 1H), 2.55-2.51 (m, 1H), 2.43 (d, J = 9.8 Hz, 1H), 2.36-2.13 (m, 2H), 1.87-1.74 (m, 3H), 1.71-1.56 (m, 2H), 1.28-1.18 (m, 1H), 0.97 (d, J = 6.7 Hz, 3H). |
| 279 | 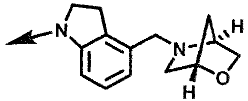 | ¹H-NMR (400 MHz, DMSO-d₆) δ: 7.88-7.83 (m, 2H), 7.10 (t, J = 7.6 Hz, 1H), 7.01 (d, J = 7.9 Hz, 1H), 6.93 (d, J = 9.8 Hz, 1H), 6.52 (s, 1H), 5.01 (s, 2H), 4.33 (s, 1H), 4.22 (t, J = 8.5 Hz, 2H), 3.89 (d, J = 7.3 Hz, 1H) , 3.70-3.60 (m, 2H), 3.52-3.50 (m, 1H), 3.39 (s, 1H), 3.20 (t, J = 8.2 Hz, 2H), 2.70 (d, J = 8.5 Hz, 1H), 2.55-2.51 (m, 1H), 2.41 (d, J = 9.8 Hz, 1H), 2.35-2.16 (m, 2H), 1.84-1.73 (m, 3H), 1.70-1.56 (m, 2H), 1.27-1.18 (m, 1H), 0.96 (d, J = 6.7 Hz, 3H). |
| 280 | 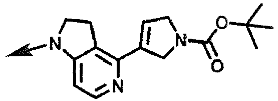 | LC-MS: [M+H]⁺ / Rt (min) 518.4 / 1.075 (Method A) |
| 281 | 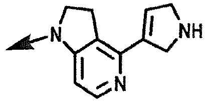 | LC-MS: [M+H]⁺ / Rt (min) 418.5 / 0.884 (Method A) |
| 282 | 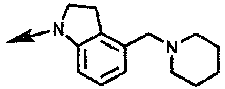 | ¹H-NMR (400 MHz, DMSO-d₆) δ: 7.89-7.85 (m, 2H), 7.11 (t, J = 7.6 Hz, 1H), 6.96-6.93 (m, 2H), 6.53 (s, 1H), 5.02 (s, 2H), 4.23 (t, J = 8.2 Hz, 2H), 3.37 (s, 2H), 3.21 (t, J = 8.5 Hz, 2H), 2.55-2.52 (m, 1H), 2.37-2.16 (m, 6H), 1.87-1. 60 (m, 3H), 1.51-1.34 (m, 6H), 1.28-1.18 (m, 1H), 0.97 (d, J = 6.7 Hz, 3H). |
| 283 | 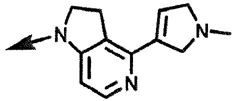 | ¹H-NMR (400 MHz, DMSO-d₆) δ: 8.31-8.28 (m, 1H), 7.88 (d, J = 10.4 Hz, 1H), 7.73 (d, J = 4.9 Hz, 1H), 6.95 (d, J = 9.8 Hz, 1H), 6.53 (s, 1H), 6.30 (s, 1H), 5.07 (s, 2H), 4.30 (t, J = 8.2 Hz, 2H), 3.87-3.84 (m, 2H), 3.64-3.61 (m, 2H), 3.36-3.31 (m, 2H), 2.54-2.50 (m, 1H), 2.42 (s, 3H), 2.35-2.16 (m, 2H), 1.86-1.73 (m, 2H), 1.70-1.60 (m, 1H), 1.29-1.16 (m, 1H), 0.96 (d, J = 6.7 Hz, 3H). |
| 284 | 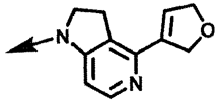 | ¹H-NMR (400 MHz, DMSO-d₆) δ: 8.30 (d, J = 5.5 Hz, 1H), 7.89 (d, J = 9.8 Hz, 1H), 7.76 (d, J = 5.5 Hz, 1H), 6.95 (d, J = 9.8 Hz, 1H), 6.54-6.49 (m, 2H), 5.08 (s, 2H), 5.02-4.99 (m, 2H), 4.84-4.81 (m, 2H), 4.33 (t, J = 8.2 Hz, 2H), 3.40-3.34 (m, 2H), 2.35-2.16 (m, 2H), 1.86-1.74 (m, 2H), 1.70-1.60 (m, 1H), 1.28-1.16 (m, 2H), 0.96 (d, J = 6.1 Hz, 3H) . |
| 285 | 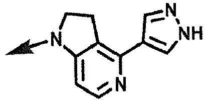 | ¹H-NMR (400 MHz, DMSO-d₆) δ: 13.15 (s, 1H), 8.30 (d, J = 5.5 Hz, 1H), 8.20 (s, 1H), 8.02 (s, 1H), 7.88 (d, J = 9.8 Hz, 1H), 7.67 (d, J = 4.9 Hz, 1H), 6.95 (d, J = 9.8 Hz, 1H), 6.53 (s, 1H), 5.08 (s, 2H), 4.34 (t, J = 8.2 Hz, 2H), 3.38 (t, J = 8.5 Hz, 2H), 2.54-2.50 (m, 1H), 2.36-2.15 (m, 2H), 1.86-1.72 (m, 2H), 1.70-1.59 (m, 1H), 1.29-1.16 (m, 1H), 0.96 (d, J = 6.7 Hz, 3H) . |
| 286 | 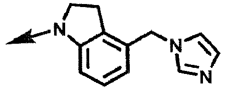 | ¹H-NMR (400 MHz, DMSO-d₆) δ: 7.92-7.87 (m, 2H), 7.73 (s, 1H), 7.18-7.13 (m, 2H), 6.96-6.92 (m, 2H), 6.77 (d, J = 7.3 Hz, 1H), 6.53 (s, 1H), 5.19 (s, 2H), 5.03 (s, 2H), 4.25 (t, J = 8.2 Hz, 2H), 3.14 (t, J = 8.2 Hz, 2H), 2.69-2.53 (m, 1H), 2.38-2.15 (m, 2H), 1.88-1.59 (m, 3H), 1.29-1.17 (m, 1H), 0.97 (d, J = 6.7 Hz, 3H). |
| 287 | 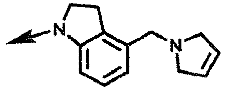 | ¹H-NMR (400 MHz, CDCl₃) δ: 8.26 (d, J = 7.9 Hz, 1H), 7.71 (d, J = 9.8 Hz, 1H), 7.30 (t, J = 7.9 Hz, 1H), 7.20 (d, J = 7.9 Hz, 1H) , 7.08 (d, J = 10.4 Hz, 1H), 6.49 (s, 1H), 5.95 (s, 2H), 5.22-5.13 (m, 2H), 4.36 (t, J = 8.2 Hz, 2H), 3.92 (s, 2H), 3.68-3.63 (m, 1H), 3.47 (t, J = 8.2 Hz, 2H), 2.79-2.70 (m, 1H), 2.52-2.40 (m, 2H), 2.04-1.70 (m, 6H), 1.49-1.38 (m, 1H), 1.16 (d, J = 6.7 Hz, 3H). |
| 288 | 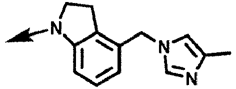 | LC-MS: [M+H]⁺ / Rt (min) 444.4 / 0.827 (Method A) |
| 289 | 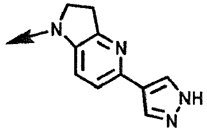 | LC-MS: [M+H]⁺ / Rt (min) 417.3 / 0.826 (Method A) |
| 290 | 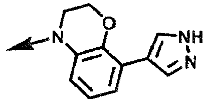 | ¹H-NMR (400 MHz, DMSO-d₆) δ: 12.93 (s, 1H), 8.14 (s, 1H), 7.96 (s, 1H), 7.86 (d, J = 9.8 Hz, 1H), 7.66-7.45 (m, 2H), 6.91-6.86 (m, 2H), 6.52 (s, 1H), 5.15 (s, 2H), 4.45-4.41 (m, 2H), 3.95 (s, 2H), 2.58-2.51 (m, 1H), 2.37-2.18 (m, 2H), 1.87-1.61 (m, 3H), 1.29-1.16 (m, 1H), 0.97 (d, J = 6.7 Hz, 3H). |
| 291 | 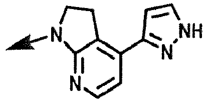 | ¹H-NMR (400 MHz, DMSO-d₆) δ: 13.36 (s, 1H), 8.20 (s, 2H), 8.11 (d, J = 5.5 Hz, 1H), 7.88 (d, J = 10.4 Hz, 1H), 7.35 (d, J = 5.5 Hz, 1H), 6.93 (d, J = 9.8 Hz, 1H), 6.54-6.52 (m, 1H), 5.48 (s, 2H), 4.05 (t, J = 8.5 Hz, 2H), 2.37-2.15 (m, 2H), 1. 87-1. 60 (m, 3H), 1.30-1.15 (m, 4H), 0.97 (d, J = 6.7 Hz, 3H). |
| 292 | 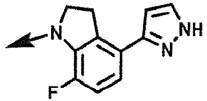 | ¹H-NMR (400 MHz, DMSO-d₆) δ: 13.07 (s, 1H), 8.07 (br s, 2H), 7.88 (d, J = 10.0 Hz, 1H), 7.37-7.33 (m, 1H), 7.10 (t, J = 9.5 Hz, 1H), 6.94 (d, J = 9.8 Hz, 1H), 6.54 (s, 1H), 5.07 (s, 2H), 4.23 (t, J = 7.6 Hz, 2H), 3.28-3.16 (m, 2H), 2.37-2.17 (m, 2H), 1.87-1.59 (m, 3H), 1.29-1.24 (m, 2H), 0.97 (d, J = 6.7 Hz, 3H) . |
| 293 | 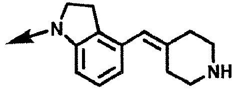 | LC-MS: [M+H]⁺ / Rt (min) 445.5 / 0.864 (Method A) |
| 294 | 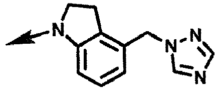 | ¹H-NMR (400 MHz, DMSO-d₆) δ: 8.62 (s, 1H), 7.96 (s, 1H), 7.91-7.84 (m, 2H), 7.13 (t, J = 7.9 Hz, 1H), 6.92 (d, J = 9.8 Hz, 1H), 6.82 (d, J = 7.3 Hz, 1H), 6.51 (s, 1H), 5.38 (s, 2H), 5.00 (s, 2H), 4.23 (t, J = 8.5 Hz, 2H), 3.29-3.27 (m, 1H), 3.17 (t, J = 8.5 Hz, 2H), 2.34-2.13 (m, 2H), 1.83-1.58 (m, 3H), 1.25-1.17 (m, 1H), 0.94 (d, J = 6.7 Hz, 3H). |
| 295 | 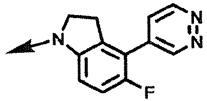 | ¹H-NMR (400 MHz, DMSO-d₆) δ: 9.40-9.37 (m, 2H), 8.09 (dd, J = 8.9, 4.6 Hz, 1H), 7.90-7.87 (m, 2H), 7.24 (t, J = 9.5 Hz, 1H), 6.95 (d, J = 9.8 Hz, 1H), 6.55-6.53 (m, 1H), 5.06 (s, 2H), 4.27 (t, J = 8.2 Hz, 2H), 3.24 (t, J = 8.2 Hz, 2H), 2.56-2.52 (m, 1H), 2.37-2.18 (m, 2H), 1.88-1.61 (m, 3H), 1.29-1.20 (m, 1H), 0.98 (d, J = 6.7 Hz, 3H). |
| 296 | 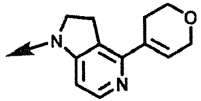 | LC-MS: [M+H]⁺ / Rt (min) 433.4 / 0.700 (Method A) |
| 297 | 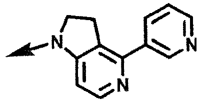 | ¹H-NMR (400 MHz, CDCl₃) δ: 8.99 (d, J = 1.8 Hz, 1H), 8.68 (dd, J = 4.9, 1.8 Hz, 1H), 8.54 (d, J = 5.5 Hz, 1H), 8.15-8.08 (m, 2H), 7.61 (d, J = 10.0 Hz, 1H), 7.45 (dd, J = 7.6, 4.6 Hz, 1H), 6.96 (d, J = 10.0 Hz, 1H), 6.39-6.36 (m, 1H), 5.07 (s, 2H), 4.31 (t, J = 8.2 Hz, 2H), 3.52 (t, J = 8.2 Hz, 2H), 2.63-2.58 (m, 1H), 2.38-2.29 (m, 2H), 1.90-1.83 (m, 2H), 1.36-1.26 (m, 2H), 1.03 (d, J = 6.1 Hz, 3H). |
| 298 | 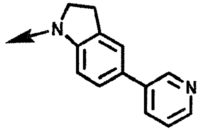 | ¹H-NMR (400 MHz, DMSO-d₆) δ: 8.85 (d, J = 1.8 Hz, 1H), 8.50 (dd, J = 4.6, 1.5 Hz, 1H), 8.05-8.01 (m, 2H), 7.87 (d, J = 9.8 Hz, 1H), 7.65 (s, 1H), 7.53 (dd, J = 8.5, 1.8 Hz, 1H), 7.43 (dd, J = 7.9, 4.9 Hz, 1H), 6.94 (d, J = 9.8 Hz, 1H), 6.53-6.51 (m, 1H), 5.04 (s, 2H), 4.27 (t, J = 8.5 Hz, 2H), 3.29-3.24 (m, 2H), 2.55-2.50 (m, 1H) , 2.35-2.15 (m, 2H), 1.86-1.60 (m, 3H), 1.27-1.18 (m, 1H), 0.95 (d, J = 6.1 Hz, 3H). |
| 299 | 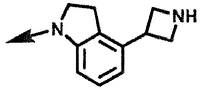 | LC-MS: [M+H]+ / Rt (min) 405.3 / 0.657 (Method A) |
| 300 | 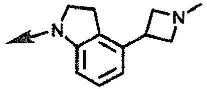 | LC-MS: [M+H]⁺ / Rt (min) 419.3 / 0.670 (Method A) |
| 301 | 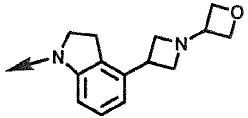 | LC-MS: [M+H]⁺ / Rt (min) 461.4 / 0.787 (Method A) |
| 302 | 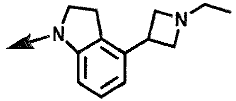 | ¹H-NMR (400 MHz, CDCl₃) δ: 8.00 (d, J = 8.5 Hz, 1H), 7.48 (d, J = 9.8 Hz, 1H), 7.12 (t, J = 7.9 Hz, 1H), 6.93-6.84 (m, 2H), 6.26 (s, 1H), 4.96-4.91 (m, 2H), 4.12 (t, J = 7.9 Hz, 2H), 3.86-3.66 (m, 3H), 3.11-3.01 (m, 3H), 2.55-2.46 (m, 3H), 2.30-1.85 (m, 3H), 1.81-1.57 (m, 3H), 1.25-1.15 (m, 1H), 1.00-0.92 (m, 6H) . |
| 303 | 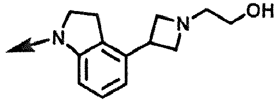 | LC-MS: [M+H]⁺ / Rt (min) 449.4 / 0.750 (Method A) |
| 304 | 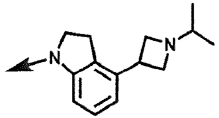 | ¹H-NMR (400 MHz, DMSO-d₆) δ: 7.85 (dd, J = 21.7, 8.9 Hz, 2H), 7.15 (t, J = 7.9 Hz, 1H), 7.01 (d, J = 7.3 Hz, 1H), 6.94 (d, J = 9.8 Hz, 1H), 6.54-6.52 (m, 1H), 5.02 (s, 2H), 4.21 (t, J = 8.5 Hz, 2H), 3.64-3.53 (m, 3H), 3.08 (t, J = 7.9 Hz, 2H), 2.99-2.95 (m, 2H), 2.68-2.64 (m, 1H), 2.35-2.21 (m, 3H), 1.86-1.75 (m, 2H), 1.70-1.63 (m, 1H), 1.28-1.19 (m, 1H), 0.97 (d, J = 6.1 Hz, 3H), 0.87 (d, J = 6.1 Hz, 6H). |
| 305 | 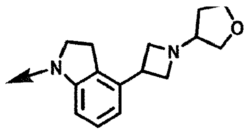 | LC-MS: [M+H]⁺ / Rt (min) 475.4 / 0.908 (Method A) |
| 306 | 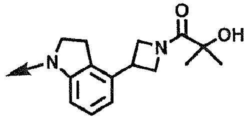 | ¹H-NMR (400 MHz, DMSO-d₆) δ: 7.90-7.86 (m, 2H), 7.21 (t, J = 7.9 Hz, 1H), 7.11 (d, J = 7.3 Hz, 1H), 6.95 (d, J = 9.8 Hz, 1H), 6.55-6.52 (m, 1H), 5.12 (s, 1H), 5.03 (s, 2H), 4.78 (t, J = 8.9 Hz, 1H), 4.43-4.39 (m, 1H), 4.24 (t, J = 8.5 Hz, 3H), 3.91-3.86 (m, 2H), 3.15-3.10 (m, 2H), 2.36-2.18 (m, 2H), 1.87-1.75 (m, 2H), 1.72-1.60 (m, 1H), 1.31-1.21 (m, 7H), 0.97 (d, J = 6.7 Hz, 3H) . |
| 307 | 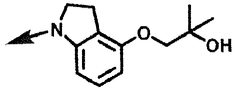 | ¹H-NMR (400 MHz, CDCl₃) δ: 7.83 (d, J = 8.5 Hz, 1H), 7.57 (d, J = 9.8 Hz, 1H), 7.15 (t, J = 8.2 Hz, 1H), 6.94 (d, J = 9.8 Hz, 1H), 6.59 (d, J = 7.9 Hz, 1H), 6.35 (s, 1H), 5.06-4.98 (m, 2H), 4.22 (t, J = 8.5 Hz, 2H), 3.85 (s, 2H), 3.24 (t, J = 8.5 Hz, 2H), 2.65-2.56 (m, 1H), 2.38-2.27 (m, 2H), 2.01 (br s, 2H), 1.89-1.81 (m, 2H), 1.76-1.67 (m, 1H), 1.37 (s, 6H), 1.01 (d, J = 6.1 Hz, 3H). |
| 308 | 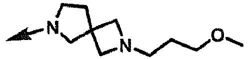 | LC-MS: [M+H]⁺ / Rt (min) 415.4 / 0.671 (Method A) |
| 309 | 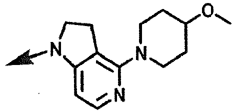 | ¹H-NMR (400 MHz, DMSO-d₆) δ: 7.94 (d, J = 5.5 Hz, 1H), 7.87 (d, J = 9.8 Hz, 1H), 7.40 (d, J = 5.5 Hz, 1H), 6.93 (d, J = 9.8 Hz, 1H), 6.52 (s, 1H), 5.02 (s, 2H), 4.22-4.18 (m, 2H), 3.69-3.63 (m, 2H), 3.39-3.32 (m, 1H), 3.25 (s, 3H), 3.16 (t, J = 7.8 Hz, 2H), 3.03-2.96 (m, 2H), 2.35-2.15 (m, 2H), 1.94-1.73 (m, 4H), 1.69-1.58 (m, 1H), 1.50-1.41 (m, 2H), 1.23-1.21 (m, 2H), 0.96 (d, J = 6.7 Hz, 3H). |
| 310 | 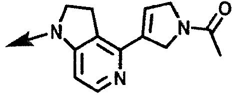 | LC-MS: [M+H]⁺ / Rt (min) 460.4 / 0.788 (Method A) |
| 311 | 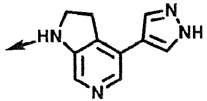 | ¹H-NMR (400 MHz, DMSO-d₆) δ: 13.19 (s, 1H), 8.94 (s, 1H), 8.54 (s, 1H), 8.22-8.01 (m, 2H), 7.87 (d, J = 9.8 Hz, 1H), 6.94 (d, J = 9.8 Hz, 1H), 6.52 (s, 1H), 5.06 (s, 2H), 4.29 (t, J = 8.5 Hz, 2H), 3.44-3.33 (m, 2H), 2.35-2.15 (m, 2H), 1.86-1.57 (m, 4H), 1.29-1.16 (m, 1H), 0.95 (d, J = 6.1 Hz, 3H). |
| 312 | 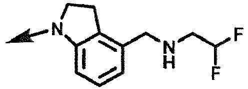 | ¹H-NMR (400 MHz, DMSO-d₆) δ: 7.90-7.85 (m, 2H), 7.13 (t, J = 7.6 Hz, 1H), 7.03 (d, J = 7.3 Hz, 1H), 6.95 (d, J = 9.8 Hz, 1H), 6.54 (s, 1H), 6.17-5.85 (m, 1H), 5.03 (s, 2H), 4.24 (t, J = 8.2 Hz, 2H), 3.72 (s, 2H), 3.20 (t, J = 8.2 Hz, 2H), 2.86 (t, J = 15.9 Hz, 2H), 2.68-2.54 (m, 2H), 2.36-2.15 (m, 2H), 1.88-1.60 (m, 3H), 1.29-1.19 (m, 1H), 0.97 (d, J = 6.1 Hz, 3H). |
| 313 | 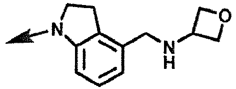 | ¹H-NMR (400 MHz, DMSO-d₆ δ: 7.90-7.84 (m, 2H), 7.11 (t, J = 7.6 Hz, 1H), 7.01-6.93 (m, 2H), 6.54 (s, 1H), 5.03 (s, 2H), 4.58-4.54 (m, 2H), 4.31-4.22 (m, 4H), 3.92-3.82 (m, 1H), 3.58 (s, 2H), 3.21 (t, J = 8.2 Hz, 2H), 2.85-2.75 (m, 1H), 2.37-2.16 (m, 2H), 1.88-1.58 (m, 3H), 1.30-1.17 (m, 1H), 0.97 (d, J = 6.1 Hz, 3H) . |
| 314 | 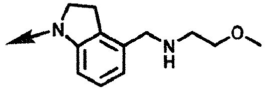 | ¹H-NMR (400 MHz, DMSO-d₆) δ: 7.90-7.83 (m, 2H), 7.12 (t, J = 7.9 Hz, 1H), 7.02 (d, J = 7.3 Hz, 1H), 6.95 (d, J = 10.4 Hz, 1H), 6.54 (s, 1H), 5.03 (s, 2H), 4.23 (t, J = 8.5 Hz, 2H), 3.67 (s, 2H), 3.40 (t, J = 5.8 Hz, 2H), 3.25-3.16 (m, 5H), 2.65 (t, J = 5.5 Hz, 2H), 2.37-2.17 (m, 2H), 2.09-1.59 (m, 4H), 1.28-1.17 (m, 1H), 0.97 (d, J = 6.7 Hz, 3H). |
| 315 | 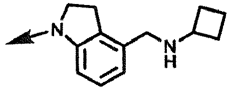 | ¹H-NMR (400 MHz, DMSO-d₆) δ: 7.90-7.83 (m, 2H), 7.10 (t, J = 7.9 Hz, 1H), 7.00 (d, J = 7.3 Hz, 1H), 6.94 (d, J = 9.8 Hz, 1H), 6.54 (s, 1H), 5.03 (s, 2H), 4.23 (t, J = 8.2 Hz, 2H), 3.55 (s, 2H), 3.22-3.11 (m, 3H), 2.36-2.04 (m, 5H), 1.85-1.49 (m, 7H), 1.28-1.18 (m, 1H), 0.97 (d, J = 6.7 Hz, 3H). |
| 316 | 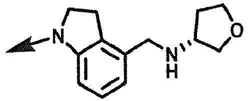 | ¹H-NMR (400 MHz, DMSO-d₆) δ: 7.89-7.84 (m, 2H), 7.12 (t, J = 7.6 Hz, 1H), 7.03 (d, J = 7.3 Hz, 1H), 6.94 (d, J = 9.8 Hz, 1H), 6.53 (s, 1H), 5.03 (s, 2H), 4.24 (t, J = 8.2 Hz, 2H), 3.78-3.59 (m, 5H), 3.47-3.42 (m, 1H), 3.30-3.19 (m, 3H), 2.36-2.11 (m, 3H), 1.97-1.62 (m, 5H), 1.28-1.18 (m, 1H), 0.97 (d, J = 6.7 Hz, 3H). |
| 317 | 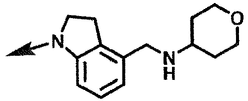 | ¹H-NMR (400 MHz, DMSO-d₆) δ: 7.90-7.83 (m, 2H), 7.13-7.03 (m, 2H), 6.94 (d, J = 9.8 Hz, 1H), 6.53 (s, 1H), 5.03 (s, 2H), 4.24 (t, J = 8.2 Hz, 2H), 3.85-3.80 (m, 2H), 3.68 (s, 2H), 3.31-3.19 (m, 4H), 2.63-2.55 (m, 1H), 2.36-2.18 (m, 2H), 1.96-1.62 (m, 6H), 1.33-1.18 (m, 3H), 0.97 (d, J = 6.1 Hz, 3H). |
| 318 | 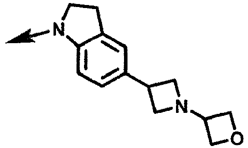 | ¹H-NMR (400 MHz, CDCl₃) δ: 8.14-8.09 (br m, 1H), 7.56 (d, J = 9.8 Hz, 1H), 7.18-7.16 (br m, 1H), 7.08-7.04 (br m, 1H) , 6.92 (d, J = 9.8 Hz, 1H), 6.33 (br s, 1H), 5.01 (s, 2H), 4.77-4.74 (m, 1H), 4.63-4.60 (m, 1H), 4.50-4.41 (m, 1H), 4.23-4.17 (m, 2H), 4.14-4.10 (m, 1H), 4.03 (s, 2H), 3.92-3.84 (m, 2H), 3.30-3.25 (m, 2H), 2.60-2.56 (br m, 1H), 2.34-2.26 (br m, 2H), 1.87-1.79 (br m, 2H), 1.74-1.65 (m, 1H), 1.30-1.24 (m, 3H), 0.99 (d, J = 6.7 Hz, 3H). |
| 319 | 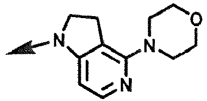 | ¹H-NMR (400 MHz, CDCl₃) δ: 8.09-8.07 (br m, 1H), 7.69 (br s, 1H), 7.57 (d, J = 9.8 Hz, 1H), 6.92 (d, J = 9.8 Hz, 1H), 6.35-6.33 (br m, 1H), 5.00 (br s, 2H), 4.24-4.20 (br m, 2H), 3.83 (t, J = 4.9 Hz, 4H), 3.38-3.24 (br m, 6H), 2.61-2.54 (br m, 1H), 2.36-2.26 (br m, 2H), 1.88-1.78 (m, 2H), 1.75-1.66 (br m, 1H), 1.33-1.23 (m, 1H), 1.00 (d, J = 6.7 Hz, 3H). |
| 320 | 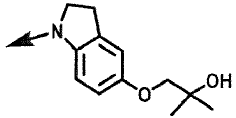 | ¹H-NMR (400 MHz, CDCl₃) δ: 8.09 (d, J = 8.6 Hz, 1H), 7.55 (d, J = 9.8 Hz, 1H), 6.92 (d, J = 9.8 Hz, 1H), 6.79 (d, J = 2.4 Hz, 1H), 6.71 (dd, J = 8.6, 2.4 Hz, 1H), 6.33 (br s, 1H), 5.04-4.96 (m, 2H), 4.19 (t, J = 8.3 Hz, 2H), 3.76 (s, 2H), 3.25 (t, J = 8.3 Hz, 2H), 2.62-2.56 (br m, 1H), 2.36-2.27 (br m, 2H), 2.23 (s, 1H), 1.88-1.79 (m, 2H), 1.74-1.66 (br m, 1H), 1.33 (s, 6H), 1.31-1.22 (m, 1H), 1.00 (d, J = 6.7 Hz, 3H). |
| 321 | 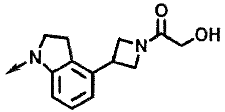 | LC-MS: [M+H]⁺ / Rt (min) 463.1 / 0.799 (Method A) |
| 322 | 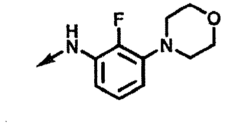 | ¹H-NMR (400 MHz, CDCl₃) δ: 8.92 (s, 1H), 8.03-7.93 (m, 1H), 7.60 (d, J = 5.1 Hz, 1H), 7.06-7.00 (m, 1H), 6.98 (d, J = 5.1 Hz, 1H), 6.86-6.75 (m, 1H), 6.42-6.32 (m, 1H), 5.00 (d, J = 14.6 Hz, 1H), 4.95 (d, J = 14.6 Hz, 1H), 3.93-3.85 (m, 4H), 3.15-3.07 (m, 4H), 2.68-2.55 (m, 1H), 2.40-2.25 (m, 2H), 1.92-1.80 (m, 2H), 1.80-1.65 (m, 2H), 1.01 (d, J = 6.1 Hz, 3H). |
| 323 | 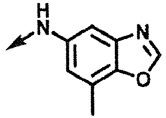 | ¹H-NMR (400 MHz, CDCl₃) δ: 9.09 (s, 1H), 8.05 (s, 1H), 7.87-7.83 (m, 1H), 7.60 (d, J = 9.8 Hz, 1H), 7.29 (s, 1H), 6.98 (d, J = 9.8 Hz, 1H), 6.37 (s, 1H), 5.00 (s, 2H), 2.70-2.58 (m, 1H), 2.46 (s, 3H), 2.36-2.30 (m, 2H), 1.92-1.79 (m, 2H), 1.77-1.69 (m, 2H), 1.02 (d, J = 6.1 Hz, 3H). |
| 324 | 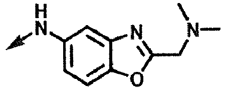 | ¹H-NMR (400 MHz, CDCl₃) δ: 9.10 (s, 1H), 8.06 (s, 1H), 7.63 (t, J = 7.6 Hz, 1H), 7.48-7.44 (m, 2H), 7.01 (d, J = 9.8 Hz, 1H), 6.40 (s, 1H), 5.03 (s, 2H), 4.11-4.06 (m, 2H), 2.73-2.61 (m, 7H), 2.38-2.26 (m, 2H), 2.94-1.80 (m, 2H), 1.80-1.69 (m, 2H), 1.03 (d, J = 6.1 Hz, 3H). |
| 325 | 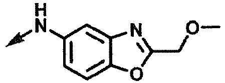 | ¹H-NMR (400 MHz, CDCl₃) δ: 9.17 (s, 1H), 7.98 (s, 1H), 7.60 (d, J = 10.4 Hz, 1H), 7.45-7.35 (m, 2H), 6.98 (t, J = 10.7 Hz, 1H), 6.36 (s, 1H), 5.00 (s, 2H), 4.69 (s, 2H), 3.51 (s, 3H), 2.67-2.57 (m, 1H), 2.39-2.328 (m, 2H), 1.90-1.79 (m, 2H), 1.78-1.62 (m,, 1H), 1.33-1.21 (m, 1H), 1.00 (d, J = 6.7 Hz, 3H). |
| 326 | 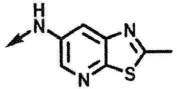 | ¹H-NMR (400 MHz, CDCl₃) δ: 9.43 (s, 1H), 8.61-8.57 (m, 2H), 7.64 (d, J = 9.8 Hz, 1H), 7.02 (d, J = 9.8 Hz, 1H), 6.40 (s, 1H), 5.05 (s, 2H), 2.84 (s, 3H), 2.82 (s, 1H), 2.70-2.60 (m, 1H), 2.43-2.29 (m, 2H), 1.92-1.83 (m, 2H), 1.65-1.75 (m, 1H), 1.38-1.25 (m, 1H), 1.0 (t, J = 7.3 Hz, 3H). |
| 327 | 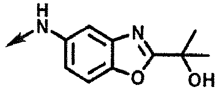 | ¹H-NMR (400 MHz, CDCl₃) δ: 9.16 (s, 1H), 7.90 (s, 1H), 7.60 (d, J = 7.0 Hz, 1H), 7.46-7.39 (m, 2H), 7.29-7.37 (m, 1H), 6.36 (s, 1H), 5.00 (s, 2H), 2.38-2.25 (m, 3H), 1.90-1.79 (m, 3H), 1.75 (s, 1H), 1.73 (t, 6H), 0.99 (t, J = 7.3 Hz, 3H). |
| 328 | 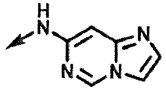 | ¹H-NMR (400 MHz, CD₃OD) δ: 9.13 (s, 1H), 8.17 (s, 1H), 7.85 (d, J = 9.2 Hz, 2H), 7.53 (s, 1H), 6.95 (d, J = 9.8 Hz, 1H), 6.51 (s, 1H), 5.02 (s, 2H), 2.67-2.57 (m, 1H), 2.42-2.28 (m, 2H), 1.90-1.80 (m, 2H), 1.80-1.65 (s, 1H), 1.35-1.25 (m, 1H), 1.01 (d, J = 6.7 Hz, 3H). |
| 329 | 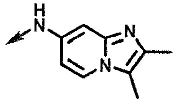 | ¹H-NMR (400 MHz, CD₃OD) δ: 7.98 (d, J = 7.3 Hz, 1H), 7.85-7.80 (m, 2H), 7.09-7.02 (m, 1H), 6.93 (d, J = 9.8 Hz, 1H), 6.53-6.45 (m, 1H), 4.97 (s, 2H), 2.67-2.55 (m, 1H), 2.36 (s, 3H), 2.36-2.26 (m, 2H), 2.30 (s, 3H), 1.87-1.80 (m, 2H), 1.73-1.68 (m, 1H), 0.98 (t, J = 7.0 Hz, 3H). |
| 330 | 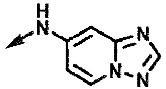 | ¹H-NMR (400 MHz, CDCl₃) δ: 9.98 (s, 1H), 8.32 (d, J = 4.3 Hz, 1H), 8.23 (s, 1H), 8.01 (s, 1H), 7.63 (d, J = 9.8 Hz, 1H), 7.09 (dd, J = 7.3, 2.4 Hz, 1H), 6.98 (d, J = 9.8 Hz, 1H), 6.40 (s, 1H), 5.03 (s, 2H), 2.65-2.55 (m, 1H), 2.40-2.25 (m, 2H), 1.91-1.80 (m, 2H), 1.80-1.65 (m, 1H), 1.34-1.20 (m, 1H), 1.00 (d, J = 6.7 Hz, 3H). |
| 331 | 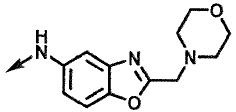 | ¹H-NMR (400 MHz, CDCl₃) δ: 9.12 (s, 1H), 7.94 (s, 1H), 7.59 (d, J = 9.8 Hz, 1H), 7.41-7.34 (m, 2H), 6.97 (d, J = 9.8 Hz, 1H), 6.36 (s, 1H), 4.99 (s, 2H), 3.83 (s, 2H), 3.78-3.74 (m, 4H), 2.68-2.58 (m, 5H), 2.38-2.28 (m, 2H), 1.90-1.81 (m, 2H), 1.75-1.65 (m, 1H), 1.35-1.26 (m, 1H), 0.99 (t, J = 8.6 Hz, 3H). |
| 332 | 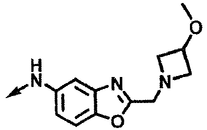 | ¹H-NMR (400 MHz, CDCl₃) δ: 9.08 (s, 1H), 7.93 (s, 1H), 7.59 (d, J = 9.8 Hz, 1H), 7.41-7.33 (m, 2H), 6.98 (d, J = 7.9 Hz, 1H), 6.36 (s, 1H), 4.98 (s, 2H), 4.17-4.05 (m, 2H), 3.92-3.88 (m, 2H), 3.85-3.76 (m, 2H), 3.24 (s, 3H), 3.21-3.11 (m, 2H), 2.68-2.57 (m, 1H), 2.38-2.29 (m, 2H), 1.90-1.79 (m, 2H), 1.78-1.60 (m, 1H), 0.98 (d, J = 6.4 Hz, 3H). |
| 333 | 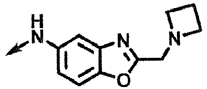 | ¹H-NMR (400 MHz, CD₃OD) δ: 8.14 (d, J = 1.8 Hz, 1H), 7.84 (d, J = 9.8 Hz, 1H), 7.59 (d, J = 8.6 Hz, 1H), 7.49 (dd, J = 8.9, 2.1 Hz, 1H), 6.93 (d, J = 9.5 Hz, 1H), 6.50 (s, J = 3.1 Hz, 1H), 4.97 (s, 2H), 4.83 (s, 2H), 4.50-4.40 (m, 2H), 4.40-4.29 (m, 2H), 3.32-3.28 (m, 5H), 2.70-2.45 (m, 3H), 1.89-1.878 (m, 2H), 1.35-1.26 (m, 1H), 1.00 (d, J = 6.7 Hz, 3H). |
| 334 | 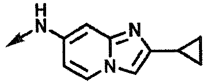 | ¹H-NMR (400 MHz, CDCl₃) δ: 10.11 (s, 1H), 7.72 (d, J = 11.3 Hz, 1H), 7.61 (s, 1H), 7.54 (d, J = 9.2 Hz, 1H), 7.11 (s, 1H), 7.06-7.02 (m, 1H), 6.89 (d, J = 4.9 Hz, 1H), 6.30 (s, 1H), 4.95 (s, 2H), 2.65-2.41 (m, 2H), 2.35-2.20 (m, 2H), 2.00-1.91 (m, 1H), 1.86-1.75 (m, 2H), 1.75-1.65 (m, 1H), 0.98 (d, J = 6.7 Hz, 3H), 0.94-0.83 (m, 4H). |
| 335 | 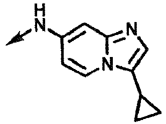 | ¹H-NMR (400 MHz, CDCl₃) δ: 10.06 (s, 1H), 7.97 (d, J = 7.3 Hz, 1H), 7.82 (s, 1H), 7.65-7.56 (m, 1H), 7.25-7.15 (m, 2H), 6.99-6.93 (m, 1H), 6.35 (s, 1H), 5.05 (s, 2H), 2.65-2.56 (m, 1H), 2.50-2.22 (m, 3H), 1.90-1.60 (m, 3H), 1.34-1.20 (m, 1H), 0.98 (d, J = 5.8 Hz, 3H), 1.02-0.92 (m, 1H), 0.88 (t, J = 6.7 Hz, 1H), 0.69-0.62 (m, 2H). |
| 336 | 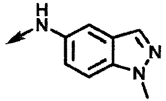 | ¹H-NMR (400 MHz, CDCl₃) δ: 8.93 (s, 1H), 8.04 (d, J = 1.2 Hz, 1H), 7.91 (s, 1H), 7.61 (d, J = 9.8 Hz, 1H), 7.41-7.38 (m, 1H), 7.29 (d, J = 8.5 Hz, 1H), 6.99 (d, J = 9.8 Hz, 1H), 6.38 (s, 1H), 4.98 (s, 2H), 2.70-2.59 (m, 1H), 2.41-2.28 (m, 2H), 1.93-1.79, 2H), 1.55-1.42 (m, 4H), 1.38-1.23 (m, 1H), 1.01 (d, J = 6.7 Hz, 3H). |
| 337 | 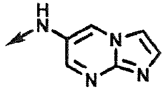 | ¹H-NMR (400 MHz, CDCl₃) δ: 11.01 (s, 1H), 9.45 (s, 1H), 8.57 (s, 1H), 7.69 (s, 1H), 7.57 (d, J = 9.1 Hz, 1H), 7.43 (s, 1H), 6.94 (d, J = 9.8 Hz, 1H), 6.33 (s, 1H), 5.14 (s, 2H), 2.63-2.51 (m, 1H), 2.38-2.25 (m, 2H), 1.90-1.75 (m, 2H), 1.75-1.60 (m, 1H), 1.30-1.21 (m, 1H), 0.98 (d, J = 6.1 Hz, 3H). |
| 338 | 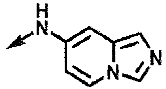 | ¹H-NMR (400 MHz, CDCl₃) δ: 9.52 (s, 1H), 7.94 (s, 1H), 7.79 (s, 1H) , 7.68 (d, J = 7.3 Hz, 1H), 7.61 (d, J = 4.9 Hz, 1H), 7.19 (s, 1H), 6.96 (d, J = 9.8 Hz, 1H), 6.56 (dd, J = 7. 6, 2.1 Hz, 1H), 6.37 (s, 1H), 4.99 (s, 2H), 2.67-2.56 (m, 1H) , 2.39-2.25 (m, 2H), 2.25-2.08 (m, 1H), 1.89-1.78 (m, 2H), 1. 35-1.21 (m, 1H), 0.99 (d, J = 6.7 Hz, 3H) . |
| 339 | 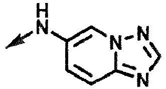 | ¹H-NMR (400 MHz, CDCl₃) δ: 9.65 (s, 1H), 9.48 (s, 1H), 8.31 (s, 1H), 7.67 (dd, J = 13.7, 9.5 Hz, 2H), 7.41-7.35 (m, 1H), 7.01 (d, J = 9.8 Hz, 1H), 6.40 (s, 1H), 5.03 (s, 2H), 2.68-2.57 (m, 1H), 2.40-2.31 (m, 2H), 1.93-1.83 (m, 2H), 1.35-1.21 (m, 1H). 1.01 (d, J = 6.7 Hz, 3H). |
| 340 | 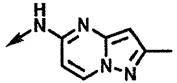 | ¹H-NMR (400 MHz, CDCl₃) δ: 8.99 (s, 1H), 8.46 (d, J = 7.3 Hz, 1H), 7.75 (d, J = 6.7 Hz, 1H), 7.59 (d, J = 9.8 Hz, 1H), 6.98 (d, J = 9.8 Hz, 1H), 6.38 (s, 1H), 6.18 (s, 1H), 4.99 (s, 2H), 2.65-2.56 (m, 1H), 2.46 (s, 3H), 2.40-2.28 (m, 2H), 1.89-1.80 (m, 1H), 1.78-1.65 (m, 2H), 1.35-1.20 (m, 1H), 1.01 (d, J = 6.7 Hz, 3H) . |
| 341 | 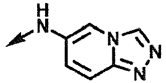 | ¹H-NMR (400 MHz, CDCl₃) δ: 10.90-10.70 (m, 1H), 9.48-9.35 (m, 1H), 9.15-9.00 (m, 1H), 7.95-7.50 (m, H), 7.61 (d, J = 9.8 Hz, 1H), 6.98 (d, J = 9.8 Hz, 1H), 6.36 (s, 1H), 5.12 (s, 2H), 2.65-2.40 (m, 2H), 2.39-2.23 (m, 2H), 1.90-1.79 (m, 1H), 1.75-1.65 (m, 1H), 0.99 (d, J = 6.1 Hz, 3H) . |
| 342 | 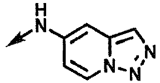 | ¹H-NMR (400 MHz , CDCl₃) δ: 9.67 (s, 1H), 8.58 (d, J = 7.9 Hz, 1H), 8.19 (d, J = 1.2 Hz, 1H), 7.90 (s, 1H), 7.64 (t, J = 8.2 Hz, 1H), 7.01 (d, J = 9.8 Hz, 1H), 6.93 (dd, J = 7.6, 2.1 Hz, 1H), 6.40 (s, 1H), 5.01 (s, 2H), 2.70-2.55 (m, 1H), 2.40-2.25 (m, 2H), 1.90-1.64 (m, 2H), 1.35-1.26 (m, 1H), 1.02 (t, J = 8.5 Hz, 5H). |
| 343 | 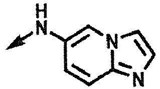 | ¹H-NMR (400 MHz, CDCl₃) δ: 9.70 (s, 1H), 9.11 (s, 1H), 7.64-7.52 (m, 3H), 7.47 (d, J = 9.2 Hz, 1H), 7.08-6.95 (m, 2H), 6.38 (s, 1H), 5.03 (s, 2H), 2.70-2.55 (m, 1H), 2.40-2.28 (m, 1H), 2.25-2.10 (m, 2H), 1.93-1.78 (m, 1H), 1.78-1.65 (m, 1H), 1.35-1.21 (m, 1H), 1.01 (d, J = 6.7 Hz, 3H). |
| 344 | 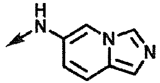 | ¹H-NMR (400 MHz, CDCl₃) δ: 9.96 (s, 1H), 9.02 (s, 1H), 8.49 (s, 1H), 7.60 (d, J = 9.8 Hz, 1H), 7.47 (s, 1H), 7.33 (d, J = 9.8 Hz, 1H), 6.98 (d, J = 9.5 Hz, 1H), 6.80 (d, J = 9.2 Hz, 1H), 6.35 (s, 1H), 5.08 (s, 2H), 2.65-2.53 (m, 1H), 2.40-2.25 (m, 2H), 1.90-1.78 (m, 2H), 1.73-1.65 (m, 1H), 1.33-1.25 (m, 1H), 1.01 (d, J = 6.7 Hz, 3H). |
| 345 | 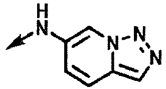 | ¹H-NMR (400 MHz, CD₃OD) δ: 9.61 (s, 1H), 8.08 (s, 1H), 7.86 (d, J = 4.9 Hz, 2H), 7.32 (d, J = 7.9 Hz, 1H), 6.96 (d, J = 9.8 Hz, 1H), 6.53 (s, 1H), 5.01 (s, 2H), 2.65-2.51 (m, 1H), 2.42-2.30 (m, 2H), 1.90-1.75 (m, 2H), 1.75-1.65 (m, 1H), 1.40-1.25 (m, 1H), 1.01 (d, J = 6.7 Hz, 3H). |
| 346 | 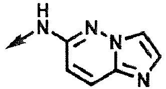 | ¹H-NMR (400 MHz, CD₃OD) δ: 9.31 (s, 1H), 8.04 (d, J = 9.8 Hz, 1H), 7.91 (d, J = 9.8 Hz, 1H), 7.79 (s, 1H), 7.70 (d, J = 1.2 Hz, 1H), 7.62 (d, J = 9.8 Hz, 1H), 7.01 (d, J = 9.8 Hz, 1H), 6.39 (s, 1H), 5.02 (s, 2H), 2.70-2.58 (m, 1H), 2.45-2.30 (m, 2H), 1.95-1.83 (m, 2H), 1.80-1.65 (m, 1H), 1.35-1.20 (m, 1H), 1.01 (d, J = 6.7 Hz, 3H). |
| 347 | 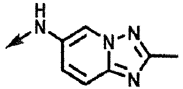 | ¹H-NMR (400 MHz, CDCl₃) δ: 9.53 (s, 1H), 9.30 (s, 1H), 7.64 (d, J = 9.8 Hz, 1H), 7.46 (dd, J = 9.4, 2.7 Hz, 1H), 7.21 (d, J = 9.8 Hz, 1H), 6.99 (d, J = 9.8 Hz, 1H), 6.39 (s, 1H), 5.01 (s, 2H), 2.68-2.58 (m, 1H), 2.54 (s, 3H), 2.40-2.38 (m, 2H), 1.92-1.80 (m, 2H), 1.80-1.65 (m, 1H), 1.35-1.21 (m, 1H), 1.01 (d, J = 6.7 Hz, 3H). |
| 348 | 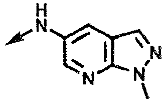 | ¹H-NMR (400 MHz, CDCl₃) δ: 9.18 (s, 1H), 8.48 (d, J = 1.8 Hz, 1H), 8.41 (d, J = 2.4 Hz, 1H), 7.90 (s, 1H), 7.63 (d, J = 9.8 Hz, 1H), 7.00 (d, J = 9.8 Hz, 1H), 6.39 (s, 1H), 5.02 (s, 2H), 4.11 (s, 3H), 2.70-2.59 (m, 1H), 2.40-2.38 (m, 2H), 1.90-1.80 (m, 2H), 1.79-1.63 (m, 1H), 1.36-1.24 (m, 1H), 1.01 (d, J = 6.7 Hz, 3H). |
| 349 | 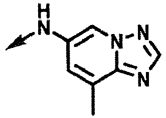 | ¹H-NMR (400 MHz, CDCl₃) δ: 9.46 (s, 1H), 9.29 (s, 1H), 8.23 (s, 1H), 7.65 (d, J = 9.8 Hz, 1H), 7.04 (s, 1H), 7.01 (d, J = 9.8 Hz, 1H), 6.40 (s, 1H), 5.01 (s, 2H), 2.68-2.58 (m, 1H), 2.56 (s, 3H), 2.40-2.38 (m, 2H), 1.90-1.79 (m, 2H), 1.78-1.64 (m, 1H), 1.38-1.29 (m, 1H), 1.01 (d, J = 6.7 Hz, 3H). |
| 350 | 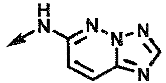 | ¹H-NMR (400 MHz, CDCl₃) δ: 9.67 (s, 1H), 8.50 (d, J = 9.8 Hz, 1H), 8.41 (d, J = 15.2 Hz, 1H), 8.09 (d, J = 9.8 Hz, 1H), 7.61 (d, J = 9.8 Hz, 1H), 7.00 (d, J = 9.8 Hz, 1H), 6.38 (d, J = 2.4 Hz, 1H), 5.11 (s, 2H), 2.65-2.55 (m, 1H), 2.40-2.25 (m, 2H), 1.93-1.81 (m, 2H), 1.75-1.60 (m, 1H), 1.35-1.22 (m, 1H), 1.00 (d, J = 6.4 Hz, 3H). |
| 351 | 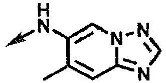 | ¹H-NMR (400 MHz, CDCl₃) δ: 9.52 (s, 1H), 9.15 (s, 1H), 8.23 (s, 1H), 7.61 (d, J = 9.8 Hz, 1H), 7.50 (s, 1H), 6.99 (d, J = 9.8 Hz, 1H), 6.38 (s, 1H), 5.04 (s, 2H), 2.68-2.57 (m, 1H), 2.40 (s, 3H), 2.40-2.30 (m, 2H), 1.91-1.80 (m, 2H), 1.80-1.65 (m, 1H), 1.35-1.25 (m, 1H), 1.01 (d, J = 6.7 Hz, 3H). |
| 352 | 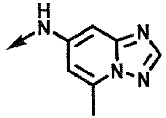 | ¹H-NMR (400 MHz, CDCl₃) δ: 9.41 (s, 1H), 8.25 (s, 1H), 7.90 (s, 1H), 7.61 (d, J = 10.4 Hz, 1H), 6.99 (d, J = 4.9 Hz, 2H), 6.38 (s, 1H), 4.99 (s, 2H), 2.73 (s, 3H), 2.68-2.58 (m, 1H), 2.43-2.30 (m, 2H), 1.93-1.82 (m, 2H), 1.78-1.72 (m, 1H), 1.38-1.25 (m, 1H), 1.02 (d, J = 6.7 Hz, 3H). |
| 353 | 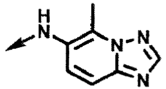 | ¹H-NMR (400 MHz, CDCl₃) δ: 8.82 (s, 1H), 8.33 (s, 1H), 7.81 (d, J = 9.8 Hz, 1H), 7.63 (dd, J = 13.1, 9.4 Hz, 2H), 7.01 (d, J = 9.8 Hz, 1H), 6.40 (s, 1H), 5.05 (s, 2H), 2.73 (s, 3H), 2.68-2.58 (m, 1H), 2.40-2.25 (m, 2H), 1.93-1.80 (m, 2H), 1.79-1.65 (,m 1H), 1.34-1.21 (m, 1H), 1.01 (d, J = 6.7 Hz, 3H) . |
| 354 | 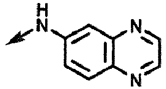 | ¹H-NMR (400 MHz, CDCl₃) δ: 9.99 (s, 1H), 8.68 (s, 1H), 8.63 (s, 1H), 8.29 (t, J = 7.6 Hz, 1H), 7.83-7.78 (m, 2H), 7.61-7.55 (m, 1H), 7.00-6.94 (m, 1H), 6.32 (s, 1H), 5.10 (s, 2H), 2.65-2.53 (m, 1H), 2.33-2.22 (m, 2H), 1.90-1.78 (m, 2H), 1.78-1.60 (m, 1H), 1.31-1.20 (m, 1H), 1.00 (d, J = 6.5 Hz, 3H). |
| 355 | 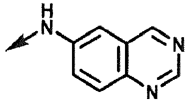 | ¹H-NMR (400 MHz, CDCl₃) δ: 10.00 (s, 1H), 9.10 (d, J = 3.7 Hz, 1H), 8.97 (t, J = 4.3 Hz, 1H), 8.22 (s, 1H), 7.77 (d, J = 6.7 Hz, 1H), 7.71-7.60 (m, 2H), 7.02 (dd, J = 9.8, 3.7 Hz, 1H), 6.39 (s, 1H), 5.14 (s, 2H), 2.68-2.57 (m, 1H), 2.40-2.31 (m, 2H), 1.95-1.90 (m, 2H), 1.80-1.65 (m, 1H), 1.35-1.21 (m, 1H), 1.02 (d, J = 6.0 Hz, 3H). |
| 356 | 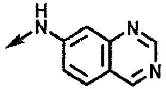 | ¹H-NMR (400 MHz, CDCl₃) δ: 9.59 (s, 1H), 9.27 (s, 2H), 8.28 (s, 1H), 7.85 (q, J = 6.7 Hz, 2H), 7.65 (d, J = 9.8 Hz, 1H), 7.03 (d, J = 9.8 Hz, 1H), 6.41 (s, 1H), 5.05 (s, 2H), 2.70-2.60 (m, 1H), 2.41-2.30 (m, 2H), 1.95-1.83 (m, 2H), 1.80-1.68 (m, 1H), 1.35-1.21 (m, 1H), 1.03 (d, J = 6.1 Hz, 3H). |
| 357 | 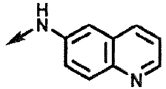 | ¹H-NMR (400 MHz, CDCl₃) δ: 9.65 (s, 1H), 8.93 (s, 1H), 8.40 (d, J = 8.5 Hz, 1H), 8.20 (d, J = 7.3 Hz, 1H), 7.94 (d, J = 7.9 Hz, 1H), 7.72-7.64 (m, 2H), 7.48 (s, 1H), 7.06 (d, J = 9.8 Hz, 1H), 6.41 (s, 1H), 5.12 (s, 2H), 2.73-2.60 (m, 1H), 2.43-2.30 (m, 2H),1.93-1.83 (m, 2H), 1.80-1.70 (m, 1H), 1.37-1.22 (m, 1H), 1.05 (d, J = 6.0 Hz, 3H) . |
| 358 | 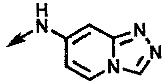 | ¹H-NMR (400 MHz, DMSO-d₆) δ: 10.78 (s, 1 H), 9.11 (s, 1 H), 8.54-8.44 (m, 1H), 8.09-8.02 (m, 1 H), 7.93-7.82 (m, 1 H), 7.01-6.89 (m, 1 H), 6.54 (s, 1 H), 4.91 (s, 2 H), 2.35-2.16 (m, 2 H), 1.91-1.75 (m, 2 H), 1.70-1. 60 (m, 1 H), 1.29-1.16 (m, 1H), 0.99 (d, J = 8.0 Hz, 3H), |
| 359 | 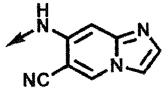 | ¹H-NMR (400 MHz, CDCl₃) δ: 9.16 (s, 1H), 8.60 (s, 1H), 8.49 (s, 1H), 7.71 (d, J = 1.2 Hz, 1H), 7.63 (d, J = 9.8 Hz, 1H), 7.57 (s, 1H), 7.04 (d, J = 9.8 Hz, 1H), 6.40 (s, 1H), 5.10 (s, 2H), 2.68-2.59 (m, 1H), 2.41-2.28 (m, 2H), 1.93-1.80 (m, 2H), 1.65-1.78 (m, 1H), 1.39-1.22 (m, 1H), 1.01 (d, J = 6.7 Hz, 3H). |
| 360 | 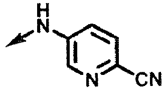 | ¹H-NMR (400 MHz, CDCl₃) δ: 9.77 (s, 1H), 8.62 (d, J = 2.4 Hz, 1H), 8.29 (dd, J = 8.5, 2.4 Hz, 1H), 7.67-7.58 (m, 2H), 7.01 (d, J = 9.8 Hz, 1H), 6.40 (s, 1H), 5.01 (s, 2H), 2.68-2.57 (m, 1H), 2.41-2.27 (m, 2H), 1.93-1.80 (m, 2H), 1.80-1.65 (m, 1H), 1.35-1.21 (m, 1H), 1.0 (d, J = 6.7 Hz, 3H) . |
| 361 | 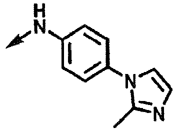 | ¹H-NMR (400 MHz, CDCl₃) δ: 9.42 (s, 1H), 7.69 (d, J = 8.6 Hz, 2H), 7.62 (d, J = 9.8 Hz, 1H), 7.21 (d, J = 8.6 Hz, 2H), 7.09 (s, 1H), 7.10-6.90 (m, 2H), 6.38 (s, 1H), 5.00 (s, 2H), 2.68-2.58 (m, 1H), 2.40-2.25 (m, 2H), 1.95-1.80 (m, 2H), 1.80-1.60 (m, 1H), 1.31-1.23 (m, 1H), 1.01 (d, J = 6.7 Hz, 3H). |
| 362 | 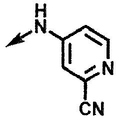 | ¹H-NMR (400 MHz, CDCl₃) δ: 9.90 (s, 1H), 8.51 (d, J = 6.1 Hz, 1H), 7.93 (d, J = 1.8 Hz, 1H), 7.66 (d, J = 9.8 Hz, 1H), 7.60-7.56 (m, 1H), 7.01 (d, J = 9.8 Hz, 1H), 6.41 (s, 1H), 4.99 (s, 2H), 2.67-2.56 (m, 1H), 2.41-2.28 (m, 2H), 1.93-1.81 (m, 2H), 1.80-1.65 (m, 1H), 1.32-1.25 (m, 1H), 1.01 (d, J = 6.7 Hz, 3H). |
| 363 | 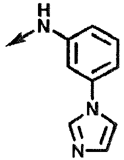 | ¹H-NMR (400 MHz, CDCl₃) δ: 9.79 (s, 1H), 8.29 (s, 1H), 7.83 (s, 1H), 7.63 (d, J = 9.8 Hz, 1H), 7.46 (d, J = 7.9 Hz, 1H), 7.32 (t, J = 7.9 Hz, 1H), 7.28 (s, 1H), 7.23 (s, 1H), 7.09 (d, J = 7.9 Hz, 1H), 6.97 (d, J = 9.8 Hz, 1H), 6.41-6.35 (m, 1H), 5.06 (s, 2H), 2.67-2.56 (m, 1H), 2.41-2.28 (m, 2H), 1.90-1.80 (m, 2H), 1.79-1.65 (m, 1H), 1.33-1.23 (m, 1H), 1.00 (d, J = 6.7 Hz, 3H). |
| 364 | 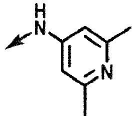 | ¹H-NMR (400 MHz, CDCl₃) δ: 9.35 (s, 1H), 7.63 (d, J = 9.8 Hz, 1H), 7.18 (s, 2H), 7.00 (d, J = 9.8 Hz, 1H), 6.40 (s, 1H), 4.97 (s, 2H), 2.67-2.58 (m, 1H), 2.50 (s, 6H), 2.41-2.30 (m, 2H), 1.93-1.83 (m, 2H), 1.79-1.65 (m, 1H), 1.35-1.22 (m, 1H), 1.02 (d, J = 6.7 Hz, 3H). |
| 365 | 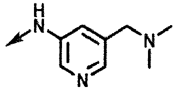 | ¹H-NMR (400 MHz, CDCl₃) δ: 9.20 (s, 1H), 8.61 (s, 1H), 8.25 (s, 1H), 7.99 (s, 1H), 7.60 (d, J = 9.8 Hz, 1H), 6.97 (d, J = 9.8 Hz, 1H), 6.37 (s, 1H), 4.99 (s, 2H), 3.44 (s, 2H), 2.66-2.55 (m, 1H), 2.38-2.28 (m, 2H), 2.25 (s, 6H), 2.15-2.00 (m, 1H), 1.87-1.81 (m, 1H), 1.78-1.65 (s, 1H), 1.33-1.25 (m, 1H), 1.00 (d, J = 6.7 Hz, 3H). |
| 366 | 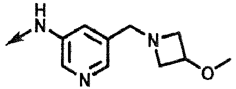 | ¹H-NMR (400 MHz, CDCl₃) δ: 9.07 (s, 1H), 8.55 (d, J = 2.4 Hz, 1H), 8.23 (d, J = 1.8 Hz, 1H), 7.97 (d, J = 9.2 Hz, 1H), 7.61 (d, J = 9.8 Hz, 1H), 6.98 (d, J = 9.8 Hz, 1H), 6.38 (s, 1H), 4.99 (s, 2H), 3.63-3.53 (m, 4H), 3.25 (s, 3H), 2.97-2.93 (m, 2H), 2.67-2.56 (m, 1H), 2.36-2.31 (m, 2H), 2.15-2.00 (m, 1H), 1.90-1.75 (m, 1H), 1.78-1.66 (m, 1H), 1.38-1.25 (m, 1H), 1.01 (d, J = 6.7 Hz, 3H). |
| 367 | 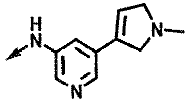 | ¹H-NMR (400 MHz, CDCl₃) δ: 9.38 (s, 1H), 8.44 (s, 1H), 8.26 (s, 1H), 8.02 (s, 1H), 7.62 (d, J = 9.8 Hz, 1H), 6.98 (d, J = 9.8 Hz, 1H), 6.38 (s, 1H), 6.20 (s, 1H), 4.99 (s, 2H), 3.90 (s, 2H), 3.76 (s, 2H), 2.68-2.55 (m, 4H), 2.40-2.25 (m, 2H), 1.91-1.80 (m, 2H), 1.75-1.66 (m, 1H), 1.34-1.20 (m, 1H), 1.00 (d, J = 6.1 Hz, 3H). |
| 368 | 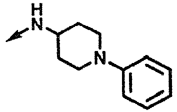 | LC-MS: [M+H]⁺ / Rt (min) 407.4 / 1.79 (Method C) |
| 369 | 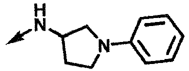 | LC-MS: [M+H]⁺ / Rt (min) 393.4 / 1.82 (Method C) |
| 370 | 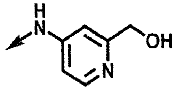 | ¹H-NMR (400 MHz, CDCl₃) δ: 9.44 (s, 1H), 8.36 (d, J = 5.5 Hz, 1H), 7.63 (d, J = 9.8 Hz, 1H), 7.41 (s, 1H), 7.29 (dd, J = 5.8, 2.1 Hz, 1H), 6.99 (t, J = 4.9 Hz, 1H), 6.39 (s, 1H), 4.98 (s, 2H), 4.67 (s, 2H), 3.75-3.65 (m, 1H), 2.67-2.57 (m, 1H), 2.40-2.28 (m, 2H), 1.93-1.78 (m, 2H), 1.77-1.65 (m, 1H), 1.34-1.21 (m, 1H), 1.01 (q, J = 6.3 Hz, 3H). |
| 371 | 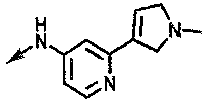 | ¹H-NMR (400 MHz, CDCl₃) δ: 9.49 (s, 1H), 8.35 (d, J = 5.5 Hz, 1H), 7.63 (d, J = 10.4 Hz, 1H), 7.52 (s, 1H), 7.30-7.23 (m, 1H), 6.99 (d, J = 9.8 Hz, 1H), 6.45-6.33 (m, 2H), 4.97 (s, 2H), 4.01 (d, J = 1.8 Hz, 2H), 3.81 (s, 2H), 2.70-2.55 (m, 4H), 2.40-2.25 (m, 2H), 1.93-1.80 (m, 2H), 1.80-1.65 (m, 1H), 1.35-1.20 (m, 1H), 1.01 (d, J = 6.7 Hz, 3H) . |
| 372 | 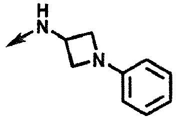 | LC-MS: [M+H]⁺ / Rt (min) 379.1 / 1.83 (Method C) |
| 373 | 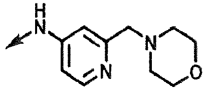 | ¹H-NMR (400 MHz, CDCl₃) δ: 9.78 (s, 1H),8.27 (d, J = 5.5 Hz, 1H), 7.61 (dd, J = 10.1, 4.6 Hz, 1H), 7.47 (d, J = 1.8 Hz, 1H), 7.22-7.18 (m, 1H), 6.95 (t, J = 7.9 Hz, 1H), 6.35 (s, 1H), 4.97 (q, J = 12.8 Hz, 2H), 3.70-3.64 (m, 4H), 3.48 (s, 2H), 2.60-2.53 (m, 1H), 2.48-2.38 (m, 4H), 2.37-2.26 (m, 2H), 1.86-1.79 (m, 2H), 1.74-1.62 (m, 1H), 1.35-1.22 (m, 1H), 0.96 (d, J = 5.8 Hz, 3H). |
| 374 | 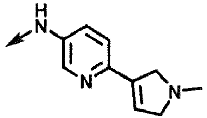 | ¹H-NMR (400 MHz, CDCl₃) δ: 9.74 (s, 1H), 8.45 (s, 1H), 8.07 (d, J = 8. 6 Hz, 1H), 7.62 (dd, J = 9.5, 3.4 Hz, 1H), 7.00-6.94 (m, 2H), 6.37 (s, 1H), 6.27 (s, 1H), 4.99 (s, 2H), 4.44 (s, 2H), 4.29 (s, 2H), 2.97 (s, 3H), 2.67-2.57 (m, 1H), 2.40-2.25 (m, 2H), 1.93-1.78 (m, 2H), 1.79-1.65 (m, 1H), 1.35-1.21 (m, 1H), 1.01 (d, J = 6.1 Hz, 3H). |
| 375 | 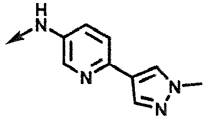 | ¹H-NMR, (400 MHz, CDCl₃) δ: 9.13 (s, 1H), 8.51 (d, J = 2.4 Hz, 1H), 8.04 (dd, J = 8.5, 2.4 Hz, 1H), 7.85 (d, J = 14.0 Hz, 2H), 7.62 (d, J = 9.8 Hz, 1H), 7.35 (d, J = 8.5 Hz, 1H), 6.99 (d, J = 9.8 Hz, 1H), 6.38 (s, 1H), 4.99 (s, 2H), 3.92 (s, 3H), 2.68-2.57 (m, 1H), 2.40-2.30 (m, 2H), 1.91-1.79 (m, 2H), 1.78-1.64 (m, 1H), 1.34-1.22 (m, 1H), 1.01 (d, J = 6.7 Hz, 3H). |
| 376 | 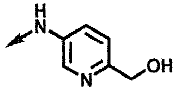 | ¹H-NMR (400 MHz, CDCl₃) δ: 9.19 (s, 1H), 8.57 (d, J = 2.4 Hz, 1H), 7.98 (d, J = 9.1 Hz, 1H), 7.63 (d, J = 9.8 Hz, 1H) , 7.18 (t, J = 7.6 Hz, 1H) , 7.00 (t, J = 4.9 Hz, 1H) , 6.39 (s, 1H), 4.99 (s, 2H), 4.75-4.65 (m, 2H) , 3.49 (s, 1H) , 2.69-2.52 (m, 1H), 2.40-2.28 (m, 2H), 1.93-1.79 (m, 2H), 1.78-1.63 (m, 1H), 1.35-1.21 (m, 1H), 1.01 (d, J = 6.1 Hz, 3H) . |
| 377 | 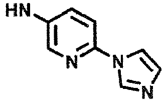 | ¹H-NMR (400 MHz, CDCl₃) δ: 9.54 (s, 1H) , 8.45 (s, 1H) , 8.27-8.15 (m, 2H), 7.64 (d, J = 9.8 Hz, 1H), 7.56-7.41 (m, 1H), 7.25-7.18 (m, 1H), 7.17 (s, 1H), 7.00 (d, J = 9.8 Hz, 1H), 6.39 (s, 1H), 5.01 (s, 2H), 2.68-2.57 (m, 1H), 2.40-2.27 (m, 2H), 1.93-1.80 (m, 2H), 1.79-1.62 (m, 1H), 1.35-1.21 (m, 1H), 1.01 (d, J = 6.1 Hz, 3H). |
| 378 | 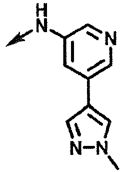 | ¹H-NMR (400 MHz, CDCl₃) δ: 9.08 (s, 1H) , 8.46 (d, J = 1.8 Hz, 1H), 8.39 (d, J = 2.4 Hz, 1H), 8.23 (t, J = 2.1 Hz, 1H), 7.77 (s, 1H), 7.66 (s, 1H) , 7.62 (d, J = 9.8 Hz, 1H) , 7.00 (d, J = 5.2 Hz, 1H) , 6.39 (s, 1H) , 4.99 (s, 2H), 3.94 (s, 3H) , 2.67-2.51 (m, 1H) , 2.40-2.29 (m, 2H), 1.90-1.80 (m, 2H), 1.78-1.65 (m, 1H) 1.35-1.21 (m, 1H), 1.01 (d, J = 6.7 Hz, 3H). |
| 379 | 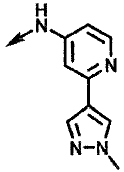 | ¹H-NMR (400 MHz, CDCl₃) δ: 9.33 (s, 1H) , 8.37 (d, J = 6.1 Hz, 1H) , 7.91 (s, 1H) , 7.85 (s, 1H) , 7.68-7.60 (m,2H), 7.21-7.17 (m, 1H), 7.00 (d, J = 9.8 Hz, 1H), 6.38 (s, 1H) , 4.98 (s, 2H), 3.92 (s, 3H), 2.67-2.56 (m, 1H), 2.40-2.26 (m, 2H), 1.90-1.80 (m, 2H), 1.75-.66 (m, 1H), 1.35-1.21 (m, 1H), 1.01 (d, J = 6.7 Hz, 3H). |
| 380 | 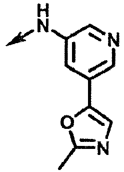 | ¹H-NMR (400 MHz, CDCl₃) δ: 9.33 (s, 1H), 8.54 (s, 1H), 8.46 (s, 1H) , 8.34 (s, 1H) , 7.63 (d, J = 9.8 Hz, 1H), 7.27 (s, 1H), 7.00 (d, J = 9.8 Hz, 1H) , 6.39 (s, 1H), 5.03 (s, 2H), 2.68-2.58 (m, 1H), 2.52 (s, 3H), 2.40-2.28 (m, 2H), 1.91-1.80 (m, 2H), 1.78-1.65 (m, 1H) , 1.35-1.21 (m, 1H) , 1.01 (d, J = 6.7 Hz, 3H). |
| 381 | 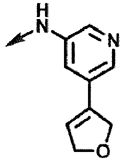 | ¹H-NMR (400 MHz, CDCl₃) δ: 9.27 (s, 1H) , 8.43 (s, 1H) , 8.26 (s, 1H) , 8.08 (s, 1H) , 7.63 (d, J = 9.1 Hz, 1H), 6.99 (d, J = 10.4 Hz, 1H), 6.41-6.32 (m, 2H) , 5. 00 (s, 2H) , 4.95 (d, J = 4.3 Hz, 2H) , 4.84 (d, J = 4.9 Hz, 2H), 2.68-2.57 (m, 1H) , 2.40-2.27 (m, 2H), 1.92-1.80 (m, 2H), 1.79-1.65 (m, 1H), 1.34-1.24 (m, 1H), 1.01 (d, J = 6.7 Hz, 3H). |
| 382 | 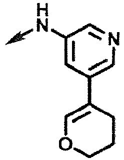 | ¹H-NMR (400 MHz, CDCl₃) δ: 9.55 (s, 1H) , 8.35 (d, J = 2.4 Hz, 1H), 8.22 (t, J = 3.0 Hz, 1H), 7.99 (d, J = 1.8 Hz, 1H), 7.59 (d, J = 10.1, 1H), 6.97-6.91 (m, 2H), 6.35 (s, 1H), 5.00 (s, 2H), 4.03-3.97 (m, 2H), 3.00 (s, 1H), 2.65-2.53 (m, 1H), 2.38-2.25 (m, 4H), 1.99-1.78 (m, 4H), 1.75-1.62 (m, 1H), 1.35-1.21 (m, 1H) , 0.99 (d, J = 6.0 Hz, 3H) . |
| 383 | 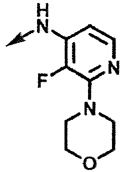 | ¹H-NMR (400 MHz, CDCl₃) δ: 9.27 (s, 1H) , 7.91 (d, J = 5.5 Hz, 1H), 7.81 (t, J = 4.9 Hz, 1H), 7.61 (d, J = 9.8 Hz, 1H), 7.00 (t, J = 4.9 Hz, 1H), 6.38 (s, 1H), 4.99 (s, 2H) , 3.84-3.76 (m, 4H), 3.48-3.40 (m, 4H), 2.65-2.55 (m, 1H), 2.40-2.27 (m, 2H), 1.92-1.80 (m, 2H), 1.80-1. 65 (m, 1H) , 1.34-1.26 (m, 1H), 1.01 (d, J = 6.7 Hz, 3H). |
| 384 | 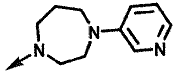 | LC-MS: [M+H]⁺ / Rt (min) 408.4 / 1.56 (Method C) |
| 385 | 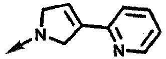 | LC-MS: [M+H]⁺ / Rt (min) 377.4 / 1.64 (Method C) |
| 386 | 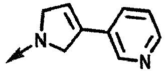 | LC-MS: [M+H]⁺ / Rt (min) 377.3 / 1.57 (Method C) |
| 387 | 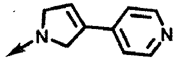 | LC-MS: [M+H]⁺ / Rt (min) 377.3 / 1.55 (Method C) |
| 388 | 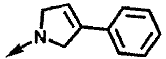 | LC-MS: [M+H]⁺ / Rt (min) 376.3 / 1.83 (Method C) |
| 389 | 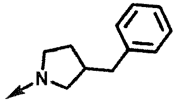 | LC-MS: [M+H]⁺ / Rt (min) 392.3 / 1.88 (Method C) |
| 390 | 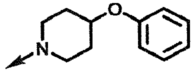 | LC-MS: [M+H]⁺ / Rt (min) 408.3 / 1.86 (Method C) |
| 391 | 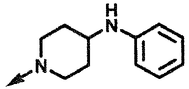 | LC-MS: [M+H]⁺ / Rt (min) 407.4 / 1.80 (Method C) |
| 392 | 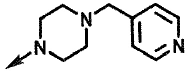 | LC-MS: [M+H]⁺ / Rt (min) 408.3 / 1.55 (Method C) |
| 393 | 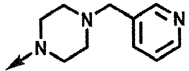 | LC-MS: [M+H]⁺ / Rt (min) 408.4 / 1.54 (Method C) |
| 394 | 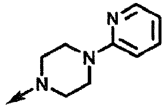 | ¹H-NMR (400 MHz, DMSO-d₆) δ: 8.14-8.12 (m, 1H), 7.83 (d, J = 10.0 Hz, 1H), 7.58-7.53 (m, 1H), 6.89 (d, J = 9.6 Hz, 1H) , 6.87-6.84 (m, 1H) , 6.68-6.65 (m, 1H), 6.51-6.48 (m, 1H), 4.97 (s, 2H), 3.65-3.49 (m, 8H), 2.68-2.64 (m, 1H), 2.34-2.27 (m, 1H), 2.25-2.15 (m, 1H), 1.84-1.73 (m, 2H), 1.71-1.60 (m, 1H), 1.28-1.17 (m, 1H), 0.96 (d, J = 6.8 Hz, 3H) . |
| 395 | 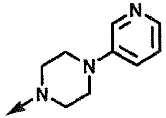 | ¹H-NMR (400 MHz, DMSO-d₆) δ: 8.33 (d, J = 2.8 Hz, 1H) , 8.03-8.02 (m, 1H), 7.83 (d, J = 10.4 Hz, 1H), 7.37-7.36 (m, 1H), 7.25-7.21 (m, 1H), 6.89 (d, J = 10.0 Hz, 1H) , 6.51-6.48 (m, 1H), 4.98 (s, 2H), 3.69-3.66 (m, 2H), 3.61-3.59 (m, 2H), 3.29-3.26 (m, 2H), 3.21-3.18 (m, 2H), 2.48-2.45 (m, 1H), 2.35-2.28 (m, 1H) , 2.24-2.15 (m, 1H), 1.84-1.73 (m, 2H), 1.68-1.61 (m, 1H), 1.26-1.16 (m, 1H), 0.96 (d, J = 6.0 Hz, 3H). |
| 396 | 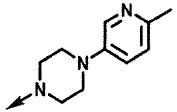 | LC-MS: [M+H]⁺ / Rt (min) 408.4 / 0.643 (Method A) |
| 397 | 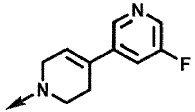 | LC-MS: [M+H]⁺ / Rt (min) 409.3 / 0.932 (Method A) |
| 398 | 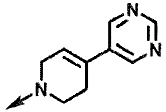 | LC-MS: [M+H]⁺ / Rt (min) 392.4 / 1.239 (Method A) |
| 399 | 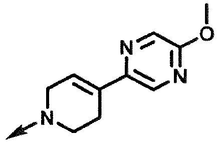 | ¹H-NMR (400 MHz, DMSO-d₆) δ: 8.40-8.37 (m, 1H), 8.29-8.28 (m, 1H) , 7.83 (d, J = 10.0 Hz, 1H), 6.89 (d, J = 10.0 Hz, 1H) , 6.65-6.63 (m, 1H) , 6.51-6.48 (m, 1H) , 5.02 (s, 1H) , 4.96 (s, 1H), 4.31-4.28 (m, 1H), 4.15-4.12 (m, 1H) , 3.91 (s, 3H), 3.75-3.66 (m, 2H), 2.69-2.63 (m, 2H), 2.57-2.51 (m, 1H), 2.34-2.14 (m, 2H), 1.84-1.70 (m, 2H), 1.70-1.59 (m, 1H) , 1.26-1.16 (m, 1H), 0.95 (d, J = 6.8 Hz, 3H) . |
| 400 | 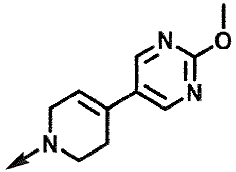 | ¹H-NMR (400 MHz, DMSO-d₆) δ: 8.71 (d, J = 7.2 Hz, 2H) , 7.83 (d, J = 10.0 Hz, 1H) , 6.89 (d, J = 10.0 Hz, 1H) , 6.51-6.48 (m, 1H) , 6.30-6.26 (m, 1H), 4.98 (d, J = 22 Hz, 2H), 4.26-4.23 (m, 1H), 4.11-4.08 (m, 1H) , 3.91 (s, 3H), 3.73 (t, J = 5.6 Hz, 1H), 3.68 (t, J = 5.6 Hz, 1H), 3.40-3.33 (m, 1H) , 2.63-2.58 (m, 1H), 2.47-2.44 (m, 1H), 2.33-2.26 (m, 1H), 2.24-2.14 (m, 1H), 1.84-1.72 (m, 2H), 1.68-1.60 (m, 1H) , 1.26-1.15 (m, 1H) , 0.95 (d, J = 6.8 Hz, 3H). |
| 401 | 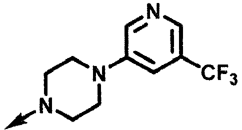 | LC-MS: (M+H]⁺ / Rt (min) 462.4 / 1.021 (Method A) |
| 402 | 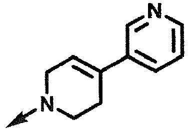 | ¹H-NMR (400 MHz, DMSO-d₆) δ: 8.70-8.68 (m, 1H), 8.48-8.47 (m, 1H), 7.87-7.82 (m, 2H) , 7.39-7.36 (m, 1H), 6.90 (d, J = 10.0 Hz, 1H), 6.51-6.48 (m, 1H), 6.31-6.29 (m, 1H), 4.99 (d, J = 23.2 Hz, 2H) , 4.28-4.26 (m, 1H), 4.13-4.10 (m, 1H) , 3.73 (t, J = 5.6 Hz, 1H) , 3.69 (t, J = 5.6 Hz, 1H), 3.39-3.33 (m, 1H), 2.68-2.61 (m, 1H) , 2.47-2.44 (m, 1H) , 2.34-2.26 (m, 1H), 2.24-2.14 (m, 1H), 1.85-1.72 (m, 2H) , 1.69-1.60 (m, 1H), 1.26-1.16 (m, 1H), 0.95 (d, J = 6.8 Hz, 3H) . |
| 403 | 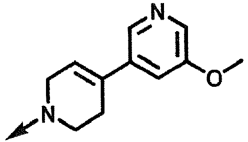 | ¹H-NMR (400 MHz, DMSO-d₆ δ: 8.30-8.27 (m, 1H), 8.20-8.19 (m, 1H), 7.84 (d, J = 10.0 Hz, 1H), 7.41-7.38 (m, 1H) , 6.90 (d, J = 10.0 Hz, 1H), 6.51-6.48 (m, 1H), 6.34-6.31 (m, 1H), 4.99 (d, J = 22 Hz, 2H), 4.28-4.25 (m, 1H), 4.13-4.10 (m, 1H), 3.85 (s, 3H), 3.73 (t, J = 5.6Hz, 1H) , 3.68 (t, J = 5.6 Hz, 1H), 3.40-3.33 (m, 1H), 2.68-2.61 (m, 1H), 2.47-2.44 (m, 1H), 2.34-2.26 (m, 1H), 2.24-2.14 (m, 1H), 1.85-1.72 (m, 2H), 1.69-1.60 (m, 1H), 1.26-1.16 (m, 1H), 0.95 (d, J = 6.8 Hz, 3H) . |
| 404 | 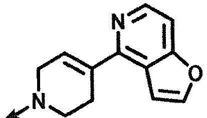 | LC-MS: [M+H]⁺ / Rt (min) 431.4 / 0.860 (Method A) |
| 405 | 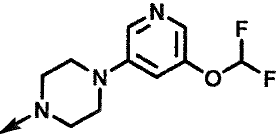 | ¹H-NMR (400 MHz, DMSO-d₆) δ: 8.24-8.22 (m, 1H), 7.90-7.89 (m, 1H), 7.83 (d, J = 9.6 Hz, 1H) , 7.18 (t, J = 2.4 Hz, 1H) , 6.90 (d, J = 10.0 Hz, 1H), 6.51-6.48 (m, 1H), 4.98 (s, 2H), 3.69-3.65 (m, 2H), 3.61-3.57 (m, 2H) , 3.39-3.34 (m, 2H), 3.29-3.25 (m, 2H), 2.67-2.63 (m, 1H), 2.54-2.50 (m, 1H), 2.35-2.30 (m, 1H), 2.26-2.14 (m, 1H), 1.85-1.72 (m, 2H), 1.70-1.60 (m, 1H), 1.27-1.17 (m, 1H) , 0.96 (d, J = 6.8 Hz, 3H). |
| 406 | 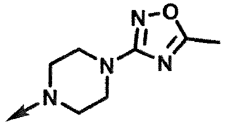 | LC-MS: [M+H]⁺ / Rt (min) 399.3 / 0.868 (Method A) |
| 407 | 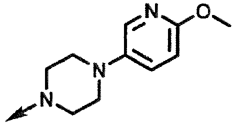 | LC-MS: [M+H]⁺ / Rt (min) 424.3 / 0.679 (Method A) |
| 408 | 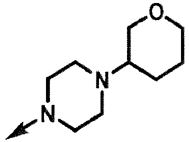 | LC-MS: [M+H]⁺ / Rt (min) 401.4 / 0.621 (Method A) |
| 409 | 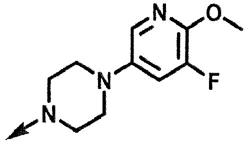 | LC-MS: [M+H]⁺ / Rt (min) 442.4 / 0.994 (Method A) |
| 410 | 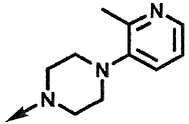 | LC-MS: [M+H]⁺ / Rt (min) 408.4 / 0.704 (Method A) |
| 411 | 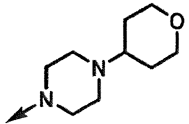 | LC-MS: [M+H]⁺ / Rt (min) 401.4 / 0.624 (Method A) |
| 412 | 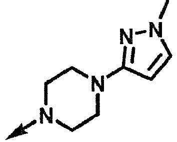 | LC-MS: [M+H]⁺ / Rt (min) 397.3 / 0.828 (Method A) |
| 413 | 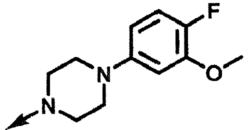 | LC-MS: [M+H]⁺ / Rt (min) 441.4 / 0.993 (Method D) |
| 414 | 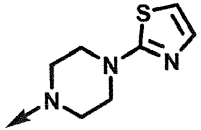 | ¹H-NMR (400 MHz, DMSO-d₆) δ: 7.83 (d, J = 9.6 Hz, 1H) , 7.19 (d, J = 3.6 Hz, 1H), 6.91-6.89 (m, 1H), 6.88 (s, 1H) , 6.51-6.47 (m, 1H), 4.97 (s, 2H), 3.68-3.65 (m, 2H), 3.60-3.58 (m, 2H), 3.51-3.48 (m, 2H), 3.41-3.39 (m, 2H), 2.48-2.45 (m, 1H), 2.35-2.26 (m, 1H), 2.26-2.14 (m, 1H), 1.85-1.73 (m, 2H), 1.69-1.60 (m, 1H), 1.26-1.18 (m, 1H), 0.96 (d, J = 6.4 Hz, 3H). |
| 415 | 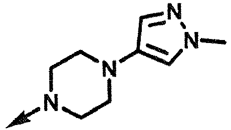 | LC-MS: [M+H]⁺ / Rt (min) 397.3 / 0.787 (Method A) |
| 416 | 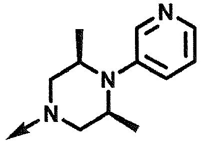 | ¹H-NMR (400 MHz , DMSO-d₆) δ: 8.36 (d, J = 2.8 Hz, 1H) , 8.03-8.01 (m, 1H), 7.84 (d, J = 9.6 Hz, 1H), 7.39-7.36 (m, 1H), 7.25-7.21 (m, 1H), 6.89 (d, J = 10.0 Hz, 1H) , 6.52-6.49 (m, 1H) , 5.01-4.90 (m, 2H), 4.48-4.39 (m, 1H) , 4.26-4.17 (m, 1H) , 3.68-3.59 (m, 2H), 3.34-3.32 (m, 1H), 3.00-2.81 (m, 2H), 2.35-2.26 (m, 1H), 2.26-2.16 (m, 1H), 1.86-1.74 (m, 2H) , 1.70-1.61 (m, 1H), 1.48-1.34 (m, 3H), 1.30-1.18 (m, 4H) , 0.96 (d, J = 6.8 Hz, 3H). |
| 417 | 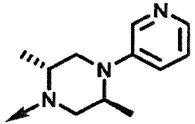 | LC-MS: [M+H]⁺ / Rt (min) 422.4 / 0.676 (Method A) |
| 418 | 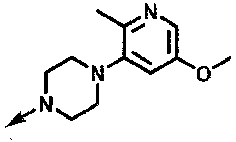 | ¹H-NMR (400 MHz, CDCl₃) δ: 7.96 (d, J = 2.4 Hz, 1H) , 7.54 (d, J = 9.8 Hz, 1H) , 6.91-6.89 (br m, 2H), 6.32 (br s, 1H) , 5.00 (d, J = 3.7 Hz, 2H) , 3.86 (s, 3H) , 3.83-3.80 (br m, 2H), 3.70-3.68 (br m, 2H), 3.00-2.92 (m, 4H), 2.62-2.60 (m, 1H) , 2.58-2.56 (m, 1H) , 2.52 (s, 3H) , 1.87-1.66 (m, 4H), 1.33-1.23 (m, 1H), 1.00 (d, J = 6.7 Hz, 3H). |
| 419 | 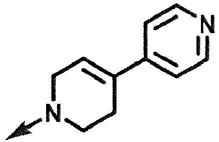 | ¹H-NMR (400 MHz, CDCl₃) δ: 8.64 (d, J = 6.1 Hz, 2H), 7.59 (br s, 2H), 7.55 (d, J = 9.8 Hz, 1H), 6.90 (d, J = 9.8 Hz, 1H), 6.60-6.51 (br m, 1H), 6.33-6.31 (m, 1H), 5.05-4.98 (m, 2H) , 4.40-4.34 (m, 2H) , 3.92-3.78 (m, 1H), 2.75-2.70 (br m, 1H), 2.64-2.53 (br m, 2H) , 2.35-2.24 (br m, 3H) , 1.88-1.78 (br m, 2H) , 1.75-1.65 (br m, 1H), 1.32-1.22 (m, 1H), 0.99 (d, J = 6.1 Hz, 3H) . |
| 420 | 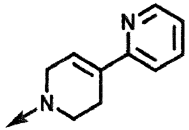 | LC-MS: [M+H]⁺ / Rt (min) 391.2 / 1.62 (Method B) |
| 421 | 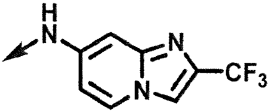 | ¹H-NMR (400 MHz, CDCl₃) δ: 9.57 (s, 1H), 7.96 (s, 1H), 7.92 (d, J = 7.6 Hz, 1H), 7.68 (s, 1H) , 7.65 (d, J = 10.0 Hz, 1H), 7.02-6.97 (m, 2H), 6.40 (s, 1H) , 5.00 (s, 2H), 2.67-2.57 (m, 1H), 2.40-2.25 (m, 2H) , 1.90-1.85 (m, 2H) , 1.77-1.67 (m, 1H), 1.35-1.25 (m, 1H), 1.01 (d, J = 6.7 Hz, 3H) . |
| 422 | 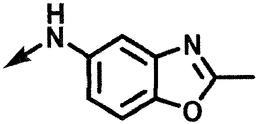 | ¹H-NMR (400 MHz, CDCl₃) δ: 8.73 (s, 1H) , 7.84 (d, J = 2.4 Hz, 1H), 7.56 (d, J = 9.8 Hz, 1H), 7.41 (dd, J = 8.6, 2.4 Hz, 1H), 7.35 (d, J = 8.6 Hz, 1H), 6.96 (d, J = 9.8 Hz, 1H) , 6.36 (s, 1H) , 4.96 (s, 2H), 2.68-2.60 (m, 1H), 2.60 (s, 3H), 2.45-2.30 (m, 2H), 1.91-1.81 (m, 2H), 1.79-1.71 (m, 1H), 1.37-1.27 (m, 1H), 1.02 (d, J = 6.7 Hz, 3H). |
| 423 | 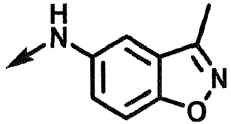 | ¹H-NMR (400 MHz, CDCl₃) δ: 8.97 (s, 1H), 8.05 (s, 1H), 7.58 (d, J = 9.8 Hz, 1H), 7.43 (s, 2H), 6.98 (t, J = 6.7 Hz, 1H), 6.38 (s, 1H), 4.98 (s, 2H), 2.69-2.60 (m, 1H), 2.53 (s, 3H), 2.43-2.32 (m, 2H) , 1.93-1.82 (m, 2H), 1.80-1.70 (m, 1H), 1. 39-1.25 (m, 1H) , 1.03 (d, J = 6.7 Hz, 3H). |
| 424 | 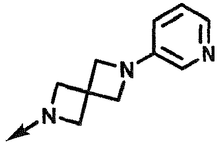 | LC-MS: [M+H]⁺ / Rt (min) 406.3 / 0.577 (Method D) |

### Examples 425 to 445

According to the method of Example 1, 37, or 50 and common reaction conditions, the compounds of Examples 425 to 445 were obtained by using corresponding material compounds.

| | | |
|---|---|---|
| 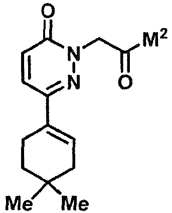 | | |

| Example | M² | Analytical data |
|---|---|---|
| 425 | 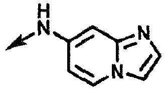 | ¹H-NMR (400 MHz, DMSO-d₆) δ: 10.55 (s, 1H), 8.45 (d, J = 7.6 Hz, 1H), 7.88-7.91 (m, 2H), 7.82 (s, 1H), 7.45-7.44 (m, 1H), 6.93-6.96 (m, 2H), 6.49-6.52 (m, 1H), 4.89 (s, 2H), 2.32-2.38 (m, 2H), 1.99-2.03 (m, 2H), 1.43 (t, J = 6.8 Hz, 2H), 0.92 (s, 6H). |
| 426 | 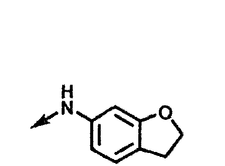 | ¹H-NMR (400 MHz, CDCl₃) δ: 8.70 (br s, 1H) , 7.60 (d, J = 10.1 Hz, 1H) , 7.09-7.06 (m, 2H), 6.96 (d, J = 10.1 Hz, 1H) , 6.91 (dd, J = 8.2, 2.1 Hz, 1H) , 6.34-6.32 (m, 1H), 4.93 (s, 2H) , 4.55 (t, J = 8.7 Hz, 2H), 3.14 (t, J = 8.7 Hz, 2H) , 2.48-2.44 (m, 2H) , 2.04-2.02 (m, 2H), 1.48 (t, J = 6.4 Hz, 2H), 0.95 (s, 6H). |
| 427 | 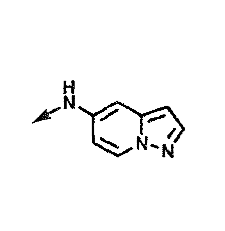 | ¹H-NMR (400 MHz, CDCl₃) δ: 8.34 (d, J = 7.3 Hz, 1H), 7.99 (s, 1H), 7.88 (d, J = 2.4 Hz, 1H), 7.64 (d, J = 9.8 Hz, 1H), 7.00 (d, J = 9.8 Hz, 1H), 6.73-6.71 (m, 1H), 6.40 (d, J = 2.4 Hz, 1H), 6.37-6.34 (br m, 1H), 4.98 (s, 2H), 2.49-2.45 (m, 2H), 2.04-2.03 (m, 2H), 1.51-1.48 (br m, 2H), 0.96 (s, 6H) . |
| 428 | 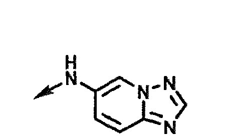 | ¹H-NMR (400 MHz, CDCl₃) δ: 9.54 (s, 1H) , 9.47 (s, 1H) , 8.29 (d, J = 4.3 Hz, 1H), 7.68-7.63 (m, 2H), 7.31-7.28 (m, 1H), 7.02 (t, d = 6.7 Hz, 1H) , 6.37 (s, 1H), 5.03 (s, 2H), 2.50-2.46 (m, 2H) , 2.08-2.03 (m, 2H) , 1.53-1.547 (m, 2H), 0.96 (s, 6H). |
| 429 | 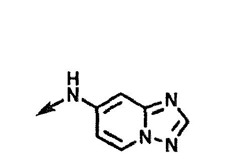 | ¹H-NMR (400 MHz, CDCl₃) δ: 9.69 (s, 1H) , 8.42 (d, J = 7.3 Hz, 1H) , 8.24 (s, 1H) , 8.08 (d, J = 1.8 Hz, 1H) , 7.66 (d, J = 9.8 Hz, 1H), 7.13 (dd, J = 7.6, 2.1 Hz, 1H), 7.01 (d, J = 9.8 Hz, 1H), 6.36 (s, 1H), 5.01 (s, 2H), 2.50-2.43 (m, 2H), 2.15-2.03 (m, 2H), 1.53-1.45 (m, 2H), 0.95 (s, 6H). |
| 430 | 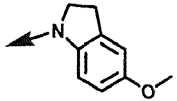 | ¹H-NMR (400 MHz, DMSO-d₆) δ: 7.89-7.84 (m, 2H) , 6.93 (d, J = 10.0 Hz, 1H) , 6.88-6.86 (m, 1H) , 6.70 (dd, J = 2.4, 4.0 Hz, 1H) , 6.50-6.47 (m, 1H) , 5.00 (s, 2H) , 4.20 (t, J = 8.0 Hz, 2H) , 3.71 (s, 3H) , 3.17 (t, J = 8.0 Hz, 2H) , 2.36-2.31 (m, 2H) , 2.03-1.99 (m, 2H), 1.42 (t, J = 6.8 Hz, 2H), 0.91 (s, 6H). |
| 431 | 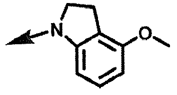 | ¹H-NMR (400 MHz, DMSO-d₆) δ: 7.88 (d, J = 10.0 Hz, 1H), 7.57 (d, J = 8.0 Hz, 1H) , 7.13 (t, J = 8.0 Hz, 1H) , 6.93 (d, J = 9.6 Hz, 1H), 6.70 (d, J = 8.0 Hz, 1H), 6.50-6.47 (m, 1H), 5.01 (s, 2H), 4.25-4.20 (m, 2H), 3.79 (s, 3H) , 3.09-3.04 (m, 2H) , 2.36-2.31 (m, 2H), 2.02-1.99 (m, 2H) , 1.44-1.40 (m, 2H), 0.91 (s, 6H). |
| 432 | 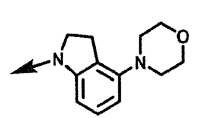 | ¹H-NMR (400 MHz, CDCl₃) δ: 7.91 (d, J = 8.4 Hz, 1H), 7.57 (d, J = 10.1 Hz, 1H), 7.16 (t, J = 8.4 Hz, 1H) , 6.92 (d, J = 10.1 Hz, 1H), 6.69 (d, J = 7.3 Hz, 1H), 6.30-6.28 (m, 1H), 5.02 (s, 2H) , 4.19 (t, J = 8.2 Hz, 2H) , 3.86-3.83 (m, 4H) , 3.19 (t, J = 8.2 Hz, 2H) , 2.99-2.97 (m, 4H) , 2.44-2.40 (m, 2H), 2.03-2.01 (m, 2H), 1.45 (t, J = 6.4 Hz, 2H), 0.94 (s, 6H). |
| 433 | - | ¹H-NMR (400 MHz, DMSO-d₆) δ: 7.89 (d, J = 10.0 Hz, 1H), 7.64 (d, J = 8.0 Hz, 1H) , 7.09 (t, J = 8.0 Hz, 1H) , 6.93 (d, J = 9.6 Hz, 1H), 6.66 (d, J = 8.0 Hz, 1H), 6.51-6.48 (m, 1H), 5.01 (s, 2H), 4.19 (t, J = 8.0 Hz, 2H), 3.09 (t, J = 8.0 Hz, 2H), 2.95-2.90 (m, 4H), 2.47-2.43 (m, 4H), 2.36-2.30 (m, 2H), 2.22 (s, 3H), 2.02-1.99 (m, 2H) , 1.42 (t, J = 6.8 Hz, 2H), 0.91 (s, 6H). |
| 434 | 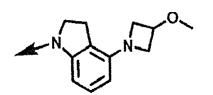 | LC-MS: [M+H]⁺ / Rt (min) 449.2 / 1.098 (Method A) |
| 435 | 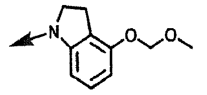 | ¹H-NMR (400 MHz, CDCl₃) δ: 7.84 (d, J = 7.9 Hz, 1H), 7.56 (d, J = 9.8 Hz, 1H) , 7.12 (t, J = 8.2 Hz, 1H) , 6.92 (d, J = 9.8 Hz, 1H), 6.78 (d, J = 7.9 Hz, 1H), 6.30-6.28 (m, 1H), 5.20 (s, 2H), 5.01 (s, 2H) , 4.21 (t, J = 8.5 Hz, 2H), 3.49 (s, 3H), 3.23 (t, J = 8.5 Hz, 2H), 2.42 (br s, 2H), 2.03-2.01 (m, 2H) , 1.46 (t, J = 6.4 Hz, 2H), 0.94 (s, 6H). |
| 436 | 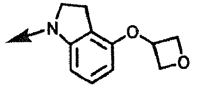 | LC-MS: [M+H]⁺ / Rt (min) 436.4 / 1.054 (Method A) |
| 437 | 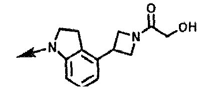 | LC-MS: [M+H]⁺ / Rt (min) 477.4 / 0.895 (Method A) |
| 438 | 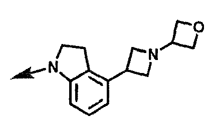 | ¹H-NMR (400 MHz, CDCl₃) δ: 8.02 (d, J = 7.9 Hz, 1H), 7.50 (d, J = 9.8 Hz, 1H) , 7.13 (t, J = 7.9 Hz, 1H), 6.93 (d, J = 7.9 Hz, 1H), 6.85 (d, J = 9.8 Hz, 1H), 6.24-6.21 (m, 1H), 4.94 (s, 2H) , 4.69 (t, J = 6.7 Hz, 2H), 4.54 (t, J = 6.1 Hz, 2H), 4.14 (t, J = 8.5 Hz, 2H), 3.86 (s, 4H), 3.33 (s, 2H) , 3.07 (t, J = 8.2 Hz, 2H) , 2.37-2.33 (m, 2H), 1.96-1.94 (m, 2H), 1.39 (t, J = 6.4 Hz, 2H), 0.87 (s, 6H). |
| 439 | 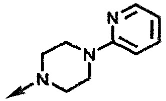 | LC-MS: [M+H]⁺ / Rt (min) 408.4 / 0.770 (Method A) |
| 440 | 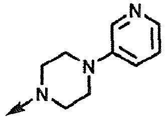 | ¹H-NMR (400 MHz, DMSO-d₆) δ: 8.33-8.32 (m, 1H), 8.03-8.02 (m, 1H), 7.85 (d, J = 10.0 Hz, 1H), 7.37-7.34 (m, 1H), 7.25-7.21 (m, 1H), 6.90 (d, J = 9.6 Hz, 1H), 6.48-6.45 (m, 1H), 4.98 (s, 2H), 3.69-3.66 (m, 2H) , 3.62-3.58 (m, 2H) , 3.29-3.26 (m, 2H), 3.22-3.17 (m, 2H), 2.34-2.29 (m, 2H) , 2.01-1.98 (m, 2H) , 1.41 (t, J = 6.0 Hz, 2H) , 0.91 (s, 6H) . |
| 441 | 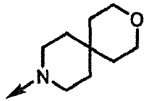 | LC-MS: [M+H]⁺ / Rt (min) 400.4 / 0.947 (Method A) |
| 442 | 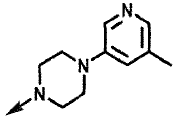 | ¹H-NMR (400 MHz, DMSO-d₆) δ: 8.14-8.12 (m, 1H), 7.87 (s, 1H), 7.85 (d, J = 10.0 Hz, 1H), 7.20-7.18 (m, 1H), 6.90 (d, J = 9.6 Hz, 1H), 6.48-6.44 (m, 1H), 4.98 (s, 2H), 3.68-3.64 (m, 2H), 3.61-3.57 (m, 2H) , 3.29-3.24 (m, 2H), 3.20-3.16 (m, 2H), 2.34-2.29 (m, 2H), 2.24 (s, 3H), 2.02-1.98 (m, 2H), 1.41 (t, J = 6.4 Hz, 2H) , 0.91 (s, 6H) . |
| 443 | 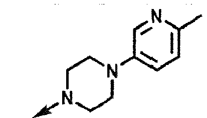 | LC-MS: [M+H]⁺ / Rt (min) 422.4 / 0.754 (Method A) |
| 444 | 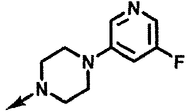 | LC-MS: [M+H]⁺ / Rt (min) 426.4 / 0.858 (Method D) |
| 445 | 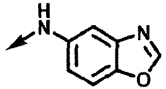 | ¹H-NMR (400 MHz, DMSO-d₆) δ: 10.49 (s, 1H) , 8.71-8.70 (m, 1H), 8.11-8.10 (m, 1H) , 7.90-7.87 (m, 1H) , 7.73-7.70 (m, 1H), 7.53-7.49 (m, 1H), 6.95-6.92 (m, 1H), 6.51-6.49 (m, 1H) , 4.89-4.86 (m, 2H) , 2.37-2.31 (m, 2H) , 2.03-1.99 (m, 2H) , 1.44-1.40 (m, 2H) , 0.93-0.89 (m, 6H) . |

### Examples 446 to 455

According to the method of Example 1, 37, or 50 and common reaction conditions, the compounds of Examples 446 to 455 were obtained by using corresponding material compounds.

| | | | | |
|---|---|---|---|---|
| 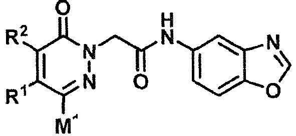 | | | | |

| Example | M¹ | R¹ | R² | Analytical data |
|---|---|---|---|---|
| 446 | 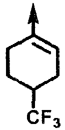 | H | H | ¹H-NMR (400 MHz, CDCl₃) δ: 8.93 (s, 1H) , 8.06 (s, 2H) , 7.59 (d, J = 9.8 Hz, 1H), 7.51-7.44 (m, 2H), 7.01 (d, J = 9.8 Hz, 1H), 6.37-6.33 (m, 1H), 4.98 (s, 2H), 2.86-2.77 (m, 1H), 2.55-2.45 (m, 1H), 2.45-2.25 (m, 3H), 2.20-2.12 (m, 1H), 1.64-1.58 (m, 1Hz) . |
| 447 | 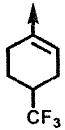 | H | OMe | ¹H-NMR (400 MHz, CDCl₃) δ: 9.10 (s, 1H) , 8.08-8.05 (m, 2H) , 7.48 (s, 2H) , 6.45-6.40 (s, 1H) , 6.27 (s, 1H) , 4.95 (s, 2H), 3.87 (s, 3H), 2.67-2.57 (m, 1H) , 2.42-2.225 (m, 4H), 2.16-2.08 (m, 1H) , 1.70-1.60 (m, 1H). |
| 448 | 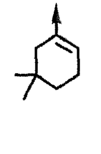 | H | OMe | ¹H-NMR (400 MHz, CDCl₃) δ: 9.35 (s, 1H), 8.01 (d, J = 5.5 Hz, 2H), 7.48-7.35 (m, 2H), 6.33-6.27 (m, 2H), 6.21 (s, 1H), 4.93 (s, 2H), 3.82 (s, 3H), 2.24-2.17 (m, 2H), 2.10 (s, 2H), 1.37 (t, J = 6.4 Hz, 2H), 1.21 (s, 6H). |
| 449 | 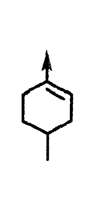 | H | OMe | ¹H-NMR (400 MHz, CDCl₃) δ: 9.13 (s, 1H), 8.07 (s, 2H), 7.49 (s, 2H), 6.38 (s, 1H), 6.25 (s, 1H), 4.95 (s, 2H), 3.86 (s, 3H), 2.48-2.28 (m, 3H), 1.87-1.67 (m, 2H), 1.38-1.23 (m, 2H) , 1.00 (d, J = 6.1 Hz, 3H) . |
| 450 | 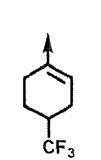 | OMe | H | ¹H-NMR (400 MHz, CDCl₃) δ: 9.07 (s, 1H) , 8.07-8.06 (m, 2H) , 7.48-7.47 (m, 2H), 6.43-6.41 (m, 1H), 6.27 (s, 1H), 4.94 (s, 2H) , 3.87 (s, 3H) , 2.65-2.59 (m, 1H), 2.49-2.26 (m, 4H), 2.15-2.10 (m, 1H), 1.69-1.58 (m, 1H) . |
| 451 | 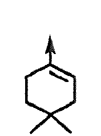 | OMe | H | ¹H-NMR (400 MHz, CDCl₃) δ: 9.35 (s, 1H) , 8.08-8.05 (m, 2H), 7.44 (s, 1H), 6.40 (s, 1H), 6.28 (s, 1H), 5.00 (s, 2H), 3.88 (s, 3H) , 2.45-2.38 (m, 2H) , 2.05-2.00 (m, 2H) , 1.49 (t, J = 6.4 Hz, 2H) , 0.98 (s, 6H). |
| 452 | 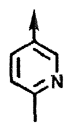 | H | H | ¹H-NMR (400 MHz, CDCl₃) δ: 9.13 (s, 1H), 8.74 (s, 1H), 7.98-7.93 (m, 1H) , 7.93-7.86 (m, 2H), 7.68-7.64 (m, 1H), 7.49-7.39 (m, 1H) , 7.29-7.38 (m, 1H) , 7.13-7.05 (m, 1H) , 6.99-6.94 (m, 1H), 4.94 (s, 2H), 2.44 (s, 3H) . |
| 453 | 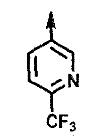 | H | H | ¹H-NMR (400 MHz, CDCl₃) δ: 9.10 (s, 1H) , 8.74 (s, 1H) , 8.34 (d, J = 7.9 Hz, 1H), 8.13-8.03 (m, 2H), 7.85-7.75 (m, 2H), 7.65-7.35 (m, 3H) , 5.10 (s, 2H). |
| 454 | 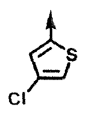 | H | H | ¹H-NMR (400 MHz, CD₃OD) δ: 8.45 (s, 1H) , 8.11 (d, J = 1.8 Hz, 1H) , 7.99 (dd, J = 9.8, 4.9 Hz, 1H), 7.63 (d, J = 8.6 Hz, 1H), 7.60-7.53 (m, 2H), 7.39 (d, J = 1.2 Hz, 1H), 7.09 (dd, J = 9.8, 4.3 Hz, 1H), 5.04 (s, 2H). |
| 455 | 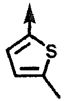 | H | H | ¹H-NMR (400 MHz, CDCl₃) δ: 8.95 (s, 1H), 8.04 (s, 2H) , 7.63 (d, J = 9.8 Hz, 1H), 7.50-7.40 (m, 2H), 7.20 (q, J = 4.7 Hz, 1H) , 7.06 (t, J = 11.0 Hz, 1H) , 6.72 (t, J = 1.8 Hz, 1H) , 5.00 (s, 2H), 7.49 (s, 3H). |

### Examples 456 to 467

According to the method of Example 1, 37, or 50 and common reaction conditions, the compounds of Examples 456 to 467 were obtained by using corresponding material compounds.

| | | | | |
|---|---|---|---|---|
| 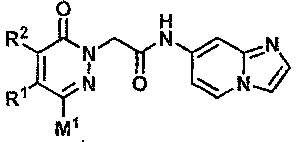 | | | | |

| Example | M¹ | R¹ | R² | Analytical data |
|---|---|---|---|---|
| 456 | 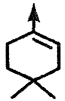 | H | OMe | ¹H-NMR (400 MHz, CDCl₃) δ: 9.98 (s, 1H), 8.06-7.98 (m, 1H), 7.88 (s, 1H), 7.57 (s, 1H), 7.54-7.48 (m, 1H), 7.26-7.17 (m, 1H), 6.35-6.39 (m, 1H) , 6.27 (s, 1H) , 5.06-5.02 (m, 2H), 3.89 (s, 3H), 2.44-2.39 (m, 2H) , 2.05-1. 99 (m, 2H) , 1.49 (t, J = 6.4 Hz, 2H), 0.99 (s, 6H). |
| 457 | 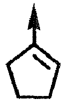 | H | H | ¹H-NMR (400 MHz, CDCl₃) δ: 10.40 (s, 1H), 7.89 (d, J = 7.6 Hz, 1H), 7.85 (s, 1H), 7.62 (d, J = 9.6 Hz, 1H), 7.53 (s, 1H) , 7.41 (s, 1H), 7.12 (dd, J = 7.6, 2.1 Hz, 1H), 6.98 (d, J = 9.6 Hz, 1H), 6.36 (s, 1H), 5.07 (s, 2H), 2.79-2.71 (m, 2H), 2.64-2.57 (m, 2H), 2.04 (q, J = 7.2 Hz, 2H) . |
| 458 | 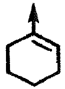 | H | H | ¹H-NMR (400 MHz, CDCl₃) δ: 10.38 (s, 1H) , 7.86-7.80 (m, 2H), 7.56 (d, J = 9.8 Hz, 1H), 7.46 (s, 1H), 7.34 (s, 1H) , 7.05 (dd, J =7.2, 2.0 Hz, 1H), 6.93 (d, J = 9.8 H, 1H z), 6.36 (s, 1H), 5.02 (s, 2H), 2.40 (s, 4H), 1.75-1.60 (m, 4H). |
| 459 | 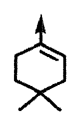 | OMe | H | ¹H-NMR (400 MHz, CDCl₃) δ: 9.58 (s, 1H), 7.97-7.83 (m, 1H), 7.81 (s, 1H), 7.49 (s, 1H) , 7.39 (s, 1H) , 6.98 (d, J= 6.7 Hz, 1H) , 6.36-6.30 (m, 1H), 6.23 (s, 1H), 4.94 (s, 2H), 3.86 (s, 3H) , 2.40-2.35 (m, 2H) , 2.00-1.96 (m, 2H), 1.44 (2H, t, J = 6.7 Hz), 0.94 (s, 6H). |
| 460 | 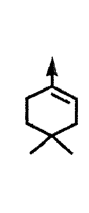 | H | Me | ¹H-NMR (400 MHz, DMSO-d₆) δ: 10.57 (s, 1H) 8.40 (d, J = 7.3 Hz, 1H), 7.85 (s, 1H) , 7.75 (d, J = 7.3 Hz, 2H) , 7.39 (s, 1H) , 6.92 (dd, J = 7.3, 1.8 Hz, 1H), 6.41 (s, 1H) , 4.83 (s, 2H) , 2.28 (s, 2H) , 2.04 (s, 3H) , 1.95 (s, 2H) , 1.38-1.34 (m, 2H) , 0.85 (s, 6H). |
| 461 | 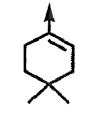 | Me | H | ¹H-NMR (400 MHz, CDCl₃) δ: 10.10 (s, 1H) , 7.98-7.93 (m, 2H) , 7.50-7.43 (m, 2H), 7.16-7.08 (s, 1H), 6.83 (s, 1H) , 5.78 (s, 1H), 5.03 (s, 2H), 2.30-2.23 (m, 5H), 1.98 (s, 2H), 1.51-1.48 (m, 2H), 0.98 (s, 6H). |
| 462 | 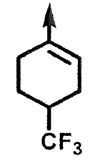 | H | OMe | ¹H-NMR (400 MHz, DMSO-d₆) δ: 10.55 (s, 1H) , 8.46 (d, J = 7.3 Hz, 1H) , 7.91 (s, 1H), 7.83 (s, 1H), 7.46 (s, 1H), 6.95 (d, J = 7.3 Hz, 1H), 6.37 (s, 2H), 4.86 (s, 2H), 3.85 (s, 3H), 2.67-2.53 (m, 2H), 2.46-2.16 (m, 3H) , 2.05-1.99 (m, 1H) , 1.57-1.45 (m, 1H). |
| 463 | 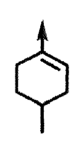 | H | OMe | ¹H-NMR (400 MHz, CDCl₃) δ: 9.39 (br s, 1H), 7.97 (d, J = 7.3 Hz, 1H) , 7.82 (s, 1H), 7.55 (s, 1H), 7.46 (s, 1H), 7.06-7.04 (br m, 1H), 6.38-6.37 (br m, 1H) , 6.25 (s, 1H) , 4.94 (s, 2H), 3.86 (s, 3H), 2.47-2.28 (m, 3H), 1.85-1.78 (m, 2H), 1.37-1.24 (m, 2H), 1.00 (d, J = 6.1 Hz, 3H). |
| 464 | 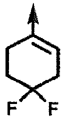 | H | H | ¹H-NMR (400 MHz, DMSO-d₆) δ: 10.58 (s, 1H), 8.46 (dd, J = 7.3, 1.2 Hz, 1H), 7.95-7.90 (m, 2H) , 7.83-7.82 (m, 1H) , 7.46 (d, J = 1.2 Hz, 1H) , 7.00 (d, J = 9.8 Hz, 1H) , 6.96 (dd, J = 7.3, 2.4 Hz, 1H), 6.46 (s, 1H), 4.92 (s, 2H), 2.83-2.75 (m, 2H), 2.66-2.60 (m, 2H), 2.19-2.09 (m, 2H) . |
| 465 | 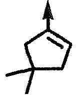 | H | H | LC-MS: [M+H]⁺ / Rt (min) 364.3 / 0.582 (Method D) |
| 466 | 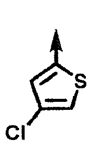 | H | OMe | ¹H-NMR (400 MHz, CDCl₃) δ: 9.86 (s, 1H) , 7.90 (s, 1H), 7.78 (s, 1H), 7.62 (s, 1H), 7.52 (d, J = 6.7 Hz, 1H), 7.39 (s, 1H), 7.18 (s, 1H), 7.11 (d, J = 6.7 Hz, 1H), 6.34 (s, 1H), 5.02 (s, 2H), 3.99 (s, 3H). |
| 467 | 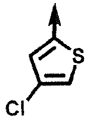 | H | H | ¹H-NMR (400 MHz, CD₃OD) δ: 8.38 (d, J = 7.3 Hz, 1H), 8.01 (d, J = 9.8 Hz, 2H), 7.75 (s, 1H), 7.57 (d, J= 1.2 Hz, 1H), 7.48 (dd, J = 8.8, 1.5 Hz, 1H), 7.40 (d, J = 1.2 Hz, 1H), 7.10 (t, J = 4.9 Hz, 2H), 5.05 (s, 2H) . |

### Examples 468 to 481

According to the method of Example 1, 37, 38, or 50, and common reaction conditions, the compounds of Examples 468 to 481 were obtained by using corresponding material compounds.

| | | | | |
|---|---|---|---|---|
| 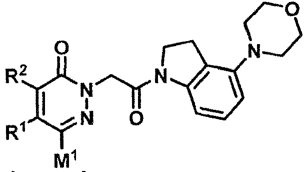 | | | | |

| Example | M¹ | R¹ | R² | Analytical data |
|---|---|---|---|---|
| 468 | 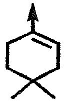 | H | OMe | ¹H-NMR (400 MHz, CDCl₃) δ: 7.91 (d, J = 8.0 Hz, 1H), 7.15 (t, J = 8.0 Hz, 1H), 6.68 (d, J = 8.0 Hz, 1H), 6.27 (s, 1H), 6.19 (s, 1H), 4.98 (s, 2H), 4.16 (t, J = 8.0 Hz, 2H), 3.90-3.78 (m, 7H), 3.17 (t, J = 8.0 Hz, 2H) , 2.98 (m, 4H), 2.36 (s, 2H), 1.98 (m, 2H), 1.45 (t, J = 6.4 Hz, 2H), 0.95 (6H, s). |
| 469 | 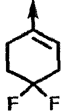 | H | H | LC-MS: [M+H]⁺ / Rt (min) 457.4 / 0.824 (Method D) |
| 470 | 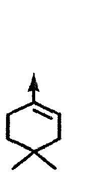 | OMe | H | ¹H-NMR (400 MHz, CDCl₃) δ: 7.89 (d, J = 8.0 Hz, 1H), 7.13 (t, J = 8.0 Hz, 1H), 6.66 (d, J = 8.0 Hz, 1H), 6.25 (s, 1H), 6.17 (s, 1H), 4.97 (s, 2H), 4.14 (t, J = 8.0 Hz, 2H), 3.90-3.78 (m, 7H), 3.15 (t, J = 8.0 Hz, 2H), 2.96 (m, 4H), 2.34 (s, 2H), 1.96 (m, 2H), 1.43 (t, J = 6.4 Hz, 2H), 0.93 (6H, s) . |
| 471 | 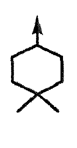 | H | H | ¹H-NMR (400 MHz, CDCl₃) δ: 7.84 (d, J = 7.9 Hz, 1H), 7.56 (d, J = 9.8 Hz, 1H), 7.12 (t, J = 8.2 Hz, 1H), 6.92 (d, J = 9.8 Hz, 1H), 6.78 (d, J = 7.9 Hz, 1H), 6.30-6.28 (m, 1H), 5.20 (s, 2H), 5.01 (s, 2H), 4.21 (t, J = 8.5 Hz, 2H), 3.49 (s, 3H), 3.23 (t, J = 8.5 Hz, 2H), 2.42 (br s, 2H), 2.03-2.01 (m, 2H), 1.46 (t, J = 6.4 Hz, 2H), 0.94 (s, 6H). |
| 472 | 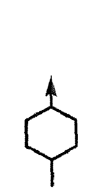 | H | H | ¹H-NMR (400 MHz, CDCl₃) δ: 7.91 (d, J = 7.3 Hz, 1H), 7.24-7.13 (m, 2H), 6.93 (dd, J = 9.1, 1.2 Hz, 1H), 6.69 (d, J = 7.9 Hz, 1H), 5.00 (d, J = 5.5 Hz, 2H), 4.18 (t, J = 8.2 Hz, 2H), 3.86-3.83 (m, 4H), 3.19 (t, J = 8.2 Hz, 2H), 2.99-2.97 (br m, 4H), 2.67-2.44 (m, 1H), 1. 93-1. 90 (br m, 1H) , 1.82-1.61 (m, 4H), 1.48-0.99 (m, 4H), 0.96-0.91 (m, 3H). |
| 473 | 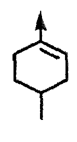 | H | OMe | ¹H-NMR (400 MHz, CDCl₃) δ: 7.91 (br m, 1H), 7.15 (br m, 1H), 6.68 (br m, 1H), 6.28 (s, 1H), 6.19 (s, 1H), 4.98 (s, 2H), 4.18-4.14 (m, 2H), 3.85-3.81 (m, 7H), 3.19-3.15 (m, 2H), 2.99-2.97 (m, 4H), 2.44-2.25 (m, 3H), 1.84-1.73 (m, 3H), 1.35-1.24 (m, 1H), 0.99 (d, J = 6.7 Hz, 3H). |
| 474 | 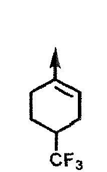 | H | OMe | ¹H-NMR (400 MHz, DMSO-d₆) δ: 7.67 (d, J = 7.9 Hz, 1H), 7.12 (t, J = 7.9 Hz, 1H), 6.68 (d, J = 7.9 Hz, 1H), 6.37-6.34 (m, 2H), 5.00 (s, 2H), 4.20 (t, J = 8.2 Hz, 2H), 3.85 (s, 3H), 3.73 (t, J = 4.3 Hz, 4H), 3.12 (t, J = 8.2 Hz, 2H), 2.93 (t, J = 4.6 Hz, 4H), 2.68-2.52 (m, 2H), 2.47-2.30 (m, 2H), 2.24-2.15 (m, 1H), 2.04-1.98 (m, 1H), 1.55-1.44 (m, 1H). |
| 475 | 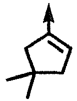 | H | H | ¹H-NMR (400 MHz, DMSO-d₆) δ: 7.90 (d, J = 9.6 Hz, 1H), 7.66 (d, J = 8.0 Hz, 1H), 7.11 (t, J = 8.0 Hz, 1H), 6.95 (d, J = 9.6 Hz, 1H), 6.68 (d, J = 8.0 Hz, 1H), 6.46 (s, 1H), 5.00 (s, 2H), 4.20 (t, J = 8.0 Hz, 2H), 3.74-3.70 (m, 4H), 3.11 (t, J = 8.0 Hz, 2H), 2.94-2.90 (m, 4H), 2.44-2.41 (m, 2H), 2.35-2.31 (m, 2H), 1.09 (s, 6H) . |
| 476 | 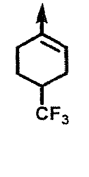 | OMe | H | ¹H-NMR (400 MHz, CDCl₃) δ: 7.90 (d, J = 7.9 Hz, 1H), 7.15 (t, J = 7.9 Hz, 1H), 6.69 (d, J = 7.3 Hz, 1H), 6.35 (br s, 1H), 6.21 (s, 1H), 4.98 (s, 2H), 4.17 (t, J = 8.2 Hz, 2H), 3.85-3.83 (m, 4H), 3.83 (s, 3H), 3.18 (t, J = 8.2 Hz, 2H), 2.99-2.97 (m, 4H), 2.61-2.56 (m, 1H), 2.48-2.25 (m, 4H), 2.12-2.06 (m, 1H), 1.67-1.56 (m, 1H) . |
| 477 | 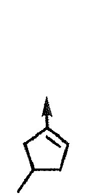 | H | H | ¹H-NMR (400 MHz, DMSO-d₆) δ: 7.90 (d, J = 9.6 Hz, 1H), 7.66 (d, J = 8.0 Hz, 1H), 7.11 (t, J = 8.0 Hz, 1H), 6.95 (d, J = 10.0 Hz, 1H), 6.68 (d, J = 8.0 Hz, 1H), 6.51-6.49 (m, 1H), 5.01 (s, 2H), 4.20 (t, J = 8.0 Hz, 3H), 3.73-3.71 (m, 4H), 3.15-3.10 (m, 2H), 2.94-2.90 (m, 4H), 2.67-2.63 (m, 1H), 2.54-2.52 (m, 1H), 2.24-2.16 (m, 1H), 2.15-2.06 (m, 1H), 1.03 (d, J = 6.8 Hz, 3H) . |
| 478 | 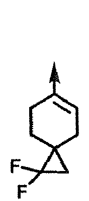 | H | H | ¹H-NMR (400 MHz, DMSO-d₆) δ: 7.91 (d, J = 9.8 Hz, 1H), 7.68 (d, J = 7.9 Hz, 1H), 7.12 (t, J = 7.9 Hz, 1H), 6.97 (d, J = 9.8 Hz, 1H), 6.69 (d, J = 7.9 Hz, 1H), 6.61-6.58 (m, 1H), 5.04 (s, 2H), 4.22 (t, J = 8.2 Hz, 2H), 3.74-3.71 (m, 4H), 3.13 (t, J = 8.2 Hz, 2H), 2.95-2.91 (m, 4H), 2.49-2.41 (m, 3H), 2.34-2.22 (m, 1H), 1.77-1.70 (m, 2H), 1.38-1.23 (m, 2H). |
| 479 | 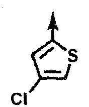 | H | OMe | LC-MS: [M+H]⁺ / Rt (min) 487.3 / 0.982 (Method A) |
| 480 | 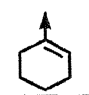 | H | H | LC-MS: [M+H]⁺ / Rt (min) 421.4 / 1.72 (Method C) |
| 481 | 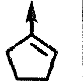 | H | H | LC-MS: [M+H]⁺ / Rt (min) 407.3 / 1.65 (Method C) |

### Examples 482 to 490

According to the method of Example 1, 37, or 50, and common reaction conditions, the compounds of Examples 482 to 490 were obtained by using corresponding material compounds.

| | | | | |
|---|---|---|---|---|
| 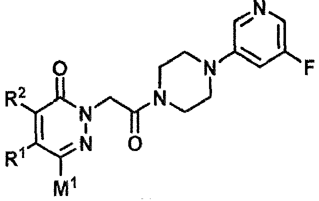 | | | | |

| Example | M¹ | R¹ | R² | Analytical data |
|---|---|---|---|---|
| 482 | 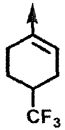 | H | OMe | ¹H-NMR (400 MHz, DMSO-d₆) δ: 8.21-8.20 (m, 1H), 7.95 (d, J = 2.4 Hz, 1H), 7.29 (dt, J = 2.4, 6.4 Hz, 1H), 6.32-6.29 (m, 2H), 4.96 (s, 2H), 3.82 (s, 3H), 3.67-3.64 (m, 2H), 3.59-3.57 (m, 2H), 3.38-3.34 (m, 2H), 3.28-3.26 (m, 2H), 2.61-2.51 (m, 1H), 2.48-2.41 (m, 1H), 2.41-2.29 (m, 2H), 2.23-2.13 (m, 1H), 2.03-1.96 (m, 1H), 1.53-1.43 (m, 1H) . |
| 483 | 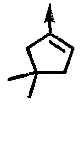 | H | H | ¹H-NMR (400 MHz, DMSO-d₆) δ: 8.21 (s, 1H), 7.96 (s, 1H), 7.89-7.85 (m, 1H), 7.32-7.26 (m, 1H), 6.94-6.90 (m, 1H), 6.43 (s, 1H), 4.97 (s, 2H) , 3.69-3.57 (m, 4H), 3.40-3.34 (m, 3H), 2.67-2.63 (m, 1H), 2.43-2.40 (m, 1H), 2.34-2.31 (m, 3H), 1.08 (s, 6H) . |
| - 484 | 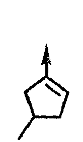 | H | H | ¹H-NMR (400 MHz, DMSO-d₆) δ: 8.21 (s, 1H), 7.96-7.95 (m, 1H), 7.87 (d, J = 10.0 Hz, 1H), 7.31-7.26 (m, 1H), 6.91 (d, J = 9.6 Hz, 1H), 6.48-6.46 (s, 1H), 4.98-4.97 (m, 2H), 3.69-3.65 (m, 2H), 3.61-3.57 (m, 2H), 3.39-3.35 (m, 2H), 3.29-3.26 (m, 2H), 2.81-2.64 (m, 2H), 2.46-2.39 (m, 1H), 2.22-2.15 (m, 1H), 2.14-2.05 (m, 1H), 1.03 (d, J = 7.2 Hz, 3H) . |
| 485 | 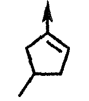 | H | OMe | LC-MS: [M+H]⁺ / Rt (min) 428.3 / 0.866 (Method A) |
| 486 | 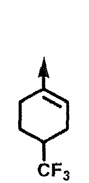 | OMe | H | ¹H-NMR (400 MHz, CDCl₃) δ: 8.14-8.13 (m, 1H), 8.01 (d, J = 2.4 Hz, 1H), 6.88 (dt, J = 11.6, 2.4 Hz, 1H), 6.33-6.32 (br m, 1H), 6.18 (s, 1H), 4.97 (s, 2H), 3.83 (s, 3H), 3.81-3.70 (br m, 4H), 3.34-3.25 (m, 4H), 2.60-2.54 (m, 1H), 2.48-2.24 (m, 4H), 2.12-2.06 (m, 1H), 1.67-1.58 (m, 1H) . |
| 487 | 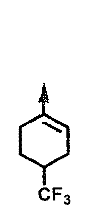 | H | H | ¹H-NMR (400 MHz, CDCl₃) δ: 8.14-8.13 (br m, 1H), 8.01 (d, J = 2.1 Hz, 1H), 7.53 (d, J = 10.1 Hz, 1H), 6.93 (d, J = 10.1 Hz, 1H), 6.89 (dt, J = 11.0, 2.1 Hz, 1H), 6.32-6.30 (br m, 1H), 5.02 (d, J = 15.2 Hz, 1H), 4.98 (d, J = 15.2 Hz, 1H), 3.84-3.71 (m, 4H), 3.36-3.26 (m, 4H), 2.80-2.75 (m, 1H), 2.52-2.45 (m, 1H), 2.37-2.29 (m, 3H), 2.15-2.11 (m, 1H), 1.64-1.55 (m, 1H). |
| 488 | 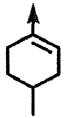 | H | OMe | ¹H-NMR (400 MHz, CDCl₃) δ: 8.13-8.12 (br m, 1H), 8.00 (d, J = 2.4 Hz, 1H), 6.88 (dt, J = 11.6, 2.4 Hz, 1H), 6.26 (d, J = 4.9 Hz, 1H), 6.16 (s, 1H), 4. 97 (s, 2H), 3.81 (s, 3H), 3.81-3.68 (m, 4H), 3.33-3.24 (m, 4H), 2.44-2.24 (m, 3H), 1.85-1.71 (m, 3H), 1.35-1.25 (m, 1H), 0. 99 (d, J = 6.7 Hz, 3H). |
| 489 | 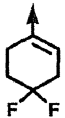 | H | H | LC-MS: [M+H]⁺ / Rt (min) 434.3 / 0.662 (Method A) |
| 490 | 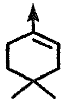 | OMe | H | ¹H-NMR (400 MHz, CDCl₃) δ: 8.13 (t, J = 1.8 Hz, 1H), 8.01 (d, J = 2.4 Hz, 1H), 6.88 (dt, J = 11.0, 2.4 Hz, 1H), 6.26-6.24 (m, 1H), 6.17 (s, 1H), 4.97 (s, 2H), 3.82 (s, 3H), 3.70 (s, 2H), 3.36-3.22 (m, 4H), 2.40-2.30 (m, 2H), 1.99-1.94 (m, 2H), 1.47-1.43 (m, 2H), 0.95 (s, 6H), 0.90-0.85 (m, 2H). |

### Examples 491 to 534

According to the method of Example 1, 37, or 50, and common reaction conditions, the compounds of Examples 491 to 534 were obtained by using corresponding material compounds.

| Example | Chemical structure | Analytical data |
|---|---|---|
| 491 | 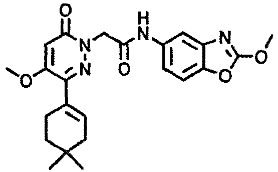 | ¹H-NMR (400 MHz, CDCl₃) δ: 9.02 (s, 1H), 7.78 (s, 1H) , 7.24-7.22 (m, 2H), 6.37 (s, 1H), 6.25 (s, 1H), 4.95 (s, 2H), 4.22 (s, 3H), 3.87 (s, 3H), 2.43-2.37 (m, 2H), 2.04-1.99 (m, 2H), 1.49 (t, J = 6.4 Hz, 2H), 0.98 (s, 6H). |
| 492 | 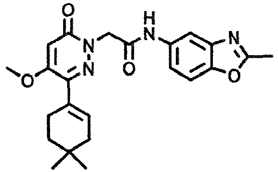 | ¹H-NMR (400 MHz, CDCl₃) δ: 9.07 (s, 1H), 7.87 (s, 1H), 7.45-7.30 (m, 2H) , 6.40-6.31 (m, 1H), 6.27-6.21 (m, 1H), 4.94 (s, 2H), 3.85 (s, 3H), 2.62 (s, 3H), 2.39 (s, 2H), 2.00 (s, 2H), 1.53-1.45 (m, 2H), 0.97 (s, 6H). |
| 493 | 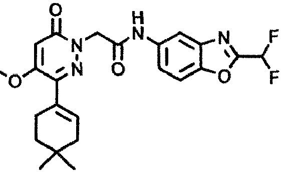 | ¹H-NMR (400 MHz, CDCl₃) δ: 9.38 (s, 1H), 8.16 (s, 1H), 7.52 (s, 1H), 6.79 (t, J = 57.4 Hz, 1H), 6.38 (s, 1H), 6.27 (s, 1H), 4.98 (s, 2H), 3.88 (s, 3H), 2.42-2.39 (m, 2H), 2.04-2.00 (m, 2H), 1.50 (t, J = 6.4 Hz, 2H), 0.98 (s, 6H) . |
| 494 | 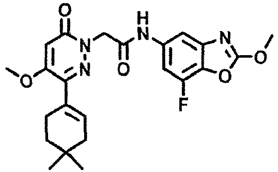 | ¹H-NMR (400 MHz, CDCl₃) δ: 9.34 (s, 1H), 7.39 (d, J = 1.5 Hz, 1H), 7.29 (dd, J = 11.6, 1.5 Hz, 1H), 6.35-6.33 (m, 1H), 6.23 (s, 1H), 4.93 (s, 2H), 4.21 (s, 3H), 3.85 (s, 3H), 2.42-2.35 (m, 2H), 2.02-1.98 (m, 2H), 1.47 (t, J = 6.5 Hz, 2H), 0.95 (s, 6H). |
| 495 | 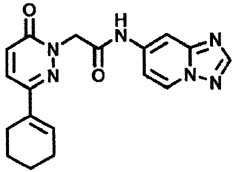 | ¹H-NMR (400 MHz, CDCl₃) δ: 9.70 (s, 1H), 8.43 (d, J = 7.3 Hz, 1H), 8.26 (s, 1H), 8.09 (s, 1H), 7.66 (d, J = 9.8 Hz, 1H), 7.15 (d, J = 7.9 Hz, 1H), 7.03 (d, J = 9.1 Hz, 1H), 6.45 (s, 1H), 5.03 (s, 2H), 2.49-2.43 (m, 2H), 2.33-2.23 (m, 2H), 1.82-1.63 (m, 4H) . |
| 496 | 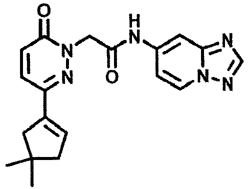 | ¹H-NMR (400 MHz, CDCl₃) δ: 10.13 (s, 1H), 8.30 (d, J = 7.3 Hz, 1H), 8.20 (d, J = 5.5 Hz, 1H), 7.99 (s, 1H), 7.60 (d, J = 10.7 Hz, 1H), 7.14 (dt, J = 16.9, 6.9 Hz, 1H), 7.03-6.95 (m, 1H), 6.26 (s, 1H), 5.04 (s, 2H), 2.54 (s, 2H), 2.39 (s, 2H), 1.18 (s, 6H) . |
| 497 | 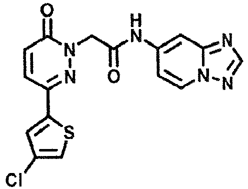 | ¹H-NMR (400 MHz, DMSO-d₆) δ: 10.97 (s, 1H), 8.86 (d, J = 7.9 Hz, 1H), 8.37 (s, 1H), 8.13 (dd, J = 8.8, 5.8 Hz, 2H), 7.82 (d, J = 1.2 Hz, 1H), 7.69 (d, J = 1.2 Hz, 1H), 7.21 (dd, J = 7.3, 1.8 Hz, 1H), 7.14 (d, J = 9.8 Hz, 1H) , 4.98 (s, 2H) . |
| 498 | 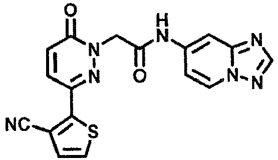 | ¹H-NMR (400 MHz, CDCl₃) δ: 9.25 (s, 1H), 8.46 (d, J = 7.3 Hz, 1H), 8.28 (s, 1H), 8.18 (d, J = 9.8 Hz, 1H), 8.10 (d, J = 1.8 Hz, 1H), 7.50 (d, J = 5.5 Hz, 1H), 7.36 (d, J = 4.9 Hz, 1H), 7.25-7.18 (m, 2H), 5.12 (s, 2H). |
| 499 | 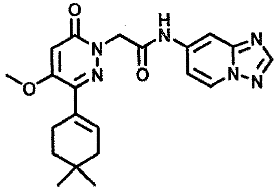 | ¹H-NMR (400 MHz, CDCl₃) δ: 10.20 (s, 1 H), 8.27-8.22 (m, 1 H), 8.15 (s, 1 H), 8.00-7.93 (m, 1H), 7.09-7.02 (m, 1 H), 6.30 (s, 1 H), 6.21 (s, 1 H), 4.98 (s, 2 H), 3.83 (s, 3 H), 2.34 (s, 2 H), 1.95 (s, 2 H), 1.50-1.38 (m, 2 H), 0.94 (s, 6 H). |

| 500 | 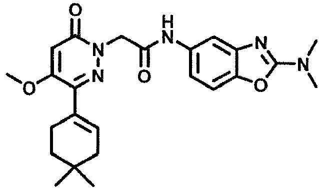 | ¹H-NMR (400 MHz, CD₃OD) ð:8.89 (s, 1 H), 7.49-7.41 (m, 1 H) , 7.19-7.12 (m, 1 H), 7.12-7.03 (m, 1 H), 6.30 (s, 1 H), 6.18 (s, 1H), 4.88 (s, 2 H), 3.77 (s, 3 H), 3.14 (s, 6 H), 2.33 (s, 2 H), 1.99-1.92 (m, 2 H) , 1.49-1.38 (m, 2 H), 0.93 (s, 6 H) . |
|---|---|---|
| 501 | 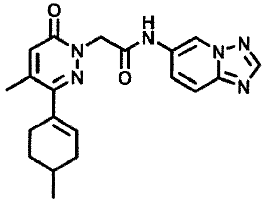 | ¹H-NMR (400 MHz, CDCl₃) δ: 10.00 (s, 1H), 9.31-9.20 (m, 1 H), 8.08 (s, 1 H), 7.45-7.35 (m, 1 H), 7.23-7.15 (m, 1 H), 6.75 (s, 1 H), 5.75 (s, 1 H), 4.96 (s, 2 H), 2.34-2.14 (m, 6 H), 1.86-1.61 (m, 3 H), 1.38-1.23 (m, 1 H) , 0.95 (d, J = 8.0 Hz, 3H), |
| 502 | 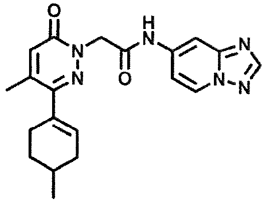 | ¹H-NMR (400 MHz, CDCl₃) δ: 10.10 (s, 1H), 8.25-8.22 (m, 1 H), 8.11 (s, 1 H), 7.96-7.90 (m, 1 H), 7.06-7.00 (m, 1 H), 6.76 (s, 1 H), 5.76 (s, 1H), 4.95 (s, 2H), 2.30-2.14 (m, 6 H), 1.79-1.64 (m, 3 H), 1.35-1.23 (m, 1 H) , 0.96 (d, J = 8.0 Hz, 3H), |
| 503 | 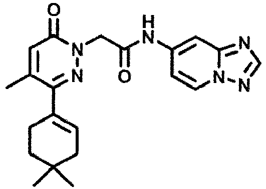 | ¹H-NMR (400 MHz, CDCl₃) δ: 8.71-8.66 (m, 1 H), 8.31 (s, 1 H), 8.23-8.20 (m, 1H), 7.32-7.26 (m, 1 H), 6.85 (s, 1 H), 5.86-5.81 (m, 1 H), 5.00 (s, 2 H), 2.36-2.29 (m, 2 H), 2.26 (s, 3 H), 2.03-1.97 (m, 2 H), 1.55-1.48 (m, 2 H), 0.99 (s, 6 H). |
| 504 | 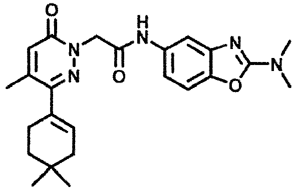 | ¹H-NMR (400 MHz, CDCl₃) δ:7.59-7.55 (m, 1 H), 7.24-7.20 (m, 1 H), 7.19-7.15 (m, 1 H), 6.82 (s, 1 H), 5.84-5.79 (m, 1H) , 4. 93 (s, 2H) , 3.16 (s, 6 H), 2.35-2.28 (m, 2 H), 2.23 (s, 3 H), 2.01-1.96 (m, 2 H), 1.53-1.47 (m, 2 H), 0.99 (s, 6 H) . |
| 505 | 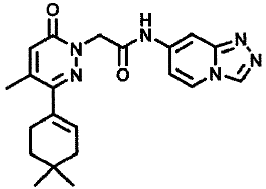 | ¹H-NMR (400 MHz, CD₃OD) δ: 9.05 (s, 1 H), 8.46-8.39 (m, 1 H), 8.26-8.15 (m, 1 H), 7.16-7.02 (m, 1 H), 6.91-6.80 (m, 1 H), 4.98 (s, 2 H) , 2.36-2.29 (m, 2 H), 2.27 (s, 3 H), 2.02-1.98 (m, 2 H) , 1.55-1.48 (m, 2 H), 0.99 (s, 6 H) . |
| 506 | 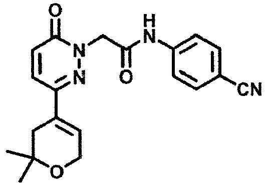 | ¹H-NMR (400 MHz, CDCl₃) δ:9.33 (s, 1 H), 7.58-7.51 (m, 3 H), 7.49-7.45 (m, 2 H), 6.99-6.93 (m, 1 H), 6.96 (s, 1 H), 4.92 (s, 2 H), 3.88-3.77 (m, 2 H), 2.44-2.36 (m, 2 H), 1.37 (s, 6 H) . |
| 507 | 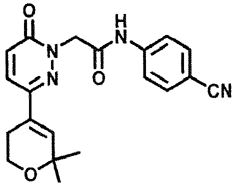 | ¹H-NMR (400 MHz, CDCl₃) δ:9.55 (s, 1 H), 7.61-7.57 (m, 1H), 7.50-7.46 (m, 2 H), 7.45-7.39 (m, 2 H), 6.99-6.95 (m, 1 H), 6.32 (s, 1 H), 4.94 (s, 2 H), 4.33-4.28 (m, 2 H), 2.38-2.32 (m, 2 H), 1.21 (s, 6 H) . |
| 508 | 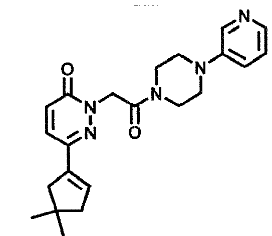 | LC-MS: [M+H]⁺ / Rt (min) 394.4 / 0.690 (Method A) |
| 509 | 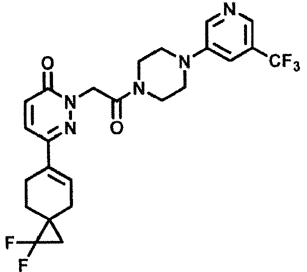 | LC-MS: [M+H]⁺ / Rt (min) 510.3 / 0.944 (Method A) |
| 510 | 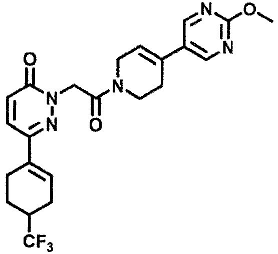 | LC-MS: [M+H]⁺ / Rt (min) 476.3 / 0.876 (Method A) |
| 511 | 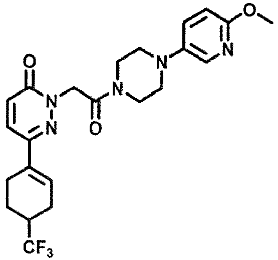 | ¹H-NMR (400 MHz, DMSO-d₆) δ: 7.86 (d, J = 10.0 Hz, 1H), 7.82 (d, J = 6.0 Hz, 1H), 6.93 (d, J = 10.0 Hz, 1H), 6.59-6.57 (m, 1H), 6.54-6.51 (m, 1H), 6.14-6.13 (m, 1H), 4.98 (s, 2H), 3.77 (s, 3H), 3.66-3.61 (m, 2H), 3.57-3.52 (m, 2H), 3.42-3.38 (m, 2H), 3.34-3.31 (m, 2H), 2.67-2.63 (m, 1H), 2.63-2.59 (m, 1H), 2.47-2.42 (m, 1H), 2.33-2.18 (m, 2H), 2.06-2.00 (m, 1H), 1.54-1.43 (m, 1H). |
| 512 | 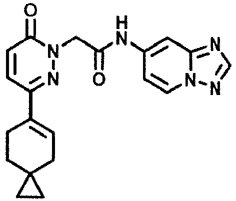 | ¹H-NMR (400 MHz, DMSO-d₆) δ: 10.92 (s, 1H) , 8.86 (d, J = 7.3 Hz, 1H), 8.37 (d, J = 9.8 Hz, 1H), 8.12 (d, J = 1.8 Hz, 1H), 7.91 (d, J = 9.8 Hz, 1H), 7.21 (dd, J = 7.3, 1.8 Hz, 1H), 6. 97 (d, J = 9.8 Hz, 1H), 6.61 (d, J = 3.7 Hz, 1H), 4.94 (s, 2H), 2.45-2.42 (m, 2H), 2.13-2.12 (m, 2H), 1.46 (t, J = 6.4 Hz, 2H), 0.36-0.31 (m, 4H) . |
| 513 | 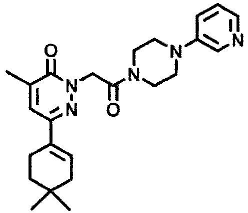 | LC-MS: [M+H]⁺ / Rt (min) 422.4 / 0.776 (Method A) |
| 514 | 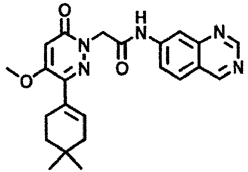 | LC-MS: [M+H]⁺ / Rt (min) 420.1 / 0.914 (Method A) |
| 515 | 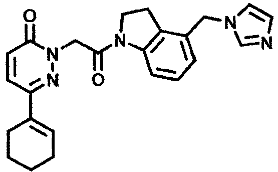 | LC-MS: [M+H]⁺ / Rt (min) 416.3 / 0.671 (Method A) |
| 516 | 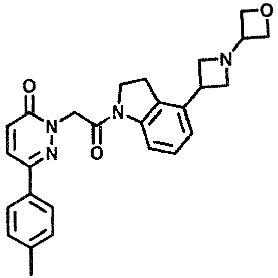 | ¹H-NMR (400 MHz, DMSO-d₆) δ: 8.23 (d, J = 9.8 Hz, 1H), 7.98 (d, J = 7.9 Hz, 1H), 7.93 (d, J = 7.9 Hz, 2H), 7.44 (d, J = 7.9 Hz, 2H), 7.30 (t, J = 7.9 Hz, 1H), 7.25-7.19 (m, 2H), 5.29 (s, 2H), 4.70 (t, J = 6.7 Hz, 2H), 4.51 (t, J = 5.8 Hz, 2H), 4.39 (t, J = 8.5 Hz, 2H), 3.89-3.80 (m, 4H), 3.34-3.28 (m, 2H), 3.26-3.21 (m, 2H), 2.49 (s, 3H). |
| 517 | 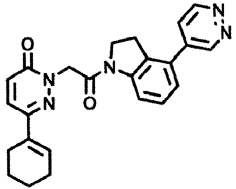 | LC-MS: [M+H]⁺ / Rt (min) 414.0 / 0.781 (Method A) |
| 518 | 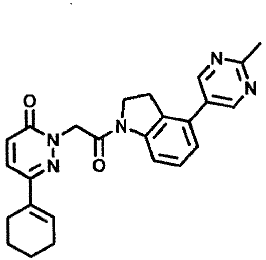 | ¹H-NMR (400 MHz, DMSO-d₆) δ: 8.85 (s, 2H), 8.05 (d, J = 7.9 Hz, 1H), 7.86 (d, J = 9.8 Hz, 1H), 7.31 (t, J = 7.9 Hz, 1H), 7.15 (d, J = 7.3 Hz, 1H), 6.93 (d, J = 9.8 Hz, 1H), 6.56-6.53 (m, 1H), 5.04 (s, 2H), 4.22 (t, J = 8.2 Hz, 2H), 3.33-3.27 (m, 2H), 2.66 (s, 3H), 2.33-2.28 (m, 2H), 2.22-2.17 (m, 2H), 1.66-1.53 (m, 4H). |
| 519 | 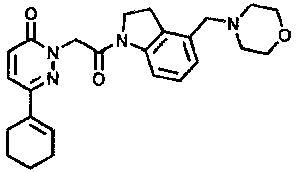 | ¹H-NMR (400 MHz, DMSO-d₆) δ: 7.85 (d, J = 9.8 Hz, 2H), 7.09 (t, J = 7.6 Hz, 1H), 6.93 (t, J = 9.2 Hz, 2H), 6.55-6.53 (m, 1H), 5.00 (s, 2H), 4.21 (t, J = 8.5 Hz, 2H), 3.55-3.52 (m, 4H), 3.40 (s, 2H), 3.21 (t, J = 8.5 Hz, 2H), 2.34-2.27 (m, 6H), 2.21-2.16 (m, 2H), 1.66-1.53 (m, 4H) . |
| 520 | 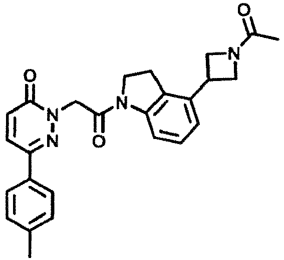 | ¹H-NMR (400 MHz, DMSO-d_{d} δ: 8.10 (d, J = 9.8 Hz, 1H), 7.89 (d, J = 7.9 Hz, 1H), 7.79 (d, J = 8.5 Hz, 2H), 7.31 (d, J = 7.9 Hz, 2H), 7.21 (t, J = 7.9 Hz, 1H), 7.14-7.10 (m, 2H), 5.16 (s, 2H), 4.49 (t, J = 8.2 Hz, 1H), 4.30-4.18 (m, 4H), 3.94-3.85 (m, 2H), 3.14 (t, J = 8.5 Hz, 2H), 2.35 (s, 3H), 1.80 (s, 3H) . |
| 521 | 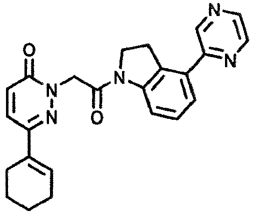 | ¹H-NMR (400 MHz, DMSO-d₆) δ: 9.05 (d, J = 1.8 Hz, 1H), 8.74 (t, J = 2.1 Hz, 1H), 8.62 (d, J = 2.4 Hz, 1H), 8.12 (d, J = 7.9 Hz, 1H), 7.86 (d, J = 9.8 Hz, 1H), 7.52 (d, J = 7.9 Hz, 1H), 7.33 (t, J = 7.9 Hz, 1H), 6.94 (d, J = 9.8 Hz, 1H), 6.56-6.54 (m, 1H), 5.04 (s, 2H), 4.25 (t, J = 8.5 Hz, 2H), 3.49 (t, J = 8.4 Hz, 2H), 2.33-2.28 (m, 2H), 2.23-2.16 (m, 2H), 1.67-1.53 (m, 4H). |
| 522 | 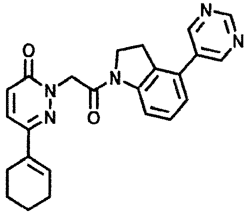 | ¹H-NMR (400 MHz, DMSO-d₆) δ: 9.20 (s, 1H), 8.98 (s, 2H), 8.07 (d, J = 7.9 Hz, 1H), 7.86 (d, J = 9.8 Hz, 1H), 7.33 (t, J = 7.6 Hz, 1H), 7.19 (d, J = 6.7 Hz, 1H), 6.93 (d, J = 9.8 Hz, 1H), 6.56-6.53 (m, 1H), 5.05 (s, 2H), 4.23 (t, J = 8.2 Hz, 2H), 3.34-3.26 (m, 2H), 2.33-2.28 (m, 2H), 2.21-2.18 (m, 2H), 1.66-1.55 (m, 4H) . |
| 523 | 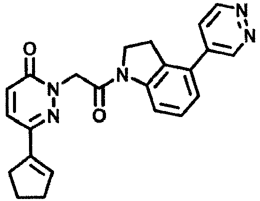 | ¹H-NMR (400 MHz, DMSO-d₆) δ: 9.47-9.45 (m, 1H), 9.33-9.31 (m, 1H), 8.14 (d, J = 7.9 Hz, 1H), 7.94 (d, J = 9.8 Hz, 1H), 7.89-7.87 (m, 1H), 7.38 (t, J = 7.6 Hz, 1H), 7.28 (d, J = 6.7 Hz, 1H), 6.98 (d, J = 9.8 Hz, 1H), 6.62-6.59 (m, 1H), 5.08 (s, 2H), 4.27 (t, J = 8.2 Hz, 2H), 3.38 (t, J = 8.2 Hz, 2H), 3.32-3.29 (m, 2H), 2.63-2.58 (m, 2H), 1.97-1.89 (m, 2H). |
| 524 | 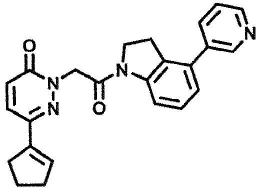 | ¹H-NMR (400 MHz, DMSO-d₆) δ: 8.70-8.69 (m, 1H), 8.58 (dd, J = 4.9, 1.8 Hz, 1H), 8.04 (d, J = 7.9 Hz, 1H), 7.94-7.90 (m, 2H), 7.48 (dd, J = 8.2, 5.2 Hz, 1H), 7.29 (t, J = 7.9 Hz, 1H), 7.11 (d, J = 6.7 Hz, 1H), 6.96 (d, J = 9.8 Hz, 1H), 6.59-6.56 (m, 1H), 5.03 (s, 2H), 4.22 (t, J = 8.2 Hz, 2H), 3.28-3.25 (m, 2H), 2.66-2.56 (m, 4H), 1.95-1.87 (m, 2H). |
| 525 | 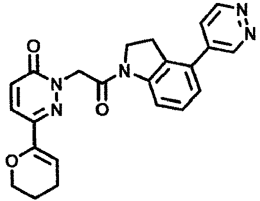 | LC-MS: [M+H]⁺ / Rt (min) 416.3 / 0.740 (Method A) |
| 526 | 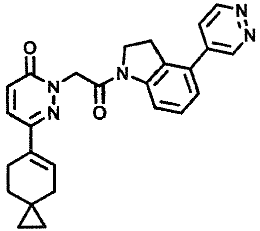 | ¹H-NMR (400 MHz, DMSO-d₆) δ: 9.44 (dd, J = 2.4, 1.2 Hz, 1H), 9.29 (dd, J = 5.5, 1.2 Hz, 1H), 8.12 (d, J = 7.9 Hz, 1H), 7.88 (d, J = 9.8 Hz, 1H), 7.85 (dd, J = 5.5, 2.4 Hz, 1H), 7.36 (t, J = 7.9 Hz, 1H), 7.25 (d, J = 6.7 Hz, 1H), 6.94 (d, J = 9.8 Hz, 1H), 6.58-6.56 (m, 1H), 5.06 (s, 2H), 4.25 (t, J = 8.2 Hz, 2H), 3.36 (t, J = 8.5 Hz, 2H), 3.28-3.26 (m, 2H), 2.12-2.09 (m, 2H), 1.43 (t, J = 6.4 Hz, 2H), 0.31 (d, J = 6.1 Hz, 4H). |
| 527 | 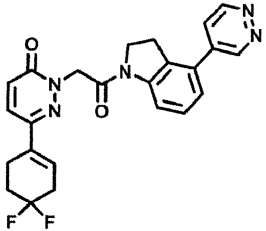 | LC-MS: [M+H]⁺ / Rt (min) 450.3 / 0.773 (Method A) |
| 528 | 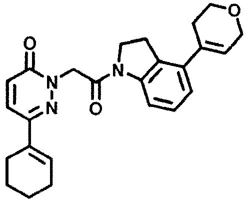 | ¹H-NMR (400 MHz, DMSO-d₆) δ: 7.93-7.87 (m, 2H), 7.17 (t, J = 7.9 Hz, 1H), 6.98-6.94 (m, 2H), 6.58-6.55 (m, 1H), 5.92-5.90 (m, 1H), 5.04 (s, 2H), 4.24-4.19 (m, 4H), 3.81 (t, J = 5.2 Hz, 2H), 3.25 (t, J = 8.2 Hz, 2H), 2.39-2.21 (m, 6H), 1.67-1.58 (m, 4H). |
| 529 | 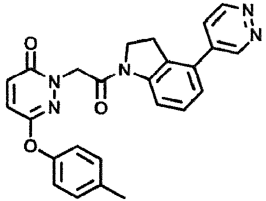 | ¹H-NMR (400 MHz, DMSO-d₆) δ: 9.44 (dd, J = 2.4, 1.2 Hz, 1H), 9.32-9.30 (m, 1H), 8.13 (d, J = 7.9 Hz, 1H), 7.86 (dd, J = 5.5, 2.4 Hz, 1H), 7.47 (d, J = 9.8 Hz, 1H), 7.38 (t, J = 7.9 Hz, 1H), 7.28-7.25 (m, 1H), 7.19 (d, J = 7.9 Hz, 2H), 7.14 (d, J = 9.8 Hz, 1H), 7.09-7.05 (m, 2H), 4.89 (s, 2H), 4.19 (t, J = 8.5 Hz, 2H), 3.34-3.30 (m, 2H), 2.27 (s, 3H). |
| 530 | 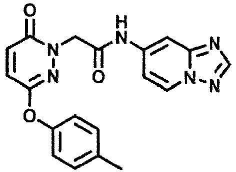 | ¹H-NMR (400 MHz, DMSO-d₆) δ: 10.79 (s, 1H), 8.82 (d, J = 7.3 Hz, 1H), 8.35 (s, 1H), 8.07 (d, J = 1.8 Hz, 1H), 7.44 (d, J = 9.8 Hz, 1H), 7.18-7.04 (m, 6H), 4.72 (s, 2H), 2.24 (s, 3H). |
| 531 | 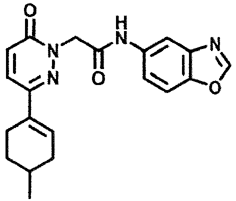 | ¹H-NMR (400 MHz, DMSO-d₆) δ: 10.49 (s, 1H), 8.71-8.70 (m, 1H), 8.11-8.10 (m, 1H), 7.90-7.87 (m, 1H), 7.73-7.70 (m, 1H), 7.53-7.49 (m, 1H), 6.95-6.92 (m, 1H), 6.51-6.49 (m, 1H), 4.89-4.86 (m, 2H), 2.37-2.31 (m, 2H), 2.03-1.99 (m, 2H), 1.44-1.40 (m, 2H), 0.93-0.89 (m, 6H). |
| 532 | 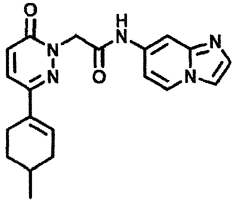 | ¹H-NMR (400 MHz, DMSO-d₆) δ: 10.53 (s, 1H), 8.46-8.44 (m, 1H), 7.88 (d, J = 9.6 Hz, 2H), 7.82 (s, 1H), 7.45-7.44 (m, 1H), 6.96-6.93 (m, 2H), 6.55-6.51 (m, 1H), 4.88 (s, 2H), 2.56-2.52 (m, 1H), 2.36-2.18 (m, 2H), 1.86-1.74 (m, 2H), 1.70-1.61 (m, 1H), 1.28-1.18 (m, 1H), 0.96 (d, J = 6.8 Hz, 3H) . |
| 533 | 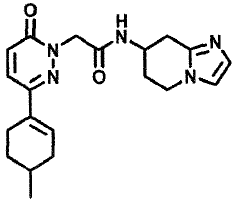 | ¹H-NMR (400 MHz, CDCl₃) δ: 7.55 (d, J = 9.8 Hz, 1H), 7.00-6.98 (m, 1H), 6.88 (d, J = 9.8 Hz, 1H), 6.83-6.79 (m, 2H), 6.34 (s, 1H), 4.83-4.74 (m, 2H), 4.42-4.38 (m, 1H), 4.09-3.96 (m, 2H), 3.24-3.17 (m, 1H), 2.82-2.75 (m, 1H), 2.61-2.54 (m, 1H), 2.35-2.07 (m, 4H), 1.87-1.81 (m, 3H), 1.33-1.24 (m, 2H), 1.01 (d, J = 6.7 Hz, 3H). |
| 534 | 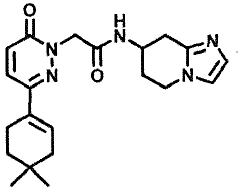 | ¹H-NMR (400 MHz, CDCl₃) δ: 7.57 (d, J = 9.8 Hz, 1H), 6.98 (d, J = 1.2 Hz, 1H), 6.89 (d, J = 9.8 Hz, 1H), 6.80 (d, J = 1.2 Hz, 1H), 6.78 (d, J = 7.3 Hz, 1H), 6.32-6.30 (m, 1H), 4.83-4.74 (m, 2H), 4.44-4.36 (m, 1H), 4.09-3.96 (m, 2H), 3.23-3.17 (m, 1H), 2.81-2.74 (m, 1H), 2.44-2.40 (m, 2H), 2.26-2.19 (m, 1H), 2.16-2.06 (m, 1H), 2.04-2.02 (m, 2H), 1.48 (t, J = 6.4 Hz, 2H), 0.95 (s, 6H). |

### Examples 535 and 536

Cis-trans isomers of the compound (35.5 mg) obtained in Example 472 were resolved by chiral column chromatography to obtaine the following compounds (Example 535 and 536):

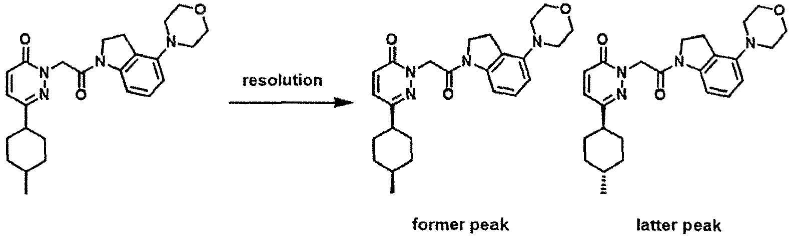

### [Resolution conditions]

Detection apparatus: SPD-M20A (Shimadzu Corporation)
HPLC: LC-20AT (Shimadzu Corporation)
Column: Waters ACQUITY UPLC (trademark) BEH C18(1.7 um, 2.1 mm x 30 mm)
Column: CHIRALPAK IA (Daicel Corporation) (S - 5 µm, 20 x 250 mm)
Elution condition: 0.0 - 60.0 (min): A/B = 55:45
Solvent A: hexane with 0.1% diethylamine
Solvent B: (isopropylalcohol:methanol = 2:1) with 0.1% diethylamine
Flow rate: 10 ml/min
UV: 220 nm

### Column temperature: 40°C

| | Example | Retention time (min.) | Yield (mg) | Purity |
|---|---|---|---|---|
| Former peak | 535 | 31.5 | 13.5 | 99.9% |
| Latter peak | 536 | 41.5 | 14.0 | 99.8% |

### Examples 537 and 538

Optical isomers of the compound obtained in Example 330 were resolved by chiral column chromatography to obtain the following compounds (Examples 537 and 538):

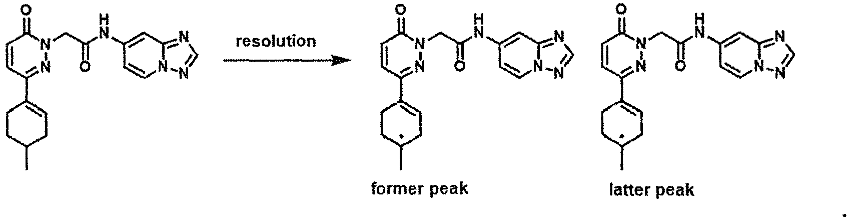

### [Resolution conditions]

Detection apparatus: SPD-M20A (Shimadzu Corporation)
HPLC: LC-20AT(Shimadzu Corporation)
Column: CHIRALPAK AY-H (Daicel Corporation) (S - 5 µm, 20 x 250 mm) Elution condition: 0.0 - 80.0 (min): A/B = 65:35
Solvent A: hexane
Solvent B: isopropylalcohol
Flow rate: 10 ml/min
UV: 220 nm

### Column temperature: 40°C

| | Example | Retention time (min.) | Yield (mg) | Optical purity |
|---|---|---|---|---|
| Former peak | 537 | 43.5 | 4.5 | 87.8%ee |
| Latter peak | 538 | 56 | 4.8 | 98.6%ee |

### Examples 539 and 540

Optical isomers of the compound obtained in Example 339 (13.7 mg) were resolved by chiral column chromatography to obtaine the following compounds (Examples 539 and 540):

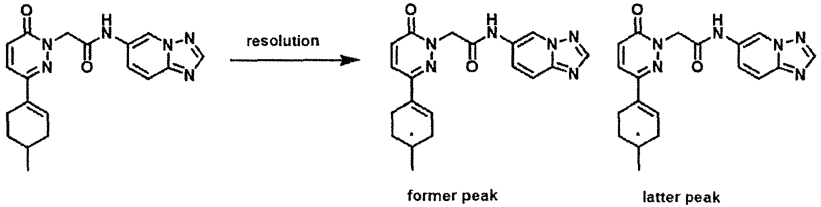

### [Resolution conditions]

Detection apparatus: SPD-M20A (Shimadzu Corporation)
HPLC: LC-20AT (Shimadzu Corporation)
Column : CHIRALPAK AY-H (Daicel Corporation) (S - 5 µm, 20 x 250 mm)
Elution condition: 0.0 - 80.0 (min): A/B = 50:50
Solvent A: hexane Solvent B: isopropylalcohol
Flow rate: 10 ml/min
UV: 220 nm

### Column temperature: 40°C

| | Example | Retention time (min. ) | Yield (mg) | Optical purity |
|---|---|---|---|---|
| Former peak | 539 | 39.8 | 4.5 | 99%ee |
| Latter peak | 540 | 52.5 | 7.6 | 98.2%ee |

### Examples 541 to 571

According to the method of Example 50 and common reaction conditions, the compounds of Examples 541 to 571 were obtained by using corresponding material compounds.

| Example | Chemical structure | Analytical data |
|---|---|---|
| 541 | 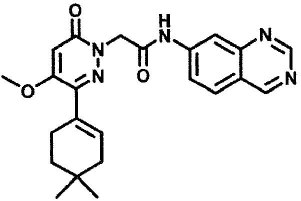 | LC-MS: [M+H]⁺ / Rt (min) 420.1 / 0.914 (Method A) |
| 542 | 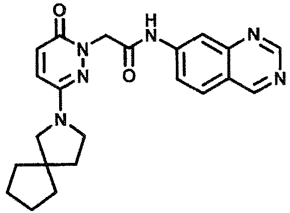 | LC-MS: [M+H]⁺ / Rt (min) 391.3 / 0.761 (Method A) |
| 543 | 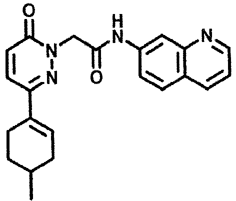 | LC-MS: [M+H]⁺ / Rt (min) 375.1 / 0.661 (Method B) |
| 544 | 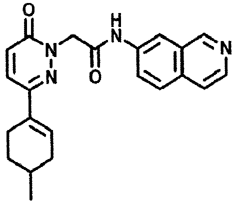 | LC-MS: [M+H]⁺ / Rt (min) 375.0 / 0.745 (Method A) |
| 545 | 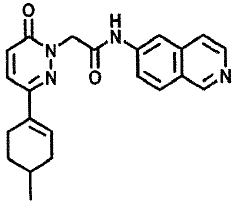 | LC-MS: [M+H]⁺ / Rt (min) 375.0 / 0.725 (Method A) |
| 546 | 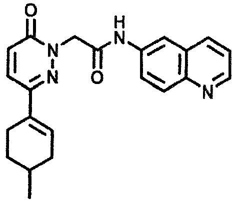 | LC-MS: [M+H]⁺ / Rt (min) 375.0 / 0.784 (Method A) |
| 547 | 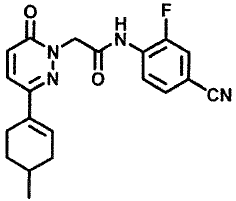 | ¹H-NMR (400 MHz, CDCl₃) δ: 9.36 (s, 1H), 8.52 (t, J = 8.2 Hz, 1H), 7.61 (d, J = 10.4 Hz, 1H), 7.42 (d, J = 8.4 Hz, 1H), 7.37 (dd, J = 10.4, 2.0 Hz), 7.00 (d, J = 9.8 Hz, 1H), 6.38 (s, 1H), 4.99 (s, 2H), 2.66-2.57 (m, 1H), 2.39-2.27 (m, 2H), 1.91-1.80 (m, 2H), 1.77-1.65 (m, 1H), 1.42-1.23 (m, 1H), 1.01 (d, J = 6.7 Hz, 3H). |
| 548 | 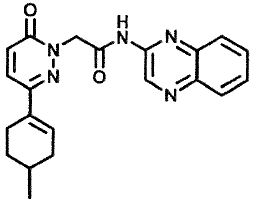 | LC-MS: [M+H]⁺ / Rt (min) 376.0 / 0.995 (Method A) |
| 549 | 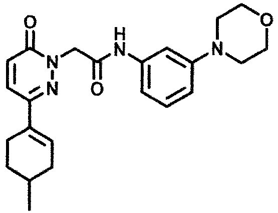 | LC-MS: [M+H]+ / Rt (min) 409.1 / 0.958 (Method A) |
| 550 | 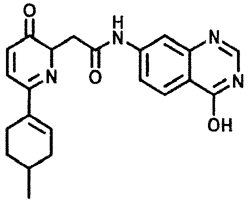 | LC-MS: [M+H]⁺ / Rt (min) 392.0 / 0.791 (Method A) |
| 551 | 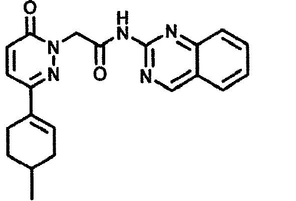 | LC-MS: [M+H]⁺ / Rt (min) 376.1 / 0.964 (Method A) |
| 552 | 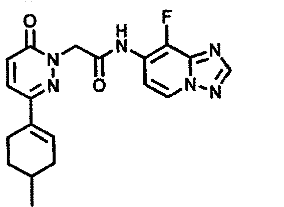 | LC-MS: [M+H]⁺ / Rt (min) 383.0 / 0.838 (Method A) |
| 553 | 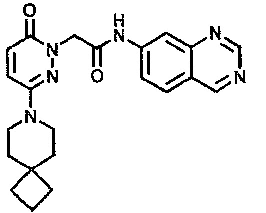 | LC-MS: [M+H]⁺ / Rt (min) 405.0 / 0.834 (Method A) |
| 554 | 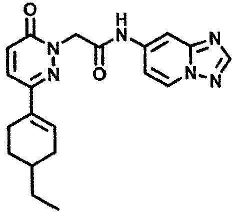 | ¹H-NMR (400 MHz, CDCl₃) δ: 9.95 (s, 1H), 8.36 (d, J = 7.3 Hz, 1H), 8.23 (s, 1H), 8.05 (d, J = 1.8 Hz, 1H), 7.66 (d, J = 9.8 Hz, 1H), 7.13 (dd, J = 7.3, 1.8 Hz, 1H), 7.01 (d, J = 9.8 Hz, 1H), 6.42 (s, 1H), 5.04 (s, 2H), 2.72-2.59 (m, 1H), 2.47-2.26 (m, 2H), 1.98-1.83 (m, 2H), 1.58-1.45 (m, 1H), 1.43-1.25 (m, 3H), 0.93 (t, J = 7.2 Hz, 3H). |
| 555 | 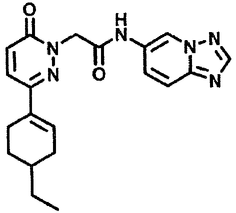 | ¹H-NMR (400 MHz, CDCl₃) δ:9.77 (s, 1H), 9.37 (s, 1H), 8.22 (s, 1H), 7.66 (d, J = 9.6 Hz, 1H), 7.56 (d, J = 9.6 Hz, 1H), 7.30-7.25 (m, 1H), 7.02 (t, J = 10.1 Hz, 1H), 6.41 (s, 1H), 5.05 (s, 2H), 2.70-2.59 (m, 1H), 2.45-2.23 (m, 2H), 1.97-1.80 (m, 2H), 1.59-1.42 (s, 1H), 1.41-1.24 (m, 3H), 0.95 (t, J = 7.2 Hz, 3H). |
| 556 | 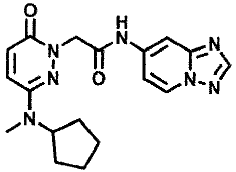 | LC-MS: [M+H]⁺ / Rt (min) 368.2 / 0.693 (Method A) |
| 557 | 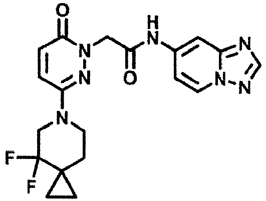 | LC-MS: [M+H]⁺ / Rt (min) 416.2 / 0.710 (Method A) |
| 558 | 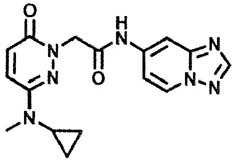 | LC-MS: [M+H]⁺ / Rt (min) 340.1 / 0.609 (Method A) |
| 559 | 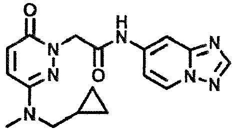 | LC-MS: [M+H]⁺ / Rt (min) 354.2 / 0.651 (Method A) |
| 560 | 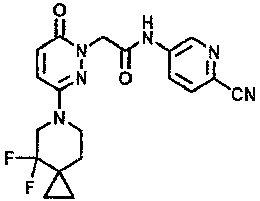 | LC-MS: [M+H]⁺ / Rt (min) 401.1 / 0.804 (Method A) |
| 561 | 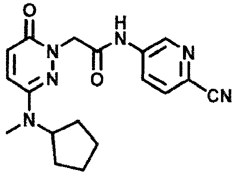 | LC-MS: [M+H]⁺ / Rt (min) 353.2 / 0.834 (Method A) |
| 562 | 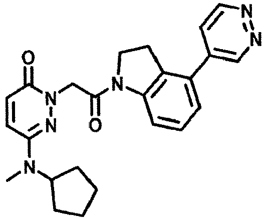 | LC-MS: [M+H]⁺ / Rt (min) 431.3 / 0.822 (Method A) |
| 563 | 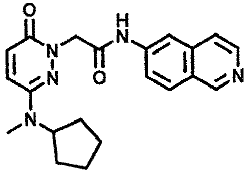 | ¹H-NMR (400 MHz, CDCl₃) δ: 9.85 (s, 1H), 9.10 (s, 1H), 8.43 (d, J = 6.1 Hz, 1H), 8.24 (s, 1H), 7.86 (d, J = 8.6 Hz, 1H), 7.54-7.51 (m, 2H), 7.23 (d, J = 10.4 Hz, 1H), 6.96 (d, J = 9.8 Hz, 1H), 4.90 (s, 2H), 4.37-4.29 (m, 1H), 2.82 (s, 3H), 1.91-1.79 (m, 2H), 1.76-1.65 (m, 2H), 1.40-1.21 (m, 2H), 0.94-0.80 (m, 2H). |
| 564 | 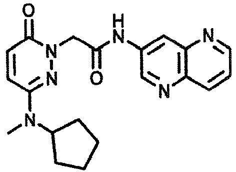 | LC-MS: [M+H]⁺ / Rt (min) 379.3 / 0.749 (Method A) |
| 565 | 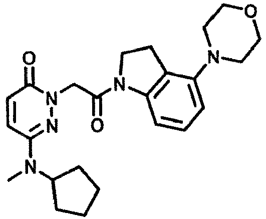 | ¹H-NMR (400 MHz, CDCl₃) δ: 7.94 (d, J = 7.9 Hz, 1H), 7.20-7.13 (m, 2H), 6.88 (d, J = 9.8 Hz, 1H), 6.68 (d, J = 7.9 Hz, 1H), 4.89 (s, 2H), 4.30-4.20 (m, 1H), 3.88-3.82 (m, 4H), 3.16 (t, J = 8.2 Hz, 2H), 3.02-2.93 (m, 4H), 2.77 (s, 3H), 1.90-1.77 (m, 2H), 1.75-1.63 (m, 2H), 1.63-1.50 (m, 6H). |
| 566 | 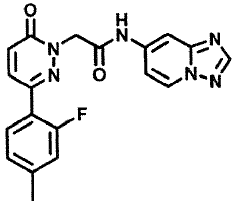 | LC-MS: [M+H]⁺ / Rt (min) 379.3 / 0.721 (Method A) |
| 567 | 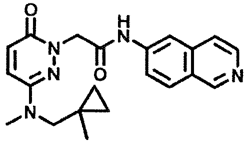 | ¹H-NMR (400 MHz, CDCl₃) δ: 9.71 (s, 1H), 9.09 (s, 1H), 8.43 (d, J = 5.5 Hz, 1H), 8.19 (d, J = 1.8 Hz, 1H), 7.83 (d, J = 9.2 Hz, 1H), 7.57-7.50 (m, 2H), 7.21 (d, J = 9.6 Hz, 1H), 6.95 (d, J = 9.6 Hz, 1H), 4.90 (s, 2H), 3.25 (s, 2H), 3.00 (s, 3H), 1.03 (s, 3H), 0.45-0.42 (m, 2H), 0.39-0.36 (m, 2H). |
| 568 | 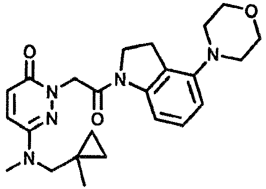 | ¹H-NMR(400 MHz, CDCl₃) δ: 7.92 (s, 1H), 7.17-7.13 (m, 2H), 6.85 (d, J = 9.8 Hz, 1H), 6.68 (d, J = 7.9 Hz, 1H), 4.90 (s, 2H), 3.90-3.79 (m, 4H), 3.25-3.07 (m, 2H), 3.18 (s, 2H), 3.05-2.95 (m, 4H), 2.98 (s, 3H), 1.70-1.55 (m, 2H), 1.03 (s, 3H), 0.45-0.38 (m, 2H), 0.367-0.32 (m, 2H) . |
| 569 | 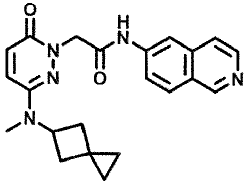 | ¹H-NMR (400 MHz, CDCl₃) δ: 9.91 (s, 1H), 9.04 (s, 1H), 8.38 (d, J = 5.5 Hz, 1H), 8.18 (s, 1H), 7.79 (d, J = 8.6 Hz, 1H), 7.48 (dd, J = 8.4, 2.0 Hz, 1H), 7.43 (d, J = 6.0 Hz, 1H), 7.21 (d, J = 10.4 Hz, 1H), 6.96 (d, J = 10.0 Hz, 1H), 4.93 (s, 2H), 4.50-4.40 (m, 1H), 2.93 (s, 3H), 2.44-2.39 (m, 2H), 2.23-2.18 (m, 2H), 0.55-0.51 (m, 2H), 0.45-0.39 (m, 2H). |
| 570 | 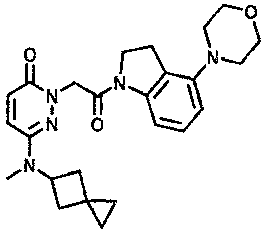 | ¹H-NMR (400 MHz, CDCl₃) δ: 7.95-7.91 (m, 1H), 7.18-7.10 (m, 2H), 6.87 (d, J = 10.1 Hz, 1H), 6.67 (d, J = 8.2 Hz, 1H), 4.88 (s, 2H), 4.40-4.30 (m, 1H), 3.85-3.80 (m, 4H), 3.18-3.10 (m, 2H), 2.98-2.93 (m, 4H), 2.85 (s, 3H), 2.85-2.77 (m, 2H), 2.40-2.32 (m, 1H), 2.17-2.10 (m, 1H), 2.05-2.00 (m, 1H), 1.85-1.70 (m, 1H), 0.50-0.45 (m, 2H), 0.40-0.35 (m, 2H). |
| 571 | 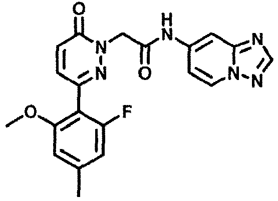 | LC-MS: [M+H]⁺ / Rt (min) 409.4 / 0.714 (Method A) |

### Tests

Hereinafter, results of pharmacological tests for representative compounds herein are described, and the pharmacologic effects of each compound are explained, but the present invention is not limited to the following Tests.

### Test 1: Evaluation of the amplification of Nav1.1-derived voltage-dependent sodium current (Nav1.1 current) by using a cell line stably expressing human Nav1.1

### (1) Preparation of test compounds

Test compounds were prepared by dissolving in DMSO at 200 times of the concentration at evaluation, and diluting the obtained solution to twice the concentration of evaluation with an extracellular fluid (135 mmol/L NaCl, 4 mmol/L KCl, 1 mmol/L MgCl₂, 5 mmol/L CaCl₂, 5 mmol/L Glucose, 10 mmol/L HEPES).

### (2) Induction and measurement of Nav1.1 current

A HEK293 cell line stably expressing human Nav1.1 (cat#CYL3009, Millipore, USA, Human Embryonic Kidney 293) was purchased, and used in the present test. Nav1.1 current was induced by stimulating Ramp wave voltage. Detection of current which accompanied with the stimulation of voltage was carried out by a patch-clamp voltage-clamp method using HTS automated patch clamp system (SynchroPatch 768PE, Nanion Technologies GmbH, Germany). Only cells with more than 500 pA of voltage-dependent sodium channel current were used for the evaluation of the activity of compounds on Nav1.1 current because there was the possibility that currents derived from endogenous voltage-dependent sodium channels accounted for large proportion in cells with less than 500 pA of voltage-dependent sodium channel current which was induced by stimulating Ramp wave voltage.

### (3) Pharmacologic effect on Nav1.1 current

The effect of test compounds on the amplification of Nav1.1-derived voltage-dependent sodium current was evaluated by using a cell line stably expressing human Nav1.1 and HTS automated patch clamp system. In other words, test compounds were added in an extracellular fluid containing 1% DMSO, and evaluated as a change of the peak value of Nav1.1 current and the area under the curve (AUC).

### (4) Method for pharmacological evaluation

The Nav1.1 current amplification rates of test compounds were calculated by the following formula: Nav1.1 current amplification rate (%) = 100 x [the peak value of Nav1.1 current or the area under the curve after the addition of test compounds] / [the peak value of Nav1.1 current or the area under the curve before the addition of test compounds] - 100

### Test 2: Evaluation of the amplification in Nav1.5-derived voltage-dependent sodium current (Navl.5 current) by using a cell line stably expressing human Navl.5

A CHO-K1 cell line (Chinese hamster ovary) stably expressing human Navl.5 (Gene Bank Accession No: P_000326.2) was obtained by using purchased T-Rex System (ThermoFisher Scientific, USA), and used for the present test. The effect of test compounds on the amplification of Nav1.5 current was evaluated by using a cell line stably expressing human Navl.5 and HTS automated patch clamp system, similarly in the method using Nav1.1. In other words, test compounds were added in an extracellular fluid which contained 1% DMSO and 500 nmol/L Tetrodotoxin (TTX), and evaluated as a change of the peak value of Navl.5 current and the area under the curve (AUC). The induction and the measurement of Navl.5 current, the pharmacologic effect on Navl.5 current, and a method for pharmacological evaluation were carried out by the same method as in Nav1.1.

### Test result 1

The effect of representative compounds herein on the activation of Nav1.1 (Nav1.1 current amplification rate) was evaluated on the basis of a change of the area under the curve of Nav1.1 current, and it was found that the representative compounds have the effect on the amplification of Nav1.1 current. The effect on the activation of Navl.5 (Navl.5 current amplification rate) was also evaluated on the basis of a change of the area under the curve of Navl.5 current. The Nav1.1 current amplification rates (%) and the Nav1.5 current amplification rates (%) when the concentration of each compound was 10 µmol/L are shown in the following table.

| **Example** | **Nav1.1 current amplification rate (%)** | **Nav1.5 current amplification rate (%)** | **Example** | **Nav1.1 current amplification rate (%)** | **Navl.5 current amplification rate (%)** |
|---|---|---|---|---|---|
| **50** | **155** | **4** | **95** | **221** | **18** |
| **51** | **196** | **31** | **96** | **105** | **7** |
| **52** | **248** | **19** | **97** | **296** | **19** |
| **53** | **161** | **11** | **98** | **15** | **4** |
| **55** | **111** | **27** | **99** | **103** | **4** |
| **59** | **180** | **56** | **100** | **197** | **56** |
| **60** | **57** | **21** | **103** | **58** | **13** |
| **61** | **77** | **8** | **107** | **37** | **24** |
| **63** | **49** | **3** | **109** | **57** | **23** |
| **66** | **49** | **31** | **112** | **46** | **9** |
| **67** | **177** | **18** | **114** | **83** | **12** |
| **69** | **137** | **0** | **115** | **56** | **12** |
| **70** | **68** | **0** | **119** | **120** | **25** |
| **71** | **131** | **13** | **120** | **176** | **5** |
| **72** | **119** | **3** | **121** | **184** | **98** |
| **73** | **92** | **1** | **122** | **173** | **18** |
| **74** | **70** | **-2** | **123** | **246** | **30** |
| **77** | **93** | **28** | **124** | **205** | **70** |
| **78** | **78** | **4** | **125** | **220** | **34** |
| **79** | **165** | **4** | **126** | **167** | **13** |
| **80** | **131** | **-3** | **127** | **229** | **58** |
| **81** | **69** | **21** | **128** | **240** | **56** |
| **82** | **121** | **5** | **129** | **314** | **25** |
| **84** | **315** | **123** | **130** | **181** | **3** |
| **86** | **73** | **3** | **131** | **227** | **7** |
| **87** | **255** | **22** | **132** | **108** | **-2** |
| **88** | **183** | **31** | **133** | **285** | **12** |
| **89** | **187** | **34** | **134** | **44** | **-5** |
| **91** | **93** | **9** | **135** | **195** | **2** |
| **94** | **198** | **29** | | | |

| **Example** | **Nav1.1 current amplification rate (%)** | **Nav1.5 current amplification rate (%)** | **Example** | **Nav1.1 current amplification rate (%)** | **Nav1.5 current amplification rate (%)** |
|---|---|---|---|---|---|
| **156** | **33** | **4** | **192** | **137** | **2** |
| **158** | **122** | **30** | **193** | **219** | **0** |
| **159** | **32** | **26** | **194** | **252** | **33** |
| **160** | **324** | **33** | **195** | **209** | **29** |
| **161** | **21** | **-1** | **196** | **225** | **18** |
| **162** | **6** | **0** | **197** | **318** | **56** |
| **163** | **118** | **1** | **198** | **36** | **-9** |
| **164** | **187** | **34** | **199** | **36** | **1** |
| **165** | **146** | **11** | **200** | **103** | **5** |
| **166** | **150** | **45** | **201** | **139** | **1** |
| **167** | **129** | **21** | **202** | **131** | **3** |
| **168** | **205** | **23** | **203** | **267** | **12** |
| **169** | **155** | **43** | **204** | **189** | **8** |
| **170** | **107** | **1** | **205** | **235** | **20** |
| **172** | **117** | **4** | **206** | **154** | **33** |
| **173** | **112** | **47** | **207** | **89** | **7** |
| **174** | **75** | **4** | **208** | **200** | **18** |
| **175** | **44** | **1** | **209** | **179** | **16** |
| **176** | **66** | **2** | **210** | **178** | **24** |
| **177** | **169** | **16** | **211** | **212** | **8** |
| **178** | **44** | **1** | **212** | **74** | **3** |
| **179** | **27** | **-1** | **213** | **136** | **5** |
| **181** | **118** | **4** | **214** | **64** | **-1** |
| **182** | **32** | **17** | **215** | **183** | **18** |
| **183** | **95** | **0** | **216** | **121** | **3** |
| **184** | **35** | **16** | **217** | **25** | **1** |
| **185** | **22** | **2** | **219** | **97** | **12** |
| **187** | **37** | **17** | **220** | **50** | **5** |
| **188** | **30** | **-2** | **221** | **281** | **15** |
| **189** | **3** | **-2** | **223** | **46** | **0** |
| **190** | **100** | **12** | **224** | **118** | **11** |

| **Example** | **Nav1.1 current amplification rate (%)** | **Nav1.5 current amplification rate (%)** | **Example** | **Nav1.1 current amplification rate (%)** | **Nav1.5 current amplification rate (%)** |
|---|---|---|---|---|---|
| **225** | **146** | **2** | **421** | **199** | **34** |
| **226** | **319** | **71** | **422** | **287** | **28** |
| **232** | **37** | **8** | **423** | **11** | **4** |
| **239** | **310** | **31** | **424** | **40** | **2** |
| **240** | **241** | **174** | **425** | **283** | **60** |
| **241** | **260** | **26** | **426** | **244** | **20** |
| **242** | **97** | **10** | **427** | **172** | **63** |
| **243** | **276** | **19** | **430** | **28** | **22** |
| **246** | **136** | **145** | **431** | **11** | **3** |
| **247** | **53** | **43** | **433** | **192** | **10** |
| **255** | **32** | **15** | **434** | **232** | **142** |
| **256** | **36** | **-30** | **435** | **262** | **14** |
| **259** | **71** | **2** | **436** | **226** | **31** |
| **260** | **13** | **-6** | **439** | **87** | **-4** |
| **261** | **60** | **1** | **440** | **196** | **7** |
| **263** | **342** | **32** | **441** | **30** | **1** |
| **359** | **26** | **6** | **442** | **414** | **27** |
| **394** | **98** | **1** | **443** | **114** | **1** |
| **395** | **343** | **15** | **444** | **292** | **10** |
| **396** | **136** | **8** | **445** | **320** | **52** |
| **397** | **273** | **49** | **446** | **333** | **10** |
| **398** | **175** | **29** | **447** | **148** | **6** |
| **399** | **102** | **11** | **448** | **236** | **27** |
| **400** | **238** | **24** | **449** | **250** | **12** |
| **401** | **286** | **5** | **450** | **187** | **5** |
| **402** | **323** | **21** | **451** | **365** | **30** |
| **403** | **270** | **12** | **452** | **10** | **2** |
| **404** | **42** | **6** | **453** | **15** | **1** |
| **414** | **40** | **-2** | **454** | **157** | **35** |
| **415** | **93** | **2** | **455** | **131** | **21** |
| **416** | **383** | **86** | **456** | **224** | **29** |

| **Example** | **Nav1.1 current amplification rate (%)** | **Nav1.5 current amplification rate (%)** | **Example** | **Nav1.1 current amplification rate (%)** | **Navl.5 current amplification rate (%)** |
|---|---|---|---|---|---|
| **458** | **24** | **0** | **477** | **398** | **39** |
| **459** | **118** | **21** | **479** | **420** | **135** |
| **460** | **221** | **46** | **482** | **219** | **8** |
| **461** | **337** | **50** | **483** | **175** | **7** |
| **462** | **124** | **5** | **484** | **224** | **8** |
| **463** | **218** | **9** | **485** | **264** | **3** |
| **464** | **18** | **1** | **486** | **148** | **8** |
| **465** | **186** | **8** | **487** | **190** | **109** |
| **466** | **233** | **38** | **488** | **376** | **174** |
| **469** | **243** | **25** | **489** | **29** | **0** |
| **470** | **522** | **131** | **490** | **469** | **25** |
| **472** | **387** | **56** | **508** | **24** | **-3** |
| **473** | **487** | **128** | **513** | **262** | **4** |
| **474** | **235** | **26** | **532** | **239** | **47** |
| **475** | **633** | **70** | **535** | **277** | **10** |
| **476** | **343** | **13** | **536** | **345** | **17** |

### Test result 2

The Nav1.1 current amplification rates (%) and the Navl.5 current amplification rates (%) of representative compounds herein when the concentration of each compound was 3 µmol/L are shown in the following table.

| **Example** | **Nav1.1 current amplification rate (%)** | **Nav1.5 current amplification rate (%)** | **Example** | **Nav1.1 current amplification rate (%)** | **Nav1.5 current amplification rate (%)** |
|---|---|---|---|---|---|
| **171** | **2** | **0** | **321** | **478** | **330** |
| **191** | **52** | **2** | **322** | **115** | **18** |
| **227** | **303** | **17** | **323** | **154** | **11** |
| **244** | **263** | **21** | **324** | **50** | **5** |
| **248** | **392** | **44** | **325** | **233** | **10** |
| **249** | **584** | **106** | **326** | **132** | **34** |
| **250** | **564** | **121** | **327** | **124** | **9** |
| **251** | **307** | **15** | **328** | **58** | **21** |
| **252** | **254** | **31** | **329** | **100** | **50** |
| **253** | **455** | **85** | **331** | **64** | **9** |
| **254** | **309** | **74** | **332** | **15** | **9** |
| **257** | **45** | **0** | **333** | **25** | **13** |
| **258** | **79** | **4** | **334** | **255** | **25** |
| **264** | **597** | **65** | **335** | **187** | **65** |
| **265** | **110** | **19** | **405** | **268** | **14** |
| **266** | **108** | **128** | **407** | **113** | **6** |
| **267** | **231** | **79** | **409** | **244** | **11** |
| **268** | **55** | **22** | **410** | **66** | **10** |
| **269** | **127** | **266** | **413** | **27** | **1** |
| **270** | **481** | **78** | **417** | **23** | **1** |
| **271** | **48** | **70** | **418** | **105** | **7** |
| **290** | **94** | **4.5** | **437** | **582** | **194** |
| **299** | **6** | **10** | **438** | **404** | **25** |
| **300** | **140** | **55** | **478** | **345** | **23** |
| **301** | **410** | **55** | **491** | **157** | **22** |
| **302** | **104** | **79** | **492** | **222** | **16** |
| **303** | **67** | **11** | **493** | **317** | **20** |
| **305** | **314** | **117** | **494** | **255** | **26** |
| **306** | **532** | **327** | **509** | **238** | **5** |
| **307** | **320** | **37** | **510** | **50** | **7** |
| **318** | **77** | **128** | **511** | **37** | **6** |

### Test result 3

The Nav1.1 current amplification rates (%) and the Navl.5 current amplification rates (%) of representative compounds herein when the concentration of each compound was 1 µmol/L are shown in the following table.

| **Examp le** | **Nav1.1 current amplification rate (%)** | **Navl.5 current amplification rate (%)** | **Examp le** | **Nav1.1 current amplification rate (%)** | **Nav1.5 current amplification rate (%)** |
|---|---|---|---|---|---|
| **218** | **155** | **9** | **381** | **153** | **60** |
| **222** | **137** | **11** | **383** | **84** | **51** |
| **234** | **240** | **4** | **428** | **109** | **23** |
| **235** | **198** | **7** | **429** | **101** | **28** |
| **245** | **393** | **32** | **432** | **280** | **16** |
| **276** | **207** | **47** | **468** | **275** | **75** |
| **277** | **210** | **18** | **471** | **142** | **17** |
| **278** | **204** | **27** | **480** | **110** | **7** |
| **286** | **848** | **579** | **502** | **49** | **7** |
| **287** | **73** | **123** | **503** | **58** | **10** |
| **294** | **271** | **427** | **512** | **103** | **34** |
| **311** | **265** | **52** | **515** | **255** | **287** |
| **313** | **84** | **133** | **516** | **130** | **13** |
| **319** | **134** | **30** | **517** | **359** | **16** |
| **330** | **191** | **47** | **518** | **344** | **9** |
| **338** | **241** | **49** | **520** | **253** | **252** |
| **339** | **127** | **15** | **521** | **358** | **9** |
| **342** | **109** | **52** | **522** | **367** | **25** |
| **345** | **232** | **46** | **523** | **129** | **5** |
| **346** | **96** | **27** | **524** | **143** | **5** |
| **349** | **101** | **24** | **526** | **499** | **1** |
| **352** | **155** | **34** | **527** | **324** | **36** |
| **354** | **123** | **27** | **528** | **198** | **24** |
| **355** | **130** | **28** | **531** | **183** | **8** |
| **356** | **378** | **34** | **537** | **99** | **29** |
| **360** | **200** | **100** | **538** | **135** | **45** |
| **361** | **101** | **112** | **539** | **80** | **11** |
| **362** | **133** | **24** | **540** | **112** | **15** |

### Test result 4

The Nav1.1 current amplification rates (%) of representative compounds herein when the concentration of each compound was 50 µmol/L are shown in the following table.

| **Example** | **Nav1.1 current amplification rate (%)** | **Example** | **Nav1.1 current amplification rate (%)** | **Example** | **Nav1.1 current amplification rate (%)** |
|---|---|---|---|---|---|
| **157** | **29** | **341** | **202** | **386** | **126** |
| **233** | **47** | **351** | **98** | **406** | **68** |
| **237** | **27** | **353** | **32** | **408** | **80** |
| **238** | **128** | **357** | **43** | **411** | **18** |
| **289** | **51** | **365** | **32** | **412** | **36** |
| **291** | **212** | **366** | **37** | **457** | **29** |
| **293** | **55** | **369** | **10** | **505** | **255** |
| **308** | **9** | **371** | **64** | **506** | **10** |
| **340** | **41** | **385** | **42** | **530** | **9** |

### Test result 5

The Nav1.1 current amplification rates (%) of representative compounds herein when the concentration of each compound was 10 µmol/L are shown in the following table.

| **Example** | **Nav1.1 current amplification rate (%)** | **Example** | **Nav1.1 current amplification rate (%)** | **Example** | **Nav1.1 current amplification rate (%)** |
|---|---|---|---|---|---|
| **1** | **246** | **24** | **286** | **47** | **11** |
| **2** | **148** | **25** | **163** | **48** | **33** |
| **3** | **40** | **26** | **364** | **49** | **2** |
| **4** | **3** | **27** | **5** | **136** | **63** |
| **5** | **6** | **28** | **158** | **137** | **58** |
| **6** | **35** | **29** | **20** | **138** | **58** |
| **7** | **3** | **30** | **77** | **139** | **166** |
| **8** | **4** | **31** | **220** | **140** | **93** |
| **9** | **11** | **32** | **60** | **141** | **46** |
| **10** | **124** | **33** | **220** | **142** | **34** |
| **11** | **205** | **34** | **311** | **143** | **74** |
| **12** | **241** | **35** | **301** | **144** | **48** |
| **13** | **76** | **36** | **312** | **145** | **113** |
| **14** | **9** | **37** | **93** | **146** | **148** |
| **15** | **10** | **38** | **174** | **147** | **127** |
| **16** | **59** | **39** | **125** | **148** | **97** |
| **17** | **212** | **40** | **20** | **149** | **126** |
| **18** | **11** | **41** | **324** | **150** | **157** |
| **19** | **98** | **42** | **216** | **151** | **104** |
| **20** | **152** | **43** | **174** | **152** | **50** |
| **21** | **130** | **44** | **59** | **153** | **77** |
| **22** | **110** | **45** | **202** | **154** | **75** |
| **23** | **28** | **46** | **85** | **155** | **99** |
| **180** | **22** | **374** | **18** | **393** | **19** |
| **186** | **51** | **375** | **173** | **420** | **39** |
| **262** | **124** | **376** | **44** | **481** | **128** |
| **281** | **40** | **379** | **147** | **498** | **24** |
| **304** | **167** | **388** | **37** | **507** | **10** |
| **368** | **18** | **389** | **53** | **525** | **18** |
| **370** | **35** | **390** | **50** | **533** | **22** |
| **372** | **52** | **391** | **30** | **534** | **7** |
| **373** | **35** | **392** | **53** | | |

### Test result 6

The Nav1.1 current amplification rates (%) of representative compounds herein when the concentration of each compound was 1 µmol/L are shown in the following table.

| **Example** | **Nav1.1 current amplification rate (%)** | **Example** | **Nav1.1 current amplification rate (%)** | **Example** | **Nav1.1 current amplification rate (%)** |
|---|---|---|---|---|---|
| **228** | **29** | **298** | **51** | **377** | **134** |
| **229** | **34** | **309** | **71** | **378** | **37** |
| **230** | **33** | **310** | **87** | **380** | **82** |
| **231** | **50** | **312** | **80** | **382** | **13** |
| **236** | **214** | **314** | **9** | **384** | **15** |
| **272** | **127** | **315** | **19** | **387** | **19** |
| **273** | **77** | **316** | **81** | **419** | **66** |
| **274** | **60** | **317** | **41** | **467** | **80** |
| **275** | **46** | **320** | **10** | **495** | **28** |
| **279** | **123** | **336** | **68** | **496** | **37** |
| **280** | **10** | **337** | **52** | **497** | **43** |
| **282** | **26** | **343** | **44** | **499** | **50** |
| **283** | **15** | **344** | **69** | **500** | **14** |
| **284** | **91** | **347** | **37** | **501** | **33** |
| **285** | **113** | **348** | **57** | **504** | **27** |
| **288** | **115** | **350** | **31** | **514** | **325** |
| **292** | **46** | **358** | **71** | **519** | **135** |
| **295** | **95** | **363** | **174** | **529** | **43** |
| **296** | **87** | **364** | **25** | | |
| **297** | **151** | **367** | **38** | | |

### Test result 7

The Nav1.1 current amplification rates (%) of representative compounds herein are shown in the following table.

| Example | Concentrat ion (µM) | Nav1.1 current amplification rate (%) | Example | Concentra tion (µM) | Nav1.1 current amplification rate (%) |
|---|---|---|---|---|---|
| 541 | 1 | 325 | 557 | 1 | 17 |
| 542 543 | 1 | 214 | 558 | 10 | 11 |
| | | 154 | 559 | 10 | 34 |
| 544 | 1 | 208 | 560 | 1 | 12 |
| 545 | 1 | 223 | 561 | 1 | 17 |
| 546 | 1 | 59 | 562 | 1 | 247 |
| 547 | 1 | 70 | 563 | 1 | 50 |
| 548 | 1 | 41 | 564 | 10 | 73 |
| 549 | 1 | 33 | 565 | 1 | 62 |
| 550 | 1 | 31 | 566 | 1 | 98 |
| 551 | 10 | 16 | 567 | 50 | 281 |
| 552 | 1 | 24 | 568 | 1 | 42 |
| 553 | 1 | 155 | 569 | 50 | 393 |
| 554 | 1 | 115 | 570 | 1 | 99 |
| 555 | 1 | 80 | 571 | 10 | 24 |
| 556 | 1 | 13 | | | |

For the evaluation of antiepileptic agents, evaluation in a maximal electroshock seizure (MES) model, which has high clinical predictability, evaluation in a subcutaneous pentetrazol model (minimal convulsions model, scPTZ), and evaluation in a 6 Hz psychomotor seizure model, which is resistant to existing antiepileptic agents can be used.

### Test 3: Evaluation in a maximal electroshock seizure (MES) model

This test is carried out to evaluate the anticonvulsant effect of drugs. An animal model which is used in this test is a phenotype of generalized tonic-clonic seizure and secondary generalized partial seizure. Slc:ddY male mice (20 to 30 g of body weight) are administered test compounds, and electrically stimulated (60 Hz, 25 mA, 0.2 seconds) through corneas after 15 minutes to 3 hours of the administration. The anticonvulsant effect can be confirmed by observation of the suppression of induced tonic extension seizure at hind limbs.

### Test 4: Evaluation in a subcutaneous pentetrazol model (minimal convulsions model, scPTZ)

This test is carried out to evaluate the anticonvulsant effect of drugs, similarly to the MES model. An animal model which is used in this test is a phenotype of generalized absence seizure and myoclonic seizure. Slc:ddY male mice (20 to 30 g of body weight) are administered test compounds, and after 15 minutes to 3 hours of the administration, mice are subcutaneously administered 85 mg/kg of pentetrazol. The anticonvulsant effect can be confirmed by observation of the onset or absence of clonic seizure in 30 minutes.

### Test 5: Evaluation in a 6 Hz psychomotor seizure model

This test is carried out to evaluate the anticonvulsant effect of drugs. An animal model which is used in this test is a phenotype of a seizure which is resistant to existing antiepileptic agents. Slc:ddy male mice (20 to 30 g of body weight) are administered test compounds, and electrically stimulated (6 Hz, 32 mA, 3 seconds) through corneas after 15 minutes to 3 hours of the administration. The anticonvulsant effect can be confirmed by observation of the onset or absence of induced clonic seizure at front limbs, Straub tail response, and akinesia.

### Test 6: Rotarod performance test

This test is carried out to evaluate the inhibitory effect of drugs on coordinated movements. Slc:ddy male mice (20 to 30 g of body weight) are trained to walk without falling for 3 minutes on Rotarod equipment (equipment which rotates a cylindrical bar with 4 cm in diameter, 13 rotations/min) on the day before the test or on the day of the test. Trained mice are administered test compounds, placed on the above Rotarod equipment after 1 hour of the administration, and subjected to the observation of ambulation for up to 180 seconds in the condition of 15 rotations/min. When mice fall within 180 seconds, they will be made walk again (retrial are conducted twice at maximum). The inhibitory effect on coordinated movements can be confirmed by evaluation of the longest walking time in a maximum of three trials.

### Test 7: Evaluation of the inhibition of febrile seizure by using SCNIA-mutated animals

This test is carried out to evaluate the inhibitory effect of drugs on febrile seizure which is expressed due to loss-of-function mutation on a SCN1A gene. An animal model which is used in this test has deletion mutation in a SCN1A gene, similarly to Dravet syndrome, and is a phenotype of Dravet syndrome which shows febrile seizure by the elevation of body temperature. This animal model can be purchased from RIKEN BioResource Research Center (strain: BALB/c-Scnla<+/->, catalog number: RBRC06422).

Mice (18 to 23 g) which has loss-of-function mutation on a SCN1A gene are administered test compounds. A plastic chamber is heated with a warm bath at 43°C to increase internal temperature, and in 20 minutes after the administration, mice are placed in the chamber. Heating is continued to increase their body temperature. The inhibitory effect of test compounds on the induction of febrile seizure can be confirmed by comparison in rectal temperature of test groups with untreated groups, where a test compound is not administered, at the time of the onset of seizure, or when mice do not express seizure, in one hour after the placement in the chamber.

### INDUSTRIAL APPLICABILITY

The present compound has a significant activation effect of Nav1.1, and can be a medicament that is effective for treating and/or preventing diseases involving Nav1.1 and various central nervous system diseases.

## Claims

1. A compound of Formula (I): 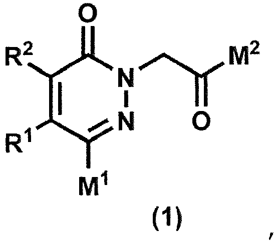 or a pharmaceutically acceptable salt thereof, wherein
M¹ is
(1-1) saturated or partially-unsaturated C₄₋₁₂ carbocyclyl, wherein the carbocyclyl may be optionally substituted with 1 to 4 the same or different substituents selected from the group consisting of:
(a) halogen atom, and
(b) C₁₋₆ alkyl optionally substituted with 1 to 3 the same or different substituents selected from the group consisting of halogen atom, hydroxy, and C₁₋₆ alkoxy;
(1-2) saturated or partially-unsaturated 4- to 12-membered heterocyclyl, wherein the heterocyclyl may be optionally substituted with 1 to 4 the same or different substituents selected from the group consisting of:
(a) halogen atom,
(b) hydroxy,
(c) methoxy,
(d) C₁₋₆ alkyl optionally substituted with 1 to 3 the same or different substituents selected from the group consisting of halogen atom, hydroxy, and C₁₋₆ alkoxy, and
(e) amino-carbonyl optionally substituted with 1 to 2 the same or different C₁₋₆ alkyl, wherein the C₁₋₆ alkyl may be optionally substituted with 1 to 3 the same or different halogen atoms;
provided that the heterocyclyl is not morpholinyl;
(1-3) 4-methylphenyl, wherein a phenyl part of the group may be optionally substituted with 1 to 4 the same or different substituents selected from the group consisting of halogen atom, hydroxy, C₁₋₆ alkyl optionally substituted with 1 to 3 the same or different halogen atoms, and C₁₋₆ alkoxy optionally substituted with 1 to 3 the same or different halogen atoms; and wherein a methyl part of the group may be optionally substituted with 1 to 3 the same or different halogen atoms;
(1-4) amino, wherein the amino may be optionally substituted with 1 to 2 the same or different substituents selected from the group consisting of:
(a) C₁₋₆ alkyl optionally substituted with 1 to 3 the same or different halogen atoms,
(b) C₃₋₁₀ cycloalkyl optionally substituted with 1 to 3 the same or different substituents selected from the group consisting of halogen atom, C₁₋₆ alkyl, and C₃₋₆ cycloalkyl, and
(c) C₃₋₁₀ cycloalkyl-C₁₋₄ alkyl optionally substituted with 1 to 3 the same or different substituents selected from the group consisting of halogen atom, C₁₋₆ alkyl, and C₃₋₆ cycloalkyl;
(1-5) 6-methylpyridin-3-yl or 6-trifluoromethylpyridin-3-yl;
(1-6) 4-chlorothiophen-2-yl, 5-methylthiophen-2-yl, or 3-cyanothiophen-2-yl, provided that when M¹ is 5-methylthiophen-2-yl, then M² is not a group shown in the following (4-2); or
(1-7) 4-methylphenyloxy;
R¹ and R² are each independently
(2-1) hydrogen atom;
(2-2) halogen atom;
(2-3) cyano;
(2-4) C₁₋₆ alkyl, wherein the C₁₋₆ alkyl may be optionally substituted with 1 to 3 the same or different substituents selected from the group consisting of:
(a) halogen atom,
(b) hydroxy,
(c) saturated or partially-unsaturated C₃₋₇ carbocyclyl,
(d) C₁₋₆ alkoxy, and
(e) amino optionally substituted with 1 to 2 the same or different substituents selected from the group consisting of C₁₋₆ alkyl optionally substituted with 1 to 3 the same or different halogen atoms; saturated or partially-unsaturated C₃₋₇ carbocyclyl; and C₂₋₇ alkylcarbonyl;
(2-5) saturated or partially-unsaturated C₃₋₇ carbocyclyl optionally substituted with 1 to 4 the same or different substituents selected from the group consisting of halogen atom, hydroxy, C₁₋₆ alkyl, and C₁₋₆ alkoxy;
(2-6) C₂₋₆ alkenyl optionally substituted with 1 to 4 the same or different halogen atoms;
(2-7) C₁₋₆ alkoxy optionally substituted with 1 to 3 the same or different substituents selected from the group consisting of halogen atom, hydroxy, saturated or partially-unsaturated C₃₋₇ carbocyclyl, and C₁₋₆ alkoxy; or
(2-8) amino optionally substituted with 1 to 2 the same or different substituents selected from the group consisting of C₁₋₆ alkyl optionally substituted with 1 to 3 the same or different halogen atoms and saturated or partially-unsaturated C₃₋₇ carbocyclyl; or
alternatively, R¹ and R² may be combined together with the carbon atoms to which they attach to form
(3-1) a 5- to 7-membered saturated or partially-unsaturated carbocycle, wherein the carbocycle may be optionally substituted with 1 to 4 the same or different substituents selected from the group consisting of:
(a) halogen atom,
(b) hydroxy,
(c) C₁₋₆ alkyl optionally substituted with 1 to 3 the same or different substituents selected from the group consisting of halogen atom, hydroxy, and C₁₋₆ alkoxy, and
(d) C₁₋₆ alkoxy optionally substituted with 1 to 3 the same or different substituents selected from the group consisting of halogen atom, hydroxy, and C₁₋₆ alkoxy; or
(3-2) a 5- to 7-membered saturated or partially-unsaturated heterocycle, wherein the heterocycle may be optionally substituted with 1 to 4 the same or different substituents selected from the group consisting of (a) to (d) in the above (3-1) of the present claim;
M² is
(4-1) a group of the following formula (2a) or (2b) : 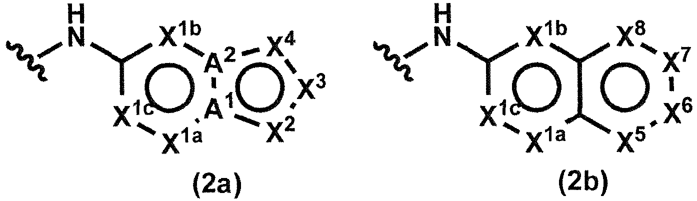 wherein X^{1a}, X^{1b}, X^{1c}, X⁵, X⁶, X⁷, and X⁸ are each independently N or CR³;
X², X³, and X⁴ are each independently CR³, O, S, N, or NR⁴;
A¹ and A² are each independently N or C;
wherein X^{1a}, X^{1b}, X^{1c}, X², X3, X⁴, X⁵, X⁶, X⁷, X⁸, A¹, and A² are selected such that a ring comprising them forms a 9-or 10-membered bicyclic aromatic heterocycle;
R³ is
(a) hydrogen atom,
(b) halogen atom,
(c) cyano,
(d) hydroxy,
(e) C₁₋₆ alkyl optionally substituted with 1 to 3 the same or different substituents selected from the group consisting of halogen atom, hydroxy, saturated or partially-unsaturated C₃₋₇ carbocyclyl, C₁₋₆ alkoxy, 4- to 7-membered saturated heterocyclyl optionally substituted with C₁₋₆ alkoxy, and amino optionally substituted with 1 to 2 the same or different C₁₋₆ alkyl,
(f) saturated or partially-unsaturated C₃₋₇ carbocyclyl optionally substituted with 1 to 4 the same or different substituents selected from the group consisting of halogen atom, hydroxy, C₁₋₆ alkyl, C₁₋₆ alkoxy, and amino optionally substituted with 1 to 2 the same or different C₁₋₆ alkyl,
(g) C₁₋₆ alkoxy optionally substituted with 1 to 3 the same or different substituents selected from the group consisting of halogen atom, hydroxy, C₁₋₆ alkoxy, and amino optionally substituted with 1 to 2 the same or different C₁₋₆ alkyl,
(h) amino optionally substituted with 1 to 2 the same or different substituents selected from the group consisting of C₁₋₆ alkyl optionally substituted with 1 to 3 the same or different substituents selected from the group consisting of halogen atom, hydroxy, and C₁₋₆ alkoxy; saturated or partially-unsaturated C₃₋₇ carbocyclyl; and C₂₋₇ alkylcarbonyl optionally substituted with 1 to 3 the same or different substituents selected from the group consisting of halogen atom, hydroxy, and C₁₋₆ alkoxy,
(i) 5- or 6-membered heteroaryl optionally substituted with 1 to 4 the same or different substituents selected from the group consisting of halogen atom, cyano, and C₁₋₆ alkyl,
(j) 4- to 7-membered saturated or partially-unsaturated heterocyclyl optionally substituted with 1 to 4 the same or different substituents selected from the group consisting of halogen atom, hydroxy, C₁₋₆ alkyl, and C₁₋₆ alkoxy, or
(k) -C(O)NR^{x}R^{y}, wherein R^{x} and R^{y} are each independently hydrogen atom, C₁₋₆ alkyl, or saturated or partially-unsaturated C₃₋₇ carbocyclyl; or alternatively, R^{x} and R^{y} may be combined together with the nitrogen atom to which they attach to form 4- to 7-membered saturated heterocyclyl,
R⁴ is
(a) hydrogen atom,
(b) C₁₋₆ alkyl optionally substituted with 1 to 3 the same or different substituents selected from the group consisting of halogen atom, hydroxy, and C₁₋₆ alkoxy, or
(c) saturated or partially-unsaturated C₃₋₇ carbocyclyl optionally substituted with 1 to 4 the same or different substituents selected from the group consisting of halogen atom, hydroxy, C₁₋₆ alkyl, and C₁₋₆ alkoxy;
provided that when R³ and R⁴ exist plurally, each of R³ and R⁴ may be the same or different;
(4-2) a group of the following formula (2c): 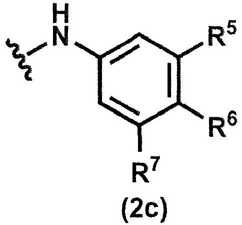 wherein R⁵, R⁶, and R⁷ are each independently
(a) hydrogen atom,
(b) halogen atom,
(c) cyano,
(d) C₁₋₆ alkyl optionally substituted with 1 to 3 the same or different substituents selected from the group consisting of halogen atom, hydroxy, saturated or partially-unsaturated C₃₋₇ carbocyclyl, and C₁₋₆ alkoxy,
(e) saturated or partially-unsaturated C₃₋₇ carbocyclyl optionally substituted with 1 to 4 the same or different substituents selected from the group consisting of halogen atom, hydroxy, C₁₋₆ alkyl, and C₁₋₆ alkoxy,
(f) C₁₋₆ alkoxy optionally substituted with 1 to 3 the same or different substituents selected from the group consisting of halogen atom, hydroxy, and C₁₋₆ alkoxy,
(g) amino optionally substituted with 1 to 2 the same or different substituents selected from the group consisting of C₁₋₆ alkyl optionally substituted with 1 to 3 the same or different halogen atoms; saturated or partially-unsaturated C₃₋₇ carbocyclyl; and C₂₋₇ alkylcarbonyl,
(h) 5- or 6-membered heteroaryl optionally substituted with 1 to 4 the same or different substituents selected from the group consisting of halogen atom, cyano, and C₁₋₆ alkyl,
(i) 4- to 7-membered saturated or partially-unsaturated heterocyclyl optionally substituted with 1 to 4 the same or different substituents selected from the group consisting of halogen atom, hydroxy, C₁₋₆ alkyl, and C₁₋₆ alkoxy,
(j) -C(O)NR^{x}R^{y}, wherein R^{x} and R^{y} are each independently hydrogen atom, C₁₋₆ alkyl, or saturated or partially-unsaturated C₃₋₇ carbocyclyl; or alternatively, R^{x} and R^{y} may be combined together with the nitrogen atom to which they attach to form 4- to 7-membered saturated heterocyclyl,
(k) C₂₋₇ alkylcarbonyl, or
(l) C₂₋₇ alkoxycarbonyl; and
either of the following condition (X) or (Y) is met:
(X) at least one of R⁵, R⁶, and R⁷ is cyano, 5- or 6-membered heteroaryl (wherein the heteroaryl may be optionally substituted with 1 to 4 the same or different substituents selected from the group consisting of halogen atom, cyano, and C₁₋₆ alkyl), 4- to 7-membered saturated or partially-unsaturated heterocyclyl (wherein the heterocyclyl may be optionally substituted with 1 to 4 the same or different substituents selected from the group consisting of halogen atom, hydroxy, C₁₋₆ alkyl, and C₁₋₆ alkoxy) , or - C(O)NR^{x}R^{y} (wherein R^{x} and R^{y} are each independently hydrogen atom, C₁₋₆ alkyl, or saturated or partially-unsaturated C₃₋₇ carbocyclyl; or alternatively, R^{x} and R^{y} are combined together with the nitrogen atom to which they attach to form 4- to 7-membered saturated heterocyclyl); or
(Y) R⁵ and R⁶ are combined together with the carbon atoms to which they attach to form a 5- to 7-membered saturated or partially-unsaturated carbocycle or heterocycle (wherein the carbocycle and heterocycle may be optionally substituted with 1 to 4 the same or different substituents selected from the group consisting of halogen atom, oxo, C_{1- 6} alkyl, C₁₋₆ alkoxy, and C₂₋₇ alkoxycarbonyl);
wherein the group of formula (2c) may further be optionally substituted with a fluorine atom at a substitutable carbon atom of the ring;
(4-3) a group of the following formula (2d), (2e), (2f), or (2g): 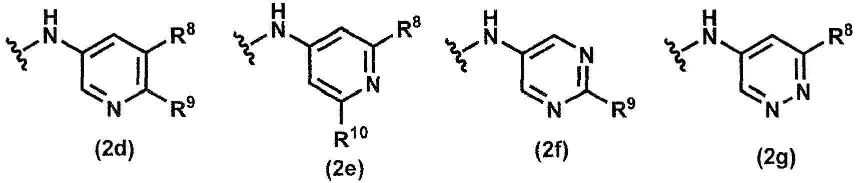 wherein R⁸, R⁹, and R¹⁰ are each independently
(a) hydrogen atom,
(b) halogen atom,
(c) cyano,
(d) C₁₋₆ alkyl optionally substituted with 1 to 3 the same or different substituents selected from the group consisting of halogen atom; hydroxy; saturated or partially-unsaturated C₃₋₇ carbocyclyl; C₁₋₆ alkoxy optionally substituted with hydroxy or C₁₋₆ alkoxy; 4- to 7-membered saturated or partially-unsaturated heterocyclyl optionally substituted with C₁₋₆ alkoxy or C₁₋₆ alkyl; 5- or 6-membered heteroaryl optionally substituted with C₁₋₆ alkyl; and amino (wherein the amino may be optionally substituted with 1 to 2 the same or different substituents selected from the group consisting of C₁₋₆ alkyl optionally substituted with 1 to 3 the same or different substituents selected from the group consisting of halogen atom, hydroxy, and C₁₋₆ alkoxy; saturated or partially-unsaturated C₃₋₇ carbocyclyl; 4- to 7-membered saturated heterocyclyl optionally substituted with C₁₋₆ alkoxy; and C₂₋₇ alkylcarbonyl optionally substituted with 1 to 3 the same or different substituents selected from the group consisting of halogen atom, hydroxy, and C₁₋₆ alkoxy);
(e) saturated or partially-unsaturated C₃₋₇ carbocyclyl optionally substituted with 1 to 4 the same or different substituents selected from the group consisting of halogen atom, hydroxy, C₁₋₆ alkyl, C₁₋₆ alkoxy, and amino optionally substituted with 1 to 2 the same or different C₁₋₆ alkyl,
(f) C₁₋₆ alkoxy optionally substituted with 1 to 3 the same or different substituents selected from the group consisting of halogen atom, hydroxy, C₁₋₆ alkoxy, and amino optionally substituted with 1 to 2 the same or different C₁₋₆ alkyl,
(g) amino optionally substituted with 1 to 2 the same or different substituents selected from the group consisting of C₁₋₆ alkyl optionally substituted with 1 to 3 the same or different substituents selected from the group consisting of halogen atom, hydroxy, and C₁₋₆ alkoxy; saturated or partially-unsaturated C₃₋₇ carbocyclyl; and C₂₋₇ alkylcarbonyl optionally substituted with 1 to 3 the same or different substituents selected from the group consisting of halogen atom, hydroxy, and C₁₋₆ alkoxy,
(h) 5- or 6-membered heteroaryl optionally substituted with 1 to 4 the same or different substituents selected from the group consisting of halogen atom, cyano, C₁₋₆ alkyl, C₁₋₆ alkoxy, and C₂₋₇ alkoxycarbonyl,
(i) 4- to 7-membered saturated or partially-unsaturated heterocyclyl optionally substituted with 1 to 4 the same or different substituents selected from the group consisting of C₁₋₆ alkyl optionally substituted with 1 to 3 the same or different substituents selected from the group consisting of halogen atom, hydroxy, and C₁₋₆ alkoxy; C₁₋₆ alkoxy; 4- to 7-membered saturated or partially-unsaturated heterocyclyl; C₂₋₇ alkylcarbonyl optionally substituted with 1 to 3 the same or different substituents selected from the group consisting of halogen atom, hydroxy, and C₁₋₆ alkoxy; and oxo,
(j) 4- to 7-membered saturated or partially-unsaturated heterocyclyl-oxy optionally substituted with 1 to 4 C₁₋₆ alkyl,
(k) -C(O)NR^{x}R^{y}, wherein R^{x} and R^{y} are each independently hydrogen atom, C₁₋₆ alkyl, or saturated or partially-unsaturated C₃₋₇ carbocyclyl; or
alternatively, R^{x} and R^{y} may be combined together with the nitrogen atom to which they attach to form 4- to 7-membered saturated heterocyclyl,
(l) -C(O)OR^{z}, wherein R^{z} is C₁₋₆ alkyl, or
(m) ethenyl optionally substituted with one 6-membered saturated heterocyclyl group;
wherein R⁸ and R⁹ may be combined together with the carbon atoms to which they attach to form a 5- to 7-membered saturated or partially-unsaturated carbocycle or heterocycle, wherein the carbocycle and heterocycle may be optionally substituted with 1 to 4 the same or different substituents selected from the group consisting of halogen atom, and C₁₋₆ alkyl,
wherein both R⁸ and R⁹ of formula (2d) are not hydrogen atoms at the same time, and the group of formula (2e) may further be optionally substituted with a fluorine atom at a substitutable carbon atom of the ring,
(4-4) a group of the following formula (2h): 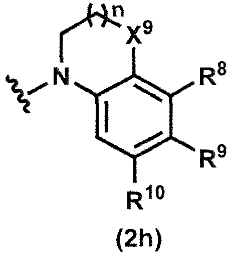 wherein R⁸, R⁹, and R¹⁰ are the same as those defined in the above (4-3) of the present claim;
n is 0, 1, or 2;
X⁹ is CH₂ or O;
wherein the group of formula (2h) may further be optionally substituted with a fluorine atom at a substitutable carbon atom at the ring;
(4-5) a group of the following formula (2i), (2j), or (2k): 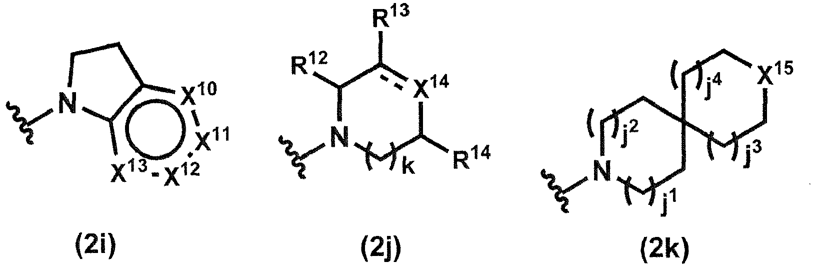 wherein X¹⁰, X¹¹, X¹², and X¹³ are each independently N or CR¹¹;
wherein X¹⁰, X¹¹, X¹², and X¹³ are selected such that a 6-membered ring comprising them forms an aromatic heterocycle;
X¹⁴ is CR¹⁵, CHR¹⁵, NR¹⁶, or O;
provided that when X¹⁴ is CR¹⁵, a bond comprising a broken line in formula (2j) denotes a double bond, and that when X¹⁴ is CHR¹⁵, NR¹⁶, or O, a bond comprising a broken line in formula (2j) denotes a single bond;
X¹⁵ is NR¹⁷ or O;
R¹¹ is
(a) hydrogen atom,
(b) halogen atom,
(c) 5- or 6-membered heteroaryl,
(d) 5- or 6-membered heteroaryl-methyl, or
(e) 4- to 7-membered saturated or partially-unsaturated heterocyclyl optionally substituted with 1 to 4 the same or different substituents selected from the group consisting of C₁₋₆ alkyl, C₁₋₆ alkoxy, C₂₋₇ alkylcarbonyl, and C₂₋₇ alkoxycarbonyl,
provided that when R¹¹ exists plurally, each R¹¹ may be the same or different;
R¹², R¹³, and R¹⁴ are each independently
(a) hydrogen atom, or
(b) methyl,
wherein R¹² and R¹⁴, or R¹³ and R¹⁴ may be combined with the carbon atoms to which they attach to form a bridged structure;
R¹⁵ is
(a) phenyl,
(b) benzyl,
(c) 5- to 10-membered heteroaryl optionally substituted with 1 to 2 the same or different substituents selected from the group consisting of fluorine atom and methoxy,
(d) hydroxy,
(e) phenyloxy, or
(f) phenylamino;
R¹⁶ is
(a) phenyl optionally substituted with 1 to 2 the same or different substituents selected from the group consisting of fluorine atom and methoxy,
(b) 5- or 6-membered heteroaryl optionally substituted with 1 to 2 the same or different substituents selected from the group consisting of methyl, methoxy, fluorine atom, trifluoromethyl, and difluoromethoxy,
(c) 5- or 6-membered heteroarylmethyl optionally substituted with 1 or 2 methyl,
(d) 5- or 6-membered saturated or partially-unsaturated carbocyclyl, or
(e) 6-membered saturated heterocyclyl;
R¹⁷ is
(a) pyridyl,
(b) 6-membered saturated heterocyclyl, or
(c) methoxypropyl;
k is 0, 1, or 2;
j¹, j², j³, and j⁴ are each independently 0 or 1;
(4-6) a group of the following formula (21): 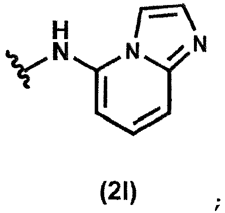 or
(4-7) a group of the following formula (2m) or (2n) : 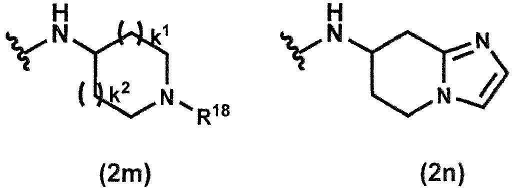 wherein R¹⁸ is
(a) phenyl, or
(b) benzyl;
k¹ and k² are each independently 0 or 1;
wherein the nitrogen-containing saturated ring in formula (2m) may be optionally substituted with oxo; provided that the compound according to Formula (1) is not the following compounds:

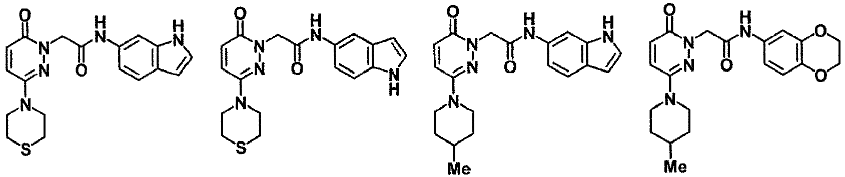

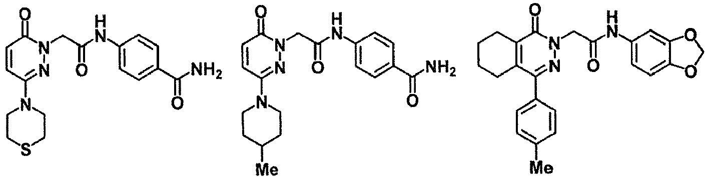

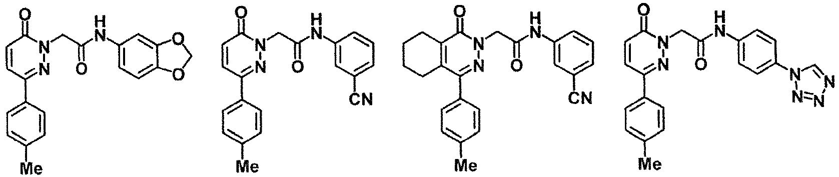

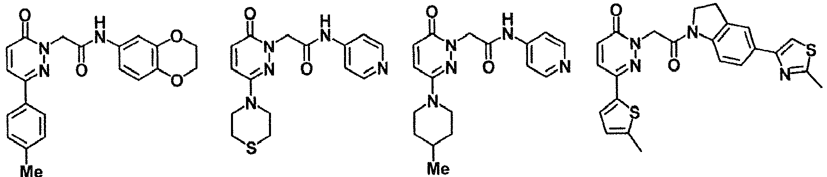

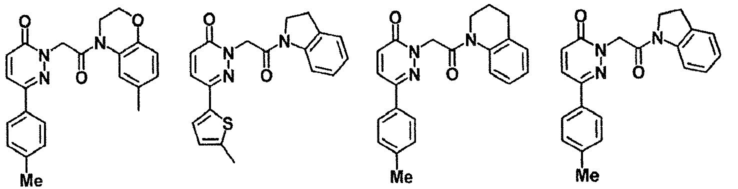

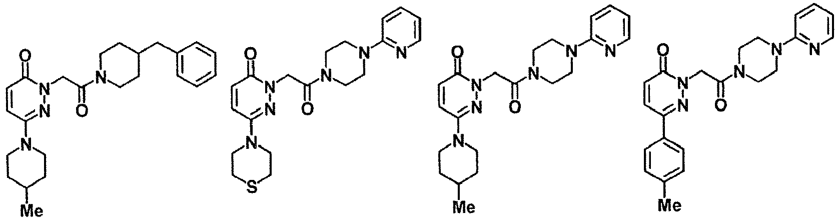

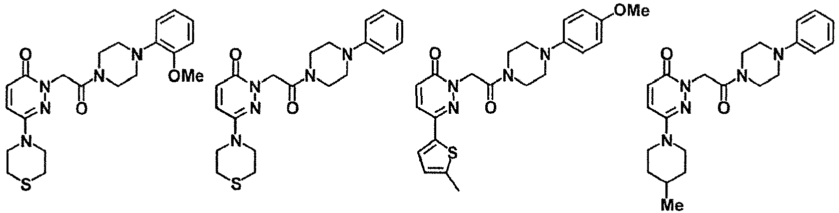

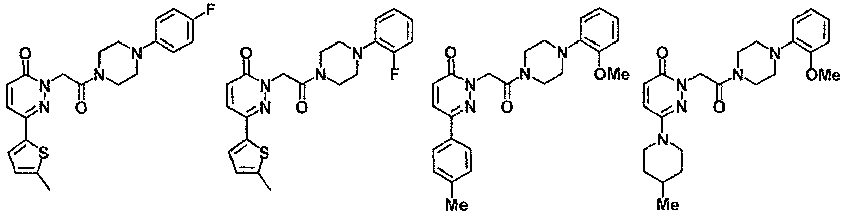

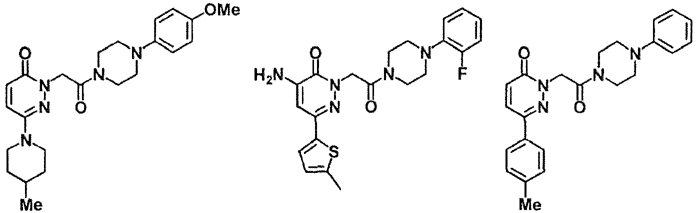

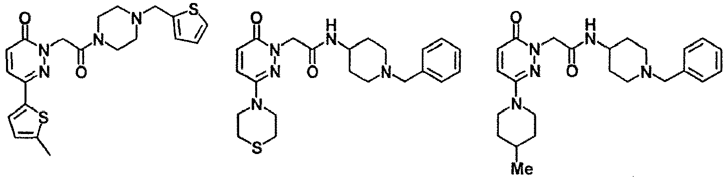

2. The compound according to claim 1, or a pharmaceutically acceptable salt thereof, wherein
R¹ and R² are each independently
(1) hydrogen atom,
(2) C₁₋₆ alkyl optionally substituted with 1 to 3 the same or different substituents selected from the group consisting of halogen atom, hydroxy, saturated or partially-unsaturated C₃₋₇ carbocyclyl, and C₁₋₆ alkoxy,
(3) C₁₋₆ alkoxy optionally substituted with 1 to 3 the same or different substituents selected from the group consisting of halogen atom, hydroxy, and C₁₋₆ alkoxy, or
(4) amino optionally substituted with 1 to 2 the same or different substituents selected from the group consisting of C₁₋₆ alkyl optionally substituted with 1 to 3 the same or different halogen atoms, and saturated or partially-unsaturated C₃₋₇ carbocyclyl, or
alternatively, R¹ and R² may be combined together with the carbon atoms to which they attach to form a 5- to 7-membered saturated or partially-unsaturated carbocycle.

3. The compound according to claim 1 or 2, or a pharmaceutically acceptable salt thereof, wherein
M² is
(1) a group of any one of the following formulae (11) to (37) : 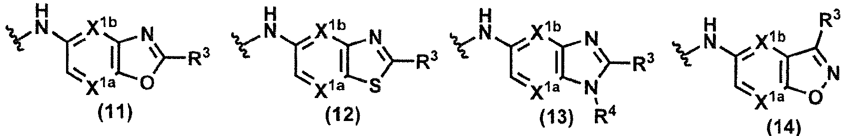 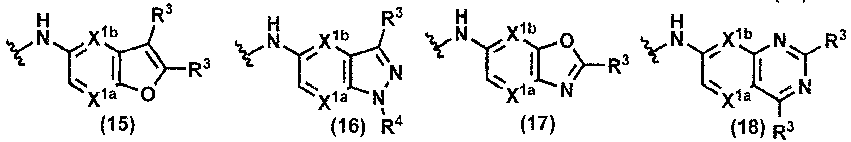 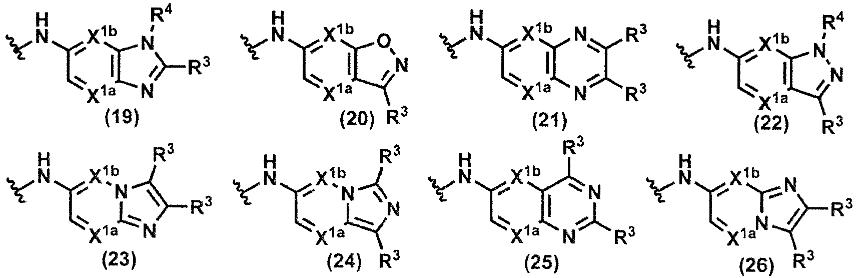 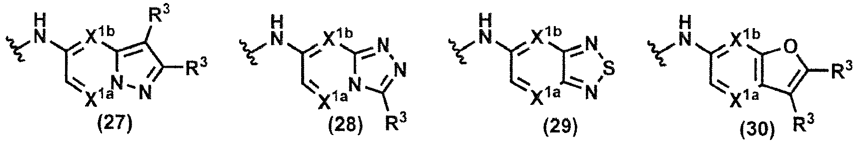 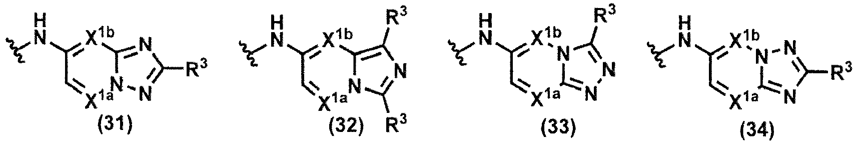 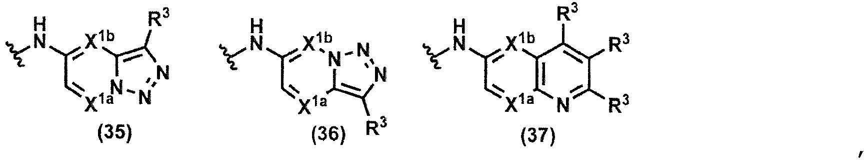 wherein X^{1a}, X^{1b}, R³, and R⁴ are the same as those defined in claim 1,
(2) 4-cyanophenylamino,
(3) a group of the following formula (2c'): 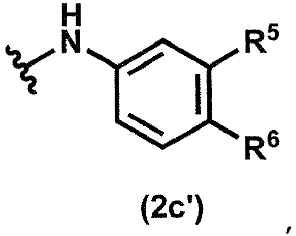 wherein R⁵ and R⁶ are each independently
(a) hydrogen atom,
(b) halogen atom,
(c) cyano,
(d) amino optionally substituted with 1 to 2 the same or different substituents selected from the group consisting of C₁₋₆ alkyl optionally substituted with 1 to 3 the same or different halogen atoms; saturated or partially-unsaturated C₃₋₇ carbocycyl; and C₂₋₇ alkylcarbonyl,
(e) 5- or 6-membered heteroaryl optionally substituted with 1 to 4 the same or different substituents selected from the group consisting of halogen atom, cyano, and C₁₋₆ alkyl,
(f) 4- to 7-membered saturated or partially-unsaturated heterocyclyl optionally substituted with 1 to 4 the same or different substituents selected from the group consisting of halogen atom, hydroxy, C₁₋₆ alkyl, and C₁₋₆ alkoxy, or
(g) -C(O)NR^{x}R^{y}, wherein R^{x} and R^{y} are each independently hydrogen atom, C₁₋₆ alkyl, or saturated or partially-unsaturated C₃₋₇ carbocyclyl; or alternatively, R^{x} and R^{y} may be combined together with the nitrogen atom to which they attach to form 4- to 7-membered saturated heterocyclyl, and
either of the following condition (X') or (Y') is met:
(X') at least one of R⁵ and R⁶ is cyano, 5- or 6-membered heteroaryl (wherein the heteroaryl may be optionally substituted with 1 to 4 the same or different substituents selected from the group consisting of halogen atom, cyano, and C₁₋₆ alkyl), 4- to 7-membered saturated or partially-unsaturated heterocyclyl (wherein the heterocyclyl may be optionally substituted with 1 to 4 the same or different substituents selected from the group consisting of halogen atom, hydroxy, C₁₋₆ alkyl, and C₁₋₆ alkoxy) , or -C(O)NR^{x}R^{y} (wherein R^{x} and R^{y} are each independently hydrogen atom, C₁₋₆ alkyl, or saturated or partially-unsaturated C₃₋₇ carbocyclyl; or alternatively, R^{x} and R^{y} may be combined together with the nitrogen atom to which they attach to form 4- to 7-membered saturated heterocyclyl); or
(Y') R⁵ and R⁶ may be combined together with the carbon atoms to which they attach to form a 5- to 7-membered saturated or partially-unsaturated carbocycle or heterocycle (wherein the carbocycle and heterocycle may be optionally substituted with 1 to 4 the same or different substituents selected from the group consisting of halogen atom, oxo, C_{1- 6} alkyl, C₁₋₆ alkoxy, and C₂₋₇ alkoxycarbonyl),
wherein the group of formula (2c') may further be optionally substituted with a fluorine atom at a substitutable carbon atom of the ring,
(4) a group of the following formula (2d), (2e), (2f), or (2g): 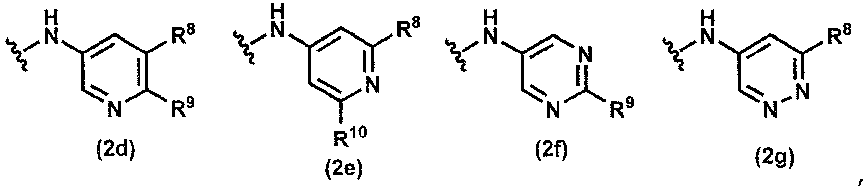 wherein R⁸, R⁹, and R¹⁰ are each independently
(a) hydrogen atom,
(b) halogen atom,
(c) cyano,
(d) C₁₋₆ alkyl optionally substituted with 1 to 3 the same or different substituents selected from the group consisting of halogen atom; hydroxy; C₁₋₆ alkoxy optionally substituted with hydroxy or C₁₋₆ alkoxy; 4- to 7-membered saturated or partially-unsaturated heterocyclyl optionally substituted with C₁₋₆ alkyl or C₁₋₆ alkoxy; 5- or 6-membered heteroaryl optionally substituted with C₁₋₆ alkyl; and amino (wherein the amino may be optionally substituted with 1 to 2 the same or different substituents selected from the group consisting of C₁₋₆ alkyl optionally substituted with 1 to 3 the same or different substituents selected from the group consisting of halogen atom, hydroxy, and C₁₋₆ alkoxy; saturated or partially-unsaturated C₃₋₇ carbocyclyl; 4- to 7-membered saturated heterocyclyl optionally substituted with C₁₋₆ alkoxy; and C₂₋₇ alkylcarbonyl optionally substituted with 1 to 3 the same or different substituents selected from the group consisting of halogen atom, hydroxy, and C₁₋₆ alkoxy);
(e) C₁₋₆ alkoxy optionally substituted with 1 to 3 the same or different substituents selected from the group consisting of halogen atom, hydroxy, and C₁₋₆ alkoxy,
(f) amino optionally substituted with 1 to 2 the same or different substituents selected from the group consisting of C₁₋₆ alkyl optionally substituted with halogen atom; saturated or partially-unsaturated C₃₋₇ carbocyclyl; and C₂₋₇ alkylcarbonyl optionally substituted with 1 to 3 the same or different substituents selected from the group consisting of halogen atom, hydroxy, and C₁₋₆ alkoxy,
(g) 5- or 6-membered heteroaryl optionally substituted with 1 to 4 the same or different substituents selected from the group consisting of halogen atom, cyano, C₁₋₆ alkyl, C₁₋₆ alkoxy, and C₂₋₇ alkoxycarbonyl,
(h) 4- to 7-membered saturated or partially-unsaturated heterocyclyl optionally substituted with 1 to 4 the same or different substituents selected from the group consisting of C₂₋₆ alkyl optionally substituted with 1 to 3 the same or different substituents selected from the group consisting of halogen atom, hydroxy, and C₁₋₆ alkoxy; C₁₋₆ alkoxy; 4- to 7-membered saturated or partially-unsaturated heterocyclyl; C₂₋₇ alkylcarbonyl optionally substituted with 1 to 3 the same or different substituents selected from the group consisting of halogen atom, hydroxy, and C₁₋₆ alkoxy; and oxo,
(i) 4- to 7-membered saturated or partially-unsaturated heterocyclyl-oxy optionally substituted with 1 to 4 C₁₋₆ alkyl,
(j) -C(O)NR^{x}R^{y}, wherein R^{x} and R^{y} are each independently hydrogen atom, C₁₋₆ alkyl, or saturated or partially-unsaturated C₃₋₇ carbocyclyl; or
alternatively, R^{x} and R^{y} may be combined together with the nitrogen atom to which they attach to form 4- to 7-membered saturated heterocyclyl,
(k) -C(O)OR^{z}, wherein R^{z} is C₁₋₆ alkyl, or
(l) ethenyl optionally substituted with one 6-membered saturated heterocyclyl group;
wherein R⁸ and R⁹ may be combined together with the carbon atoms to which they attach to form a 5- to 7-membered saturated or partially-unsaturated carbocycle or heterocycle, wherein the carbocycle and heterocycle may be optionally substituted with 1 to 4 the same or different substituents selected from the group consisting of halogen atom and C₁₋₆ alkyl,
wherein both R⁸ and R⁹ in formula (2d) are not hydrogen atoms at the same time, and the group of formula (2e) may further be optionally substituted with a fluorine atom at a substitutable carbon atom of the ring, or
(5) a group of the following formula (2h'): 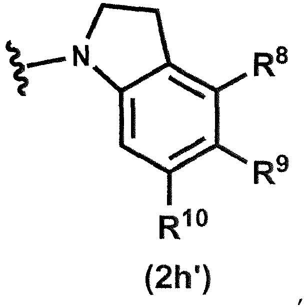 wherein R⁸, R⁹, and R¹⁰ are the same as those defined in the above (4) of the present claim.

4. The compound according to claim 3, or a pharmaceutically acceptable salt thereof, wherein
M² is
(1) a group of any one of the following formulae (11), (12), (18), (26), (31), and (34): 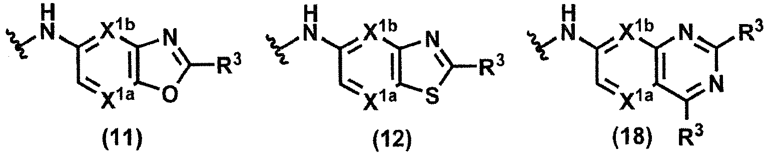 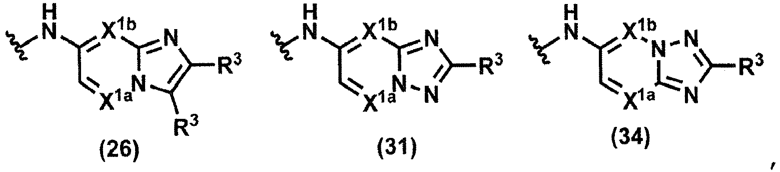 wherein X^{1a}, X^{1b}, and R³ are the same as those defined in claim 1,
(2) 4-cyanophenylamino,
(3) a group of the following formula (2h''): 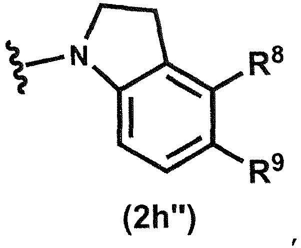 wherein R⁸ and R⁹ are the same as those defined in claim 3.

5. The compound according to any one of claims 1 to 4, or a pharmaceutically acceptable salt thereof, wherein
M¹ is
(1) saturated or partially-unsaturated C₄₋₁₂ carbocyclyl optionally substituted with 1 to 4 the same or different substituents selected from the group consisting of halogen atom and C₁₋₆ alkyl optionally substituted with 1 to 3 the same or different substituents selected from the group consisting of halogen atom, hydroxy, and C₁₋₆ alkoxy, or
(2) 4- to 12-membered saturated or partially-unsaturated heterocyclyl optionally substituted with 1 to 4 the same or different substituents selected from the group consisting of halogen atom and C₁₋₆ alkyl optionally substituted with 1 to 3 the same or different substituents selected from the group consisting of halogen atom, hydroxy, and C₁₋₆ alkoxy.

6. The compound according to claim 5, or a pharmaceutically acceptable salt thereof, wherein
M¹ is a group of the following formula (3): 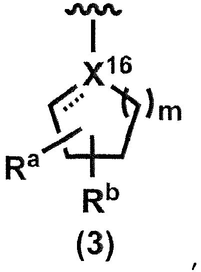 wherein X¹⁶ is N, C, or CH;
a bond comprising a broken line is a single bond or a double bond;
m is 0, 1, 2, or 3;
R^{a} and R^{b} are each independently
(1-1) hydrogen atom,
(1-2) halogen atom, or
(1-3) C₁₋₆ alkyl optionally substituted with 1 to 3 the same or different substituents selected from the group consisting of halogen atom, hydroxy, and C₁₋₆ alkoxy; or
alternatively, R^{a} and R^{b} may be combined together with the carbon atom(s) to which they attach to form a 3- to 6-membered saturated carbocycle, wherein the carbocycle may be optionally substituted with 1 to 4 the same or different substituents selected from the group consisting of:
(a) halogen atom,
(b) hydroxy,
(c) C₁₋₆ alkyl optionally substituted with 1 to 3 the same or different substituents selected from the group consisting of halogen atom, hydroxy, and C₁₋₆ alkoxy, and
(d) C₁₋₆ alkoxy optionally substituted with 1 to 3 the same or different substituents selected from the group consisting of halogen atom, hydroxy, and C₁₋₆ alkoxy.

7. The compound according to any one of claims 1 to 6, or a pharmaceutically acceptable salt thereof, wherein Formula (1) is formula (1''): 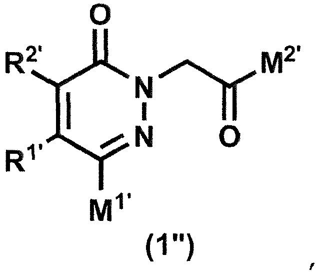 wherein M¹' is any one of the following formulae (38) to (52) : 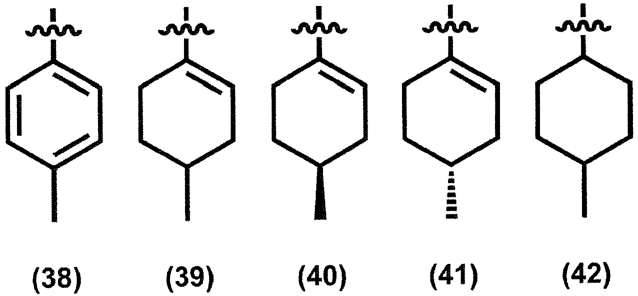 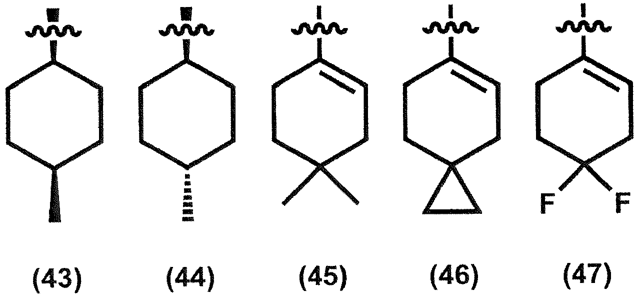 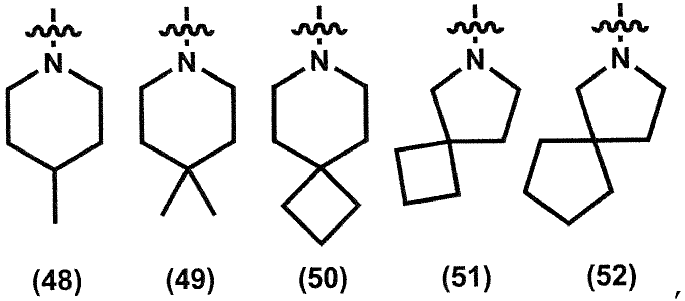 R¹' and R²' are each independently
(2-1) hydrogen atom,
(2-2) halogen atom,
(2-3) cyano,
(2-4) methyl, or
(2-5) methoxy, and
M²' is
(1) a group of any one of the following formulae (53) to (58): 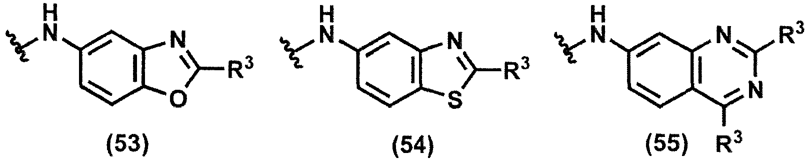 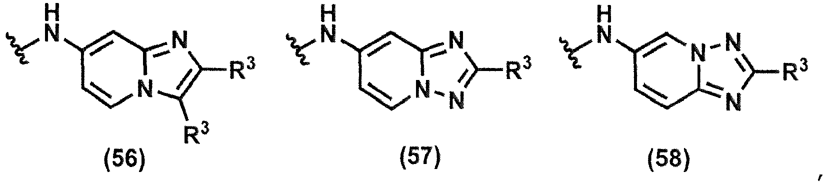 wherein R³, where R³s are each independent when existing plurally, is
(a) hydrogen atom,
(b) halogen atom,
(c) cyano,
(d) C₁₋₆ alkyl optionally substituted with 1 to 3 the same or different substituents selected from the group consisting of halogen atom, hydroxy, saturated or partially-unsaturated C₃₋₇ carbocyclyl, C₁₋₆ alkoxy, 4- to 7-membered saturated heterocyclyl optionally substituted with C₁₋₆ alkoxy, and amino optionally substituted with 1 to 2 the same or different C₁₋₆ alkyl,
(e) C₁₋₆ alkoxy optionally substituted with 1 to 3 the same or different substituents selected from the group consisting of halogen atom, hydroxy, C₁₋₆ alkoxy, and amino optionally substituted with 1 to 2 the same or different C₁₋₆ alkyl, or
(f) amino optionally substituted with 1 to 2 the same or different substituents selected from the group consisting of C₁₋₆ alkyl optionally substituted with 1 to 3 the same or different substituents selected from the group consisting of halogen atom, hydroxy, and C₁₋₆ alkoxy; saturated or partially-unsaturated C₃₋₇ carbocyclyl; and C₂₋₇ alkylcarbonyl optionally substituted with 1 to 3 the same or different substituents selected from the group consisting of halogen atom, hydroxy, and C₁₋₆ alkoxy,
(2) 4-cyanophenylamino, or
(3) a group of the following formula (2h'''): 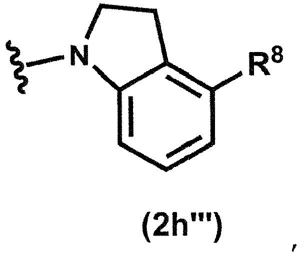 wherein R⁸ is
(a) C₁₋₆ alkyl optionally substituted with 1 to 3 the same or different substituents selected from the group consisting of halogen atom; hydroxy; C₁₋₆ alkoxy optionally substituted with hydroxy or C₁₋₆ alkoxy; 4- to 7-membered saturated or partially-unsaturated heterocyclyl optionally substituted with C₁₋₆ alkyl or C₁₋₆ alkoxy; 5- or 6-membered heteroaryl optionally substituted with C₁₋₆ alkyl; and amino (wherein the amino may be optionally substituted with 1 to 2 the same or different substituents selected from the group consisting of C₁₋₆ alkyl optionally substituted with 1 to 3 the same or different substituents selected from the group consisting of halogen atom, hydroxy, and C₁₋₆ alkoxy; saturated or partially-unsaturated C₃₋₇ carbocyclyl; 4- to 7-membered saturated heterocyclyl optionally substituted with C₁₋₆ alkoxy; and C₂₋₇ alkylcarbonyl optionally substituted with 1 to 3 the same or different substituents selected from the group consisting of halogen atom, hydroxy, and C₁₋₆ alkoxy),
(b) C₁₋₆ alkoxy optionally substituted with 1 to 3 the same or different substituents selected from the group consisting of halogen atom, hydroxy, and C₁₋₆ alkoxy,
(c) amino optionally substituted with 1 to 2 the same or different substituents selected from the group consisting of C₁₋₆ alkyl optionally substituted with halogen atom; saturated or partially-unsaturated C₃₋₇ carbocyclyl; and C₂₋₇ alkylcarbonyl optionally substituted with 1 to 3 the same or different substituents selected from the group consisting of halogen atom, hydroxy, and C₁₋₆ alkoxy,
(d) 5- or 6-membered heteroaryl optionally substituted with 1 to 4 the same or different substituents selected from the group consisting of halogen atom, cyano, C₁₋₆ alkyl, C₁₋₆ alkoxy, and C₂₋₇ alkoxycarbonyl,
(e) 4- to 7-membered saturated or partially-unsaturated heterocyclyl optionally substituted with 1 to 4 the same or different substituents selected from the group consisting of C₁₋₆ alkyl optionally substituted with 1 to 3 the same or different substituents selected from the group consisting of halogen atom, hydroxy, and C₁₋₆ alkoxy; C₁₋₆ alkoxy; 4- to 7-membered saturated or partially-unsaturated heterocyclyl; C₂₋₇ alkylcarbonyl optionally substituted with 1 to 3 the same or different substituents selected from the group consisting of halogen atom, hydroxy, and C₁₋₆ alkoxy; and oxo, or
(f) 4- to 7-membered saturated or partially-unsaturated heterocyclyl-oxy optionally substituted with 1 to 4 C₁₋₆ alkyl.

8. The compound according to claim 7, or a pharmaceutically acceptable salt thereof, wherein
M¹' is a group of the following formula (38):

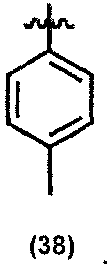

9. The compound according to claim 7, or a pharmaceutically acceptable salt thereof, wherein
M¹' is a group of the following formula (39), (40), (41), or (45) :

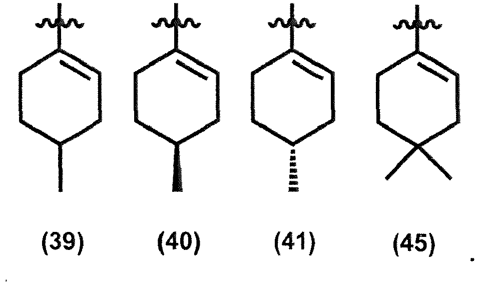

10. The compound according to claim 7, or a pharmaceutically acceptable salt thereof, wherein
M¹' is a group of the following formula (48), (50), or (51):

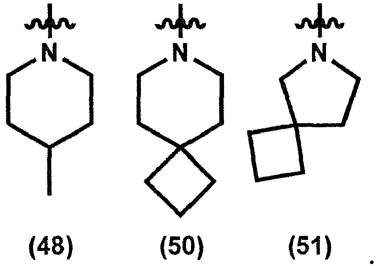

11. The compound according to any one of claims 7 to 10, or a pharmaceutically acceptable salt thereof, wherein
M²' is a group of any one of the following formulae (53) to (58) : 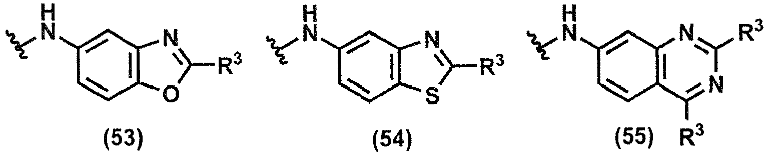 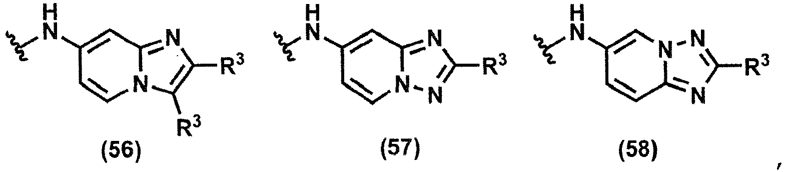 wherein R³ is hydrogen atom, halogen atom, cyano, C₁₋₆ alkyl, C₁₋₆ alkoxy, or amino optionally substituted with 1 to 2 the same or different C₁₋₆ alkyl.

12. The compound according to any one of claims 7 to 10, or a pharmaceutically acceptable salt thereof, wherein
M²' is a group of the following formula (57) or (58): 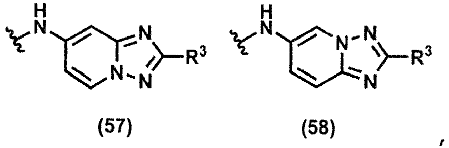 wherein R³ is hydrogen atom, halogen atom, cyano, C₁₋₆ alkyl, C₁₋₆ alkoxy, or amino optionally substituted with 1 to 2 the same or different C₁₋₆ alkyl.

13. The compound according to any one of claims 7 to 10, or a pharmaceutically acceptable salt thereof, wherein M²' is 4-cyanophenylamino.

14. The compound according to any one of claims 7 to 10, or a pharmaceutically acceptable salt thereof, wherein M²' is (3) a group of the following formula (2h'''): 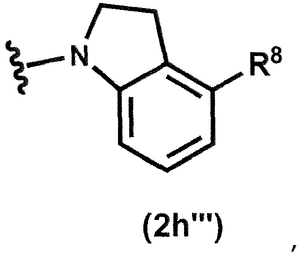 wherein R⁸ is
(a) 5- or 6-membered heteroaryl optionally substituted with 1 to 4 the same or different substituents selected from the group consisting of halogen atom, cyano, and C₁₋₆ alkyl, or
(b) 4- to 7-membered saturated or partially-unsaturated heterocyclyl optionally substituted with 1 to 4 the same or different substituents selected from the group consisting of C₁₋₆ alkyl optionally substituted with 1 to 3 the same or different substituents selected from the group consisting of halogen atom, hydroxy, and C₁₋₆ alkoxy; C₁₋₆ alkoxy; and oxo.

15. The compound according to claim 1, or a pharmaceutically acceptable salt thereof, wherein the compound is selected from the group consisting of:
N-(4-cyanophenyl)-2-[3-(4-methylpiperidin-1-yl)-6-oxopyridazin-1(6H)-yl]acetamide,
N-(1,3-benzooxazol-5-yl)-2-(3-(4-methylpiperidin-1-yl)-6-oxopyridazin-1(6H)-yl]acetamide,
2-(3-(6-azaspiro[3.4]octan-6-yl)-6-oxopyridazin-1(6H)-yl]-N-(quinazolin-7-yl)acetamide,
N-[2-(dimethylamino)-1,3-benzooxazol-5-yl]-2-[3-(4-methylcyclohex-1-en)-6-oxopyridazin-1(6H)-yl]acetamide,
2-[3-(4,4-dimethylcyclohex-1-en-1-yl)-6-oxopyridazin-1(6H)-yl]-N-([1,2,4]triazolo[1,5-a]pyridin-6-yl)acetamide,
2-[3-(4,4-dimethylcyclohex-1-en-1-yl)-6-oxopyridazin-1(6H)-yl]-N-([1,2,4]triazolo[1,5-a]pyridin-7-yl)acetamide,
N-(1,3-benzooxazol-5-yl)-2-[3-(4,4-dimethylcyclohex-1-en-1-yl)-6-oxopyridazin-1(6H)-yl]acetamide,
2-[6-oxo-3-(spiro[2.5]oct-5-en-6-yl)pyridazin-1(6H)-yl]-N-([1,2,4]triazolo[1,5-a]pyridin-7-yl)acetamide,
2-{2-oxo-2-[4-(pyridazin-4-yl)-2.3-dihydro-1H-indol-1-yl]ethyl}-6-(spiro[2.5]oct-5-en-6-yl)pyridazin-3(2H)one,
N-(1,3-benzooxazol-5-yl)-2-[3-(4-methylcyclohex-1-en-1-yl)-6-oxopyridazin-1(6H)-yl]acetamide,
2-13-[(4S)-4-methylcyclohex-1-en-1-yl]-6-oxopyridazin-1(6H)-yl}-N-([1,2,4]triazolo[1,5-a]pyridin-7-yl)acetamide,
2-{3-[(4R)-4-methylcyclohex-1-en-1-yl]-6-oxopyridazin-1(6H)-yl}-N-([1,2,4]triazolo[1,5-a]pyridin-7-yl)acetamide,
2-{3-[(4S)-4-methylcyclohex-1-en-1-yl]-6-oxopyridazin-1(6H)-yl}-N-([1,2,4]triazolo[1,5-a]pyridin-6-yl)acetamide, and
2-{3-[(4R)-4-methylcyclohex-1-en-1-yl]-6-oxopyridazin-1(6H)-yl}-N-([1,2,4]triazolo[1,5-a]pyridin-6-yl)acetamide.

16. A pharmaceutical composition comprising a compound according to any one of claims 1 to 15, or a pharmaceutically acceptable salt thereof, as an active ingredient.

17. A medicament for activating Nav1.1, comprising a compound according to any one of claims 1 to 15, or a pharmaceutically acceptable salt thereof, as an active ingredient.

18. A medicament for treating and/or preventing a central nervous system disease, comprising a compound according to any one of claims 1 to 15, or a pharmaceutically acceptable salt thereof, as an active ingredient.

19. A medicament for treating and/or preventing a disease involving Nav1.1, comprising a compound according to any one of claims 1 to 15, or a pharmaceutically acceptable salt thereof, as an active ingredient.

20. A medicament for treating and/or preventing a disease involving reduced function of Nav1.1, comprising a compound according to any one of claims 1 to 15 or a pharmaceutically acceptable salt thereof, as an active ingredient.

21. The medicament according to claim 19 or 20, wherein the disease involving Nav1.1 is a central nervous system disease.

22. The medicament according to claim 18 or 21, wherein the central nervous system disease is at least one selected from the group consisting of febrile seizure; generalised epilepsy with febrile seizure plus; epilepsy (specifically, focal epilepsy, generalized epilepsy); epileptic syndrome (such as Dravet syndrome; intractable childhood epilepsy with generalized tonic-clonic seizure; epilepsy with myoclonic-atonic seizure; West syndrome; Lennox-Gastaut syndrome; infantile spasms; sever infantile multifocal epilepsy; severe myoclonic epilepsy, borderline; benign familial neonatal-infantile seizure); schizophrenia; autism spectrum disorder; and attention deficit hyperactivity disorder.

23. Use of a compound according to any one of claims 1 to 15, or a pharmaceutically acceptable salt thereof in the manufacture of a medicament for treating and/or preventing a disease involving Nav1.1.

24. A compound according to any one of claims 1 to 15, or a pharmaceutically acceptable salt thereof, for use in treating and/or preventing a disease involving Nav1.1.

25. A method for treating and/or preventing a disease involving Nav1.1, comprising administering to a patient in need thereof a therapeutically effective amount of a compound according to any one of claims 1 to 15 or a pharmaceutically acceptable salt thereof.

26. A combination medicament comprising a compound according to any one of claims 1 to 15, or a pharmaceutically acceptable salt thereof, and one or more drugs selected from drugs classified as antiepileptic agents, antidepressant agents, antiparkinsonian agents, antischizophrenic agents, or therapeutic agents for ADHD.

27. A compound according to any one of claims 1 to 15, or a pharmaceutically acceptable salt thereof, or a pharmaceutical composition or medicament according to any one of claims 16 to 22, for use in treating and/or preventing a central nervous system disease, for combination use with one or more drugs selected from drugs classified as antiepileptic agents, antidepressant agents, antiparkinsonian agents, antischizophrenic agents, or therapeutic agents for ADHD.
